# EUROPEAN PATENT SPECIFICATION

(11) **EP 2 301 926 B1**
(45) Date of publication and mention of the grant of the patent: **21.11.2018**
(21) Application number: 09758282.9
(22) Date of filing: 01.06.2009
(51) Int. Cl.: C07D 487/04, C07D 491/048, C07D 493/04, C07D 495/04, H01L 51/00, H05B 33/22, H01L 51/50

(54) **HALOGEN COMPOUND, POLYCYCLIC COMPOUND, AND ORGANIC ELECTROLUMINESCENCE ELEMENT COMPRISING THE POLYCYCLIC COMPOUND**
HALOGENVERBINDUNG, POLYCYCLISCHE VERBINDUNG UND ORGANISCHES ELEKTROLUMINESZIERENDES ELEMENT, DAS DIE POLYCYCLISCHE VERBINDUNG ENTHÄLT
COMPOSÉ HALOGÉNÉ, COMPOSÉ POLYCYCLIQUE, ET ÉLÉMENT ÉLECTROLUMINESCENT ORGANIQUE COMPRENANT LE COMPOSÉ POLYCYCLIQUE

(30) Priority: 05.06.2008 JP 2008148514; 17.10.2008 US 253586; 16.04.2009 JP 2009100319
(43) Date of publication of application: 30.03.2011
(73) Proprietor: Idemitsu Kosan Co., Ltd., Chiyoda-ku Tokyo 100-8321 (JP)
(72) Inventor: KATO, Tomoki, Sodegaura-shi Chiba 299-0293 (JP); NUMATA, Masaki, Sodegaura-shi Chiba 299-0293 (JP); YOSHIDA, Kei, Sodegaura-shi Chiba 299-0293 (JP); NISHIMURA, Kazuki, Sodegaura-shi Chiba 299-0293 (JP); IWAKUMA, Toshihiro, Sodegaura-shi Chiba 299-0293 (JP); HOSOKAWA, Chishio, Sodegaura-shi Chiba 299-0293 (JP)
(74) Representative: Gille Hrabal
(86) International application number: PCT/JP2009/059980
(87) International publication number: WO 2009/148015

(56) References cited:
- EP-A1- 0 906 947
- EP-A1- 1 672 713
- EP-A1- 1 860 097
- EP-A1- 1 956 022
- EP-A1- 1 956 666
- EP-A1- 2 080 762
- EP-A1- 2 166 011
- EP-A1- 2 169 029
- EP-A1- 2 172 534
- EP-A1- 2 251 342
- EP-A1- 2 284 920
- EP-A1- 2 298 774
- EP-A1- 2 301 921
- WO-A1-2006/015862
- WO-A1-2006/100896
- WO-A1-2006/108497
- WO-A1-2006/122630
- WO-A1-2007/018007
- WO-A1-2007/029798
- WO-A1-2007/063754
- WO-A1-2007/063796
- WO-A1-2008/009343
- WO-A1-2008/056746
- WO-A1-2008/132103
- WO-A1-2008/146839
- WO-A1-2008/149691
- WO-A1-2009/016964
- WO-A1-2009/044615
- WO-A1-2009/124627
- WO-A2-2005/023894
- WO-A2-2010/131855
- JP-A- 11 167 215
- JP-A- 11 176 578
- JP-A- 2006 193 729
- JP-A- 2006 339 577
- JP-A- 2007 088 222
- JP-T- 2008 509 266
- US-A1- 2002 132 134
- US-A1- 2003 044 646
- US-A1- 2008 284 322
- YUNCHEOL NA ET AL.: "Formation of Pyro-products by the Pyrolysis of Monobromophenols", BULLETIN KOREAN CHEMICAL SOCIETY, vol. 24, no. 9, 2003, pages 1276-1280, XP002664291,
- ERDTMAN H. ET AL.: "STUDIES ON HUMIC ACIDS", ACTA CHEMICA SCANDINAVICA, vol. 13, no. 4, 1959, pages 653-658, XP002664292,
- MASAKI SHIMIZU ET AL.: "Palladium-Catalyzed Annulation of vic-Bis(pinacolatoboryl)alkenes and -phenanthrenes with 2,2'-Dibromobiaryls: Facile Synthesis of Functionalized Phenanthrenes and Dibenzo[g,p]chrysenes", ANGEWANDTE CHEMIE, INTERNATIONAL EDITION, vol. 47, no. 42, 15 September 2008 (2008-09-15), pages 8096-8099, XP002664293,
- TERUHISA TSUCHIMOTO ET AL.: "Indium-Catalyzed Annulation of 2-Aryl- and 2-Heteroarylindoles with Propargyl Ethers: Concise Synthesis and Photophysical Properties of Diverse Aryl- and Heteroaryl-Annulated[a]carbazoles", JOURNAL OF AMERICAN CHEMICAL SOCIETY, vol. 130, 3 November 2008 (2008-11-03), pages 15823-15835, XP002664294,
- XIA, C. ET AL.: 'Ladder-type oligo (p-phenylene)s tethered to a poly(alkylene) main chain: The orthogonal approach to functional light-emitting polymers' MACROMOLECULES vol. 34, no. 20, 2001, pages 6922 - 6928, XP008141222
- JACOB, J. ET AL.: 'Ladder-type pentaphenylenes and their polymers: Efficient blue-light emitters and electron-accepting materials via a common intermediate' J. AM. CHEM. SOC. vol. 126, no. 22, 2004, pages 6987 - 6995, XP002483843
- JACOB, J. ET AL.: 'A fully aryl-substituted poly(ladder-type pentaphenylene): A remarkably stable blue-light-emitting polymer' MACROMOLECULES vol. 38, no. 24, 2005, pages 9933 - 9938, XP008140433
- WIESENHOFER, H. ET AL.: 'Molecular origin of the temperature-dependent energy migration in a rigid-rod ladder-phenylene molecular host' ADV. MATER. vol. 18, 2006, pages 310 - 314, XP008140430
- MISHRA, A.K. ET AL.: 'Blue-emitting carbon- and nitrogen-bridged poly(ladder-type tetraphenylene)s' CHEM. MATER. vol. 18, no. 12, 2006, pages 2879 - 2885, XP008140435
- LAQUAI, F. ET AL.: 'Photophysical properties of a series of poly(ladder-type phenylene)s' ADV. FUNCT. MATER. vol. 17, 2007, pages 3231 - 3240, XP001507490
- SIRRINGHAUS, H. ET AL.: 'Dibenzothienobisbenzothiophene- a novel fused- ring oligomer with high field-effect mobility' J. METER. CHEM. vol. 9, 1999, pages 2095 - 2101, XP002244281
- SONNTAG, M. ET AL.: 'Synthesis and characterization of novel conjugated bisindenocarbazoles' TETRAHEDRON vol. 62, 2006, pages 8103 - 8108, XP025002532
- SONNTAG, M. ET AL.: 'Novel bisindenocarbazole derivative exhibiting a nematic mesophase' TETRAHEDRON LETT. vol. 47, 2006, pages 8313 - 8317, XP025003326
- BELL, T.D.M. ET AL.: 'Charge transfer enhanced annihilation leading to deterministic single photon emission in rigid perylene end-capped polephenylenes' CHEM. COMMUN. no. 39, 2005, pages 4973 - 4975, XP008140444
- ZHANG, M. ET AL.: 'Conjugated alternating copolymers containing both donor and acceptor moieties in the main chain' CHEM. COMMUN. no. 17, 2007, pages 1704 - 1706, XP002501156
- SONNTAG, M. ET AL.: 'Synthesis of a novel liquid crystalline bisindenocarbazole derivative' LIQ. CRYST. vol. 34, no. 1, 2007, pages 49 - 57, XP001504017
- FRON. E. ET AL.: 'Singlet-singlet annihilation leading to a charge-transfer intermediate in chromophore-end-capped pentaphenylenes' CHEM. PHYS. CHEM. vol. 8, 2007, pages 1386 - 1393, XP008140564
- IZQUIERDO, M.A. ET AL.: 'Switching of the fluorescence emission of a single molecules between the locally excited and charge transfer states' CHEM. PHYS. LETT. vol. 401, 2005, pages 503 - 508, XP004696551
- LEE, W.-Y. ET AL.: 'Synthesis of new fluorene- indolocarbazole alternating copolymers for light-emitting diodes and field effect transistors' POLYM. J. vol. 40, no. 3, 2008, pages 249 - 255, XP008140570
- AMARA, J.P. ET AL.: 'Conjugated polymers with geminal trifluoromethyl substituent's derived from hexafluoroacetone' MACROMOLECULES vol. 39, 2006, pages 5753 - 5759, XP008140566

## Description

### Technical Field

The present invention relates to a polycyclic compound.

### Background Art

An organic electroluminescence device (hereinafter, "electroluminescence" may be abbreviated as "EL") is a spontaneous light emitting device which utilizes the principle that a fluorescent substance emits light by energy of recombination of holes injected from an anode and electrons injected from a cathode when an electric field is applied. Since an organic EL device of the laminate type driven under a low electric voltage was reported, many studies have been conducted on organic EL devices using organic materials as the constituent materials. The devices of the laminate type use tris(8-quinolinolato) aluminum for a light emitting layer and a triphenyldiamine derivative for a hole transporting layer. Advantages of the laminate structure are that the efficiency of hole injection into the light emitting layer can be increased, that the efficiency of forming exciton which are formed by blocking and recombining electrons injected from the cathode can be increased, and that exciton formed within the light emitting layer can be enclosed. As described above, for the structure of the organic EL device, a two-layered structure having a hole transporting (injecting) layer and an electron transporting light emitting layer and a three-layered structure having a hole transporting (injecting) layer, a light emitting layer, and an electron transporting (injecting) layer are well known. To increase the efficiency of recombination of injected holes and electrons in the devices of the laminate type, the structure of the device and the process for forming the device have been studied.

As the light emitting material of the organic EL device, chelate complexes such as tris (8-quinolinolato) aluminum complexes, coumarine derivatives, tetraphenylbutadiene derivatives, distyrylarylene derivatives, and oxadiazole derivatives are known. It is reported that light in the visible region ranging from blue light to red light can be obtained by using these light emitting materials, and development of a device exhibiting color images is expected.

In addition, it has been recently proposed that a phosphorescent material as well as a fluorescent material be utilized in the light emitting layer of an organic EL device. High luminous efficiency is achieved by utilizing the singlet and triplet states of an excited state of an organic phosphorescent material in the light emitting layer of an organic EL device. Upon recombination of an electron and a hole in an organic EL device, singlet excitons and triplet excitons may be produced at a ratio of 1:3 owing to a difference in spin multiplicity between the singlet and triplet excitons, so the use of a phosphorescent material may achieve luminous efficiency three to four times as high as that of a device using fluorescence alone.

Patent Literatures 1 to 7 are exemplary inventions each describing such materials for an organic EL device.

Patent Literature 1 describes a compound using a structure obtained by crosslinking a terphenylene skeleton with, for example, a carbon atom, nitrogen atom, or oxygen atom as a mother skeleton. The document, whichmainly discloses data indicative of the potential of the compound to serve as a hole transporting material, describes that the compound is used as a host material for a phosphorescent material in a light emitting layer. However, the description is limited to a red phosphorescent device, and the luminous efficiency of the device is not high enough for the device to be put into practical use.

Patent Literature 2 describes an indolocarbazole compound having a substituent on a nitrogen atom or on an aromatic ring. The document recommends that the compound be used as a hole transporting material, and describes that a thermally and morphologically stable, thin hole transporting layer can be prepared from the compound. However, the document does not describe data indicative of the usefulness of the compound as a host material or electron transporting material to be used together with a phosphorescent material.

Patent Literature 3 describes indolocarbazole compounds each having a substituent on a nitrogen atom or on an aromatic ring. The document discloses data on a green light emitting device using any one of those compounds as a host material for a phosphorescent material in its light emitting layer. However, a high voltage must be applied to the device to drive the device, and the device shows low luminous efficiency, so the device cannot be sufficiently put into practical use.

Patent Literature 4 describes indolocarbazole compounds each having a substituent. The document describes that each of the compounds functions as a host material for a phosphorescent material in a light emitting layer. However, each of those compounds is characterized in that the compound has a dimer or trimer structure through a linking group, and each of the compounds tends to have a large molecular weight. The document discloses data on a green phosphorescent device using any one of those compounds, but all the compounds used each have a large molecular weight of 800 or more. The efficiency with which a material having a large molecular weight is deposited in a vacuum is poor, and the material may decompose owing to heating for a long time period, so the material may be insufficient in terms of practical use.

Patent Literatures 5 and 6 describe indenofluorene compounds each having a substituent on an aromatic ring, and describe that each of the compounds functions as a fluorescent material in a light emitting layer. However, none of the documents describes data indicative of the usefulness of each of the compounds as a host material or electron transporting material to be used together with a phosphorescent material.

Patent Literature 7 describes compounds each using a structure obtained by crosslinking a terphenylene skeleton with a sulfur atom, boron atom, or phosphorus atom as a mother skeleton. The document describes that each of those compounds has excellent oxidation resistance, and allows the formation of an organic semiconductor active layer by an application method. However, the document does not describe data indicative of the usefulness of each of the compounds as a host material or electron transporting material to be used together with a fluorescent material or phosphorescent material.

Patent Literature 8 is prior art under Art. 54(3) EPC. It discloses compounds (27) to (32) which are not covered by the present claims.

Patent Literature 9 is prior art under Art. 54(3) EPC, and the compound disclosed on p. 79 and 83 of this document is not covered by the present claims.

Patent Literatures 10 to 15 disclose compounds of the N,N-ladder-type corresponding to formula (7) of the present invention wherein both X₇ and X₈ are N-R¹, of formula (8) wherein both X₉ and X₁₀ are N-R¹, or of formula (9) wherein both X₁₁ and X₁₂ are N-R¹.

Patent Literature 16 discloses polycyclic compounds having a pyrene structure. Compounds 137 and 217 of said document have a p-toylpyrenyl group and a p-tolyl-t-butylphenyl group, respectively, and are not according to claim 1.

Non-Patent Literature 1 discloses compound 14 corresponding to the structure of formula (7) of the invention, which is not covered by claim 1 due to the substituent -C-ArArOH of said compound 14.

### Citation List

### Patent Literature

[PTL 1] WO 2006/122630 A1
[PTL 2] EP 0908787 A
[PTL 3] WO 2007/063796 A1
[PTL 4] WO 2007/063754 A1
[PTL 5] US 2002/0132134 A1
[PTL 6] US 2003/0044646 A1
[PTL 7] JP 2008-81494 A
[PTL 8] EP 2 284 920 A1
[PTL 9] WO 2009/124627 A1
[PTL 10] WO 2008/056746 A1
[PTL 11] WO 2007/063796 A1
[PTL 12] WO 2007/063754 A1
[PTL 13] JP 2006 193729
[PTL 14] JP 11 176578 A
[PTL 15] JP 11 167215 A
[PTL 16] WO 2007/029798 A1

### Non-Patent Literature

[NPL 1] Organic Lett. 2006, Vol. 8, No. 22, 5033-5036

### Summary of Invention

### Technical Problem

The present invention has been made with a view to solving the above-mentioned problems, and an object of the present invention is to provide a polycyclic compound for realizing an organic EL device which shows high luminous efficiency and has a long lifetime.

### Solution to Problem

The inventors of the present invention have made extensive studies to achieve the object. As a result, the inventors have found that the use of a polycyclic compound represented by any one of the following formulae (7) to (12) as a material for an organic EL device achieves the object. Thus, the inventors have completed the present invention.

That is, the present invention provides a polycyclic compound represented by any one of the following formulae (7) to (12) : 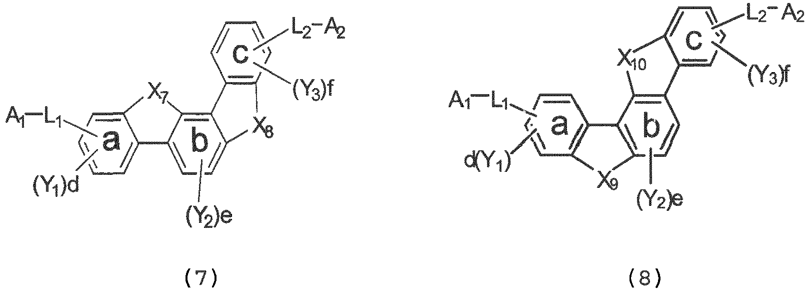 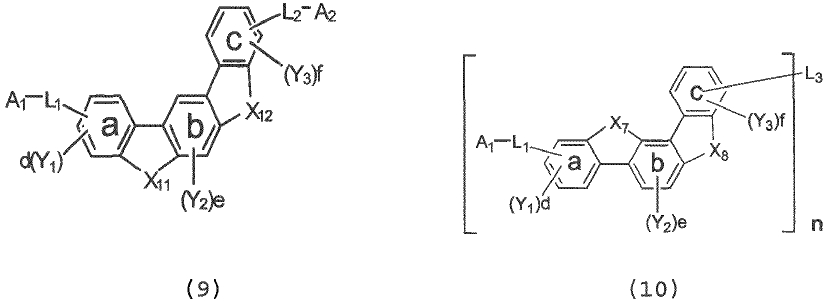 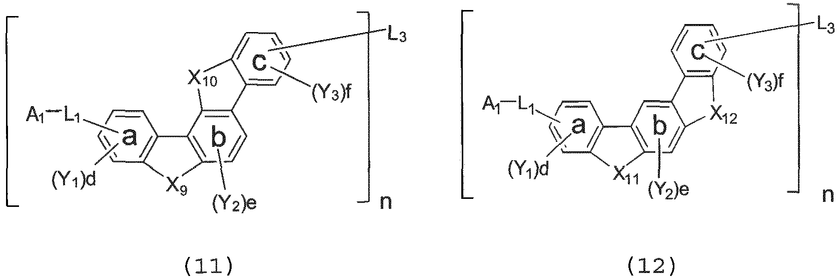 where:
X₇, X₈, X₉, X₁₀, X₁₁, and X₁₂ each independently represent oxygen (O), sulfur (S), N-R₁ or CR₂R₃; , provided that X₇, X₉ and X₁₁ in the general formulae (7) to (12) each independently represent oxygen (O), sulfur (S) or CR₂R₃, or X₈, X₁₀ and X₁₂ in the general formulae (7) to (12) each independently represent oxygen (O), sulfur (S) or CR₂R₃;
R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group, wherein the aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted aromatic heterocyclic group having a ring formed of 3 to 24 atoms;
n represents 2, 3, or 4, and the compound represented by any one of the formulae (10) to (12) comprises a dimer using L₃ as a linking group for n=2, a trimer using L₃ as a linking group for n=3, or a tetramer using L₃ as a linking group for n=4;
L₁ represents a single bond, an alkylene group having 1 to 20 carbon atoms, a substituted or unsubstituted cycloalkylene group having a ring formed of 3 to 20 carbon atoms, a divalent silyl group or divalent silyl group having 2 to 20 carbon atoms, a substituted or unsubstituted, divalent aromatic hydrocarbon group, wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with a benzene ring a through a carbon-carbon bond;
L₂ represents a single bond, an alkylene group having 1 to 20 carbon atoms, a substituted or unsubstituted cycloalkylene group having a ring formed of 3 to 20 carbon atoms, a divalent silyl group or divalent silyl group having 2 to 20 carbon atoms, a substituted or unsubstituted, divalent aromatic hydrocarbon group, wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with a benzene ring c through a carbon-carbon bond,
   provided that, when both X₇ and X₈, both X₉ and X₁₀, or both X₁₁ and X₁₂ represent CR₂R₃ and both L₁ and L₂ represent the substituted or unsubstituted, divalent aromatic hydrocarbon groups, a case where L₁ is linked to the benzene ring a at the para position with respect to the benzene ring b and L₂ is linked to the benzene ring c at the para position with respect to the benzene ring b is excluded;
when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group, wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with the benzene ring c through a carbon-carbon bond,
   when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group, wherein the trivalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, trivalent aromatic heterocyclic group which has 3 to 24 atoms and which is linked with the benzene ring c through a carbon-carbon bond, or
when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group, wherein the tetravalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, tetravalent aromatic heterocyclic group which has a ring formed of 3 to 24 atoms and which is linked with the benzene ring c through a carbon-carbon bond,
provided that, when both X₇ and X₈, both X₉ and X₁₀, or both X₁₁ and X₁₂ represent CR₂R₃ and both L₁ and L₃ represent the substituted or unsubstituted, divalent, trivalent, or tetravalent aromatic hydrocarbon groups, a case where L₁ is linked to the benzene ring a at the para position with respect to the benzene ring b and L₃ is linked to the benzene ring c at the para position with respect to the benzene ring b is excluded;
A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group, wherein the aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or an aromatic heterocyclic group which has a ring formed of 3 to 24 atoms and which is linked with L₁ through a carbon-carbon bond,
provided that, when L₁ represents an alkylene group having 1 to 20 carbon atoms, a case where A₁ represents a hydrogen atom is excluded;
   A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring, wherein the aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or an aromatic heterocyclic group which has a ring formed of 3 to 24 atoms and which is linked with L₂ through a carbon-carbon bond,
provided that, when L₂ represents an alkylene group having 1 to 20 carbon atoms, a case where A₂ represents a hydrogen atom is excluded;
Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group, wherein the aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted aromatic heterocyclic group which has a ring formed of 3 to 24 atoms and which is linked with the benzene ring a, b, or c through a carbon-carbon bond,
d and f each represent a number of 0, 1, 2, or 3, and e represents a number of 0, 1, or 2,
provided that, when both X₇ and X₈, both X₉ and X₁₀, or both X₁₁ and X₁₂ represent oxygen (O), sulfur (S), or CR₂R₃, both L₁ and L₂ represent single bonds, and both A₁ and A₂ represent hydrogen atoms, the benzene ring b has one or two Y₂'s, and a case where Y₂ represents a methyl group or an unsubstituted phenyl group is excluded;
A₁, A₂, L₁, L₂, and L₃ are each free of any carbonyl group, and
the substituent that may substitute each of the groups in formulae (7) to (12) includes alkyl groups each having 1 to 6 carbon atoms, a phenyl group, a pyridyl group, a carbazolyl group, and a dibenzofuranyl group,
provided that the following compound is excluded:

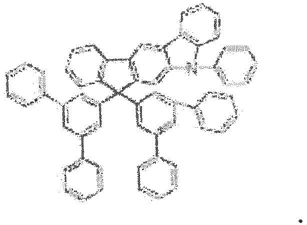

Further, disclosed is an organic EL device having one or more organic thin film layers including a light emitting layer between a cathode and an anode in which at least one layer of the organic thin film layers contains a polycyclic compound having a π-conjugated heteroacene skeleton crosslinked with a carbon atom, nitrogen atom, oxygen atom, or sulfur atom.

Further, the material for an organic EL device is effective also as a material for an organic electron device such as an organic solar cell, organic semiconductor laser, a sensor using organic matter, or an organic TFT.

### Advantageous Effects of Invention

According to the present invention, a polycyclic compound for realizing an organic EL device which shows high luminous efficiency and has a long lifetime is provided.

### Description of Embodiments

The polycyclic compound of the invention is represented by any one of the following formulae (7) to (12).

[In the formulae (7) to (12), X₇, X₈, X₉, X₁₀, X₁₁, and X₁₂ each independently represent Oxygen (O), Sulfur (S), N-R₁, or CR₂R₃, provided that provided that X₇, X₉ and X₁₁ in the general formulae (7) to (12) each independently represent oxygen (O), sulfur (S) or CR₂R₃, or X₈, X₁₀ and X₁₂ in the general formulae (7) to (12) each independently represent oxygen (O), sulfur (S) or CR₂R₃. R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group wherein the aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted aromatic heterocyclic group having a ring formed of 3 to 24 atoms.

In the formulae (10) to (12), n represents 2, 3, or 4, and the compound represented by any one of the formulae (10) to (12) includes a dimer using L₃ as a linking group for n=2, a trimer using L₃ as a linking group for n=3, or a tetramer using L₃ as a linking group for n=4.

In the formulae (7) to (12), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, , or a substituted or unsubstituted, divalent aromatic heterocyclic group which has a ring formed of 3 to 24 atoms and which is linked with a benzene ring a through a carbon-carbon bond.

In the formulae (7) to (9), L₂ represents a single bond, an alkylene group having 1 to 20 carbon atoms, a substituted or unsubstituted cycloalkylene group having a ring formed of 3 to 20 carbon atoms, a divalent silyl group or a divalent silyl group having 2 to 20 carbon atoms, a substituted or unsubstituted, divalent aromatic hydrocarbon wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with a benzene ring c through a carbon-carbon bond, provided that, when both X₇ and X₈, both X₉ and X₁₀, or both X₁₁ and X₁₂ represent CR₂R₃ and both L₁ and L₂ represent the substituted or unsubstituted, divalent aromatic hydrocarbon a case where L₁ and L₂ are simultaneously linked at para positions with respect to a benzene ring b is excluded.

In the formulae (10) to (12), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted having 2 to 20 carbon atoms, a substituted or unsubstituted, divalent aromatic hydrocarbon group wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with the benzene ring c through a carbon-carbon bond,
when n represents 3, L₃ represents a trivalent saturated hydrocarbon group having 1 to 20 carbon atoms, a substituted or unsubstituted trivalent saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon wherein the trivalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, , or a substituted or unsubstituted, trivalent aromatic heterocyclic group which has 3 to 24 atoms and which is linked with the benzene ring c through a carbon-carbon bond,
or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, tetravalent aromatic heterocyclic group which has a ring formed of 3 to 24 atoms and which is linked with the benzene ring c through a carbon-carbon bond,
provided that, when both X₇ and X₈, both X₉ and X₁₀, or both X₁₁ and X₁₂ represent CR₂R₃ and both L₁ and L₃ represent the substituted or unsubstituted, divalent, trivalent, or tetravalent aromatic hydrocarbon groups, a case where L₁ and L₃ are simultaneously linked at para positions with respect to the benzene ring b is excluded.

In the formulae (7) to (12), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or an aromatic heterocyclic group which has a ring formed of 3 to 24 atoms and which is linked with L₁ through a carbon-carbon bond,
provided that, when L₁ represents an alkylene group having 1 to 20 carbon atoms, a case where A₁ represents a hydrogen atom is excluded.

In the formulae (7) to (9), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or an aromatic heterocyclic group which has a ring formed of 3 to 24 atoms and which is linked with L₂ through a carbon-carbon bond,
provided that, when L₂ represents an alkylene group having 1 to 20 carbon atoms, a case where A₂ represents a hydrogen atom is excluded.

In the formulae (7) to (12), Y₁, Y₂, and Y₃ each represent an alkyl group having 1 to 20 carbonatoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted aromatic heterocyclic group which has a ring formed of 3 to 24 atoms and which is linked with the benzene ring a, b, or c through a carbon-carbon bond,
d and f each represent the number of 0, 1, 2, or 3, and e represents the number of 0, 1, or 2,
provided that, when both X₇ and X₈, both X₉ and X₁₀, or both X₁₁ and X₁₂ each represent oxygen (O), sulfur (S), or CR₂R₃, both L₁ and L₂ represent single bonds, and both A₁ and A₂ represent hydrogen atoms, the benzene ring b has one or two Y₂'s, and a case where Y₂ represents a methyl group or an unsubstituted phenyl group is excluded.

In the formulae (7) to (12), A₁, A₂, L₁, L₂, and L₃ are each free of any carbonyl group.]

The compounds represented by the formulae (7) to (12) are each preferably a polycyclic compound represented by any one of the following formulae (7a) to (12a), (7b) to (12b).

Thus, it is preferred that substituents such as L₁-A₁- and L₂-A₂- be bonded at meta positions, the expansion of the conjugated system is prevented, and triplet energy can be widened.

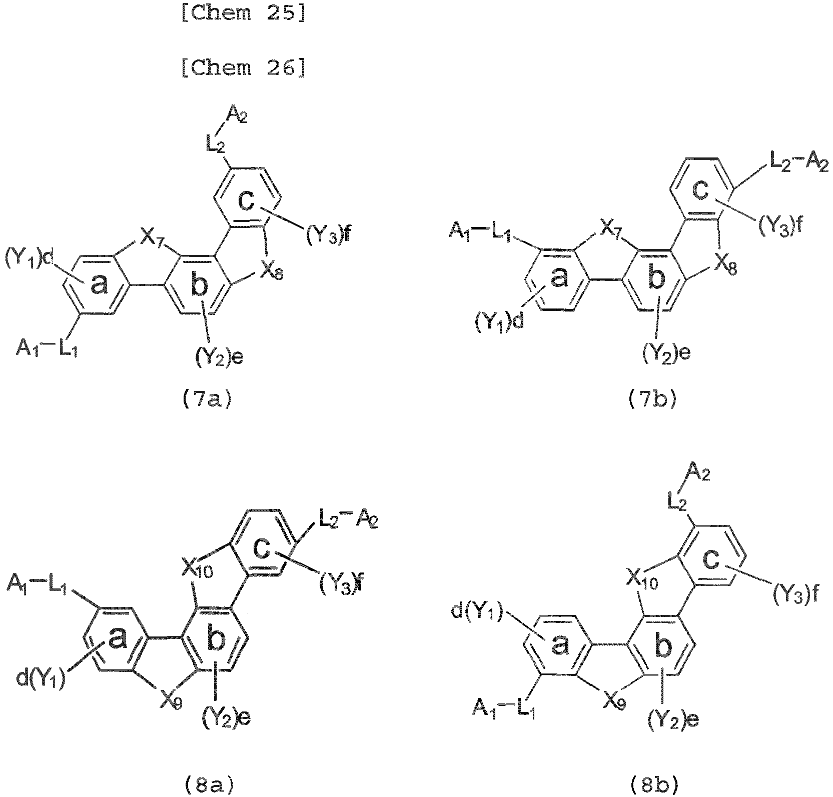

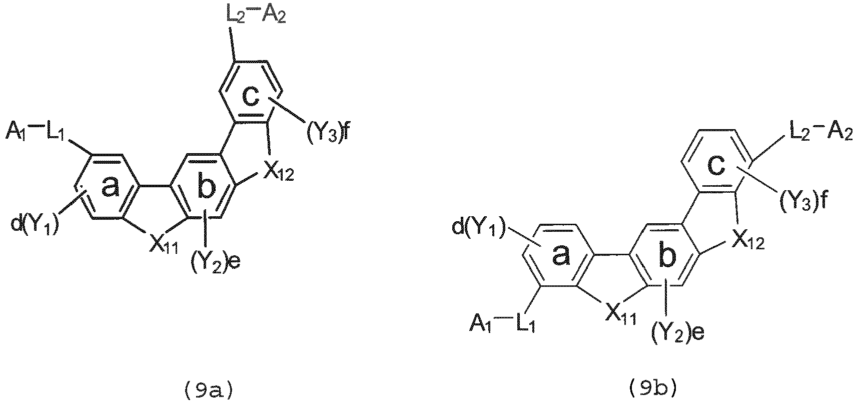

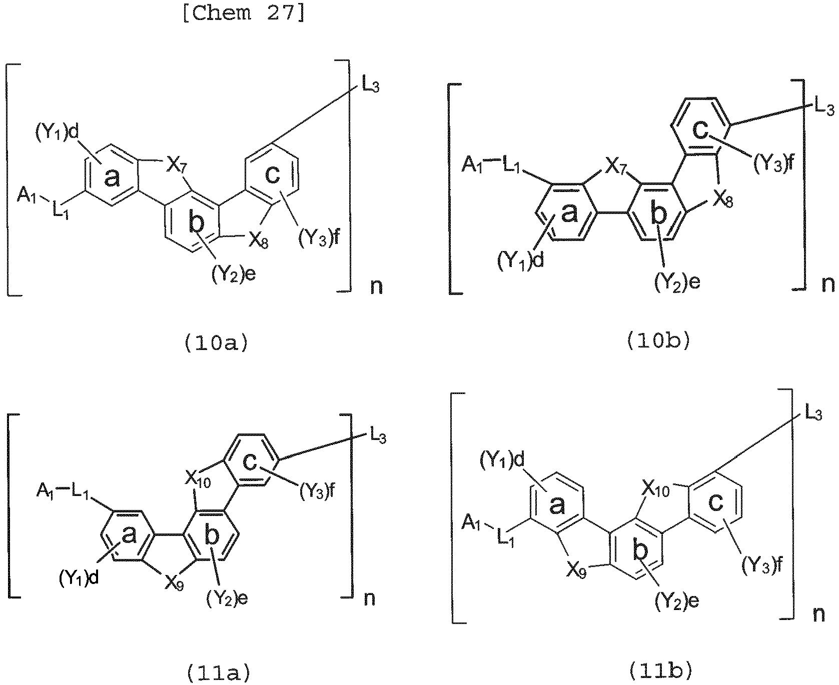

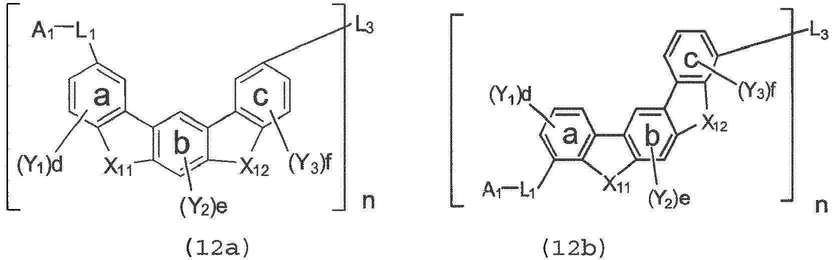

[In the formulae (7a) to (12a), and (7b) to (12b), X₇ to X₁₂, L₁, L₂, L₃, A₁, A₂, Y₁, Y₂, Y₃, n, d, f, and e each have the same meaning as that described above.]

The compounds represented by the formulae (9) and (12) are each preferably a benzofurano dibenzofuran derivative represented by one of the following formulae (17) and (18).

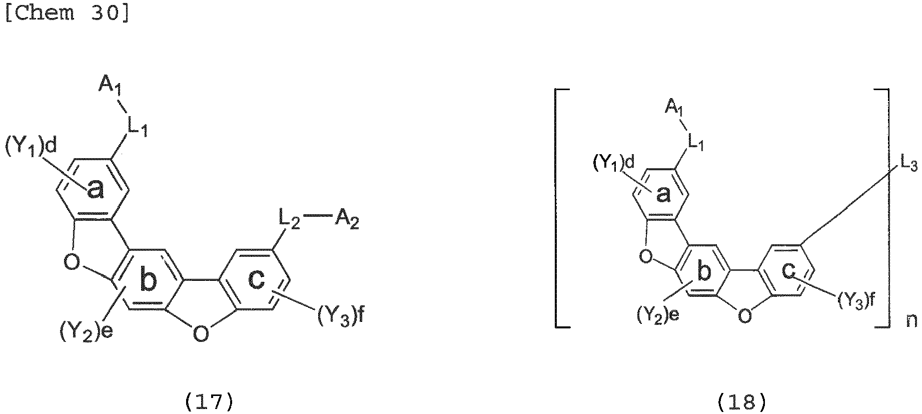

[In the formula (18), n represents 2, 3, or 4, and the compound represented by the formula (18) includes a dimer using L₃ as a linking group for n=2, a trimer using L₃ as a linking group for n=3, or a tetramer using L₃ as a linking group for n=4.

In the formulae (17) and (18), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with a benzene ring a through a carbon-carbon bond.

In the formula (17), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with a benzene ring c through a carbon-carbon bond.

In the formula (18), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with the benzene ring c through a carbon-carbon bond,
when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, trivalent aromatic heterocyclic group which has 3 to 24 atoms and which is linked with the benzene ring c through a carbon-carbon bond,
or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, tetravalent aromatic heterocyclic group which has a ring formed of 3 to 24 atoms and which is linked with the benzene ring c through a carbon-carbon bond.

In the formulae (17) and (18), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or an aromatic heterocyclic group which has a ring formed of 3 to 24 atoms and which is linked with L₁ through a carbon-carbon bond,
provided that, when L₁ represents an alkylene group having 1 to 20 carbon atoms, a case where A₁ represents a hydrogen atom is excluded.

In the formula (17), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded.

In the formulae (17) and (18), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted aromatic heterocyclic group which has a ring formed of 3 to 24 atoms and which is linked with the benzene ring a, b, or c through a carbon-carbon bond,
d and f each represent the number of 0, 1, 2, or 3, and e represents the number of 0, 1, or 2,
provided that, when both L₁ and L₂ represent single bonds, and both A₁ and A₂ represent hydrogen atoms, the benzene ring b has one or two Y₂'s, and a case where Y₂ represents a methyl group or an unsubstituted phenyl group is excluded.

In the formulae (17) and (18), A₁, A₂, L₁, L₂, and L₃ are each free of any carbonyl group.]

There is also disclosed a halogen compound represented by the following formula (30), which is not claimed, as an intermediate suitable for use in the synthesis of the polycyclic compound of the present invention.

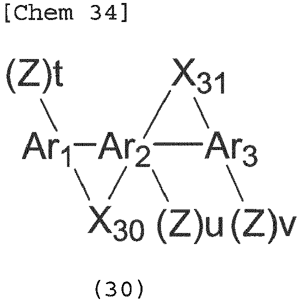

[In the formula (30), Ar₁, Ar₂, and Ar₃ each independently represent a substituted or unsubstituted aromatic hydrocarbon group having a ring formed of 6 carbon atoms or a substituted or unsubstituted aromatic heterocyclic group having a ring formed of 6 atoms, provided that Ar₁, Ar₂, and Ar₃ each may have one substituent Y or a plurality of substituents Y's, in the case of the plurality of substituents Y's, the substituents Y's may be different from each other, and Y's each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with Ar₁, Ar₂, or Ar₃ through a carbon-carbon bond.

X₃₀ and X₃₁ each independently represent oxygen (O), sulfur (S), or N-R₁.

R₁ represents an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, when both X₃₀ and X₃₁ represent N-R₁, at least one R₁ represents a substituted or unsubstituted, monovalent fused aromatic heterocyclic group having a ring formed of 8 to 24 atoms.

Z represents a halogen atom that is a fluorine atom, a chlorine atom, a bromine atom, or an iodine atom.

t, u, and v each represent 0 or 1, provided that t+u+v≥1.

The case where the formula (30) adopts a structure represented by the following formula (31) is excluded, 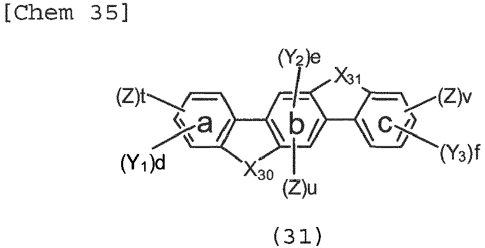 provided that, X₃₀, X₃₁, Z, t, and u in the formula (31) are identical to X₃₀, X₃₁, Z, t, and u in the formula (30), respectively.

In the formula (31), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with a benzene ring a, b, or c through a carbon-carbon bond, d and f each represent the number of 0, 1, 2, or 3, and e represents the number of 0, 1, or 2.]

The halogen compound represented by the formula (30) is preferably represented by the following formula (32), and is more preferably represented by any one of the following formulae (33) to (37).

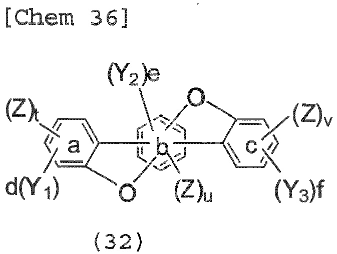

[In the formula (32), Y₁, Y₂, and Y₃ each independently represent a hydrogen atom, an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with a benzene ring a, b, or c through a carbon-carbon bond, d and f each represent the number of 0, 1, 2, or 3, and e represents the number of 0, 1, or 2.

Z represents a halogen atom that is a fluorine atom, a chlorine atom, a bromine atom, or an iodine atom.

t, u, and v each represent 0 or 1, provided that t+u+v≥1.]

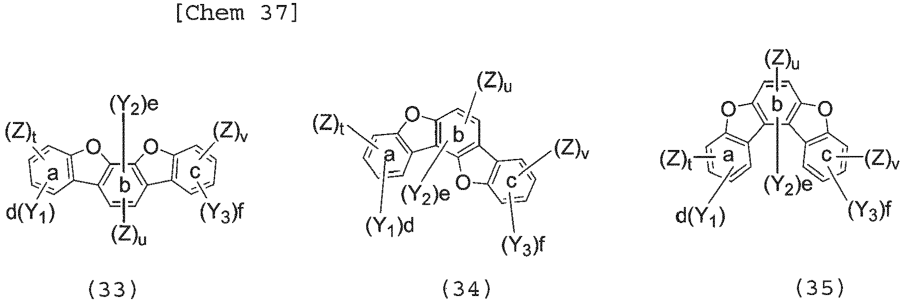

[In the formulae (33) to (35), Y₁, Y₂, and Y₃ each independently represent a hydrogen atom, an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with a benzene ring a, b, or c through a carbon-carbon bond, d and f each represent the number of 0, 1, 2, or 3, and e represents the number of 0, 1, or 2.

Z represents a halogen atom that is a fluorine atom, a chlorine atom, a bromine atom, or an iodine atom.

t, u, and v each represent 0 or 1, provided that t+u+v≥1.]

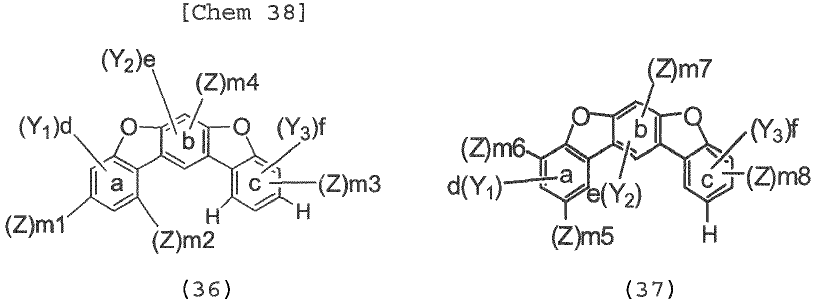

[In the formulae (36) and (37), Y₁, Y₂, and Y₃ each independently represent a hydrogen atom, an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with a benzene ring a, b, or c through a carbon-carbon bond, d and f each represent the number of 0, 1, 2, or 3, and e represents the number of 0, 1, or 2.

Z represents a halogen atom that is a fluorine atom, a chlorine atom, a bromine atom, or an iodine atom.

m1 to m8 each represent 0 or 1, provided that, m1+m2=1, m5+m6≤1, m6+m7+m8≥1.]

The halogen compound represented by any one of the formulae (33) to (37) can be obtained by subjecting an aromatic halogen compound represented by the following formula (38) to an intramolecular cyclization reaction.

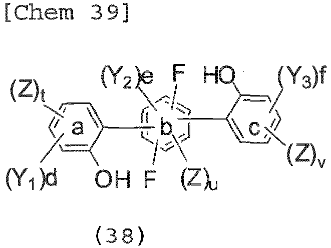

[In the formula (38), Y₁, Y₂, and Y₃ each independently represent a hydrogen atom, an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with a benzene ring a, b, or c through a carbon-carbon bond, d and f each represent the number of 0, 1, 2, or 3, and e represents the number of 0, 1, or 2.

Z represents a halogen atom that is a fluorine atom, a chlorine atom, a bromine atom, or an iodine atom.

t, u, and v each represent 0 or 1, provided that t+u+v≥1.]

The reaction of the aromatic halogen compound represented by the above-mentioned formula (38) is typically performed in the presence of a basic catalyst. Examples of the basic catalyst include sodium amide, triethylamine, tributylamine, trioctylamine, pyridine, N,N-dimethylaniline, 1,5-diazabicyclo[4,3,0]nonene-5 (DBN), 1,8-diazabicyclo[5,4,0]undecene-7 (DBU), sodium hydroxide, potassium hydroxide, sodium hydride, sodium phosphate, potassium phosphate, sodium carbonate, potassium carbonate, sodium methoxide, and potassium t-butoxide.

The basic catalyst is used for the aromatic halogen compound represented by the formula (38) as a reaction raw material in such an amount that a molar ratio of the basic catalyst to the aromatic halogen compound is about 2 to 10 and preferably 2 to 5.

A solvent can be used as required at the time of the reaction. Specific examples of the solvent include toluene, dimethylformamide (DMF), N,N-dimethylacetamide (DMAc), dimethyl sulfoxide (DMSO), N-methylpyrrolidone (NMP), tetrahydrofuran, and dimethoxyethane. One kind of them may be used alone, or two or more kinds of them may be used in combination.

The reaction of the aromatic halogen compound represented by the above-mentioned formula (38) is performed at a temperature of about 0 to 250°C and preferably 150 to 200°C in ordinary cases. When the reaction temperature is 150°C or more, a reaction rate does not reduce but becomes moderate, and hence a reaction time is shortened. In addition, when the reaction temperature is 200°C or less, the coloring of a product is suppressed. A reaction time is typically about 1 minute to 24 hours, and is desirably 1 to 10 hours.

In addition, such a halogen compound that both X₃₀ and X₃₁ in the above-mentioned formula (30) each represent N-R₁ can be obtained by subjecting an aromatic halogen compound represented by the following formula (39) to an intramolecular cyclization reaction in the same manner as in the foregoing.

Further, such a halogen compound that both X₃₀ and X₃₁ in the above-mentioned formula (30) each represent sulfur (S) can be obtained by subjecting an aromatic halogen compound represented by the following formula (40) to an intramolecular cyclization reaction in the same manner as in the foregoing, 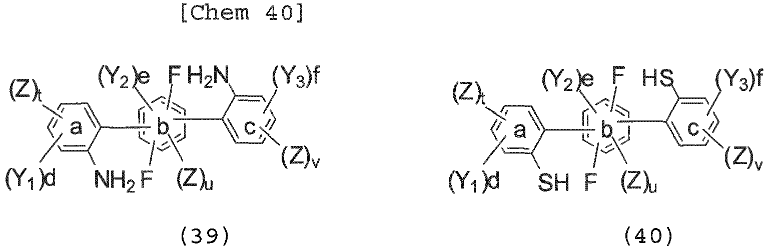 provided that Y₁, Y₂, Y₃, Z, d, e, f, t, u, and v in the formulae (39) and (40) are identical to Y₁, Y₂, Y₃, Z, d, e, f, t, u, and v in the formula (38), respectively.

In the formulae (7) to (12), (17), (18) and (30) to (40), specific examples of each group are described below.

Examples of the substituted or unsubstituted aromatic hydrocarbon group having a ring formed of 6 carbon atoms represented by each of Ar₁ to Ar₃ include substituted or unsubstituted benzene. The substituted or unsubstituted aromatic hydrocarbon group represented by each of Y₁ to Y₃, R₁ to R₃, L₁ to L₃, and A₁ and A₂ are residues having corresponding valencies of substituted or unsubstituted benzene, naphthalene, biphenyl, terphenyl, fluorene, phenanthrene, triphenylene, perylene, chrysene, fluoranthene, benzofluorene, benzotriphenylene, benzochrysene, and anthracene. Preferred are benzene, naphthalene, biphenyl, terphenyl, fluorene, and phenanthrene.

Examples of the substituted or unsubstituted aromatic heterocyclic group having a ring formed of 6 atoms represented by each of Ar₁ to Ar₃ include substituted or unsubstituted pyridine, pyridazine, pyrimidine, pyrazine, and 1,3,5-triazine. Examples of the substituted or unsubstituted aromatic heterocyclic group having a ring formed of 3 to 24 atoms represented by each of Y, Y₁ to Y₃, R₁ to R₃, L₁ to L₃, and A₁ and A₂ include residues having corresponding valencies of substituted or unsubstituted pyridine, pyridazine, pyrimidine, pyrazine, 1,3,5-triazine, carbazole, dibenzofuran, dibenzothiophene, phenoxazine, phenothiazine, and dihydroacridine. Preferred are pyridine, pyridazine, pyrimidine, pyrazine, carbazole, dibenzofuran, dibenzothiophene, phenoxazine, and dihydroacridine. In addition, examples of at least one substituted or unsubstituted, monovalent fused aromatic heterocyclic group having a ring formed of 8 to 24 atoms represented by R₁ include aromatic heterocyclic groups each having a fused structure in examples of the aromatic heterocyclic groups.

Examples of the alkyl group, alkylene group, and trivalent or tetravalent saturated hydrocarbon group, each of which has 1 to 20 carbon atoms represented by each of Y, Y₁ to Y₃, L₁ to L₃, and R₁ to R₃ include a methyl group, an ethyl group, a propyl group, an isopropyl group, an n-butyl group, an s-butyl group, a t-butyl group, an isobutyl group, an n-pentyl group, an n-hexyl group, an n-heptyl group, an n-octyl group, an n-nonyl group, ann-decyl group, an n-undecyl group, an n-dodecyl group, an n-tridecyl group, an n-tetradecyl group, an n-pentadecyl group, an n-hexadecyl group, an n-heptadecyl group, an n-octadecyl group, a neopentyl group, a 1-methylpentyl group, a 2-methylpentyl group, a 1-pentylhexyl group, a 1-butylpentyl group, a 1-heptyloctyl group, and a 3-methylpentyl group, and groups obtained by allowing those groups to have two to four valencies. Preferred are a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, a neopentyl group, a 1-methylpentyl group, a 1-pentylhexyl group, a 1-butylpentyl group, and a 1-heptyloctyl group.

Examples of the substituted or unsubstituted cycloalkyl group, cycloalkylene group, and trivalent or tetravalent cyclic saturated hydrocarbon group, each of which has a ring formed of 3 to 20 carbon atoms, represented by each of Y, Y₁ to Y₃, L₁ to L₃, R₁ to R₃, and A₁ and A₂ include a cyclopropyl group, a cyclobutyl group, a cyclopentyl group, and a cyclohexyl group, and groups obtained by allowing those group to have two to four valencies. Preferred are a cyclobutyl group, a cyclopentyl group, and a cyclohexyl group.

Examples of the alkoxy group having 1 to 20 carbon atoms and represented by each of Y and Y₁ to Y₃ include a methoxy group, an ethoxy group, a methoxy group, an i-propoxy group, an n-propoxy group, an n-butoxy group, an s-butoxy group, and a t-butoxy group. Preferred are a methoxy group, an ethoxy group, a methoxy group, an i-propoxy group, and an n-propoxy group.

Examples of the substituted silyl group having 1 to 20 carbon atoms represented by each of Y, Y₁ to Y₃, L₁ to L₃, R₁ to R₃, and A₁ and A₂ include a trimethylsilyl group, a triethylsilyl group, a tributylsilyl group, a trioctylsilyl group, a triisobutylsilyl group, a dimethylethylsilyl group, a dimethylisopropylsilyl group, a dimethylpropylsilyl group, a dimethylbutylsilyl group, a dimethyltertiary butylsilyl group, a diethylisopropylsilyl group, a phenyl dimethyl silyl group, a diphenylmethylsilyl group, a diphenyl tertiary butyl silyl group, and a triphenylsilyl group, and groups obtained by allowing those groups to have two or three valencies. Preferred are a trimethylsilyl group, a triethylsilyl group, and a tributylsilyl group.

Examples of the aralkyl group having 7 to 24 carbon atoms represented by each of Y, Y₁ to Y₃, and R₁ to R₃ include a benzyl group, a phenethyl group, and a phenylpropyl group.

Examples of the substituent that may substitute each of the group in the formulae (1) to (40) include alkyl groups each having 1 to 10 carbon atoms (such as a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, a iodomethyl group, a 1-iodoethyl group, a 2-iodoethyl group, a 2-iodoisobutyl group, a1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, and a 1,2,3-trinitropropyl group), cycloalkyl groups each having a ring formed of 3 to 40 carbon atoms (such as a cyclopropyl group, a cyclobutyl group, a cyclopentyl group, a cyclohexyl group, a 4-methylcyclohexyl group, a1-adamantyl group, a 2-adamantyl group, a 1-norbornyl group, and a 2-norbornyl group), alkoxy groups each having 1 to 6 carbon atoms (such as an ethoxy group, a methoxy group, an i-propoxy group, an n-propoxy group, an s-butoxy group, a t-butoxy group, a pentoxy group, and a hexyloxy group), cycloalkoxy groups each having a ring formed of 3 to 10 carbon atoms (such as a cyclopentoxy group and a cyclohexyloxy group), aromatic hydrocarbon groups each having a ring formed of 6 to 40 carbon atoms, aromatic heterocyclic groups each having a ring formed of 3 to 40 atoms, amino groups each substituted with an aromatic hydrocarbon group having a ring formed of 6 to 40 carbon atoms, ester groups each having an aromatic hydrocarbon group having a ring formed of 6 to 40 carbon atoms, an ester group, cyano group, and nitro group, each of which has an alkyl group having 1 to 6 carbon atoms, and halogen atom.

Of those, an alkyl group having 1 to 6 carbon atoms, a phenyl group, a pyridyl group, a carbazolyl group, and a dibenzofuranyl group are preferred and the number of substituents is preferably 1 or 2.

In the polycyclic compound represented by the formula (10) to (12) or (18), n preferably represents 2.

In the formula (7) to (9), or (17), the total number of the substituents represented by Y₁, Y₂, and Y₂ is preferably 3 or less, and the total number of the substituents represented by Y₁, Y₂, and Y₃ in the structure of []ₙ in the formula (10) to (12), or (18) is preferably 3 or less.

Compounds wherein two groups X are both NR are not according to the invention.

It is preferred that both X₁₁ and X₁₂ in the formula (9) or (12) each represent CR₂R₃. In addition, the triplet energy of such a compound represented by the formula (9) or (12) that X₁₁ and X₁₂, are on the same side with respect to the benzene rings a, b, and c can be slightly widened as compared with that in the case where the substituents are on the sides opposite to each other. As a result, luminous efficiency can be improved. The same holds true for the case where X₁₁ and X₁₂ each represent CR₂R₃.

In addition, when the benzene rings a and c are bonded at para positions with respect to the benzene ring b like any one of the formulae (5), (6), (15), (16), (19), and (25) (not claimed), the electron transporting property can be improved, and a reduction in voltage at which the device is driven can be achieved.

Alternatively, when the benzene rings a and c are bonded at meta positions with respect to the benzene ring b like any one of the formulae (7) to (12), (17) and (18), the triplet energy can be widened as compared with that in the case where the benzene rings are bonded at the para positions, and hence the luminous efficiency can be improved.

In any one of the formulae (7) to (12, at least one of X₇ and X₈, X₉ and X₁₀, X₁₁ and X₁₂, preferably represents an oxygen atom, and both the substituents more preferably represent oxygen atoms like any one of the formulae (17) and (18). An oxygen atom has a high electronegativity, and can improve the electron transporting property. As a result, a reduction in voltage at which the device is driven can be achieved. Accordingly, any such compound can be particularly suitably used as a host material or an electron transporting material. In addition, when both the substituents represent oxygen atoms, the triplet energy can be widened, and hence the luminous efficiency can be improved. In addition, when A₁, A₂, L₁, L₂, and L₃ in any one of the formulae (7) to (12), (17) and (18) are each free of a carbonyl group, that is, a terminal structure is free of a carbonyl group, the shortening of the lifetime is suppressed.

Specific examples of the polycyclic compounds represented by the formulae (7) to (12), (17) and (18) of the present invention are shown below. However, the present invention is not limited to these exemplary compounds.

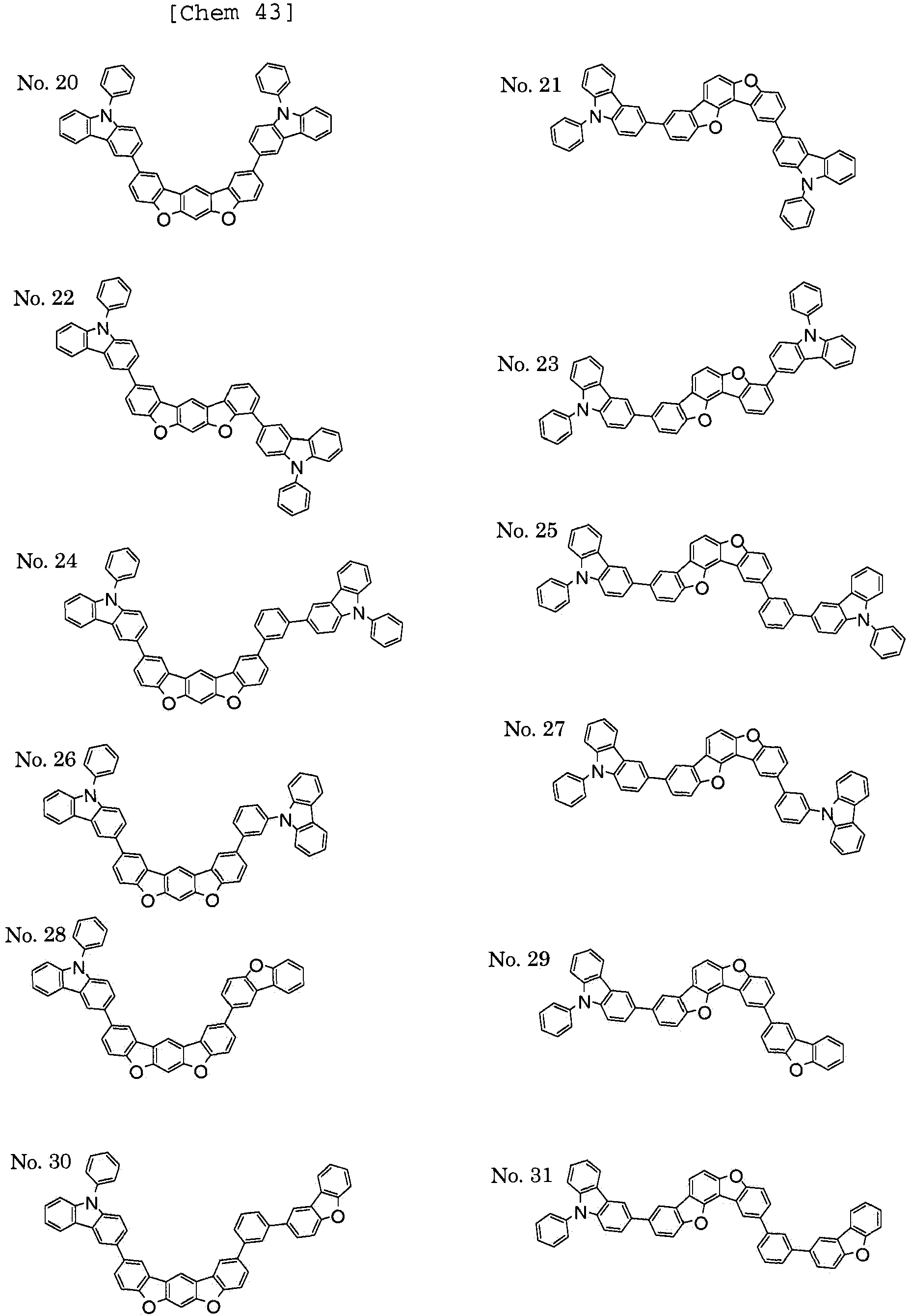

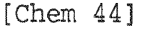

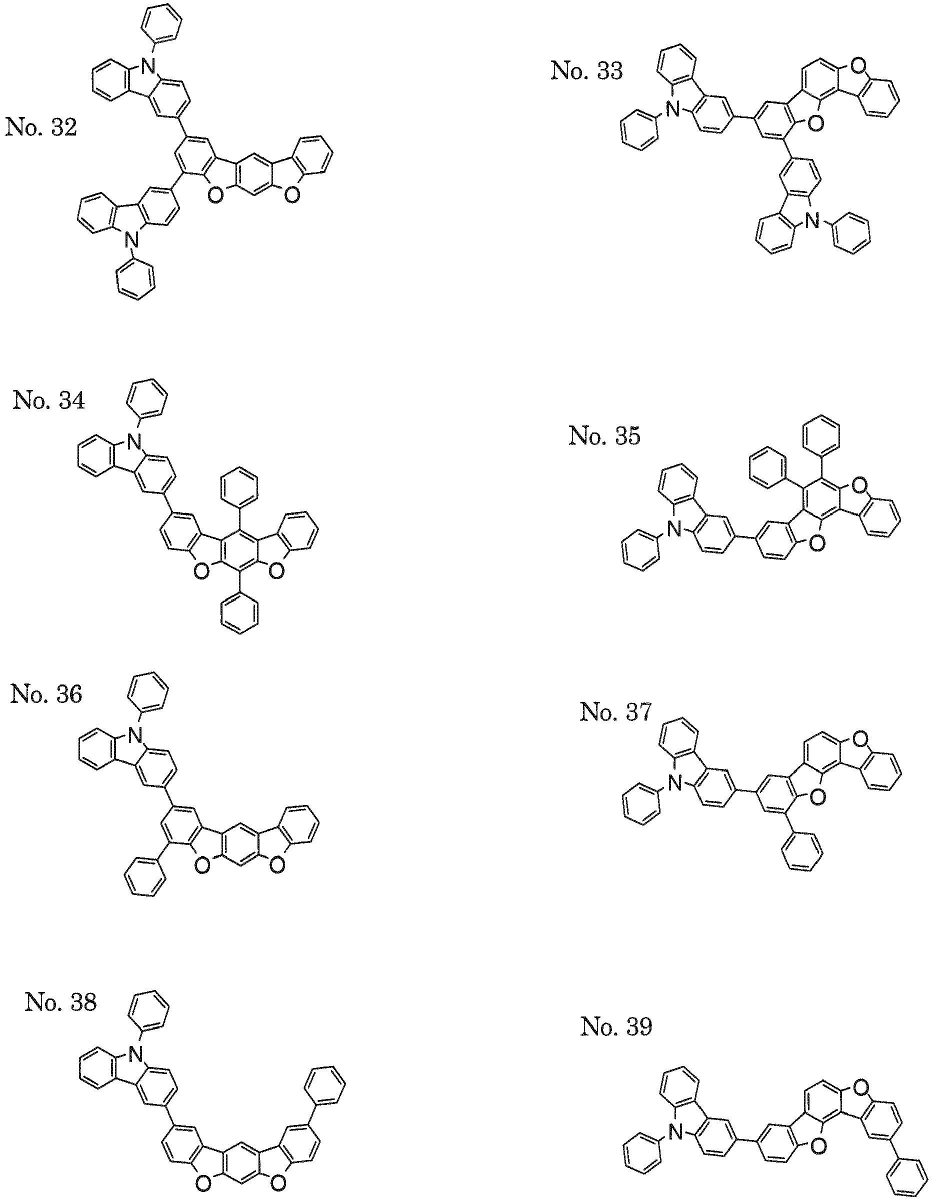

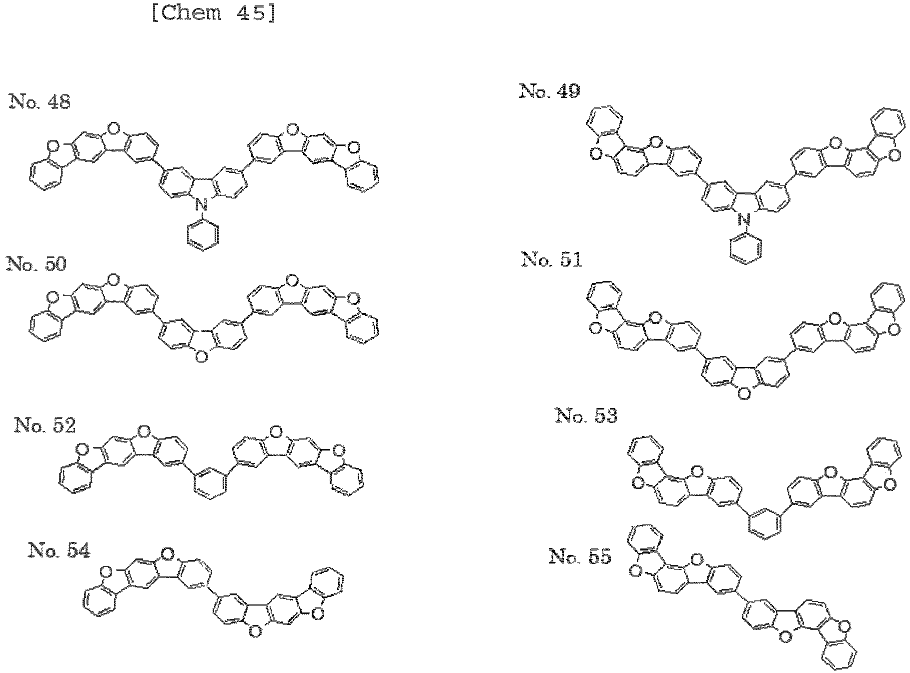

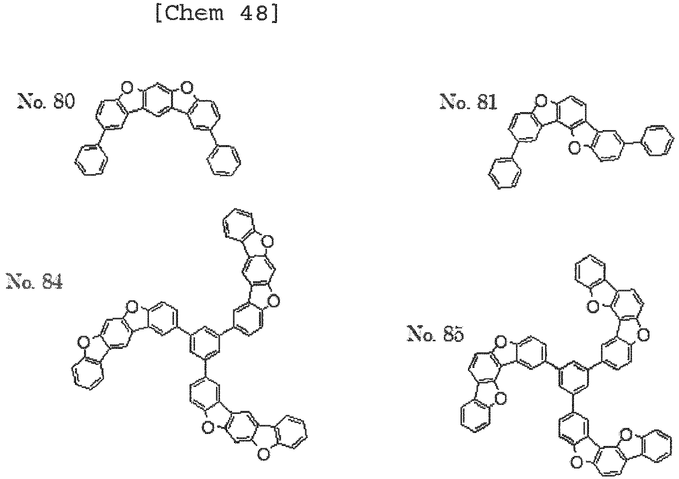

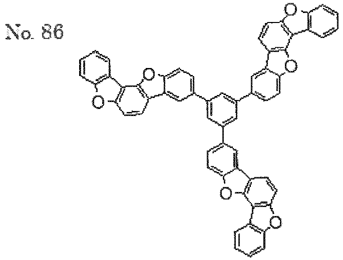

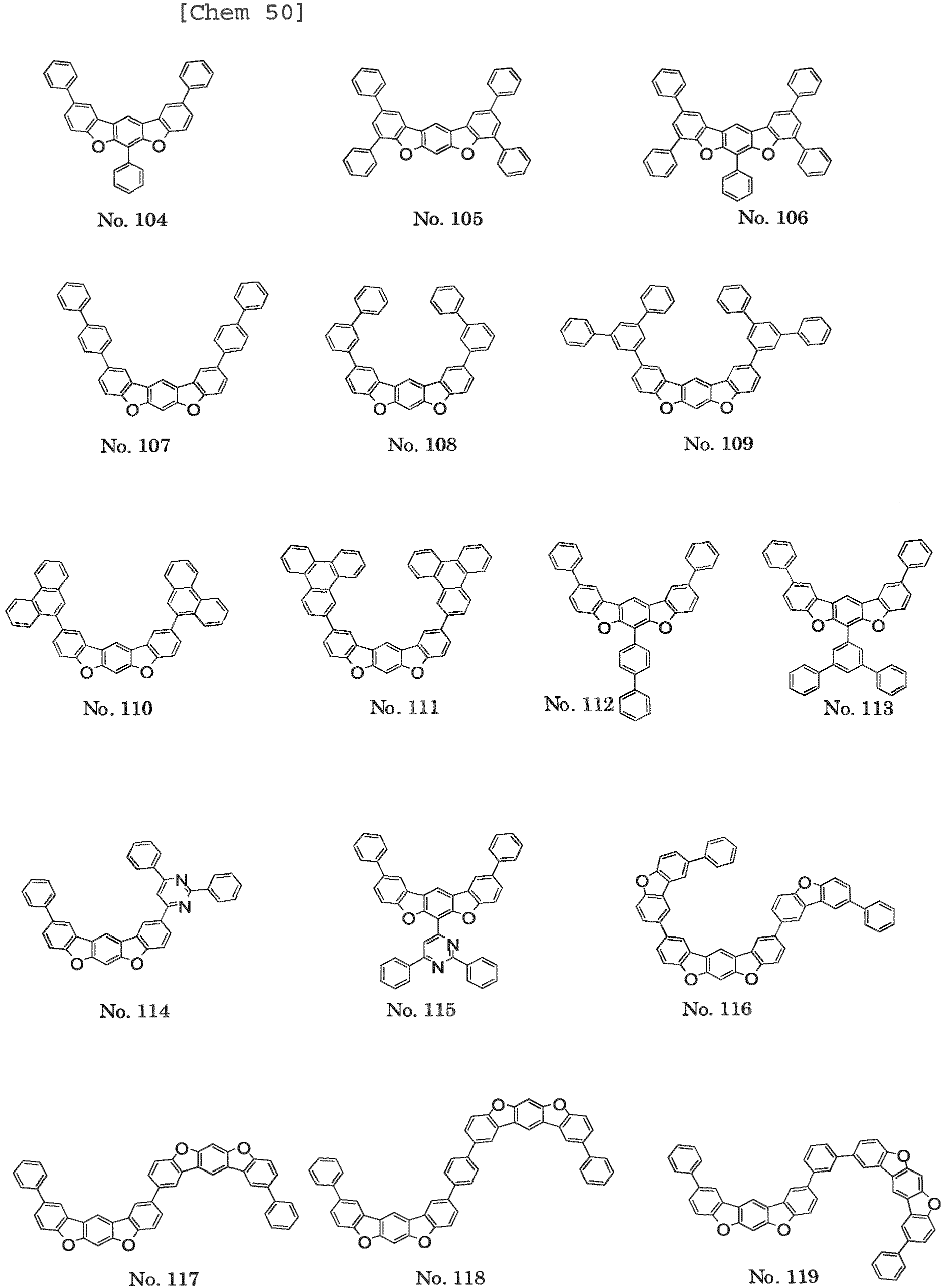

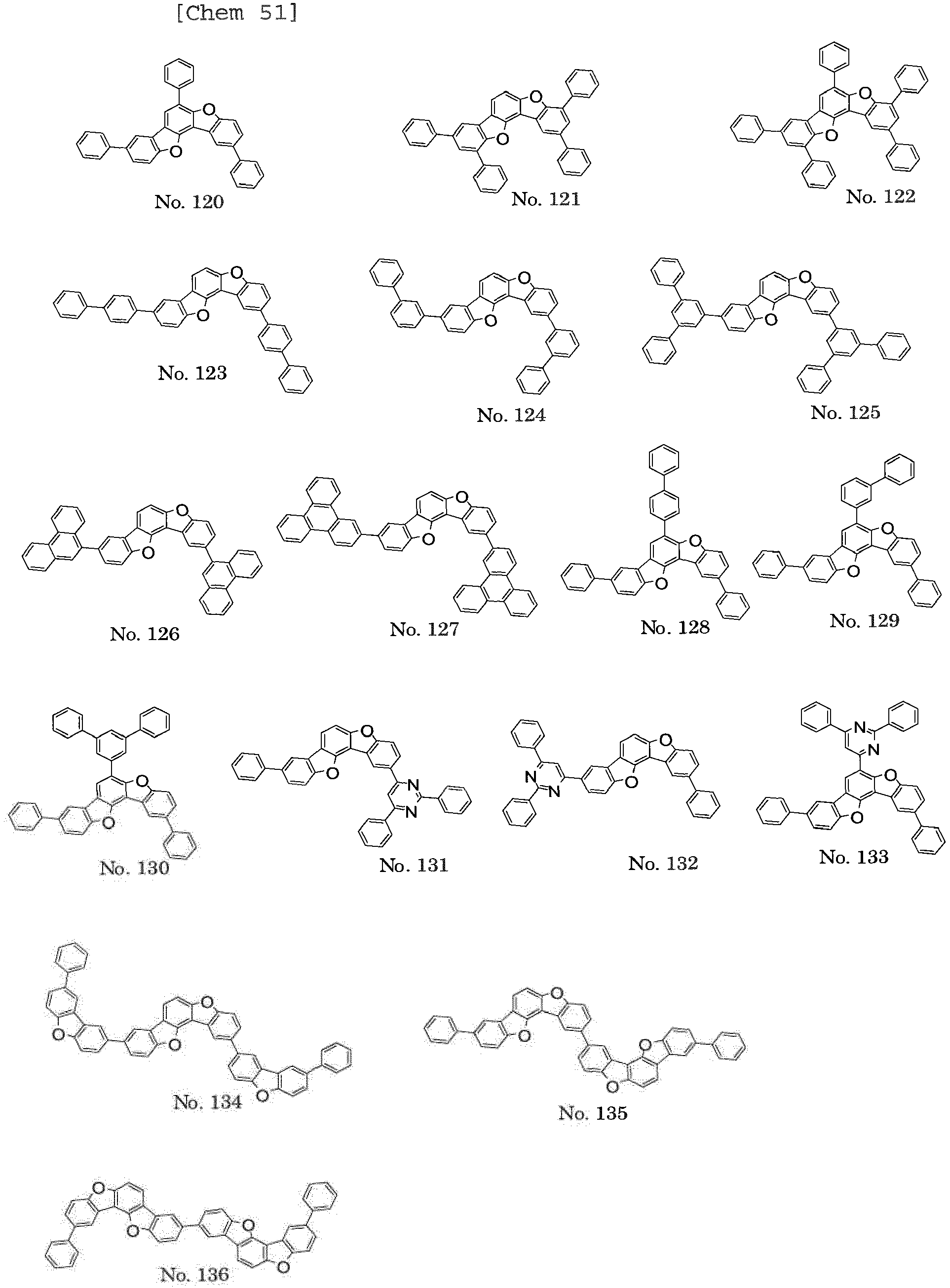

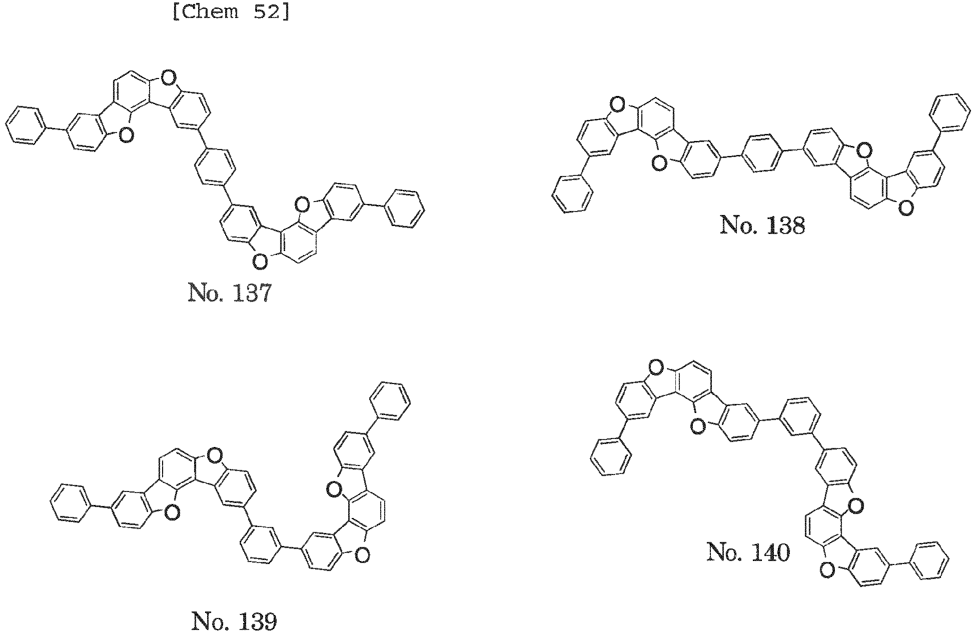

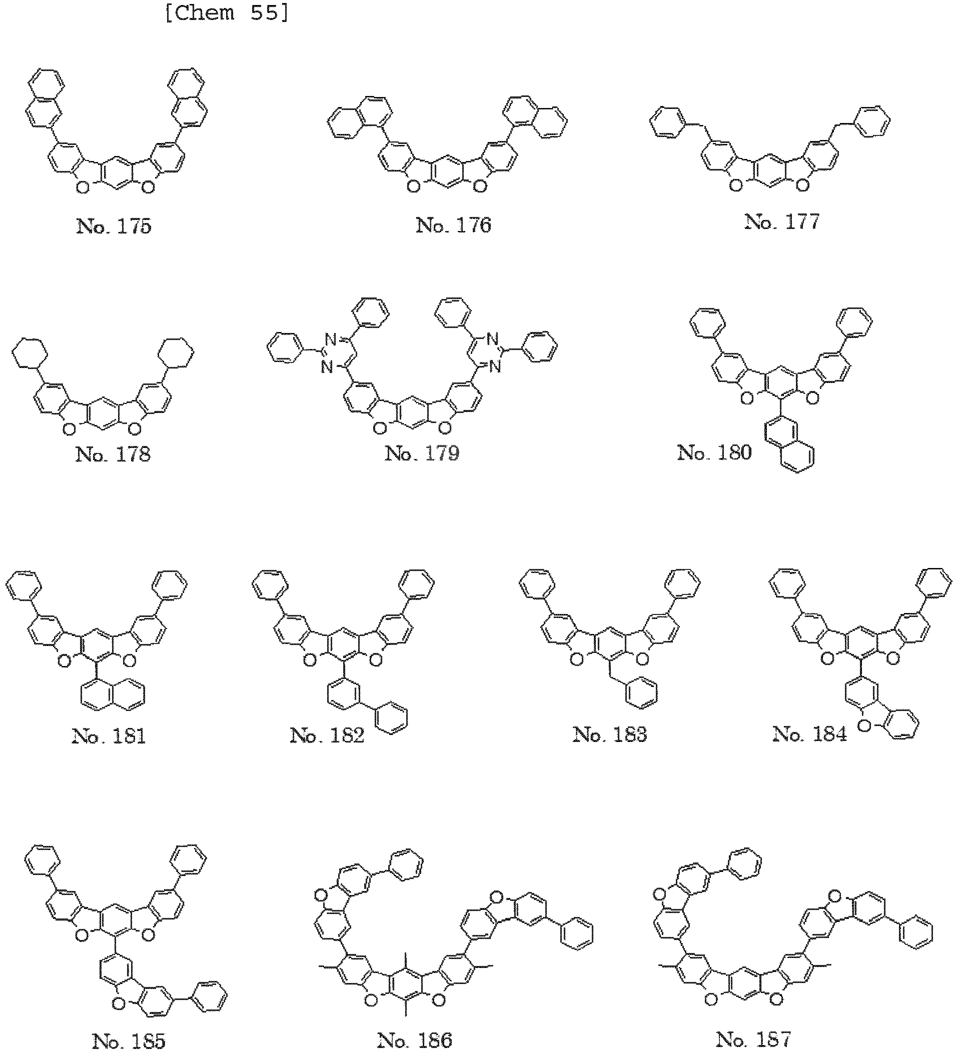

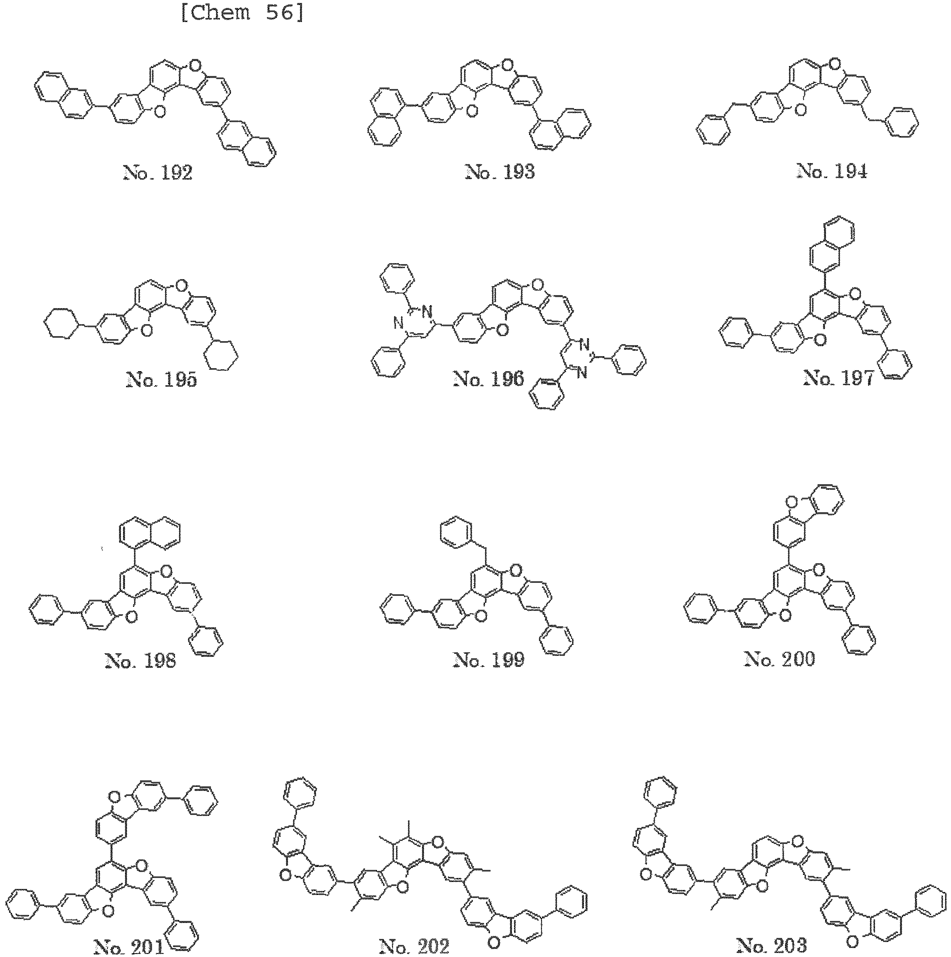

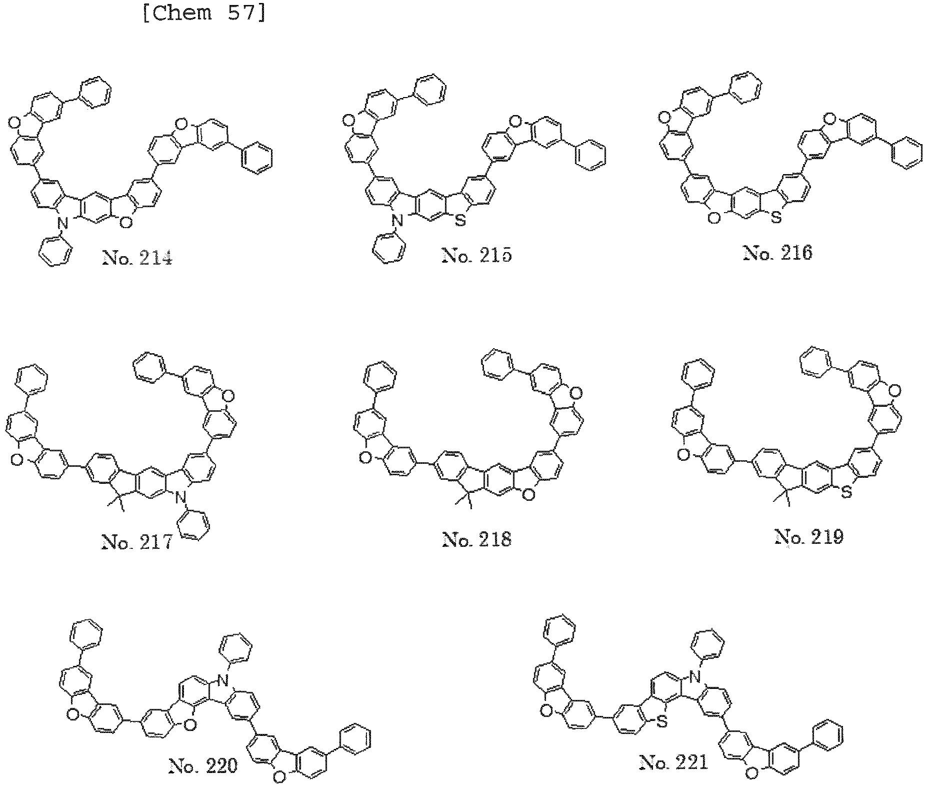

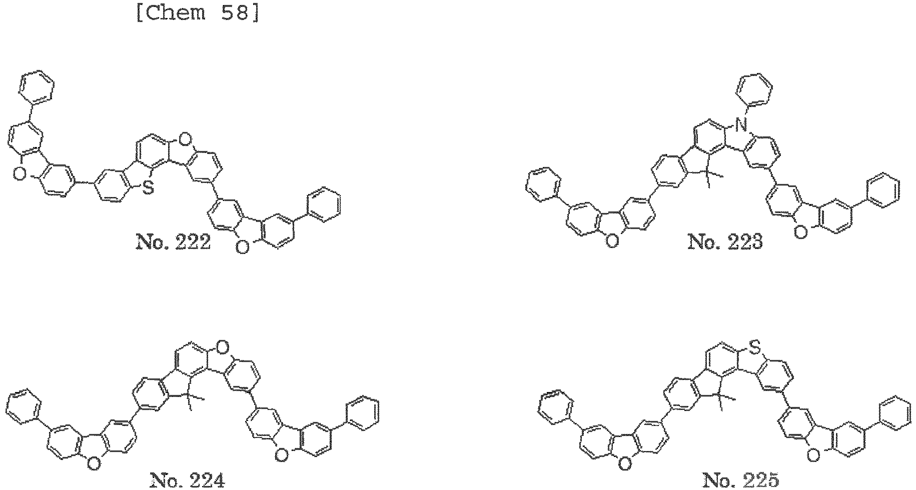

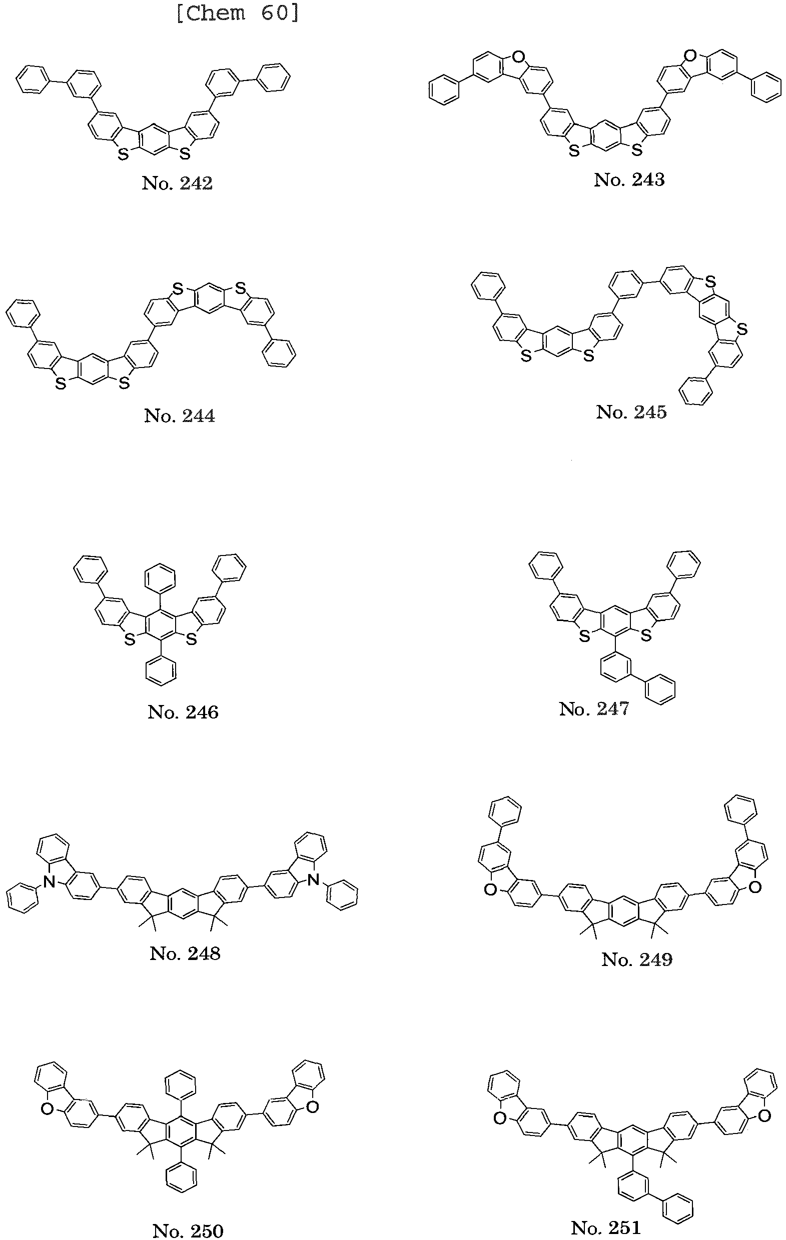

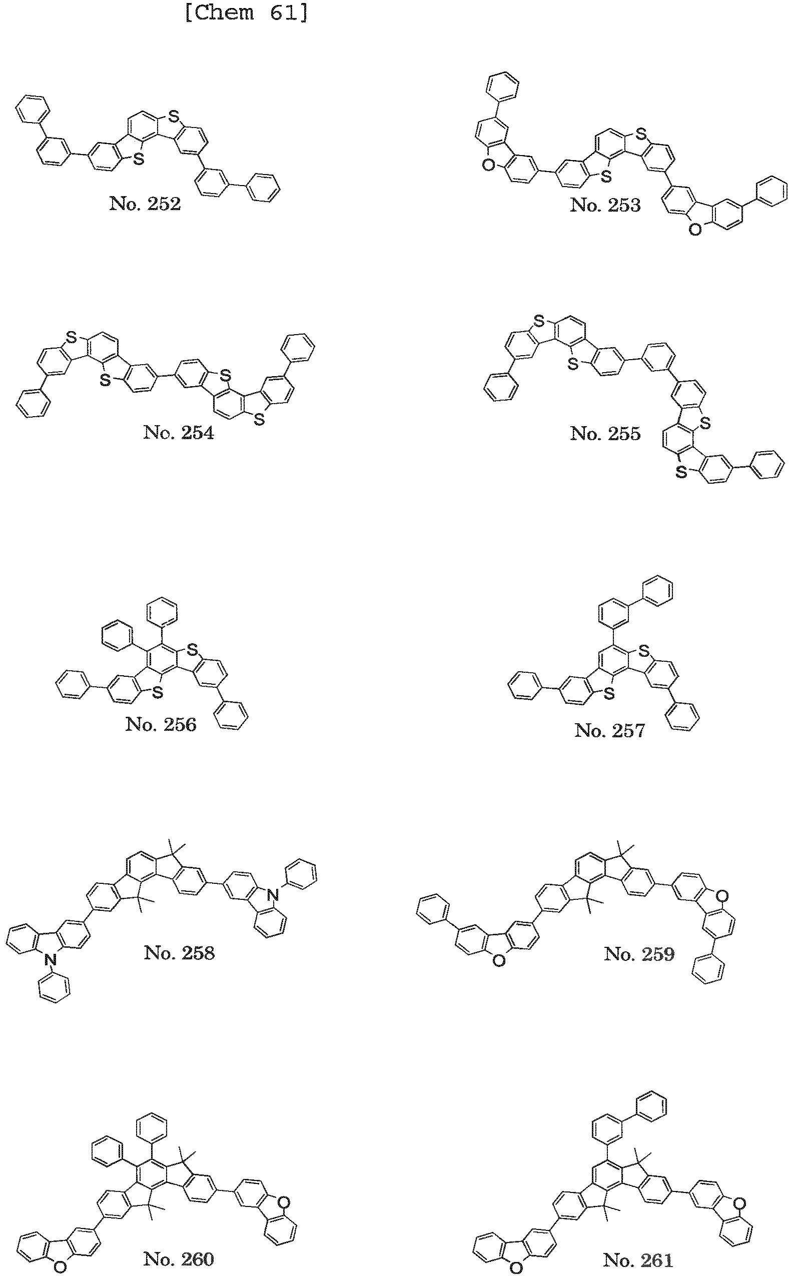

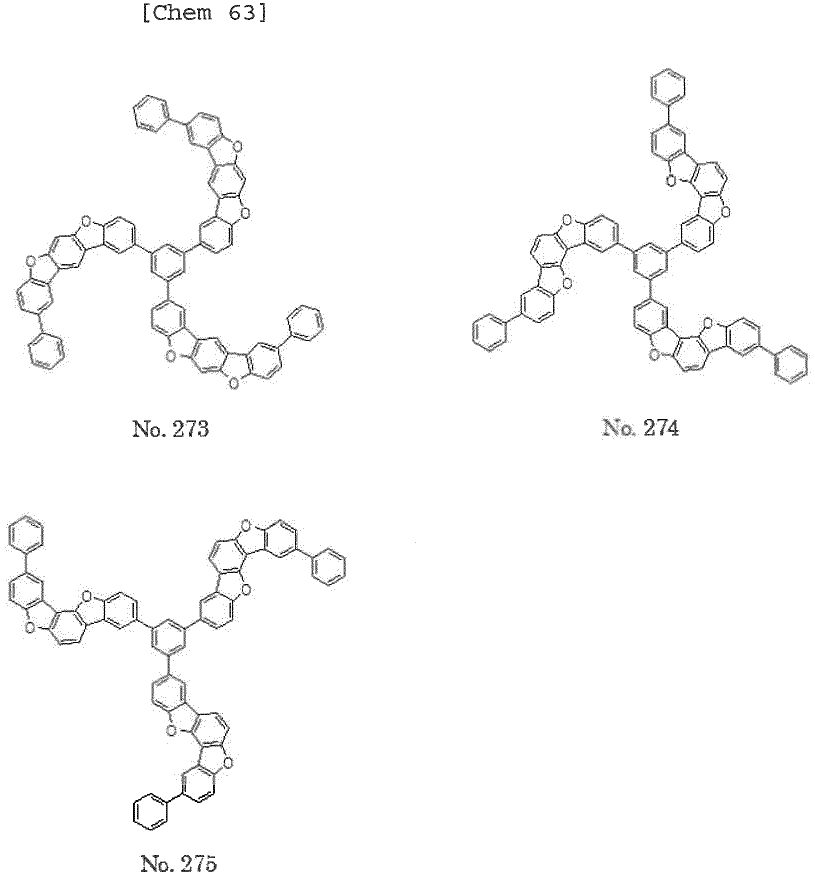

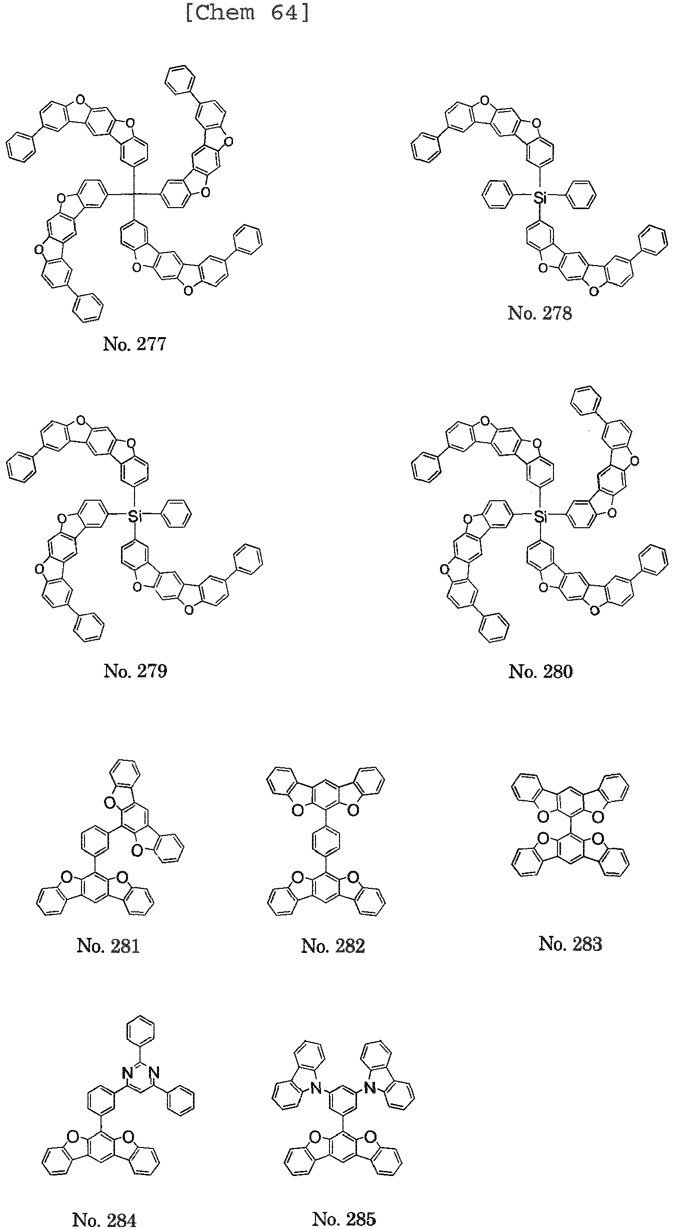

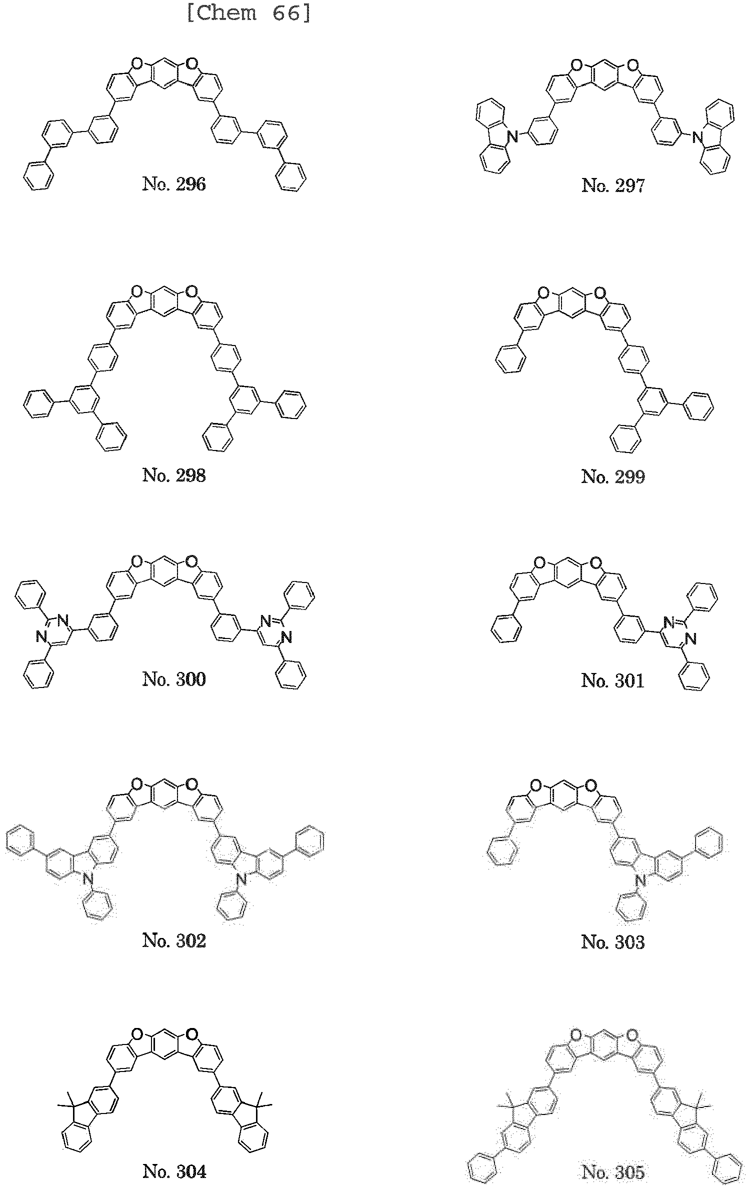

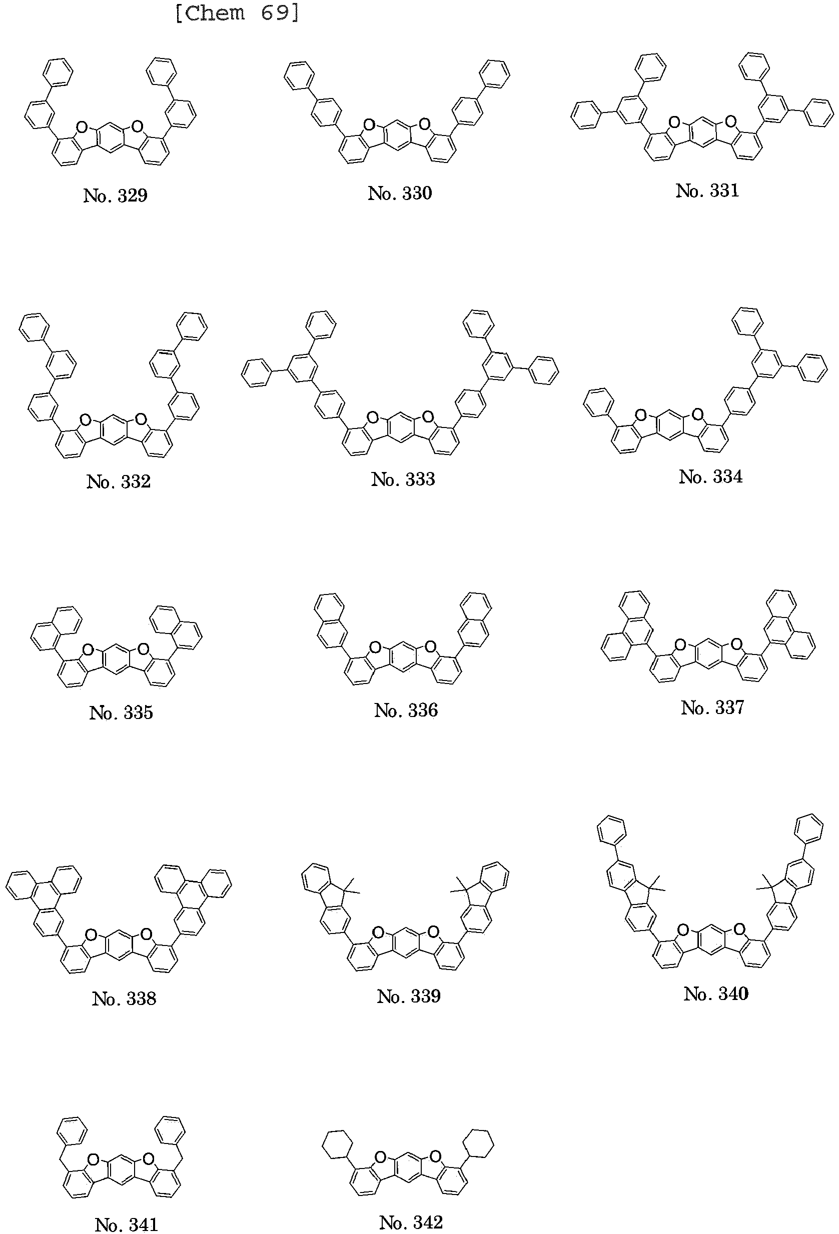

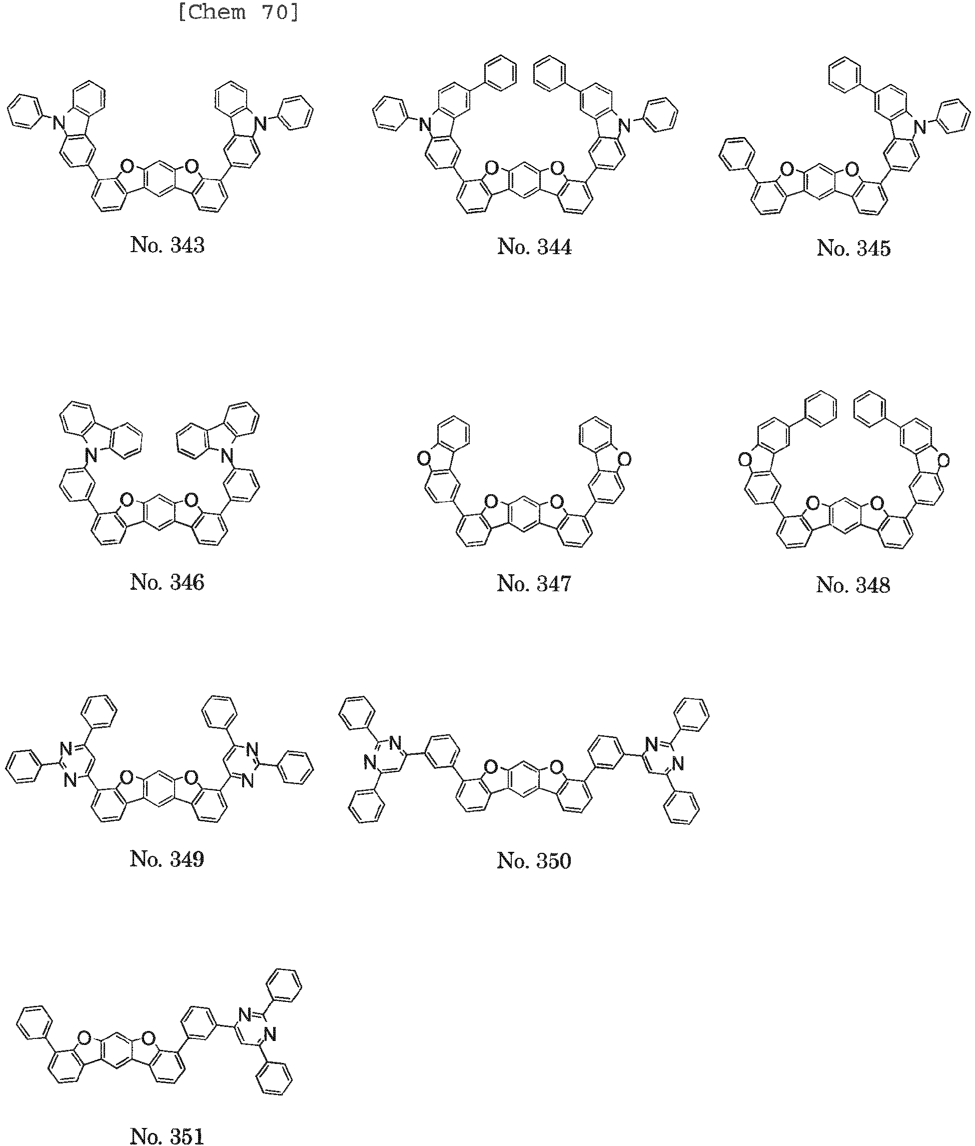

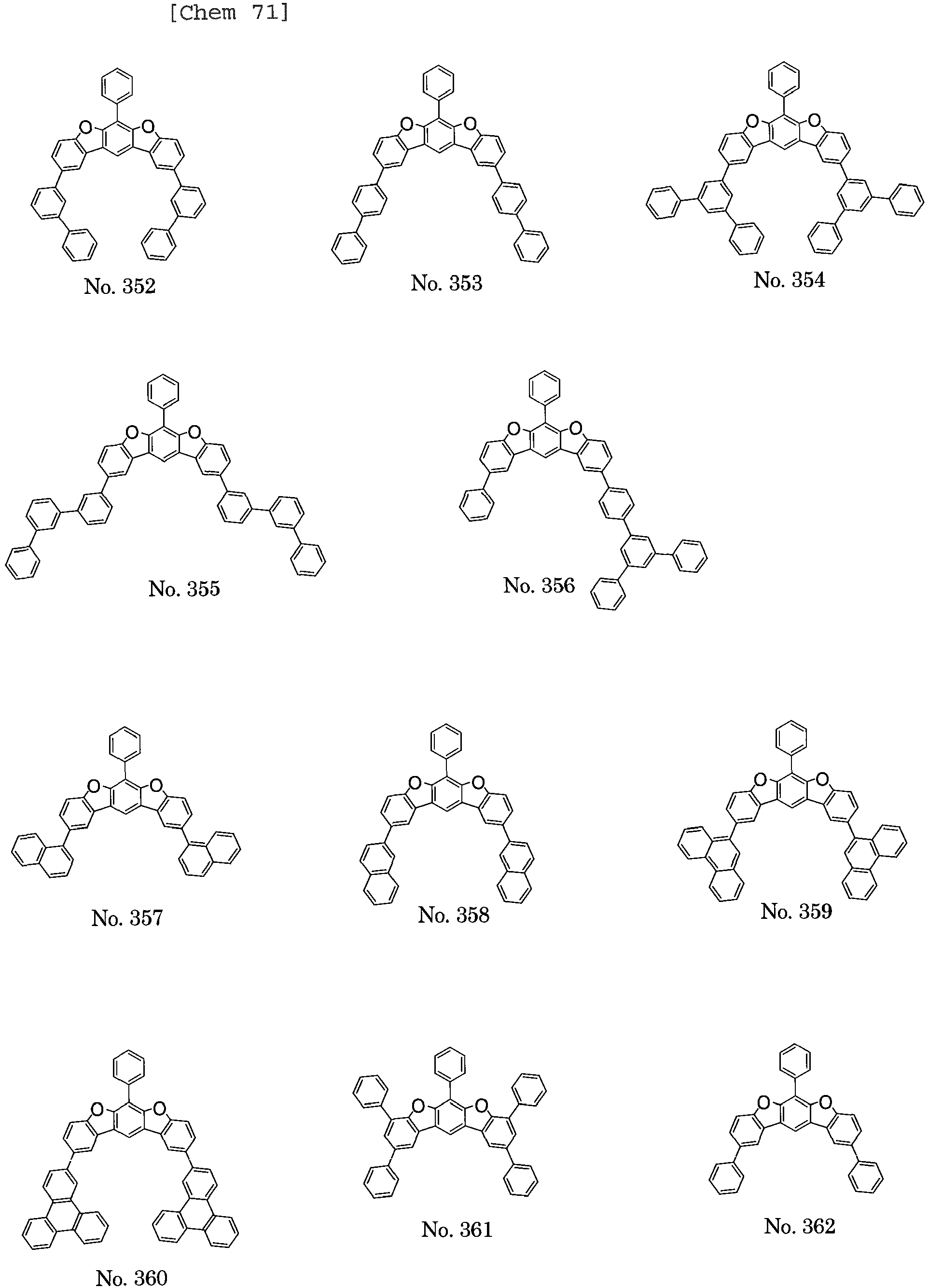

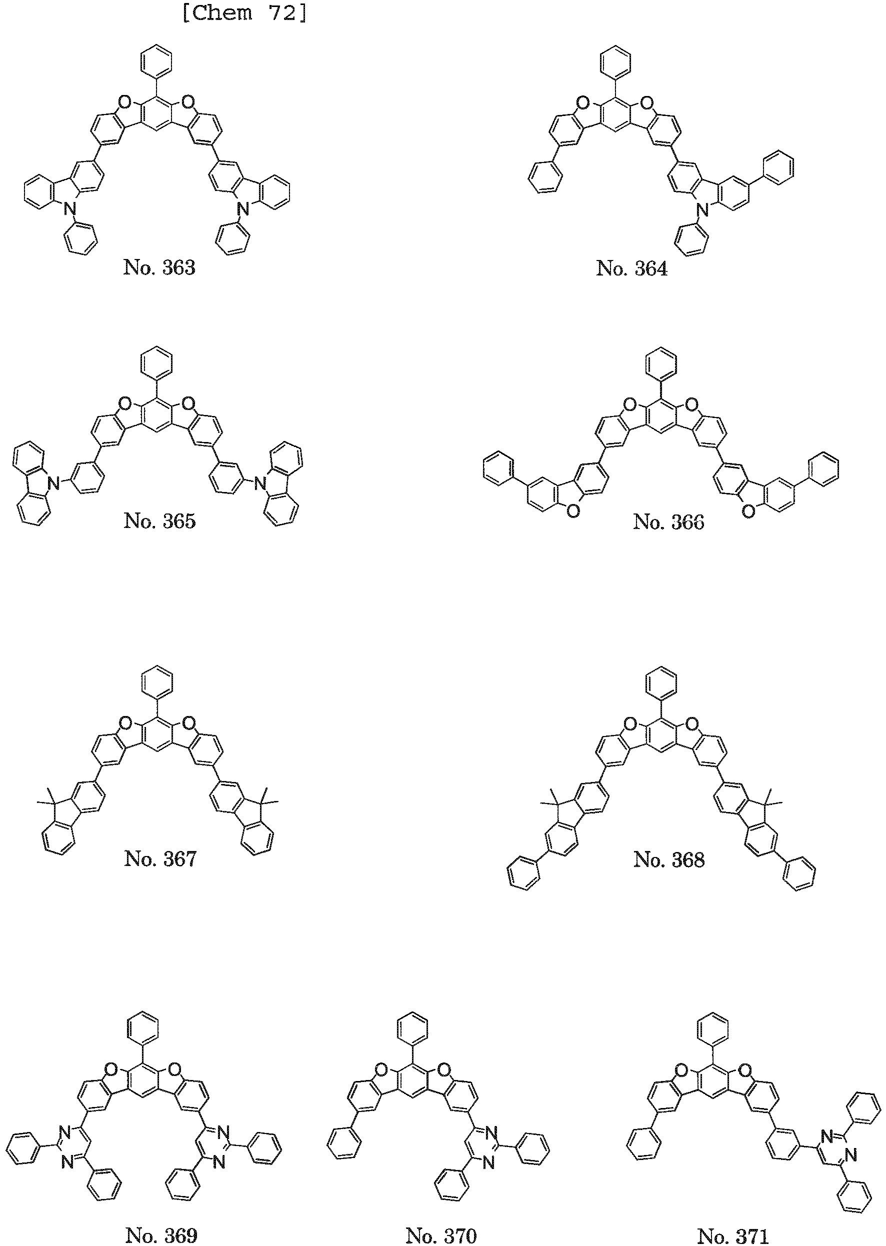

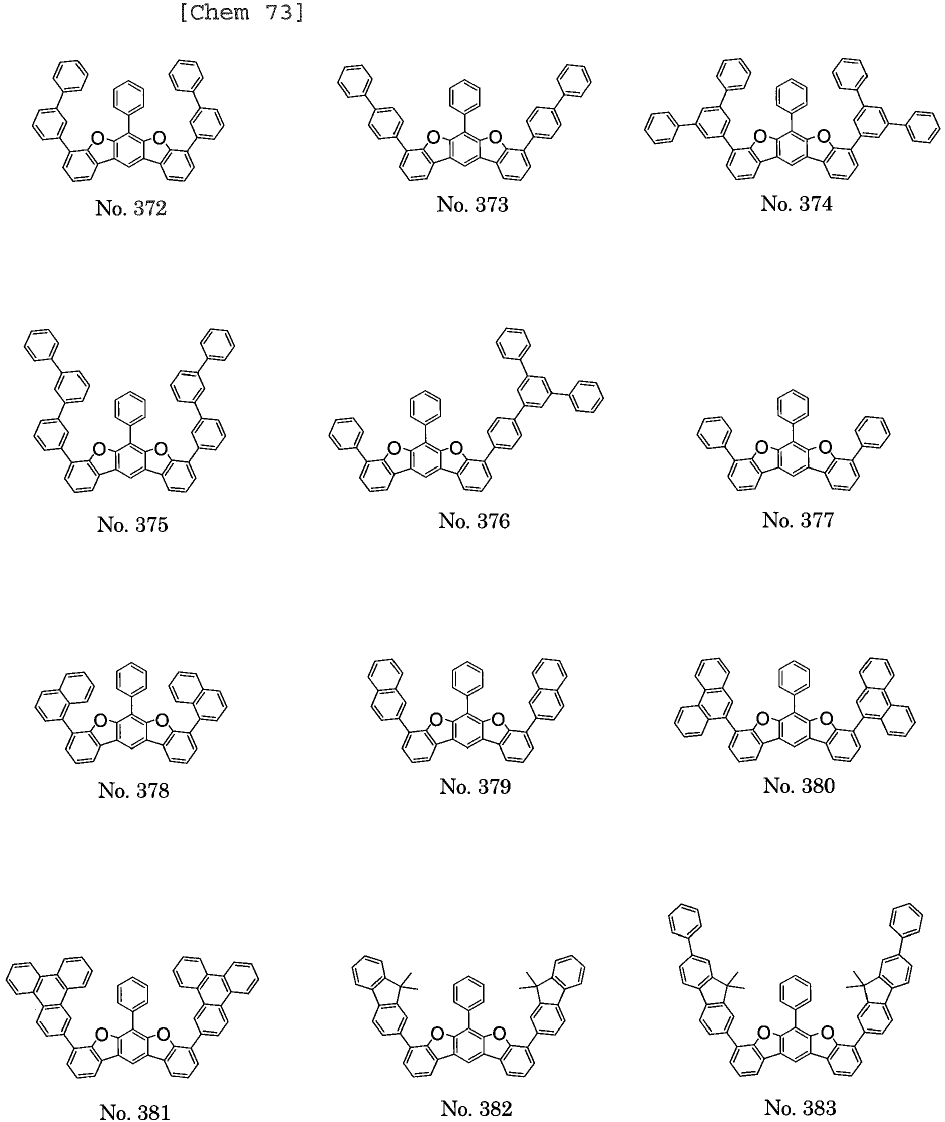

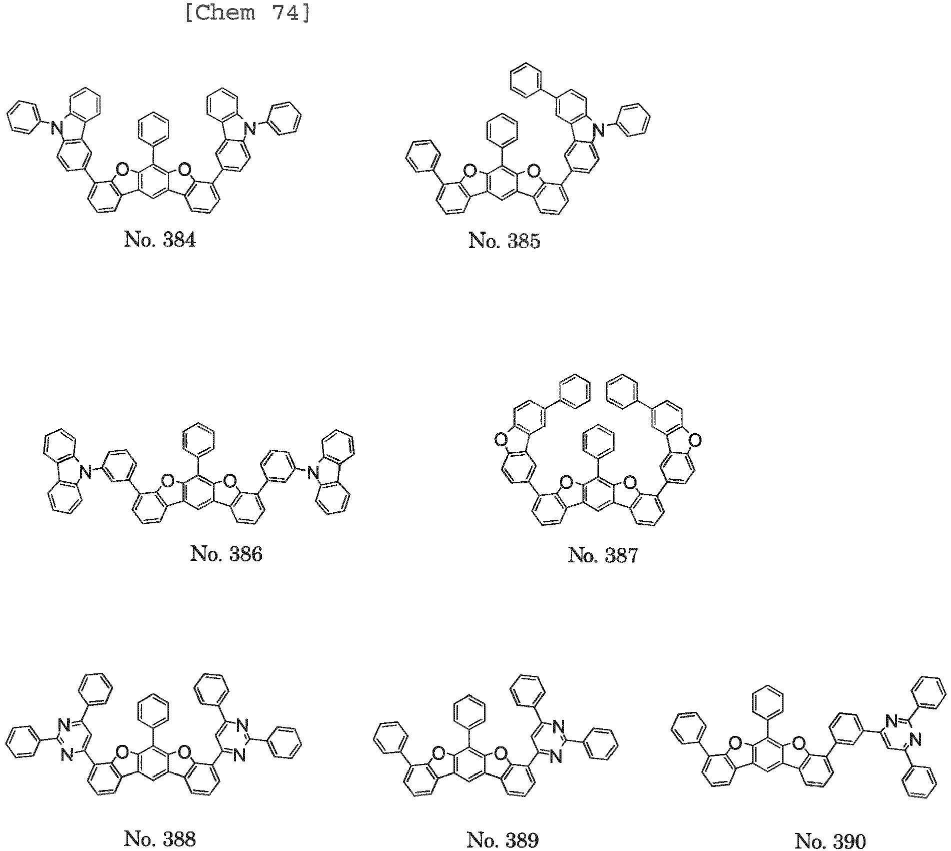

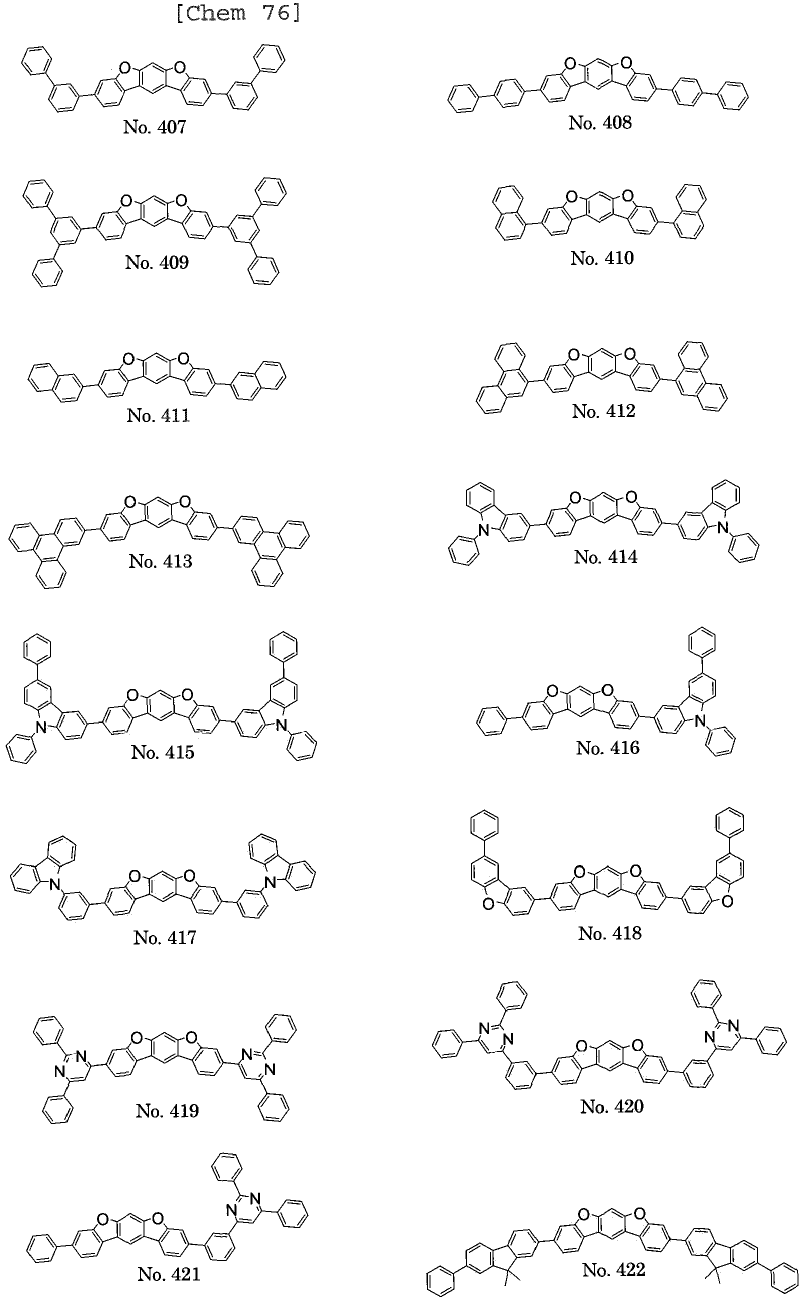

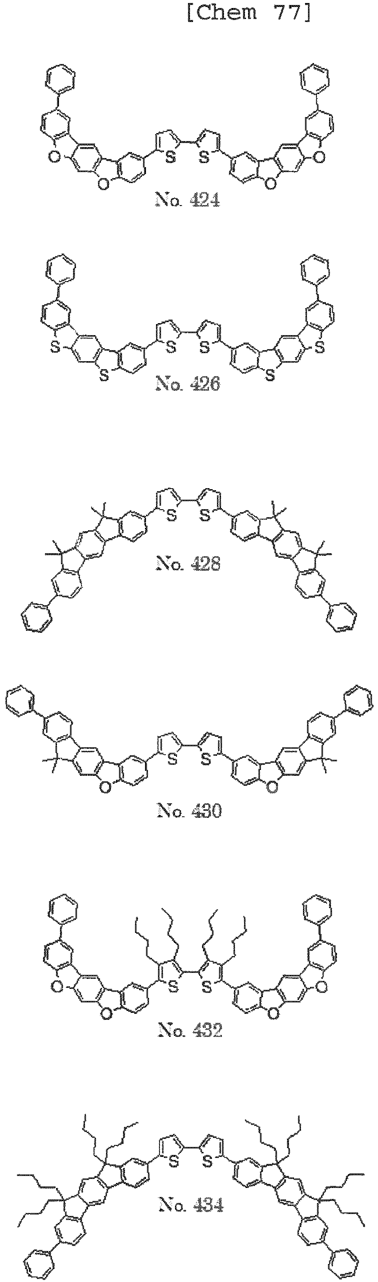

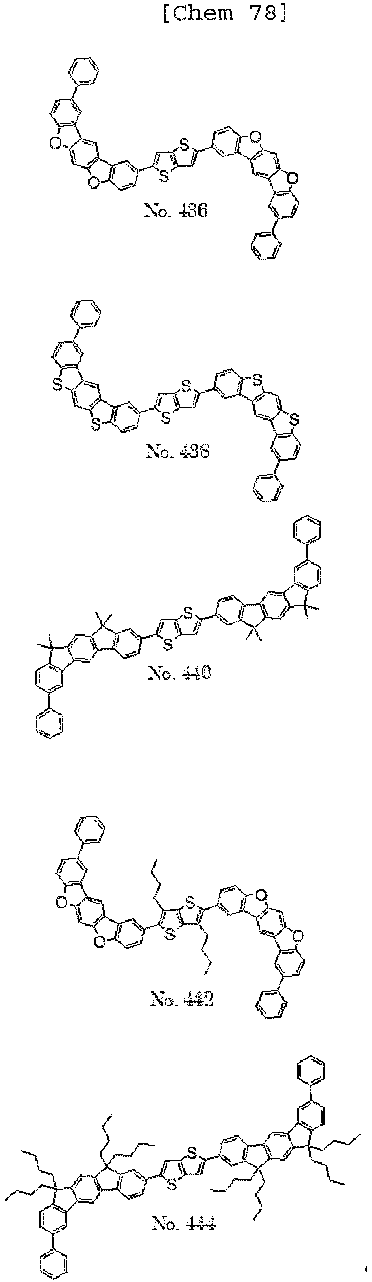

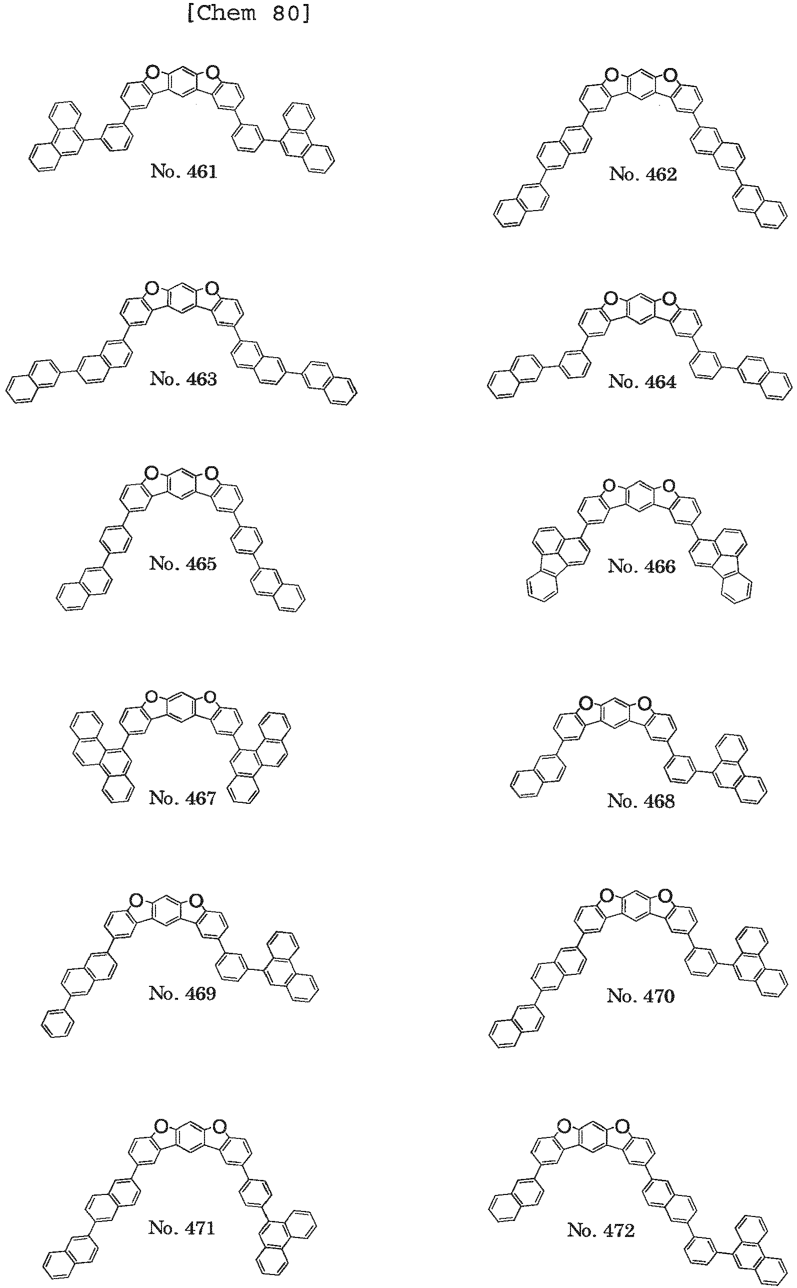

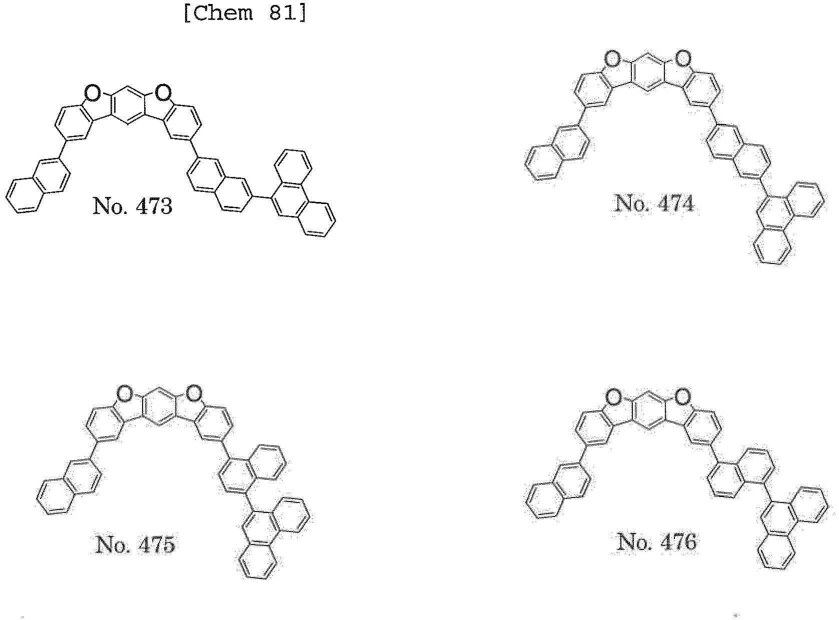

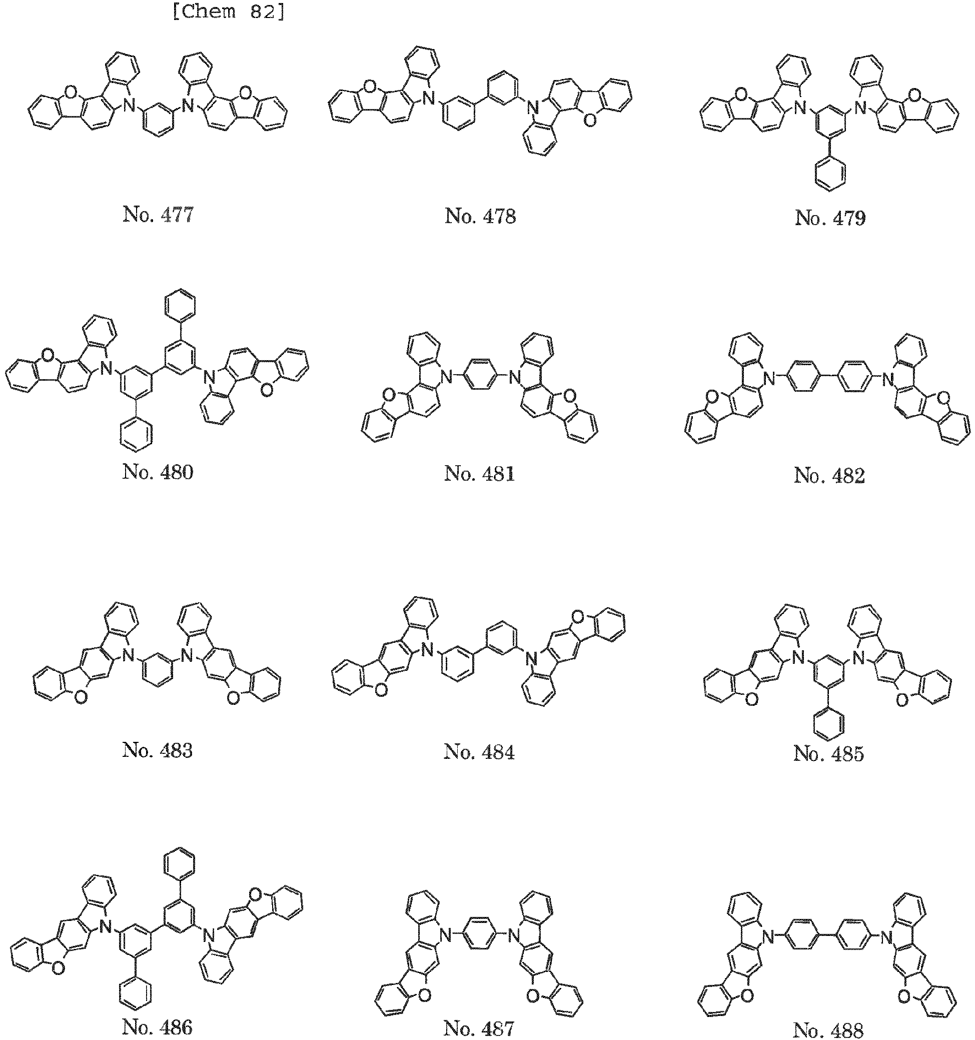

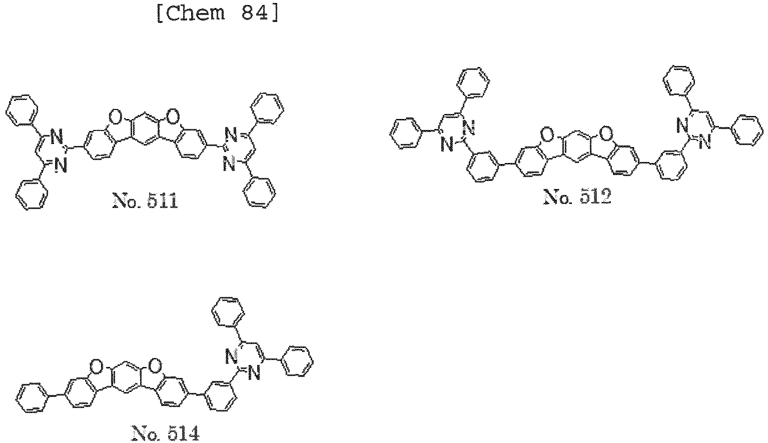

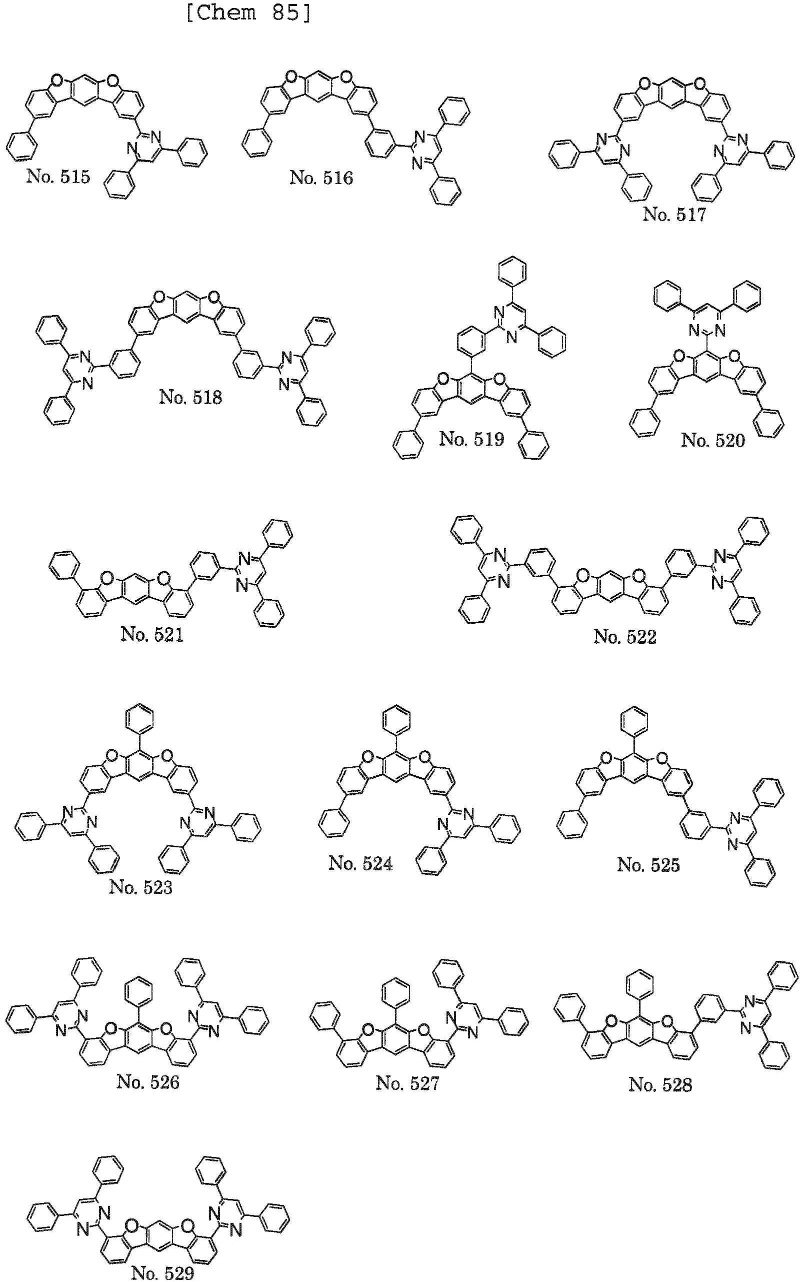

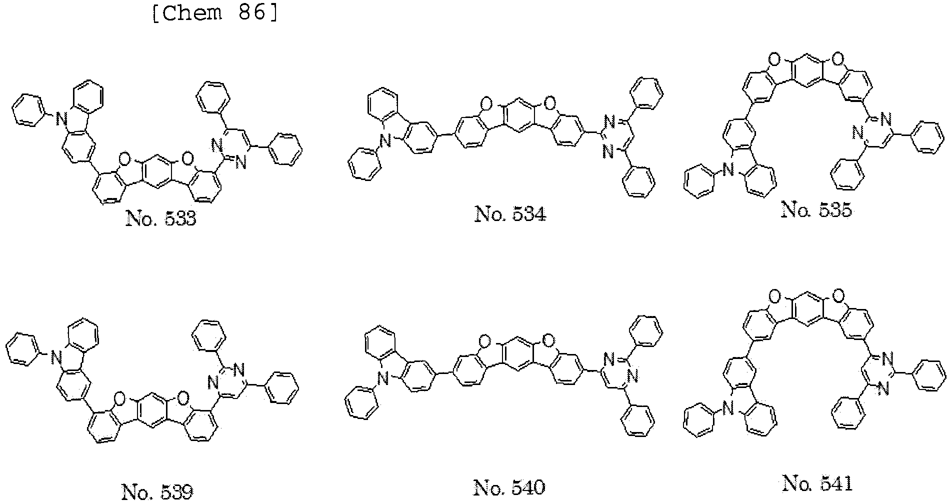

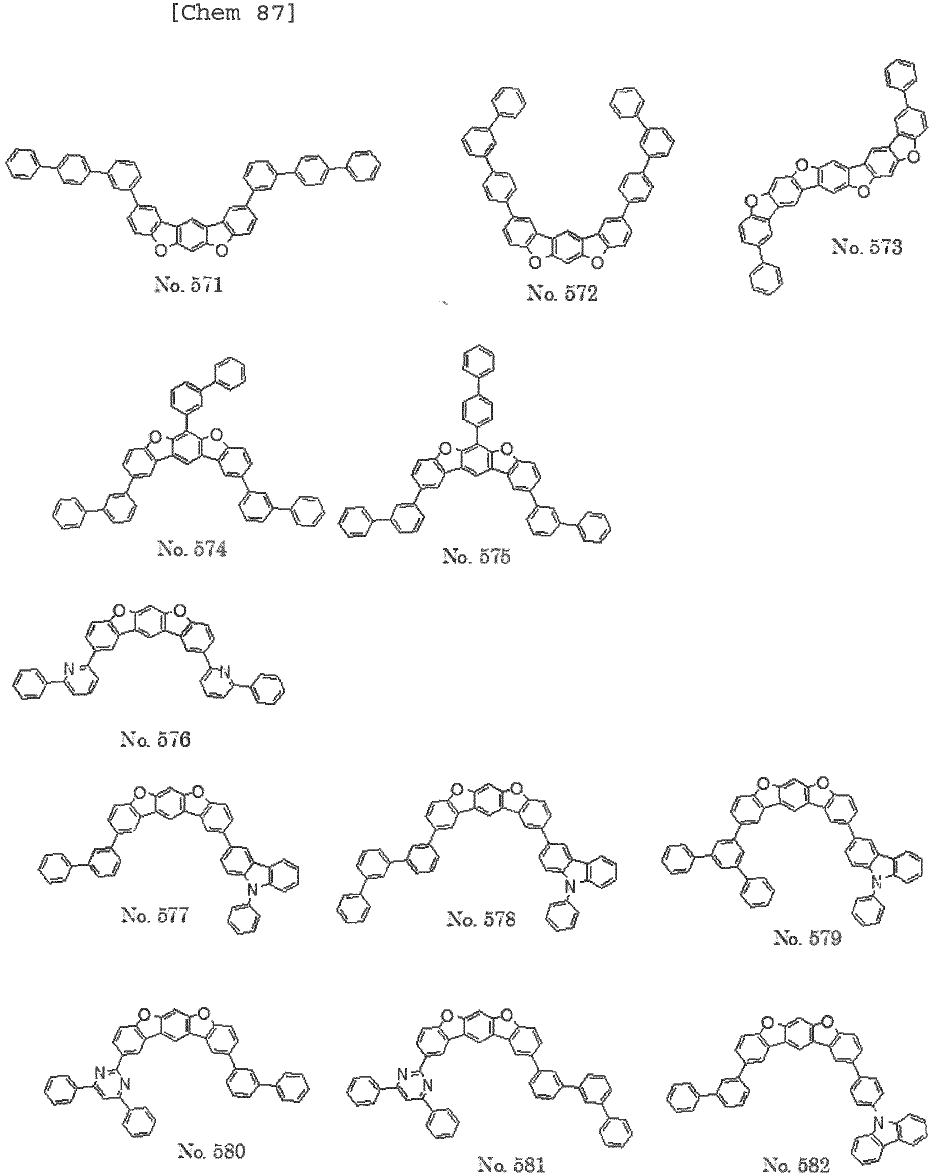

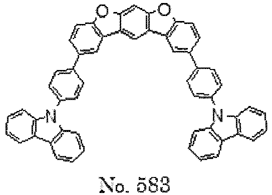

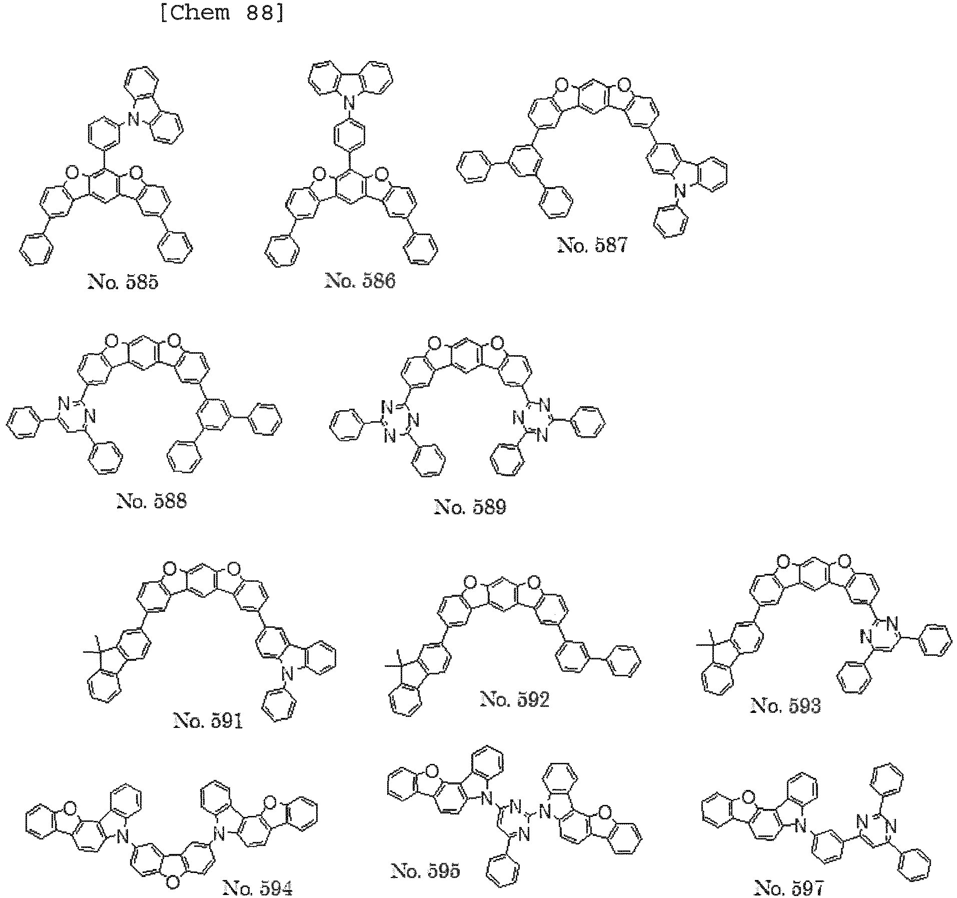

Specific examples of the halogen compound represented by the formula (30) are shown below

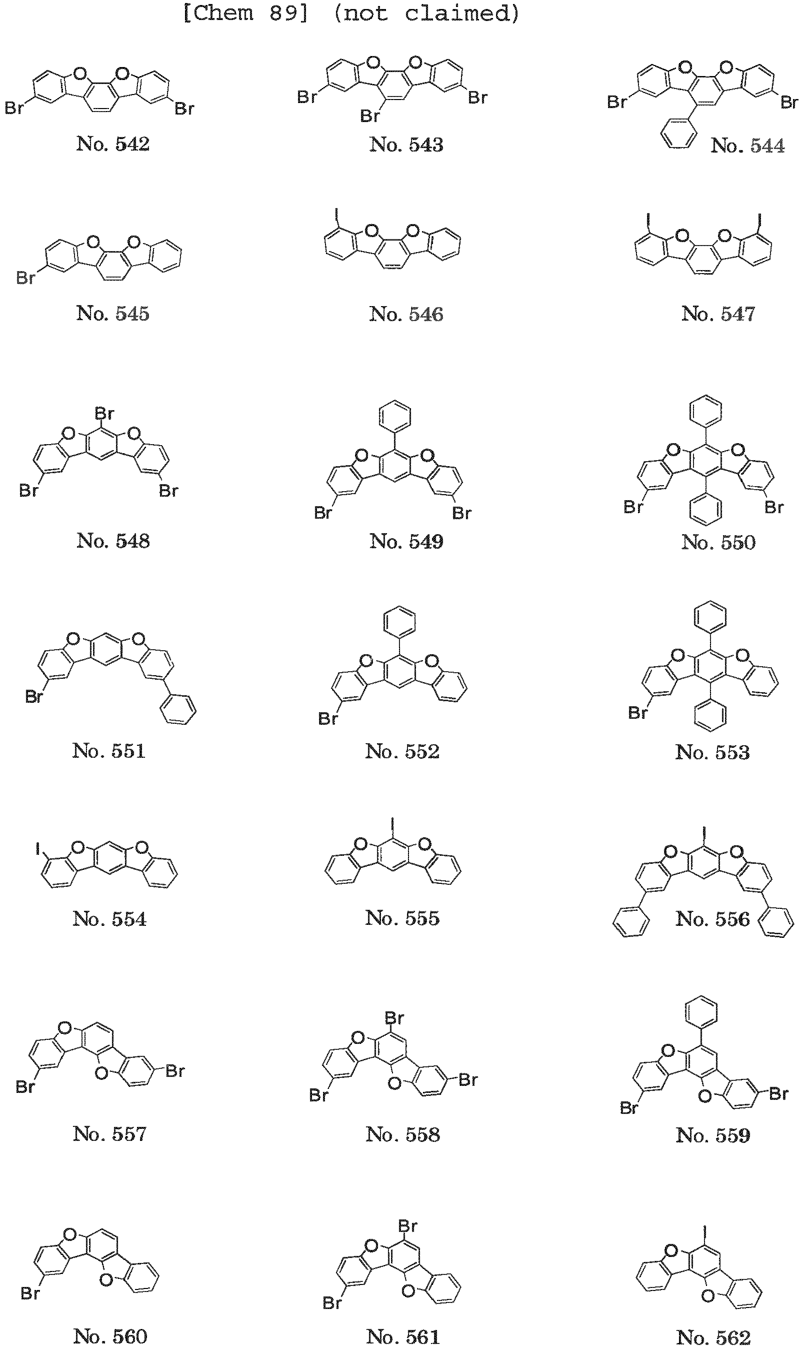

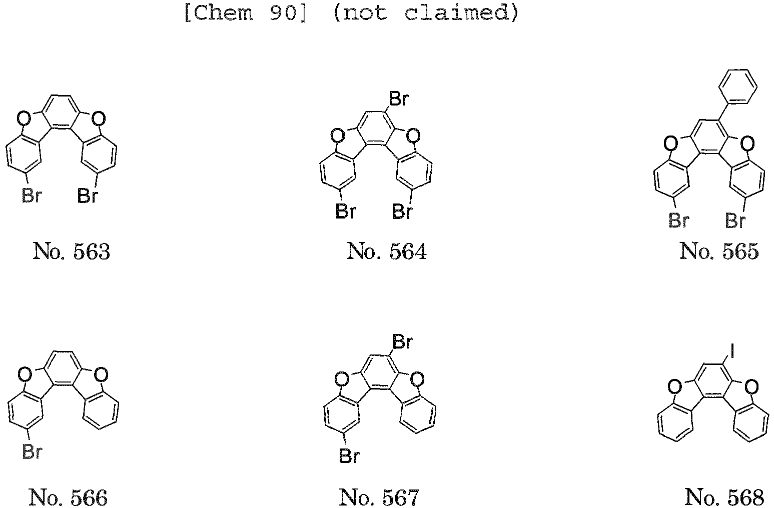

Next, an organic EL device is described.

The organic EL device disclosed herein has one or more organic thin film layers including a light emitting layer between a cathode and an anode, and at least one layer of the organic thin film layers contains a polycyclic compound having a π-conjugated heteroacene skeleton crosslinked with a carbon atom, a nitrogen atom, an oxygen atom, or a sulfur atom. Specific examples of the π-conjugated heteroacene skeleton are shown below.

Indenofluorene (crosslinked with a carbon atom) 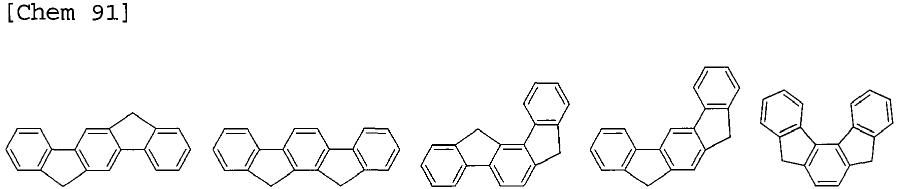 Indolocarbazole (crosslinked with a nitrogen atom) 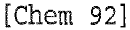 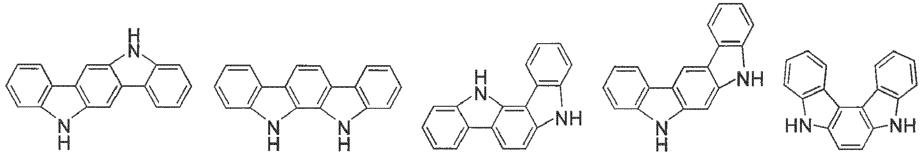 Benzofuranodibenzofuran (crosslinked with an oxygen atom) 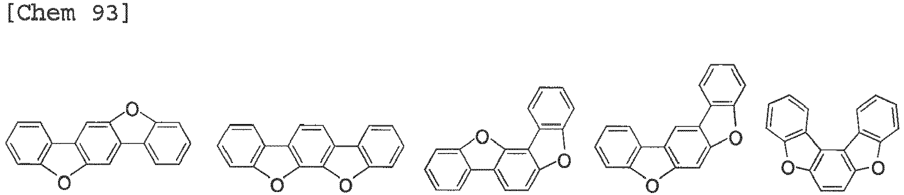 Benzothiophenodibenzothiophene (crosslinked with a sulfur atom)

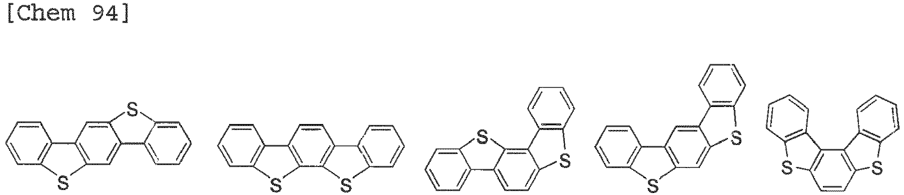

In addition to the foregoing, a π-conjugated heteroacene skeleton crosslinked with a combination of two or more of a carbon atom, a nitrogen atom, an oxygen atom, and a sulfur atom is also permitted. Specific examples of the π-conjugated heteroacene skeleton are shown below. The electron and hole transporting properties can be adjusted by combining two or more of those examples. In particular, a combination of an oxygen atom and a nitrogen atom can achieve compatibility between the electron and hole transporting properties, and reduce the voltage at which the device is driven.

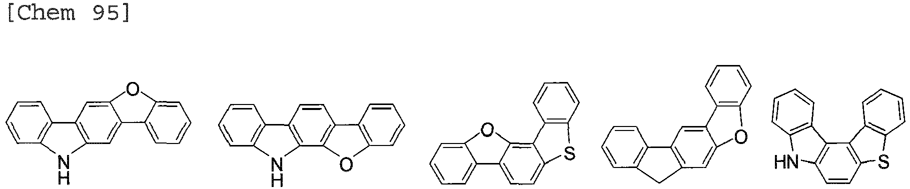

In addition, the above-mentioned polycyclic compound of the present invention is preferably used as the polycyclic compound.

The organic EL device may have an electron transporting layer between the light emitting layer and the cathode, and the electron transporting layer may contain the polycyclic compound. Further, both the light emitting layer and the electron transporting layer each preferably contain the polycyclic compound.

Alternatively, the organic EL device may have a hole transporting layer between the light emitting layer and the anode, and the hole transporting layer may contain the polycyclic compound.

Further, the polycyclic compound of the present invention is preferably incorporated into at least the light emitting layer. When the compound is used in the light emitting layer, the lifetime of the organic EL device can be lengthened. When the compound is used in the electron transporting layer or the electron injecting layer, the voltage at which the device is driven can be reduced. The compound is preferably incorporated into each of two or more layers including the light emitting layer and the electron transporting layer or the electron injecting layer at the same time because both the reduced voltage and the lengthened lifetime can be achieved.

In particular, in addition to the electron transporting layer or the electron injecting layer, the light emitting layer preferably contains the polycyclic compound of the present invention as a host material. The light emitting layer preferably contains the polycyclic compound represented by the formula (9) or (12) as a host material, more preferably contains the polycyclic compound represented by the formula (17) or (18) as a host material.

A multi-layer type organic EL device is obtained by laminating a plurality of layers; for example, the device is formed of an anode, a hole transporting layer (a hole injecting layer), a light emitting layer, and a cathode, of an anode, a light emitting layer, an electron transporting layer (an electron injecting layer), and a cathode, of an anode, a hole transporting layer (a hole injecting layer), a light emitting layer, an electron transporting layer (an electron injecting layer), and a cathode, or of an anode, a hole transporting layer (a hole injecting layer), a light emitting layer, a hole barrier layer, an electron transporting layer (an electron injecting layer), and a cathode.

In the organic EL device, the light emitting layer preferably contains the polycyclic compound as a host material. In addition, it is preferred that the light emitting layer be formed of a host material and a phosphorescent material, and the host material be the polycyclic compound.

In addition, the polycyclic compound may be a host material to be used together with a phosphorescent material, or may be an electron transporting material to be used together with a phosphorescent material. The material has a triplet energy gap of preferably 2.2 to 3.2 eV, or more preferably 2.5 to 3.2 eV.

The phosphorescent material is preferably a compound containing iridium (Ir), osmium (Os), ruthenium (Ru), or platinum (Pt) because the compound has a high phosphorescent quantum yield, and can additionally improve the external quantum efficiency of the light emitting device. The material is more preferably a metal complex such as an iridium complex, an osmium complex, a ruthenium complex, or a platinum complex. Of those, the iridium complex and the platinum complex are still more preferred, and an orthometalated iridium complex is most preferred. Specific examples of the metal complex such as an iridium complex, an osmium complex, a ruthenium complex, or a platinum complex are shown below.

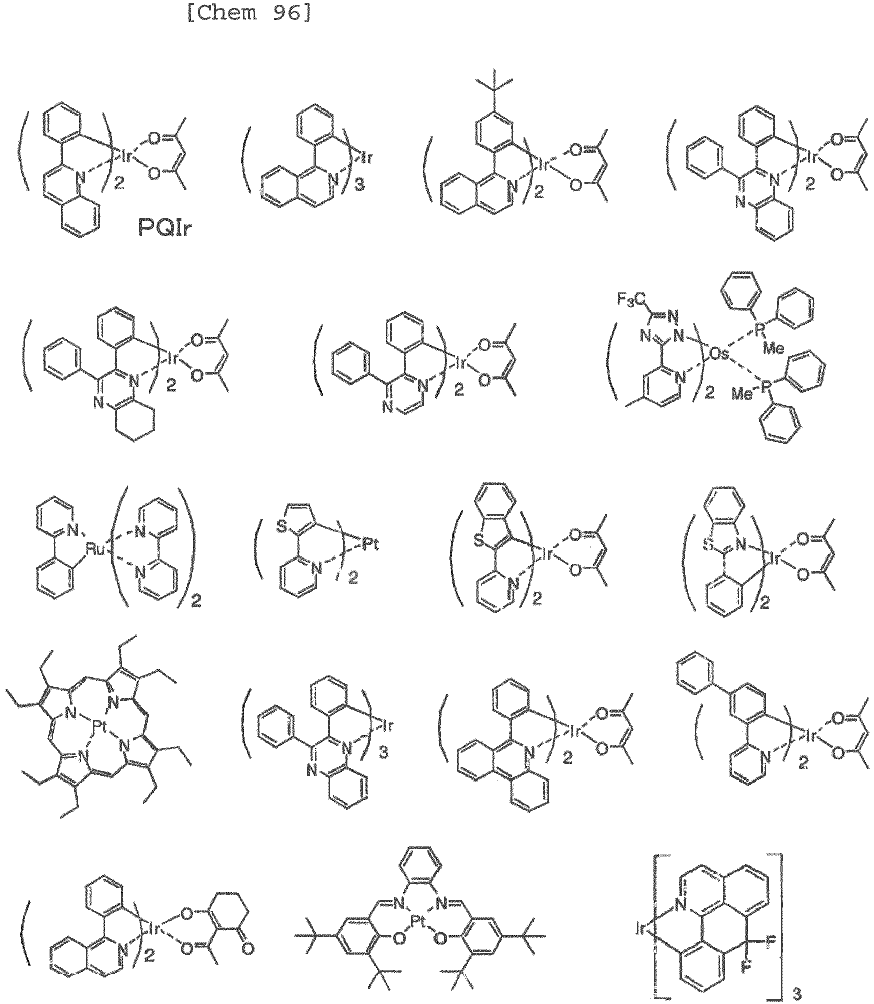

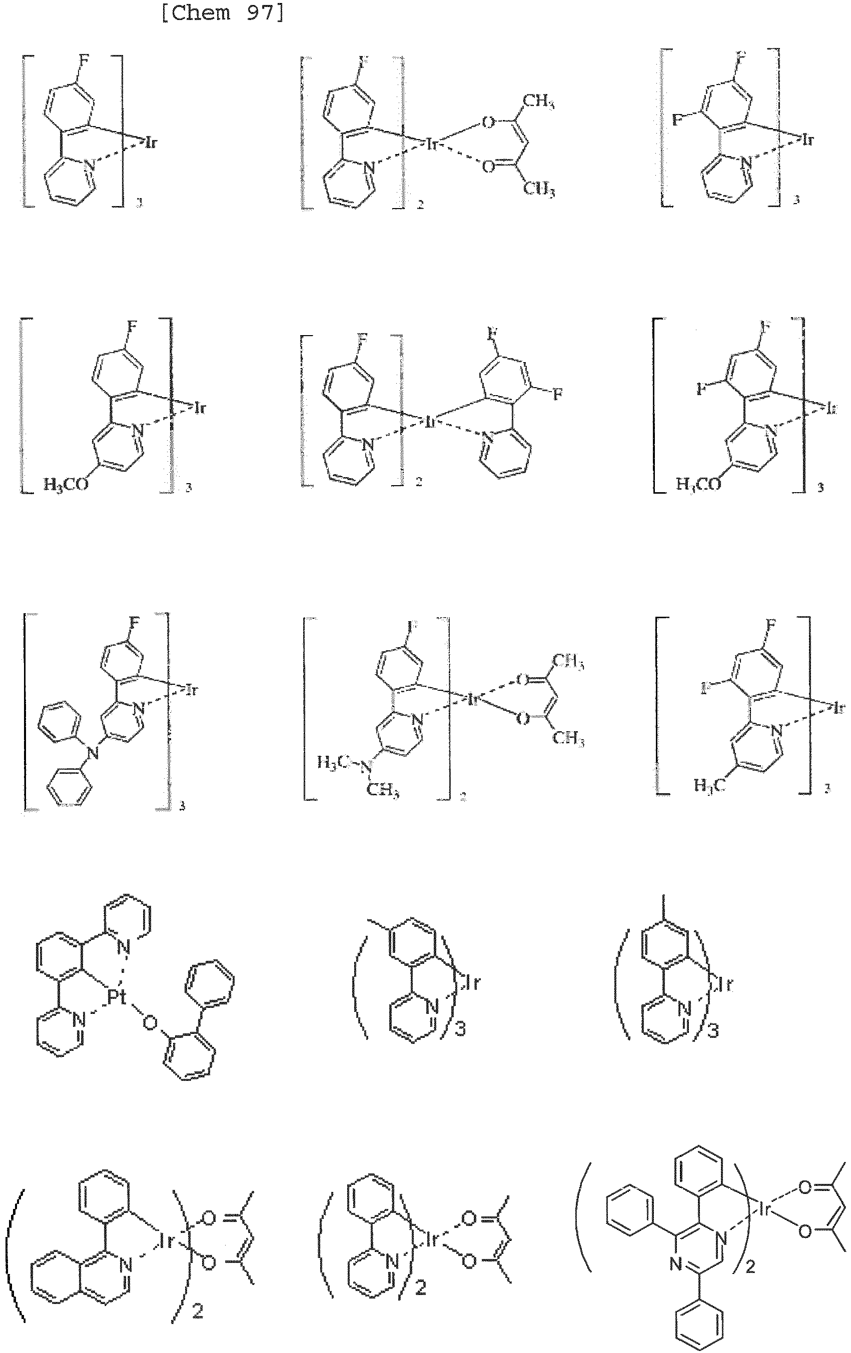

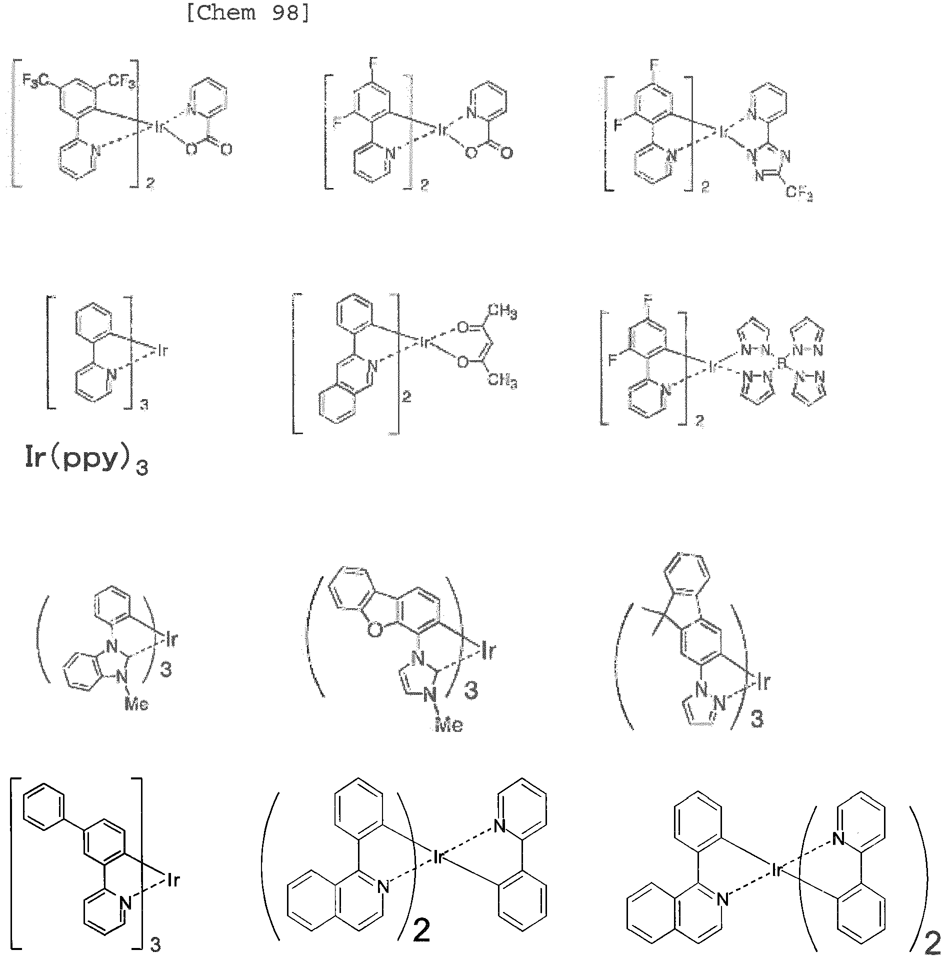

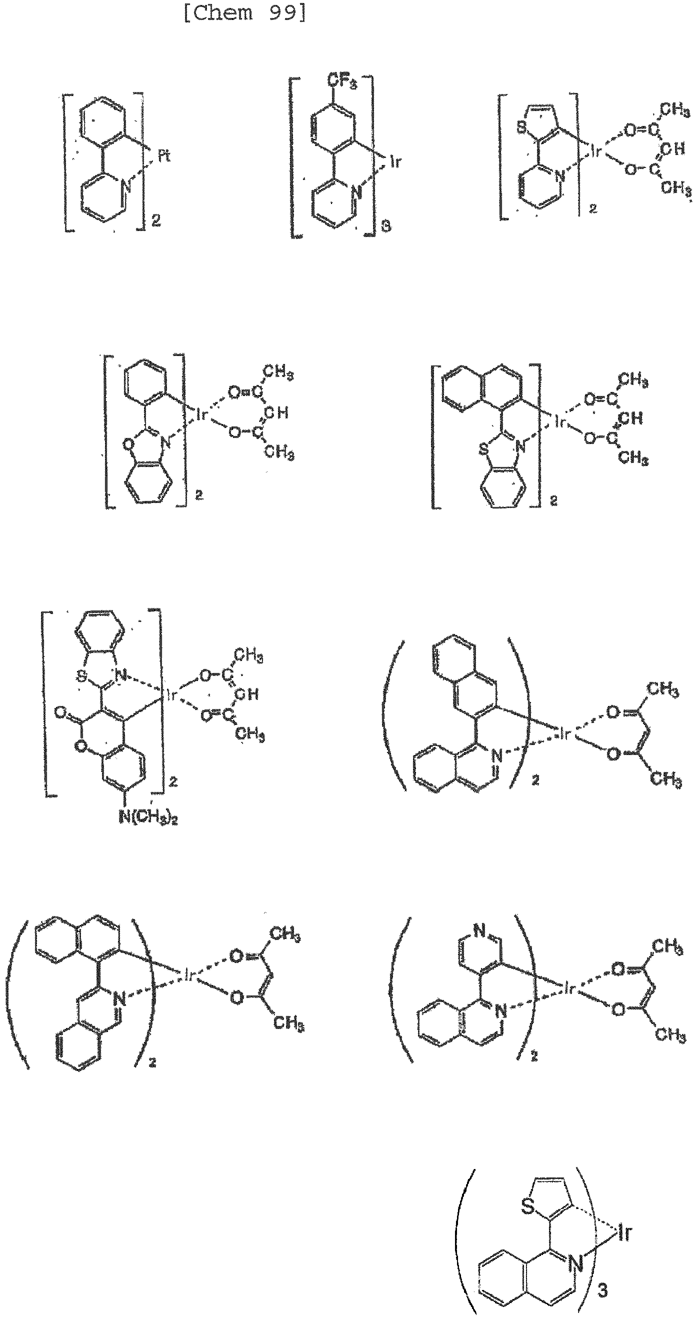

In addition, the organic EL device is preferably such that the light emitting layer contains a host material and a phosphorescent material, and contains a metal complex having a local maximum luminous wavelength of 500 nm or less. Further, the material can be used together with a fluorescent dopant. The material can be used together with a blue, green, or red fluorescent dopant. In particular, the material can be more preferably used together with the blue or green fluorescent dopant. Further, the material can be preferably used also as an electron transporting material for a fluorescent organic EL device.

The organic EL device preferably has a reductive dopant in an interfacial region between the cathode and an organic thin layer (for example, an electron injecting layer or a light emitting layer) . Examples of the reductive dopant include at least one kind selected from an alkali metal, an alkali metal complex, an alkali metal compound, an alkaline earth metal, an alkaline earth metal complex, an alkaline earth metal compound, a rare earth metal, a rare earth metal complex, and a rare earth metal compound.

Preferred examples of the alkali metal include an alkali metal having a work function of 2.9 eV or less, such as Na having a work function of 2.36 eV, K having a work function of 2.28 eV, Rb having a work function of 2.16 eV, and Cs having a work function of 1.95 eV. Of those, K, Rb, and Cs are more preferred, Rb or Cs is still more preferred, and Cs is most preferred.

Preferred examples of the alkali earth metal include an alkali earth metal having a work function of 2.9 eV or less, such as Ca having a work function of 2.9 eV, Sr having a work function of 2.0 to 2.5 eV, and Ba having a work function of 2.52 eV.

Preferred examples of the rare earth metal include a rare earth metal having a work function of 2.9 eV or less, such as Sc, Y, Ce, Tb, and Yb.

Of those metals, a preferred metal has a particularly high reductive ability, so improvement of light emission intensity and long life of organic EL device can be attained by adding a relatively small amount of the metal to an electron injecting region.

Examples of the alkali metal compound include an alkali oxide such as Li₂O, Cs₂O, or K₂O, and an alkali halide such as LiF, NaF, CsF, or KF. Of those, LiF, Li₂O, and NaF are preferred.

Examples of the alkali earth metal compound include BaO, SrO, CaO, and mixtures thereof such as BaₘSr₁₋ₘO (0<m<1) and BaₘCa₁₋ₘO (0<m<1). Of those, BaO, SrO, and CaO are preferred.

Examples of the rare earth metal compound include YbF₃, ScF₃, ScO₃, Y₂O₃, Ce₂O₃, GdF₃, and TbF₃. Of those, YbF₃, ScF₃, and TbF₃ are preferred.

The alkali metal complex, alkali earth metal complex, and rare earth metal complex are not particularly limited as long as they each include as a metal ion at least one of alkali metal ions, alkali earth metal ions, and rare earth metal ions. Meanwhile, preferred examples of a ligand include, but not limited to, quinolinol, benzoquinolinol, acridinol, phenanthridinol, hydroxyphenyloxazole, hydroxyphenylthiazole, hydroxydiaryloxadiazole, hydroxydiarylthiadiazole, hydroxyphenylpyridine, hydroxyphenylbenzoimidazole, hydroxybenzotriazole, hydroxyfluborane, bipyridyl, phenanthroline, phthalocyanine, porphyrin, cyclopentadiene, β-diketones, azomethines, and derivatives thereof.

For the addition form of the reductive dopant, it is preferred that the reductive dopant be formed in a shape of a layer or an island in the interfacial region. A preferred example of the forming method includes a method in which an organic substance which is a light emitting material or an electron injecting material for forming the interfacial region is deposited at the same time as the reductive dopant is deposited by a resistant heating deposition method, thereby dispersing the reductive dopant in the organic substance. The disperse concentration by molar ratio of the organic substance to the reductive dopant is 100:1 to 1:100 , and is preferably 5:1 to 1:5.

In a case where the reductive dopant is formed into the shape of a layer, the light emitting material or electron inj ectingmaterial which serves as an organic layer in the interface is formed into the shape of a layer. After that, the reductive dopant is solely deposited by the resistant heating deposition method to form a layer preferably having a thickness of 0.1 to 15 nm.

In a case where the reductive dopant is formed into the shape of an island, the light emitting material or electron injecting material which serves as an organic layer in the interface is formed into the shape of an island. After that, the reductive dopant is solely deposited by the resistant heating deposition method to form an island preferably having a thickness of 0.05 to 1 nm.

When the organic EL device has an electron injecting layer between the light emitting layer and the cathode, an electron transporting material to be used in the electron injecting layer is preferably an aromatic heterocyclic compound containing one or more hetero atoms in any one of its molecules, or particularly preferably a nitrogen-containing ring derivative.

The nitrogen-containing ring derivative is preferably, for example, a nitrogen-containing ring metal chelate complex represented by the following formula (A).

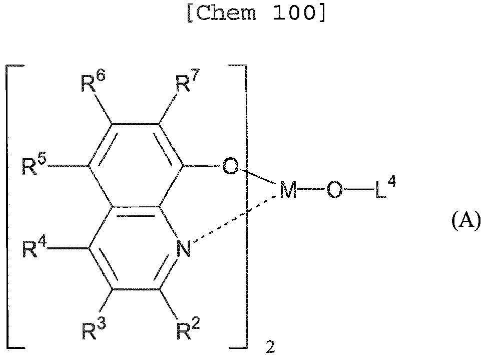

R² to R⁷ each independently represent a hydrogen atom, a halogen atom, an amino group, a hydrocarbon group each having 1 to 40 carbon atoms, an alkoxy group, an aryloxy group, an alkoxycarbonyl group, or a heterocyclic group, each of which may be substituted.

Mrepresentsaluminum (Al), gallium (Ga), or indium (In). Indium is preferred.

L⁴ in the formula (A) is a group represented by the following formula (A') or (A").

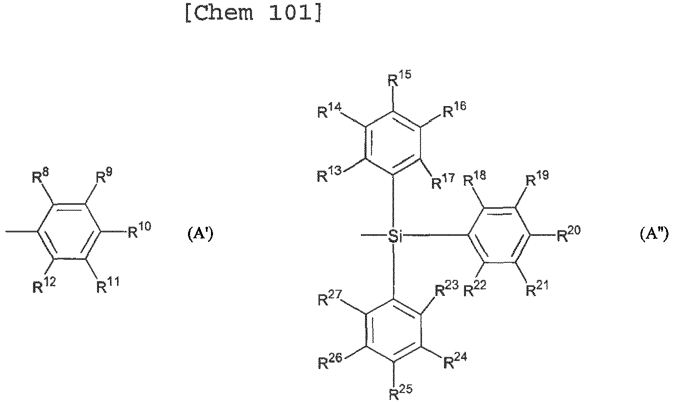

(In the formula, R⁸ to R¹² each independently represent a hydrogen atom, or a substituted or unsubstituted hydrocarbon group having 1 to 40 carbon atoms, and adjacent groups may form a cyclic structure. In addition, R¹³ to R²⁷ each independently represent a hydrogen atom, or a substituted or unsubstituted hydrocarbon group having 1 to 40 carbon atoms, and adjacent groups may form a cyclic structure.)

A nitrogen-containing heterocyclic derivative is a nitrogen-containing heterocyclic derivative formed of an organic compound having any one of the following formulae, and a nitrogen-containing compound which is not a metal complex is also an example of the derivative. Examples of the derivative include a five- or six-membered ring containing a skeleton represented by the following formula (a) and a derivative of a structure represented by the following formula (b).

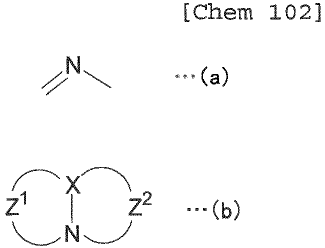

(In the formula (b), X represents a carbon atom or a nitrogen atom, and Z¹ and Z² each independently represent an atomic group capable of forming a nitrogen-containing heterocycle.)

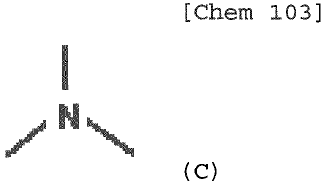

An organic compound having a nitrogen-containing aromatic polycycle formed of a five- or six-memberedring is preferred. In the case of such nitrogen-containing aromatic polycycle having a plurality of nitrogen atoms, a nitrogen-containing aromatic polycyclic organic compound having a skeleton obtained by combining the above-mentioned formulae (a) and (b) or the above-mentioned formulae (a) and (c) is more preferred.

The nitrogen-containing group of the nitrogen-containing organic compound is selected from, for example, nitrogen-containing heterocyclic groups represented by the following formulae.

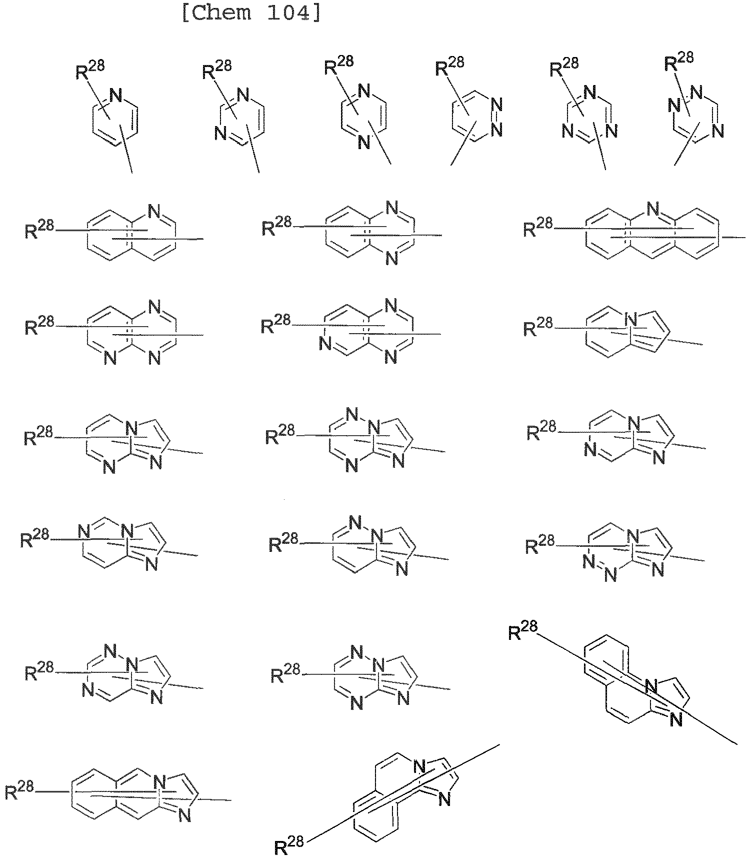

(In each of the formulae, R²⁸ represents an aryl group having 6 to 40 carbon atoms, a heteroaryl group having 3 to 40 carbon atoms, an alkyl group having 1 to 20 carbon atoms, or an alkoxy group having 1 to 20 carbon atoms, when the number of R²⁸ is two or more, a plurality of R²⁸s may be identical to or different from each other.)

Further, a preferred specific compound is, for example, a nitrogen-containing heterocyclic derivative represented by the following formula.

[Chem 105] HAr^{a}-L⁶-Ar^{b}-Ar^{c}

(In the formula, HAr^{a} represents a nitrogen-containing heterocycle which has 3 to 40 carbon atoms and which may have a substituent, L⁶ represents a single bond, an arylene group which has 6 to 40 carbon atoms and which may have a substituent, or a heteroarylene group which has 3 to 40 carbon atoms and which may have a substituent, Ar^{b} represents a divalent aromatic hydrocarbon group which has 6 to 40 carbon atoms and which may have a substituent, and Ar^{c} represents an aryl group which has 6 to 40 carbon atoms and which may have a substituent, or a heteroaryl group which has 3 to 40 carbon atoms and which may have a substituent.)

HAr^{a} is selected from, for example, the following group.

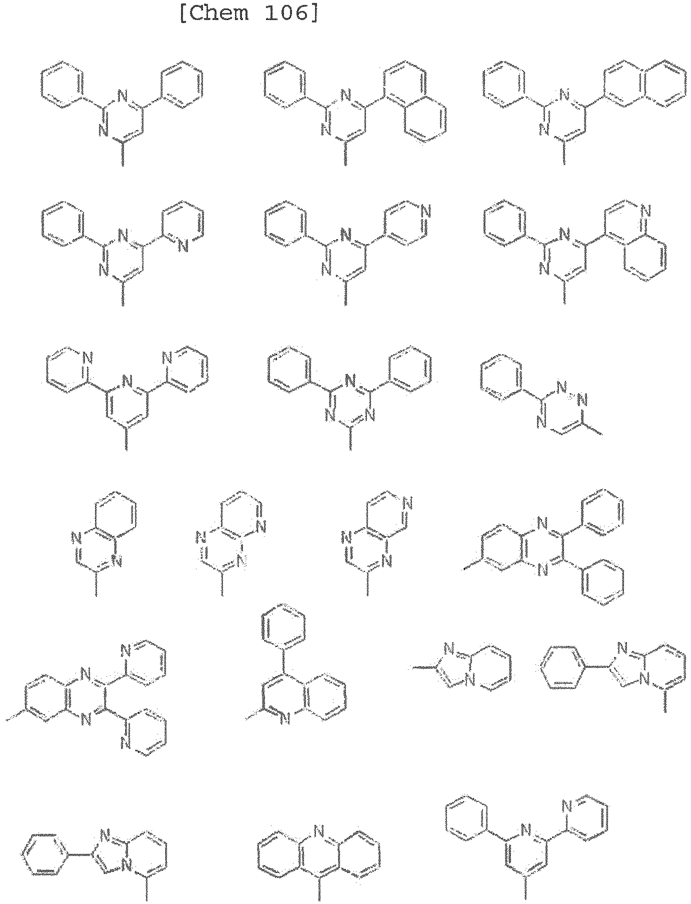

L⁶ is selected from, for example, the following group.

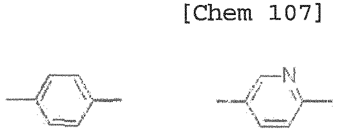

Ar^{c} is selected from, for example, the following group.

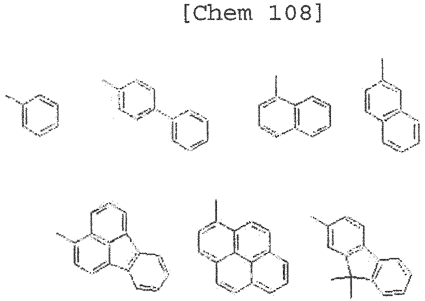

Ar^{b} is selected from, for example, the following arylanthranil group.

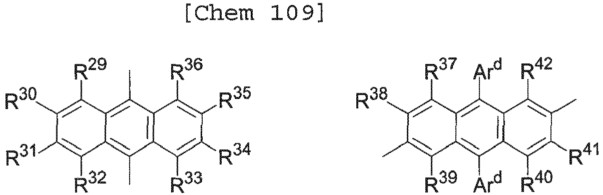

(In the formulae, R²⁹ to R⁴² each independently represent a hydrogen atom, a halogen atom, an alkyl group having 1 to 20 carbon atoms, an alkoxy group having 1 to 20 carbon atoms, an aryloxy group having 6 to 40 carbon atoms, an aryl group which has 6 to 40 carbon atoms and which may have a substituent, or a heteroaryl group having 3 to 40 carbon atoms, and Ar^{d} represents an aryl group which has 6 to 40 carbon atoms and which may have a substituent, or a heteroaryl group having 3 to 40 carbon atoms.)

In addition, a nitrogen-containing heterocyclic derivative in which R²⁹ to R³⁶ in Ar^{b} represented by the above-mentioned formula each represent a hydrogen atom is preferred.

In addition to the foregoing, the following compound (see JP 09-3448 A) is also suitably used.

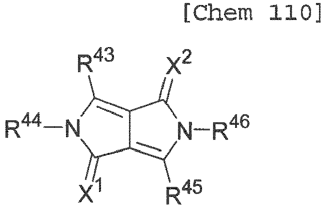

(In the formula, R⁴³ to R⁴⁶ each independently represent a hydrogen atom, a substituted or unsubstituted aliphatic group, a substituted or unsubstituted alicyclic group, a substituted or unsubstituted carbocyclic aromatic ring group, or a substituted or unsubstituted heterocyclic group, and X¹ and X² each independently represent an oxygen atom, asulfuratom, or a dicyanomethylene group.)

In addition, the following compound (see JP 2000-173774 A) is also suitably used.

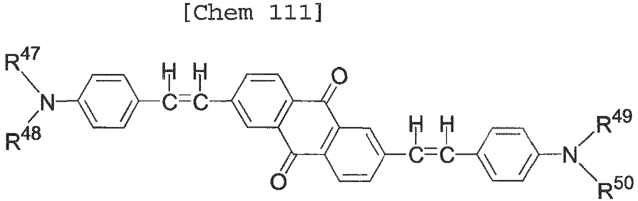

In the formula, R⁴⁷, R⁴⁸, R⁴⁹, and R⁵⁰ represent groups identical to or different from one another, and each represent an aryl group represented by the following formula.

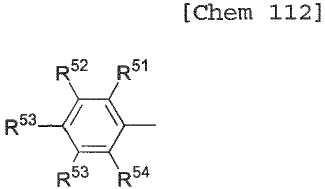

(In the formula, R⁵¹, R⁵², R⁵³, R⁵⁴, and R⁵⁵ represent groups identical to or different from one another, and each represent a hydrogen atom, or at least one of them may represent a saturated or unsaturated alkoxyl, alkyl, amino, or alkylamino group.)

Further, a polymer compound containing the nitrogen-containing heterocyclic group or nitrogen-containing heterocyclic derivative is also permitted.

In addition, the electron transporting layer preferably contains at least one of the nitrogen-containing heterocyclic derivatives represented by the following formulae (201) to (203).

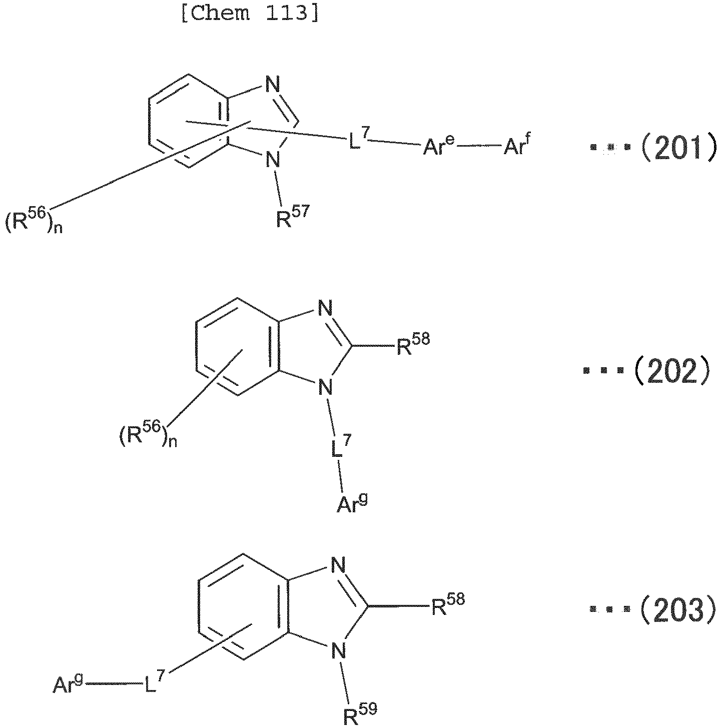

In the formulae (201) to (203), R⁵⁶ represents a hydrogen atom, an aryl group which has 6 to 60 carbon atoms and which may have a substituent, a pyridyl group which may have a substituent, a quinolyl group which may have a substituent, an alkyl group which has 1 to 20 carbon atoms and which may have a substituent, or an alkoxy group which has 1 to 20 carbon atoms and which may have a substituent, n represents an integer of 0 to 4, R⁵⁷ represents an aryl group which has 6 to 60 carbon atoms and which may have a substituent, a pyridyl group which may have a substituent, a quinolyl group which may have a substituent, an alkyl group which has 1 to 20 carbon atoms and which may have a substituent, or an alkoxy group having 1 to 20 carbon atoms, R⁵⁸ and R⁵⁹ each independently represent a hydrogen atom, an aryl group which has 6 to 60 carbon atoms and which may have a substituent, a pyridyl group which may have a substituent, a quinolyl group which may have a substituent, an alkyl group which has 1 to 20 carbon atoms and which may have a substituent, or an alkoxy group which has 1 to 20 carbon atoms and which may have a substituent, L⁷ represents a single bond, an arylene group which has 6 to 60 carbon atoms and which may have a substituent, a pyridinylene group which may have a substituent, a quinolinylene group which may have a substituent, or a fluorenylene group which may have a substituent, Ar^{e} represents an arylene group which has 6 to 60 carbon atoms and which may have a substituent, a pyridinylene group which may have a substituent, or a quinolinylene group which may have a substituent, and Ar^{f} represents a hydrogen atom, an aryl group which has 6 to 60 carbon atoms and which may have a substituent, a pyridyl group which may have a substituent, a quinolyl group which may have a substituent, an alkyl group which has 1 to 20 carbon atoms and which may have a substituent, or an alkoxy group which has 1 to 20 carbon atoms and which may have a substituent.

Ar^{g} represents an aryl group which has 6 to 60 carbon atoms and which may have a substituent, a pyridyl group which may have a substituent, a quinolyl group which may have a substituent, an alkyl group which has 1 to 20 carbon atoms and which may have a substituent, an alkoxy group which has 1 to 20 carbon atoms and which may have a substituent, or a group represented by -Ar^{e}-Ar^{f} (Ar^{e} and Ar^{f} each have the same meaning as that described above).

In addition, as the nitrogen-containing ring derivative, nitrogen-containing five-membered ring derivative are preferably exemplified. Examples of the nitrogen-containing five-membered ring include an imidazole ring, a triazole ring, a tetrazole ring, an oxadiazole ring, a thiadiazole ring, an oxatriazole ring, and a thiatriazole ring. Examples of the nitrogen-containing five-membered ring derivative include a benzoimidazole ring, a benzotriazole ring, apyridinoimidazole ring, apyrimidinoimidazole ring, and a pyridazinoimidazole ring. Particularly preferred is the compound represented by the following formula (B).

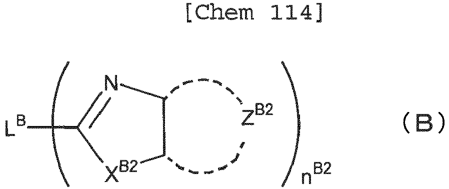

In the formula (B), L^{B} represents a divalent or more bonding group. Examples thereof include a carbon atom, a silicon atom, a nitrogen atom, a boron atom, an oxygen atom, a sulfur atom, metal atoms (for example, a barium atom, a beryllium atom), aromatic hydrocarbon rings, and aromatic heterocycles.

Of the nitrogen-containing five-membered ring derivatives each represented by the formula (B), a derivative represented by the following formula (B') is more preferred.

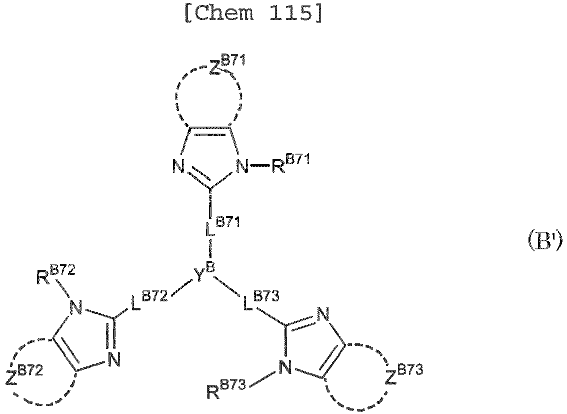

In the formula (B'), R^{B71}, R^{B72}, and R^{B73} each have the same meaning as that of R^{B2} in the formula (B).

Z^{B71}, Z^{B72}, and Z^{B73} each have the same meaning as that of Z^{B2} in the formula (B).

L^{B71}, L^{B72}, and L^{B73} each represent a linking group, and examples of the linking group include examples obtained by making the examples of L^{B} in the formula (B) divalent. The linking group is preferably a single bond, a divalent aromatic hydrocarbon ring group, a divalent aromatic heterocyclic group, or a linking group formed of a combination of two or more of them, or is more preferably a single bond. L^{B71}, L^{B72}, and L^{B73} may each have a substituent. Examples of the substituent include the same examples as those described for the substituent of the group represented by L^{B} in the formula (B) .

Y^{B} represents a nitrogen atom, a 1,3,5-benzenetriyl group, or a 2,4,6-triazinetriyl group.

A compound of which each of the electron injecting layer and the electron transporting layer is formed is, for example, a compound having a structure obtained by combining an electron-deficient, nitrogen-containing five-membered ring skeleton or electron-deficient, nitrogen-containing six-membered ring skeleton and a substituted or unsubstituted indole skeleton, substituted or unsubstituted carbazole skeleton, or substituted or unsubstituted azacarbazole skeleton as well as the polycyclic compound of the present invention. In addition, a suitable electron-deficient, nitrogen-containing five-membered ring skeleton or electron-deficient, nitrogen-containing six-membered ring skeleton is a molecular skeleton such as a pyridine, pyrimidine, pyrazine, triazine, triazole, oxadiazole, pyrazole, imidazole, quinoxaline, or pyrrole skeleton, or benzimidazole or imidazopyridine obtained when two or more of them fuse with each other. Of those combinations, a preferred combination is, for example, a combination of a pyridine, pyrimidine, pyrazine, or triazine skeleton and a carbazole, indole, azacarbazole, or quinoxaline skeleton. The skeleton may be substituted or unsubstituted.

Each of the electron injecting layer and the electron transporting layer may be of a monolayer structure formed of one or two or more kinds of the materials, or may be of a multi-layered structure formed of the plurality of layers identical to or different from each other in composition. Materials for those layers each preferably have a n-electron-deficient, nitrogen-containing heterocyclic group.

In addition, an insulator or semiconductor serving as an inorganic compound as well as the nitrogen-containing ring derivative is preferably used as a component of the electron injecting layer. When the electron injecting layer is formed of an insulator or semiconductor, current leakage can be effectively prevented, and the electron injecting property of the layer can be improved.

As the insulator, at least one metal compound selected from the group consisting of alkali metal chalcogenides, alkaline earth metal chalcogenides, alkali metal halides, and alkaline earth metal halides is preferably used. It is preferred that the electron injecting layer be formed of the alkali metal chalcogenide or the like since the electron injecting property can be further improved. To be specific, preferred examples of the alkali metal chalcogenide include Li₂O, K₂O, Na₂S, Na₂Se, and Na₂O, and preferred examples of the alkaline earth metal chalcogenide include CaO, BaO, SrO, BeO, BaS, and CaSe. In addition, preferred examples of the alkali metal halide include LiF, NaF, KF, LiCl, KCl, andNaCl. Further, preferred examples of the alkaline earth metal halide include fluorides such as CaF₂, BaF₂, SrF₂, MgF₂, and BeF₂ and halides other than the fluorides .

In addition, examples of the semiconductor include oxides, nitrides, and oxide nitrides containing at least one element selected from the group consisting of Ba, Ca, Sr, Yb, Al, Ga, In, Li, Na, Cd, Mg, Si, Ta, Sb, and Zn. One kind of them may be used alone, or two or more kinds of them may be used in combination. In addition, it is preferred that the inorganic compound composing the electron injecting layer form a crystallite or amorphous insulating thin film. When the electron injecting layer is formed of the insulating thin film, a more uniform thin film can be formed, and defects of pixels such as dark spots can be decreased. It should be noted that examples of the inorganic compound include alkali metal chalcogenides, alkaline earth metal chalcogenides, alkali metal halides, and alkaline earth metal halides.

Inaddition, the reducing dopant can be preferably incorporated into the electron injecting layer in the present invention.

It should be noted that the thickness of each of the electron injecting layer and the electron transporting layer, which is not particularly limited, is preferably 1 to 100 nm.

An aromatic amine compound such as an aromatic amine derivative represented by the formula (I) is suitably used in the hole injecting layer or hole transporting layer (a hole injecting/transporting layer is also included in this category).

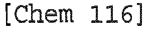

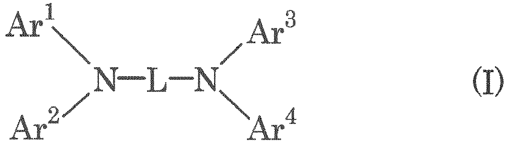

In the formula (I), Ar¹ to Ar⁴ each represent a substituted or unsubstituted aryl group having a ring formed of 6 to 50 carbon atoms, or a substituted or unsubstituted heteroaryl group having a ring formed of 5 to 50 atoms.

L represents a linking group, and specifically, a substituted or unsubstituted arylene group having a ring formed of 6 to 50 carbon atoms, a substituted or unsubstituted heteroarylene group having a ring formed of 5 to 50 atoms, or a divalent group in which two or more arylene groups or heteroarylene groups are bonded by a single bond, an ether bond, a thioether bond, with an alkylene group having 1 to 20 carbon atoms, an alkenylene group having 2 to 20 carbon atoms, and an amino group.

In addition, an aromatic amine represented by the following formula (II) is also suitably used in the formation of the hole injecting layer or hole transporting layer.

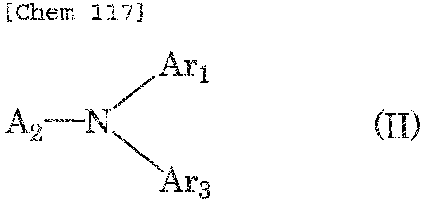

In the formula (II), the definition of Ar₁ to Ar₃ is the same as that of Ar¹ to Ar⁴ in the formula (I).

The compound of the present invention can be used in each of the hole injecting layer, the hole transporting layer, the electron injecting layer, and the electron transporting layer because the compound can transport both a hole and an electron.

The anode in the organic EL device has the function of injecting holes into the hole transporting layer or the light emitting layer. It is effective that the anode has a work function of 4.5 eV or more. Specific examples of the material for the anode used in the present invention include indium tin oxide alloys (ITO), tin oxide (NESA), gold, silver, platinum, and copper. In addition, as the cathode, a material having a small work function is preferred in view to inject an electron into an electron-injecting layer or a light-emitting layer. Examples of the cathode material are not particularly limited, and specifically, indium, aluminum, magnesium, a magnesium-indium alloy, a magnesium-aluminum alloy, an aluminum-lithium alloy, an aluminum-scandium-lithium alloy, and a magnesium-silver alloy may be used.

The method of forming the layers in the organic EL device is not particularly limited. A conventionally known process such as the vacuum vapor deposition process or the spin coating process can be used. The organic thin film layer which is used in the organic EL device and includes the compound represented by the formula (1) can be formed in accordance with a known process such as the vacuum vapor deposition process or the molecular beam epitaxy process (MBE process) or, using a solution prepared by dissolving the compounds into a solvent, in accordance with a coating process such as the dipping process, the spin coating process, the casting process, the bar coating process, or the roll coating process.

The thickness of each layer in the organic thin film layer in the organic EL device is not particularly limited. In general, an excessively thin layer tends to have defects such as pin holes, whereas an excessively thick layer requires a high applied voltage to decrease the efficiency. Therefore, a thickness in the range of several nanometers to 1 µm is typically preferred.

### Examples

Next, the present invention is described in detail by way of examples, but the present invention is not limited to the following examples. Note that, in the synthetic examples below, DMF refers to dimethylformamide, THF refers to tetrahydrofuran, DME refers to dimethoxyethane, NBS refers to N-bromosuccine imide, Ph refers to a phenyl group, AcOEt refers to ethyl acetate, and NMP refers to N-methyl pyrrolidone.

### Synthesis Example 1 (Synthesis of Compound No. 11 (not according to the invention))

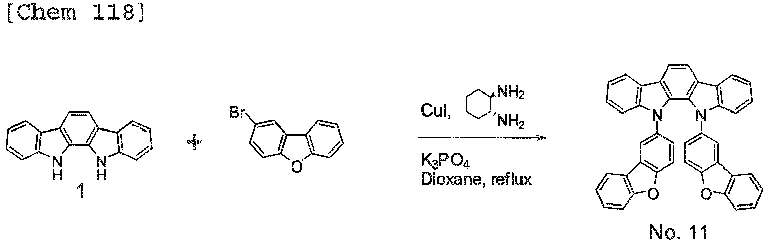

Compound 1 (2.6 g, 10 mmol), 2-bromodibenzofuran (5.0 g, 20 mmol), CuI (1.9 g, 10 mmol), trans-cyclohexane-1,2-diamine (3.4 g, 30 mmol), K₃PO₄ (8.5 g, 40 mmol), and 1,4-dioxane (10 mL) were loaded into a three-necked flask, and the mixture was refluxed under an argon atmosphere for 10 hours.

After the completion of the reaction, the resultant was cooled to room temperature. The resultant sample was transferred to a separating funnel, and water (50 mL) was charged into the funnel. Then, the mixture was extracted with CH₂Cl₂. The resultant sample was purified by column chromatography. The purified product was concentrated to dryness, and was then recrystallized twice, whereby a white powder (Compound No. 11 (not according to the invention)) was obtained. The powder was purified by sublimation, whereby a white solid was obtained in an amount of 0.7g in 12% yield.
FD-MS analysis C₄₂H₂₄N₂O₂: theoretical value 588, observed value 588

### Synthesis Example 2 (Synthesis of Compound No. 40 (not according to the invention))

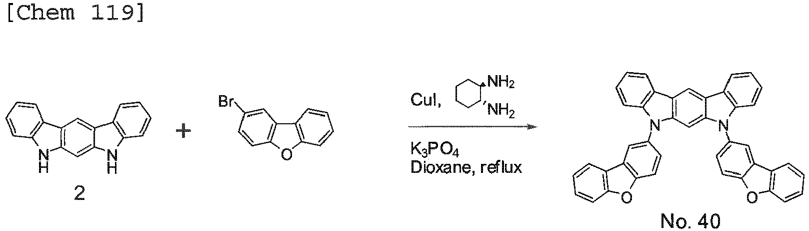

Compound 2 (2.6 g, 10 mmol), 2-bromodibenzofuran (5.0 g, 20 mmol), CuI (1.9 g, 10 mmol), trans-cyclohexane-1,2-diamine (3.4 g, 30 mmol), K₃PO₄ (8.5 g, 40 mmol), and 1,4-dioxane (10 mL) were loaded into a three-necked flask, and the mixture was refluxed under an argon atmosphere for 10 hours.

After the completion of the reaction, the resultant was cooled to room temperature. The resultant sample was transferred to a separating funnel, and water (50 mL) was charged into the funnel. Then, the mixture was extracted with CH₂Cl₂. The resultant sample was purified by column chromatography. The purified product was concentrated to dryness, and was then recrystallized twice, whereby a white powder (Compound No. 40 (not according to the invention)) was obtained. The powder was purified by sublimation, whereby a white solid was obtained in an amount of 1.9 g in 33% yield.
FD-MS analysis C₄₂H₂₄N₂O₂: theoretical value 588, observed value 588

### Synthesis Example 3 (Synthesis of Compound No. 47 (not according to the invention))

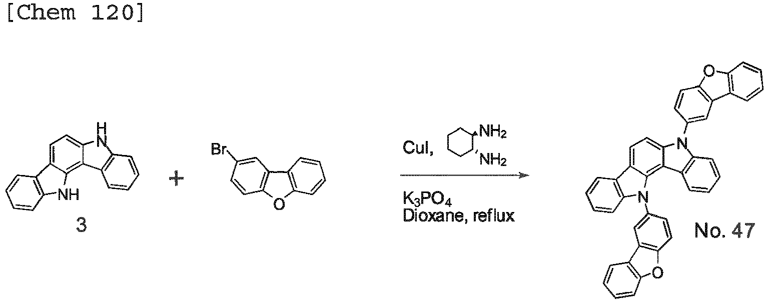

Compound 3 (2.6 g, 10 mmol), 2-bromodibenzofuran (5.0 g, 20 mmol), CuI (1.9 g, 10 mmol), trans-cyclohexane-1,2-diamine (3.4 g, 30 mmol), K₃PO₄ (8.5 g, 40 mmol), and 1,4-dioxane (10 mL) were loaded into a three-necked flask, and the mixture was refluxed under an argon atmosphere for 10 hours.

After the completion of the reaction, the resultant was cooled to room temperature. The resultant sample was transferred to a separating funnel, and water (50 mL) was charged into the funnel. Then, the mixture was extracted with CH₂Cl₂. The resultant sample was purified by column chromatography. The purified product was concentrated to dryness, and was then recrystallized twice, whereby a white powder (Compound No. 47 (not according to the invention)) was obtained. The powder was purified by sublimation, whereby a white solid was obtained in an amount of 1.5 g in 25% yield.
FD-MS analysis C₄₂H₂₄N₂O₂: theoretical value 588, observed value 588

### Synthesis Example 4 (Synthesis of Compound No. 66 (not according to the invention))

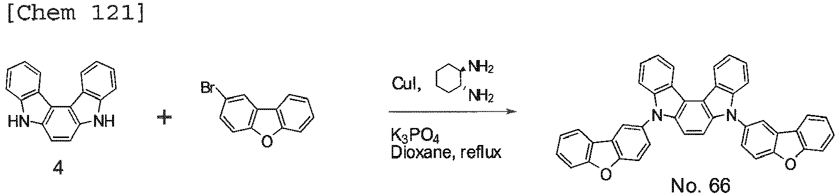

Compound 4 (2.6 g, 10 mmol), 2-bromodibenzofuran (5.0 g, 20 mmol), CuI (1.9 g, 10 mmol), trans-cyclohexane-1,2-diamine (3.4 g, 30 mmol), K₃PO₄ (8.5 g, 40 mmol), and 1,4-dioxane (10 mL) were loaded into a three-necked flask, and the mixture was refluxed under an argon atmosphere for 10 hours.

After the completion of the reaction, the resultant was cooled to room temperature. The resultant sample was transferred to a separating funnel, and water (50 mL) was charged into the funnel. Then, the mixture was extracted with CH₂Cl₂. The resultant sample was purified by column chromatography. The purified product was concentrated to dryness, and was then recrystallized twice, whereby a white powder (Compound No. 66 (not according to the invention)) was obtained. The powder was purified by sublimation, whereby a white solid was obtained in an amount of 2.4 g in 40% yield.
FD-MS analysis C₄₂H₂₄N₂O₂: theoretical value 588, observed value 588

### Synthesis Example 5 (Synthesis of Compound No. 100 (not according to the invention))

### (1) Synthesis of Compound 5

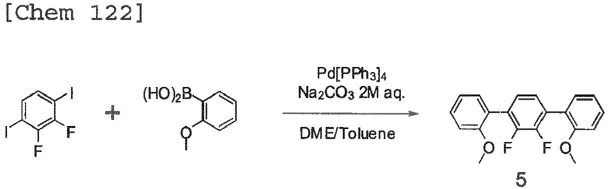

1,2-difluor-3,6-diiodobenzene (65.9 g, 180.0 mmol), 2-methoxyphenylboronic acid (65.6 g, 432.0 mmol), and a 2 M aqueous solution of Na₂CO₃ (360 mL, 720 mmol), DME (360 mL), toluene (360 mL), and Pd[PPh₃]₄ (20.8 g, 18.0 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (500 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 38.2 g in 65% yield.
FD-MS analysis C₂₀H₁₆F₂O₂: theoretical value 326, observed value 326

### (2) Synthesis of Compound 6

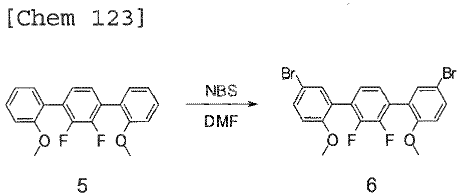

Compound 5 (38.0g, 116.4mmol), NBS (41.5g, 232.9mmol), and DMF (1000 mL) were loaded into a three-necked flask, and the mixture was stirred under an Ar atmosphere at room temperature for 8 hours . After the completion of the reaction, the resultant sample was transferred to a separating funnel, and water (1000 mL) was charged into the funnel. Then, the mixture was extracted with AcOEt. The resultant sample was purified by column chromatography, whereby a white solid was obtained in an amount of 45.1 g in 80% yield.
FD-MS analysis C₂₀H₁₄Br₂F₂O₂: theoretical value 484, observed value 484

### (3) Synthesis of Compound 7

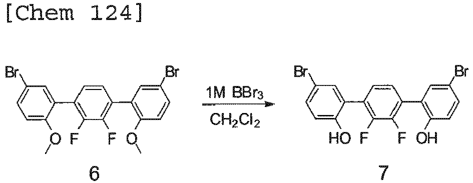

Compound 6 (45.0 g, 93.0 mmol), a 1 M solution of BBr₃ in CH₂Cl₂ (218 mL, 218 mmol), and CH₂Cl₂ (560 mL) were loaded into a three-necked flask, and the mixture was stirred under an Ar atmosphere at 0°C for 8 hours. After that, the mixture was left to stand at room temperature overnight. After the completion of the reaction, the resultant was neutralized with a saturated aqueous solution of NaHCO₃. The resultant sample was transferred to a separating funnel, and was extracted with CH₂Cl₂. The resultant sample was purified by column chromatography, whereby a white solid was obtained in an amount of 35.2 g in 83% yield.
FD-MS analysis C₁₈H₁₀Br₂F₂O₂: theoretical value 456, observed value 456

### (4) Synthesis of Compound 8

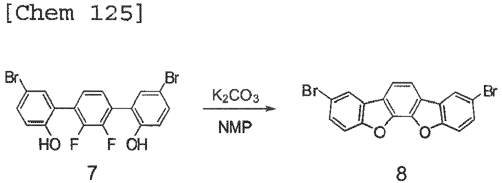

Compound 7 (35.0 g, 76.7 mmol), K₂CO₃ (23.3 g, 168.8 mmol), and NMP (320 mL) were loaded into a three-necked flask, and the mixture was stirred under an Ar atmosphere at 150°C for 8 hours. After the completion of the reaction, the resultant was cooled to room temperature. The resultant sample was transferred to a separating funnel, and water (500 mL) was charged into the funnel. Then, the mixture was extracted with AcOEt. The resultant sample was purified by column chromatography, whereby a white solid was obtained in an amount of 27.1 g in 85% yield.
FD-MS analysis C₁₈H₈Br₂O₂: theoretical value 416, observed value 416

### (5) Synthesis of Compound No. 100 (not according to the invention)

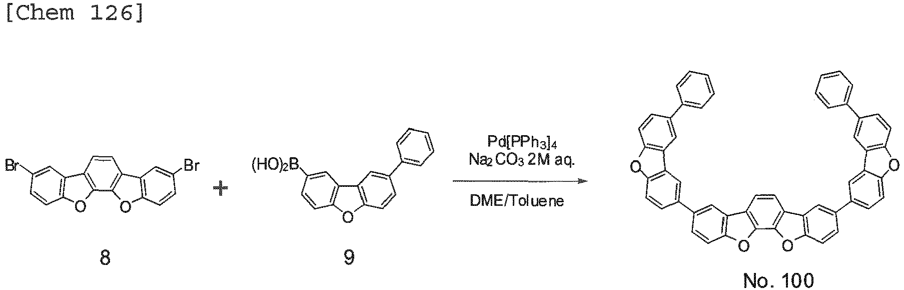

Compound 8 (2.5 g, 6.0 mmol), Compound 9 (3.8 g, 13.2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 100 (not according to the invention)) was obtained. The powder was purified by sublimation, whereby a white solid was obtained in an amount of 2.0 g in 45% yield.
FD-MS analysis C₅₄H₃₀O₄: theoretical value 742, observed value 742

### Synthesis Example 6 (Synthesis of Compound No. 103 (not according to the invention)

### (1) Synthesis of Compound 10

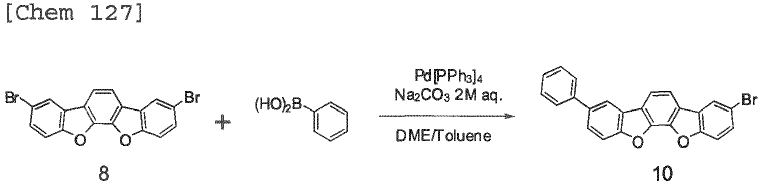

Compound 8 (16.8 g, 40 mmol), phenylboronic acid (4.8 g, 40 mmol), a 2 M aqueous solution of Na₂CO₃ (80 mL, 160 mmol), DME (80 mL), toluene (80 mL), and Pd[PPh₃]₄ (2.3 g, 2.0 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (150 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 12.4 g in 75% yield. FD-MS analysis C₂₄H₁₃BrO₂: theoretical value 413, observed value 413

### (2) Synthesis of Compound 11

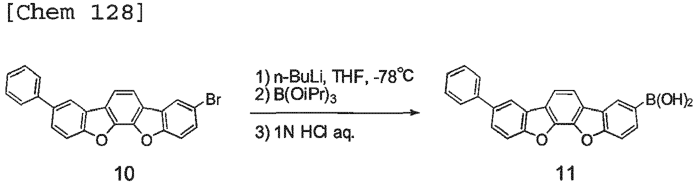

Compound 10 (12.0 g, 29.0 mmol) and THF (288 mL) were loaded into a three-necked flask, and the mixture was cooled to -78°C. Then, n-BuLi (1.65 M solution in n-hexane, 19.4 mL, 31.9 mmol) was added dropwise to the flask, and the resultant mixture was stirred at -78°C for 20 minutes. Triisopropyl borate (16.4 g, 87.0 mmol) was added to the resultant, and the mixture was stirred at -78°C for 1 hour. After that, the resultant was left to stand overnight at room temperature. Then, 1 N HCl (100 mL) was charged into the resultant, and the mixture was stirred at room temperature for 1 hour. The resultant sample was concentrated, and was then transferred to a separating funnel. Water (100 mL) was charged into the funnel, and the mixture was extracted with CH₂Cl₂. The extract was dried with MgSO₄, and was then filtered and concentrated. The resultant sample was purified by recrystallization (toluene-hexane), whereby a white solid was obtained in an amount of 7.1 g in 65% yield.

### (3) Synthesis of Compound No. 103 (not according to the invention)

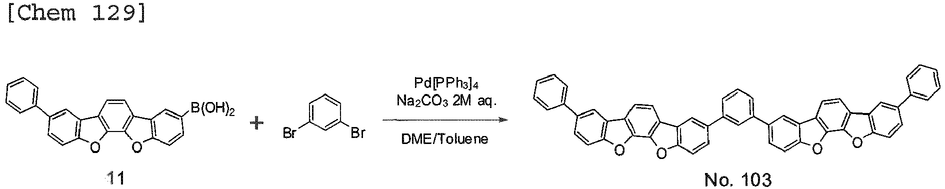

Compound 11 (5.5g, 14.5 mmol), 1,3-dibromobenzene (1.7 g, 7.3 mmol), a 2 M aqueous solution of Na₂CO₃ (15 mL, 30 mmol), DME (15 mL), toluene (15 mL), and Pd[PPh₃]₄ (0.42 g, 0.37 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 103 (not according to the invention)) was obtained. The powder was purified by sublimation, whereby a white solid was obtained in an amount of 2.4 g in 45% yield.
FD-MS analysis C₅₄H₃₀O₄: theoretical value 742, observed value 742

### Synthesis Example 7 (Synthesis of Compound No. 116)

### (1) Synthesis of Compound 12

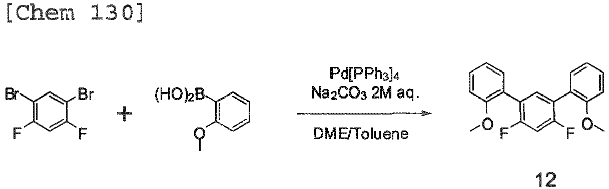

1,3-dibromo-4,6-difluorobenzene (50.0 g, 183.9 mmol), 2-methoxyphenylboronic acid (67.1 g, 441.4 mmol), a 2 M aqueous solution of Na₂CO₃ (368 mL, 736 mmol), DME (370 mL), toluene (370 mL), and Pd [PPh₃]₄ (21 g, 18.0 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (500 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 40.2 g in 67% yield. FD-MS analysis C₂₀H₁₆F₂O₂: theoretical value 326, observed value 326

### (2) Synthesis of Compound 13

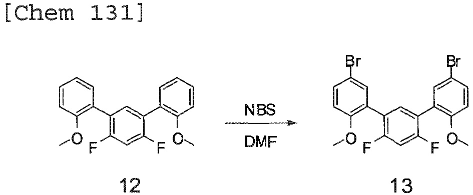

Compound 12 (40.0 g, 122.6 mmol), NBS (43.6 g, 245 mmol), and DMF (1000 mL) were loaded into a three-necked flask, and the mixture was stirred under an Ar atmosphere at room temperature for 8 hours. After the completion of the reaction, the resultant sample was transferred to a separating funnel, and water (1000 mL) was charged into the funnel. Then, the mixture was extracted with AcOEt. The resultant sample was purified by column chromatography, whereby a white solid was obtained in an amount of 42.1 g in 71% yield. FD-MS analysis C₂₀H₁₄Br₂F₂O₂: theoretical value 484, observed value 484

### (3) Synthesis of Compound 14

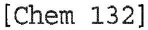

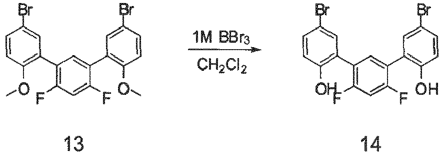

Compound 13 (40.0 g, 82.6 mmol), a 1 M solution of BBr₃ in CH₂Cl₂ (194 mL, 194 mmol), and CH₂Cl₂ (500 mL) were loaded into a three-necked flask, and the mixture was stirred under an Ar atmosphere at 0°C for 8 hours. After that, the mixture was left to stand at room temperature overnight. After the completion of the reaction, the resultant was neutralized with a saturated aqueous solution of NaHCO₃. The resultant sample was transferred to a separating funnel, and was extracted with CH₂Cl₂. The resultant sample was purified by column chromatography, whereby a white solid was obtained in an amount of 30.2 g in 80% yield.
FD-MS analysis C₁₈H₁₀Br₂F₂O₂: theoretical value 456, observed value 456

### (4) Synthesis of Compound 15

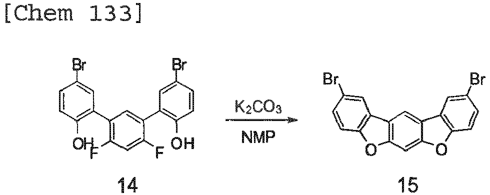

Compound 14 (30.0 g, 65.7 mmol), K₂CO₃ (19.9 g, 144.5 mmol), and NMP (270 mL) were loaded into a three-necked flask, and the mixture was stirred under an Ar atmosphere at 150°C for 8 hours. After the completion of the reaction, the resultant was cooled to room temperature. The resultant sample was transferred to a separating funnel, and water (500 mL) was charged into the funnel. Then, the mixture was extracted with AcOEt. The resultant sample was purified by column chromatography, whereby a white solid was obtained in an amount of 21.9 g in 80% yield.
FD-MS analysis C₁₈H₈Br₂O₂: theoretical value 416, observed value 416

### (5) Synthesis of Compound No. 116

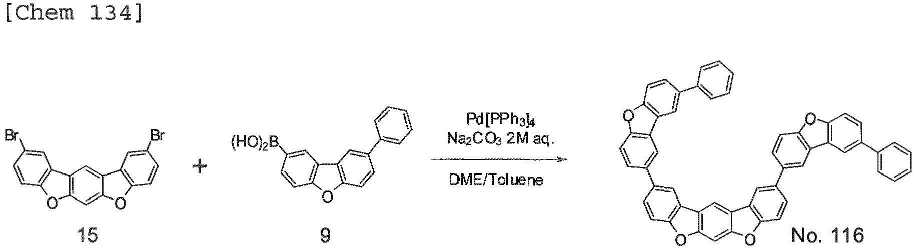

Compound 15 (2.5 g, 6.0 mmol), Compound 9 (3.8 g, 13.2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 116) was obtained. The powder was purified by sublimation, whereby a white solid was obtained in an amount of 2.7 g in 60% yield. FD-MS analysis C₅₄H₃₀O₄: theoretical value 742, observed value 742

### Synthesis Example 8 (Synthesis of Compound No. 119)

### (1) Synthesis of Compound 16

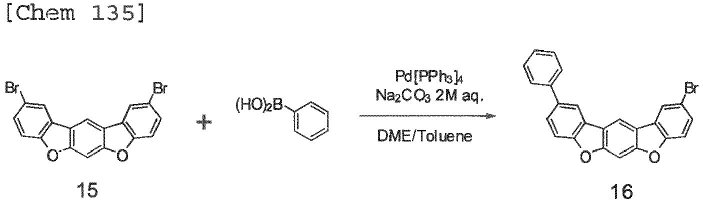

Compound 15 (16.8 g, 40. 0 mmol), phenylboronic acid (4.8 g, 40 mmol), a 2 M aqueous solution of Na₂CO₃ (80 mL, 160 mmol), DME (80 mL), toluene (80 mL), and Pd[PPh₃]₄ (2.3 g, 2.0 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (150 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 11.6 g in 70% yield. FD-MS analysis C₂₄H₁₃BrO₂: theoretical value 413, observed value 413

### (2) Synthesis of Compound 17

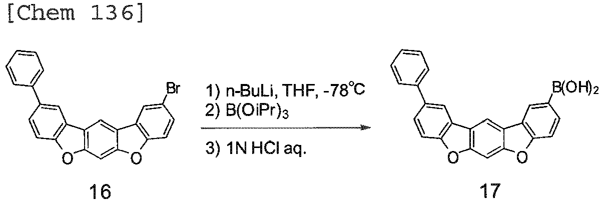

Compound 16 (11.0 g, 26.6 mmol) and THF (264 mL) were loaded into a three-necked flask, and the mixture was cooled to -78°C. Then, n-BuLi (1.65 M solution in n-hexane, 17.7 mL, 29.3 mmol) was added dropwise to the flask, and the resultant mixture was stirred at -78°C for 20 minutes. Triisopropyl borate (15.1 g, 80 mmol) was added to the resultant, and the mixture was stirred at -78°C for 1 hour. After that, the resultant was left to stand overnight at room temperature. Then, 1 N HCl (100 mL) was charged into the resultant, and the mixture was stirred at room temperature for 1 hour. The resultant sample was concentrated, and was then transferred to a separating funnel. Water (100 mL) was charged into the funnel, and the mixture was extracted with CH₂Cl₂. The extract was dried with MgSO₄, and was then filtered and concentrated. The resultant sample was purified by recrystallization (toluene-hexane), whereby a white solid was obtained in an amount of 6.1 g in 61% yield.

### (3) Synthesis of Compound No. 119

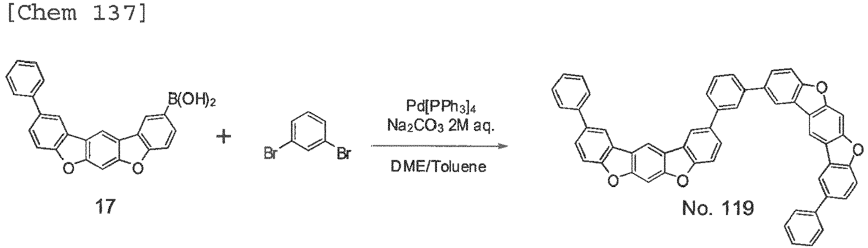

Compound 17 (5.5 g, 14.5 mmol), 1,3-dibromobenzene (1.7 g, 7.3 mmol), a 2 M aqueous solution of Na₂CO₃ (15 mL, 30 mmol), DME (15 mL), toluene (15 mL), and Pd[PPh₃]₄ (0.42 g, 0.37 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 119) was obtained. The powder was purified by sublimation, whereby a white solid was obtained in an amount of 1.9 g in 35% yield.
FD-MS analysis C₅₄H₃₀O₄: theoretical value 742, observed value 742

### Synthesis Example 9 (Synthesis of Compound No. 134)

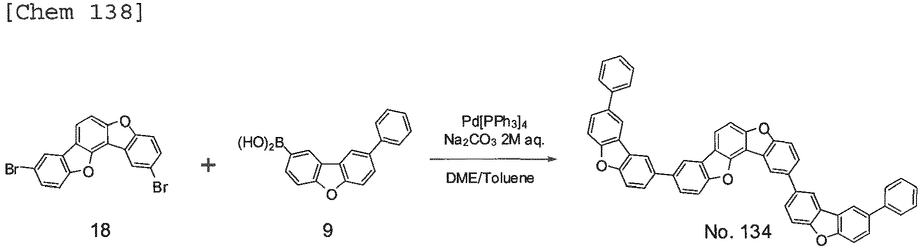

Compound 18 (2.5 g, 6.0 mmol), Compound 9 (3.8 g, 13.2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 134) was obtained. The powder was purified by sublimation, whereby a white solid was obtained in an amount of 1.8 g in 41% yield.
FD-MS analysis C₅₄H₃₀O₄: theoretical value 742, observed value 742

### Synthesis Example 10 (Synthesis of Compound No. 139)

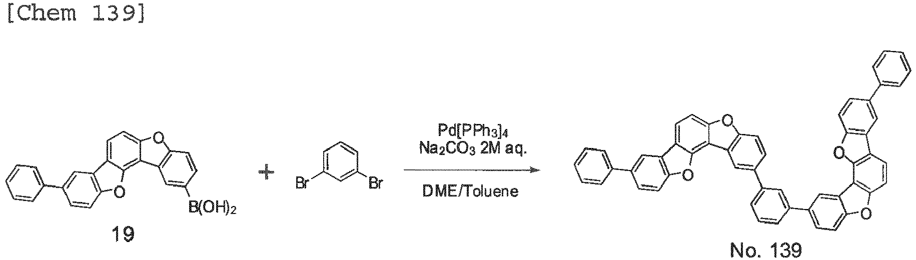

Compound 19 (5.0 g, 13.2 mmol), 1, 3-dibromobenzene (1.5 g, 6.6 mmol), a 2 M aqueous solution of Na₂CO₃ (14 mL, 28 mmol), DME (14 mL), toluene (14 mL), and Pd[PPh₃]₄ (0.38 g, 0.34 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 139) was obtained. The powder was purified by sublimation, whereby a white solid was obtained in an amount of 1.9 g in 39% yield. FD-MS analysis C₅₄H₃₈O₄: theoretical value 742, observed value 742

### Synthesis Example 11 (Synthesis of Compound No. 154 (not according to the invention))

### (1) Synthesis of Compound 20

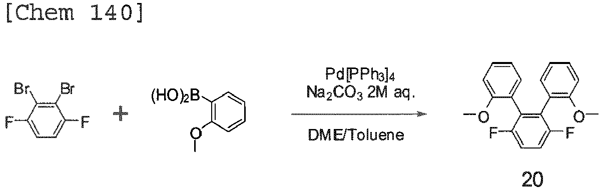

1,2-dibromo-3,6-difluorobenzene (50.0 g, 183.9 mmol), 2-methoxyphenylboronic acid (67.1 g, 441.4 mmol), a 2 M aqueous solution of Na₂CO₃ (368 mL, 736 mmol), DME (370 mL), toluene (370 mL), andPd[PPh₃]₄ (21.3g, 18.4 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (500 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 30.6 g in 51% yield. FD-MS analysis C₂₀H₁₆F₂O₂: theoretical value 326, observed value 326

### (2) Synthesis of Compound 21

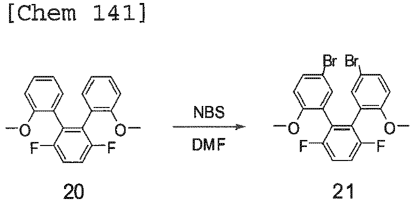

Compound 20 (30.0 g, 91.9 mmol), NBS (32.8 g, 184 mmol), and DMF (820 mL) were loaded into a three-necked flask, and the mixture was stirred under an Ar atmosphere at room temperature for 8 hours . After the completion of the reaction, the resultant sample was transferred to a separating funnel, and water (1000 mL) was charged into the funnel. Then, the mixture was extracted with AcOEt . The resultant sample was purified by column chromatography, whereby a white solid was obtained in an amount of 32 g in 72% yield. FD-MS analysis C₂₀H₁₄Br₂F₂O₂: theoretical value 484, observed value 484

### (3) Synthesis of Compound 22

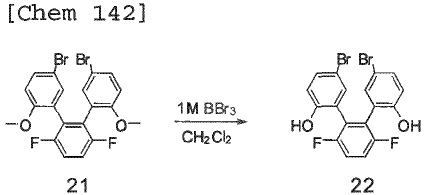

Compound 21 (32.0 g, 66.1 mmol), a 1 M solution of BBr₃ in CH₂Cl₂ (155 mL, 155 mmol), and CH₂Cl₂ (430 mL) were loaded into a three-necked flask, and the mixture was stirred under an Ar atmosphere at 0°C for 8 hours. After that, the mixture was left to stand at room temperature overnight. After the completion of the reaction, the resultant was neutralized with a saturated aqueous solution of NaHCO₃. The resultant sample was transferred to a separating funnel, and was extracted with CH₂Cl₂. The resultant sample was purified by column chromatography, whereby a white solid was obtained in an amount of 24.1 g in 80% yield.
FD-MS analysis C₁₈H₁₀Br₂F₂O₂: theoretical value 456, observed value 456

### (4) Synthesis of Compound 23

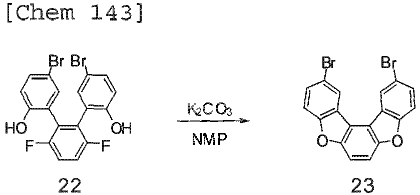

Compound 22 (24.1 g, 52.8 mmol), K₂CO₃ (16.0 g, 116 mmol), and NMP (220 mL) were loaded into a three-necked flask, and the mixture was stirred under an Ar atmosphere at 150°C for 8 hours. After the completion of the reaction, the resultant was cooled to room temperature. The resultant sample was transferred to a separating funnel, and water (500 mL) was charged into the funnel. Then, the mixture was extracted with AcOEt. The resultant sample was purified by column chromatography, whereby a white solid was obtained in an amount of 18.7 g in 85% yield.
FD-MS analysis C₁₈H₈Br₂O₂: theoretical value 416, observed value 416

### (5) Synthesis of Compound No. 154 (not according to the invention)

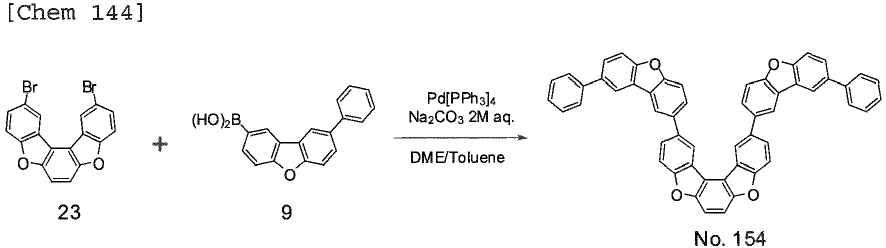

Compound 23 (2.5 g, 6.0 mmol), Compound 9 (3.8 g, 13.2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 154 (not according to the invention)) was obtained. The powder was purified by sublimation, whereby a white solid was obtained in an amount of 0.9 g in 20% yield.
FD-MS analysis C₅₄H₃₀O₄: theoretical value 742, observed value 742

### Synthesis Example 12 (Synthesis of Compound No. 157 (not according to the invention))

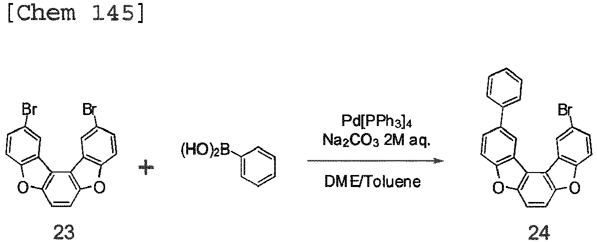

Compound 23 (16.8 g, 40 mmol), phenylboronic acid (4.8 g, 40 mmol), a 2 M aqueous solution of Na₂CO₃ (80 mL, 160 mmol), DME (80 mL), toluene (80 mL), and Pd[PPh₃]₄ (2.3 g, 2.0 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (150 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 9.1 g in 55% yield.
FD-MS analysis C₂₄H₁₃BrO₂: theoretical value 413, observed value 413

### (2) Synthesis of Compound 25

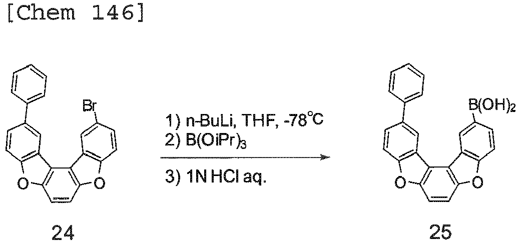

Compound 24 (9.0 g, 21.8 mmol) and THF (220 mL) were loaded into a three-necked flask, and the mixture was cooled to -78°C. Then, n-BuLi (1.65 M solution in n-hexane, 14.5 mL, 23.9 mmol) was added dropwise to the flask, and the resultant mixture was stirred at -78°C for 20 minutes. Triisopropyl borate (12.3 g, 65.3 mmol) was added to the resultant, and the mixture was stirred at -78°C for 1 hour. After that, the resultant was left to stand overnight at room temperature. Then, 1 N HCl (100 mL) was charged into the resultant, and the mixture was stirred at room temperature for 1 hour. The resultant sample was concentrated, and was then transferred to a separating funnel. Water (100 mL) was charged into the funnel, and the mixture was extracted with CH₂Cl₂. The extract was dried with MgSO₄, and was then filtered and concentrated. The resultant sample was purified by recrystallization (toluene-hexane), whereby a white solid was obtained in an amount of 4.1 g in 50% yield.

### (3) Synthesis of Compound No. 157 (not according to the invention)

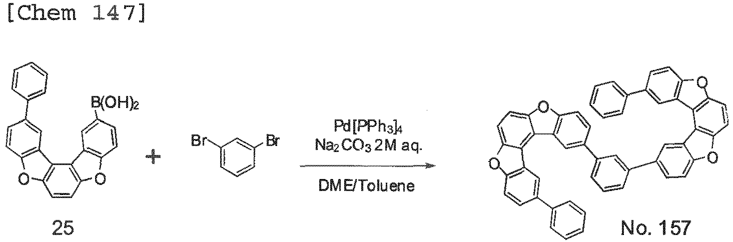

Compound 25 (4.0 g, 10.6 mmol), 1,3-dibromobenzene (1.2 g, 5.3 mmol), a 2 M aqueous solution of Na₂CO₃ (11 mL, 22 mmol), DME (11 mL), toluene (11 mL), and Pd[PPh₃]₄ (0.31 g, 0.27 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 157 (not according to the invention)) was obtained. The powder was purified by sublimation, whereby a white solid was obtained in an amount of 0.63 g in 16% yield.
FD-MS analysis C₅₄H₃₀O₄: theoretical value 742, observed value 742

### Synthesis Example 13 (Synthesis of Compound No. 239 (not according to the invention))

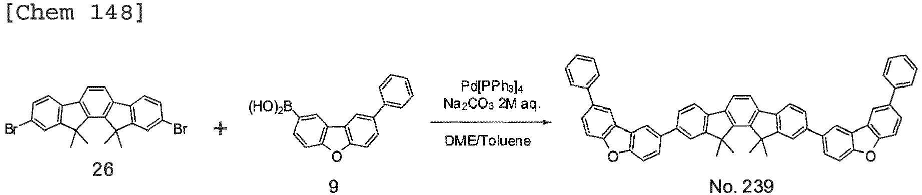

Compound 26 (2.8 g, 6.0 mmol), Compound 9 (3.8 g, 13.2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 239 (not according to the invention)) was obtained. The powder was purified by sublimation, whereby a white solid was obtained in an amount of 2.1 g in 45% yield.
FD-MS analysis C₆₀H₄₂O₂: theoretical value 794, observed value 794

### Synthesis Example 14 (Synthesis of Compound No. 249)

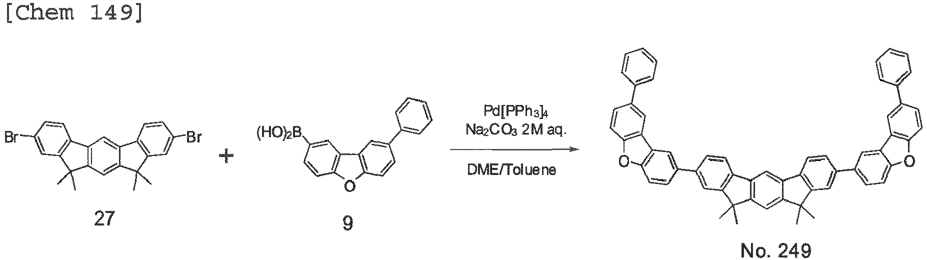

Compound 27 (2.8 g, 6.0 mmol), Compound 9 (3.8 g, 13.2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 249) was obtained. The powder was purified by sublimation, whereby a white solid was obtained in an amount of 2.5 g in 52% yield.
FD-MS analysis C₆₀H₄₂O₂: theoretical value 794, observed value 794

### Synthesis Example 15 (Synthesis of Compound No. 259)

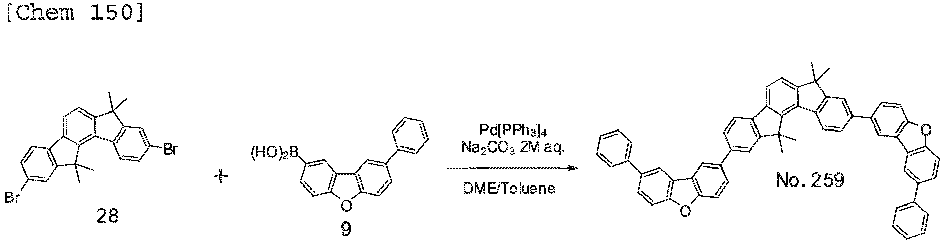

Compound 28 (2.8 g, 6.0 mmol), Compound 9 (3.8 g, 13. 2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 259) was obtained. The powder was purified by sublimation, whereby a white solid was obtained in an amount of 1.9 g in 40% yield.
FD-MS analysis C₆₀H₄₂O₂: theoretical value 794, observed value 794

### Synthesis Example 16 (Synthesis of Compound No. 269 (not according to the invention))

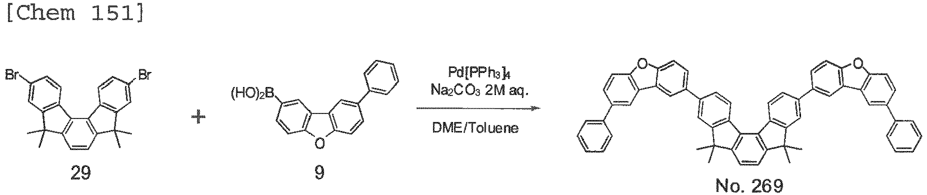

Compound 29 (2.8 g, 6.0 mmol), Compound 9 (3.8 g, 13. 2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 269 (not according to the invention)) was obtained. The powder was purified by sublimation, whereby a white solid was obtained in an amount of 1.0 g in 21% yield.
FD-MS analysis C₆₀H₄₂O₂: theoretical value 794, observed value 794

### Synthesis Example 17 (Synthesis of Compound No. 233 (not according to the invention))

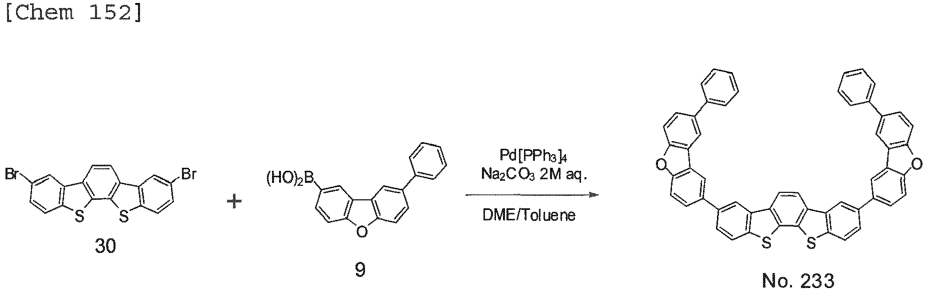

Compound 30 (2.7 g, 6.0 mmol), Compound 9 (3.8 g, 13.2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 233 (not according to the invention)) was obtained. The powder was purified by sublimation, whereby a white solid was obtained in an amount of 1.9 g in 40% yield.
FD-MS analysis C₅₄H₃₀O₂S₂: theoretical value 774, observed value 774

### Synthesis Example 18 (Synthesis of Compound No. 243)

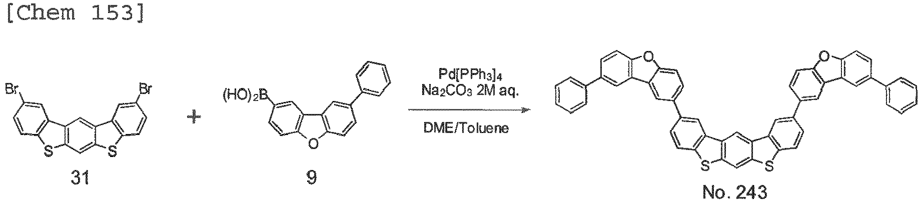

Compound 31 (2.7 g, 6.0 mmol), Compound 9 (3.8 g, 13.2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 243) was obtained. The powder was purified by sublimation, whereby a white solid was obtained in an amount of 2.1 g in 45% yield.
FD-MS analysis C₅₄H₃₀O₂S₂: theoretical value 774, observed value 774

### Synthesis Example 19 (Synthesis of Compound No. 253)

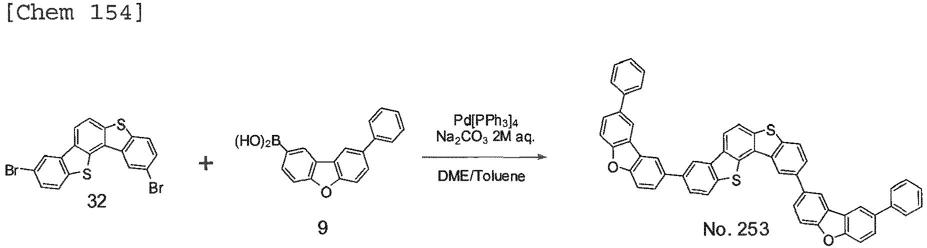

Compound 32 (2.7 g, 6.0 mmol), Compound 9 (3.8 g, 13.2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 253) was obtained. The powder was purified by sublimation, whereby a white solid was obtained in an amount of 1.8 g in 38% yield. FD-MS analysis C₅₄H₃₀O₂S₂: theoretical value 774, observed value 774

### Synthesis Example 20 (Synthesis of Compound No. 263 (not according to the invention))

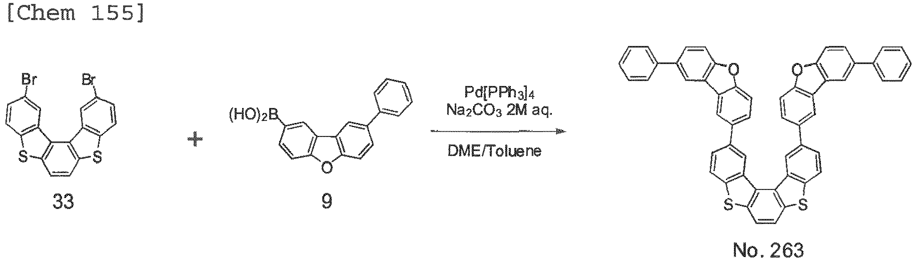

Compound 33 (2.7 g, 6.0 mmol), Compound 9 (3.8 g, 13.2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 263 (not according to the invention)) was obtained. The powder was purified by sublimation, whereby a white solid was obtained in an amount of 0.6 g in 13% yield.
FD-MS analysis C₅₄H₃₀O₂S₂: theoretical value 774, observed value 774

### Synthesis Example 21 (Synthesis of Compound No. 272 (not according to the invention))

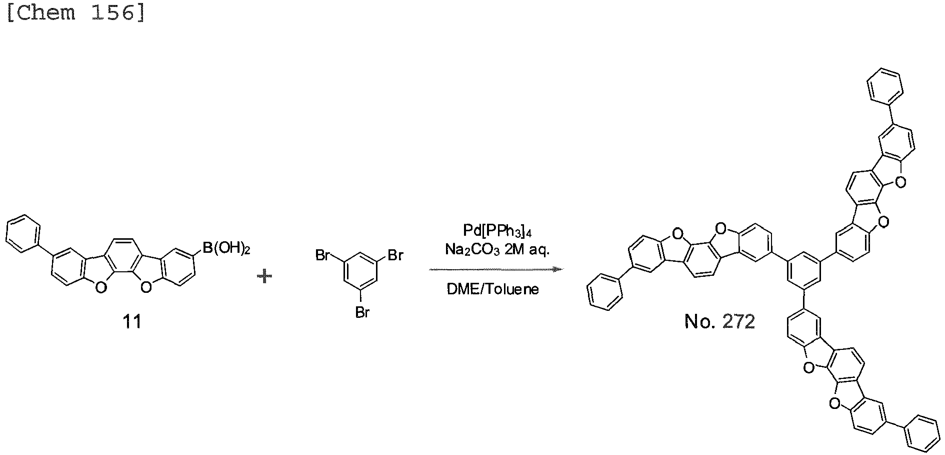

Compound 11 (8.3 g, 21.9 mmol), 1,3,5-tribromobenzene (2.3 g, 7.3 mmol), a 2 M aqueous solution of Na₂CO₃ (22.5 mL, 45 mmol), DME (15 mL), toluene (15 mL), and Pd[PPh₃]₄ (0.63 g, 0.56 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 272 (not according to the invention)) was obtained. The powder was purified by sublimation, whereby a white solid was obtained in an amount of 1.0 g in 13% yield.
FD-MS analysis C₇₈H₄₂O₆: theoretical value 1075, observed value 1075

### Synthesis Example 22 (Synthesis of Compound No. 273)

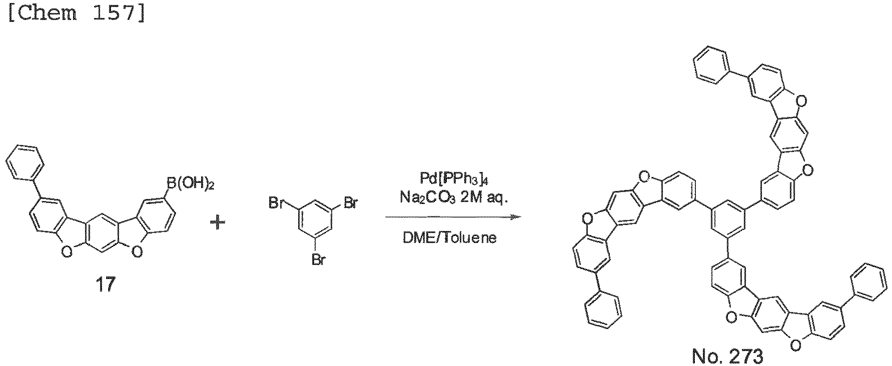

Compound 17 (8.3 g, 21.9 mmol), 1,3,5-tribromobenzene (2.3 g, 7.3 mmol), a 2 M aqueous solution of Na₂CO₃ (22.5 mL, 45 mmol), DME (15 mL), toluene (15 mL), and Pd[PPh₃]₄ (0.63 g, 0.56 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 273) was obtained. The powder was purified by sublimation, whereby a white solid was obtained in an amount of 1.2 g in 15% yield.
FD-MS analysis C₇₈H₄₂O₆: theoretical value 1075, observed value 1075

### Synthesis Example 23 (Synthesis of Compound No. 274)

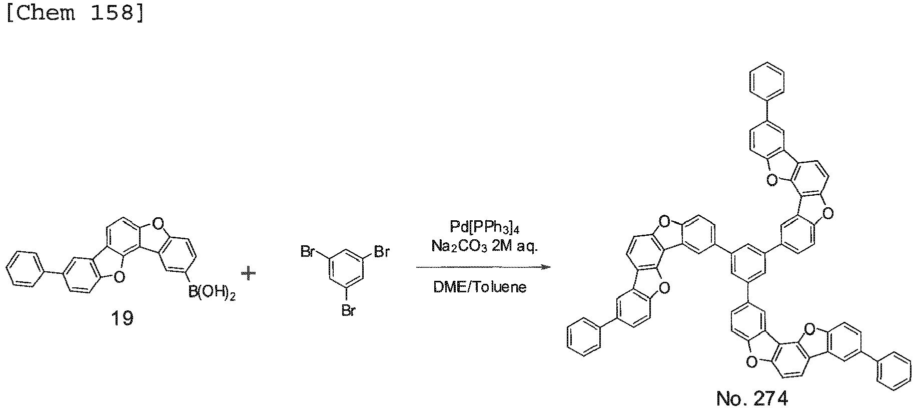

Compound 19 (8.3 g, 21.9 mmol), 1,3,5-tribromobenzene (2.3 g, 7.3 mmol), a 2 M aqueous solution of Na₂CO₃ (22.5 mL, 45 mmol), DME (15 mL), toluene (15 mL), and Pd[PPh₃]₄ (0.63 g, 0.56 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 274) was obtained. The powder was purified by sublimation, whereby a white solid was obtained in an amount of 0.94 g in 12% yield.
FD-MS analysis C₇₈H₄₂O₆: theoretical value 1075, observed value 1075

### Synthesis Example 24 (Synthesis of Compound No. 276 (not according to the invention))

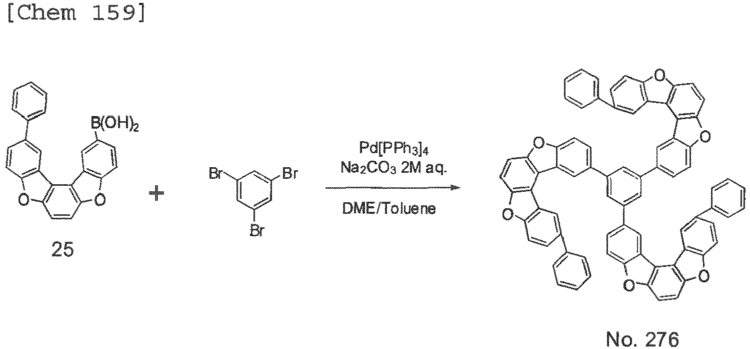

Compound 25 (8.3 g, 21.9 mmol), 1,3,5-tribromobenzene (2.3 g, 7.3 mmol), a 2 M aqueous solution of Na₂CO₃ (22.5 mL, 45 mmol), DME (15 mL), toluene (15 mL), and Pd[PPh₃]₄ (0.63 g, 0.56 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 276 (not according to the invention)) was obtained. The powder was purified by sublimation, whereby a white solid was obtained in an amount of 0.55 g in 7% yield. FD-MS analysis C₇₈H₄₂O₆: theoretical value 1075, observed value 1075

### Synthesis Example 25 (Synthesis of Compound No. 1 (not according to the invention))

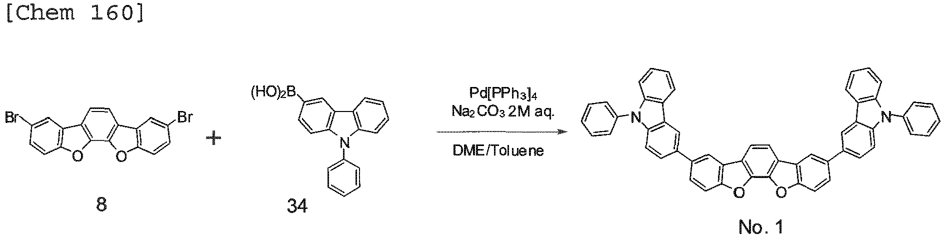

Compound 8 (2.5 g, 6.0 mmol), Compound 34 (3.8 g, 13.2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 1 (not according to the invention) was obtained. The powder was purified by sublimation, whereby a white solid was obtained in an amount of 1.2 g in 27% yield.
FD-MS analysis C₅₄H₃₂N₂O₂: theoretical value 740, observed value 740

### Synthesis Example 26 (Synthesis of Compound No. 92 (not according to the invention))

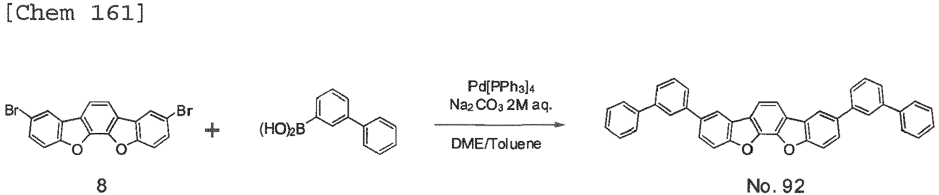

Compound 8 (2.5 g, 6.0 mmol), 3-biphenylboronic acid (2.6 g, 13.2 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 92 (not according to the invention)) was obtained. The powder was purified by sublimation, whereby a white solid was obtained in an amount of 1.1 g in 32% yield.
FD-MS analysis C₄₂H₂₆O₂: theoretical value 562, observed value 562

### Synthesis Example 27 (Synthesis of Compound No. 108)

### (1) Synthesis of Compound 35

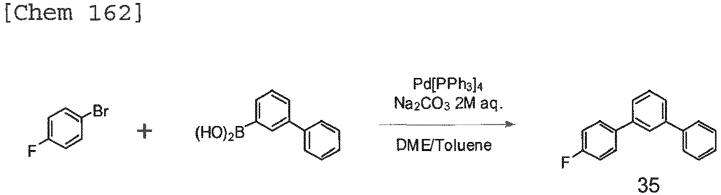

4-bromofluorobenzene (81.55 g, 466 mmol), 3-biphenylboronic acid (92.33 g, 466 mmol), a 2 M aqueous solution of Na₂CO₃ (530 mL), Pd[PPh₃]₄ (14.7 g, 12.7 mmol), DME (500 mL), and toluene (500 mL) were loaded into a three-necked flask, and the mixture was refluxed under an Ar atmosphere for 6 hours.

After the completion of the reaction, the resultant was cooled to room temperature. The resultant sample was transferred to a separating funnel, and water (350 mL) was charged into the funnel. Then, the mixture was extracted with toluene. The extract was dried with MgSO₄, and was then filtered and concentrated. The resultant sample was purified by silica gel column chromatography. The purified product was concentrated to dryness, and was then recrystallized, whereby a white solid was obtained in an amount of 31.7 g in 27% yield.
FD-MS analysis C₁₈H₁₃F: theoretical value 248, observed value 248

### (2) Synthesis of Compound 36

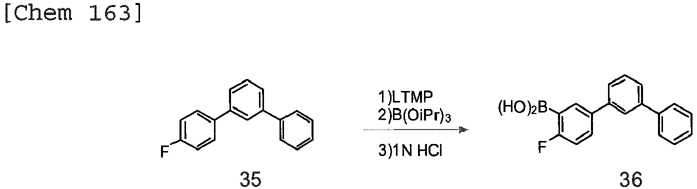

Compound 35 (31.7 g, 127 mmol) and THF (200 mL) were loaded into a three-necked flask, and then the mixture was cooled to -78°C. A lithium 2,2,6,6-tetramethylpiperidine solution prepared in advance from a 1.55-M solution of n-BuLi in hexane (82 mL, 127 mmol), 2,2,6,6-tetramethylpiperidine (17.9 g, 127 mmol), and THF (50 mL) was added to the mixture, and then the whole was stirred at -78°C under an Ar atmosphere for 2 hours. After that, triisopropyl borate (71.6 g, 381 mmol) was added to the resultant, and then the mixture was stirred at -78°C for 2 hours. After that, the temperature of the mixture was slowly returned to room temperature, and then the mixture was left to stand overnight.

After the completion of the reaction, methanol (50 mL) was added to deactivate the resultant, and then the mixture was concentrated so that its volume might be reduced by about half. CH₂Cl₂ (200 mL) and 2N HCl (120 mL) were added to the resultant, and then the mixture was stirred at room temperature for 2 hours. The resultant sample was transferred to a separating funnel and extracted with CH₂Cl₂. The solution was dried with MgSO₄, and was then purified by silica gel column chromatography. The purified product was concentrated to dryness, and then the solid was subjected to dispersion washing with toluene. Thus, a white solid was obtained in an amount of 27.5 g in 74% yield.

(2) Synthesis of Compound 37

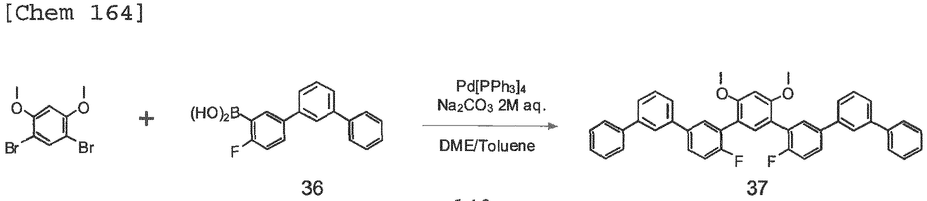

2,4-dibromo-1,5-dimethoxybenzene (5.92 g, 20 mmol), Compound 36 (14.02 g, 48 mmol), a 2 M aqueous solution of Na₂CO₃ (40 mL), Pd[PPh₃]₄ (1.15 g, 1 mmol), DME (20 mL), and toluene (20 mL) were loaded into a three-necked flask, and the mixture was refluxed under an Ar atmosphere for 36 hours.

After the completion of the reaction, the resultant was cooled to room temperature. The resultant sample was transferred to a separating funnel, and water (100 mL) was charged into the funnel. Then, the mixture was extracted with toluene. The extract was dried with MgSO₄, and was then filtered and concentrated. The resultant sample was purified by silica gel column chromatography. The purified product was concentrated to dryness, and was then recrystallized, whereby a white solid was obtained in an amount of 9.49 g in 75% yield.
FD-MS analysis C₄₄H₃₂F₂O₂: theoretical value 630, observed value 630

### (4) Synthesis of Compound 38

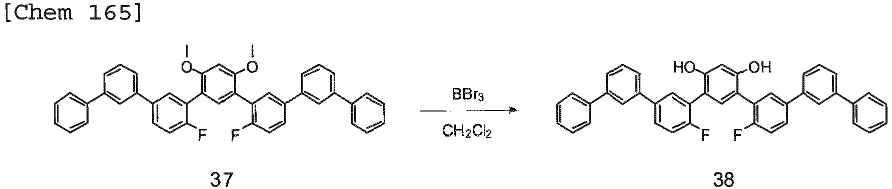

Compound 37 (9.49 g, 5 mmol) and CH₂Cl₂ (75 mL) were loaded into a three-necked flask, and the mixture was cooled to 0°C. Then, BBr₃ (15.03 g, 60 mmol) was added to the flask, and the resultant mixture was stirred under an Ar atmosphere at room temperature for 24 hours.

After the completion of the reaction, the obtained solution was cooled to -78°C, and inactivated using methanol (50 mL) and water (100 mL). The sample was transferred to a separating funnel, and water (100 mL) was charged into the funnel. Then, the mixture was extracted with CH₂Cl₂. The extract was dried with MgSO₄, and was then filtered and concentrated. The obtained sample was purified by silica gel column chromatography. The purified product was concentrated to dryness, whereby a white solid was obtained in an amount of 9.04 g in 100% yield.
FD-MS analysis C₄₂H₂₈F₂O₂: theoretical value 602, observed value 602

### (5) Synthesis of Compound No. 108

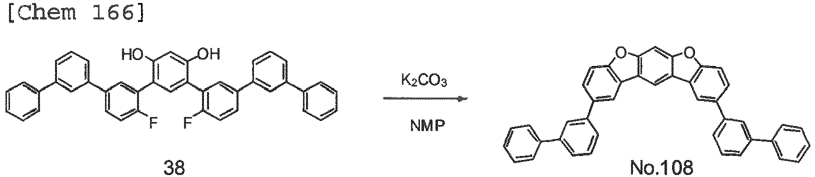

Compound 38 (9.04g, 15 mmol) andNMP (150 mL) were loaded into a three-necked flask, and Compound 38 was dissolved completely. K₂CO₃ (8.30 g, 60 mmol) was added to the flask, followed by stirring at 200°C for 2 hours.

After the completion of the reaction, the obtained solution was cooled to room temperature. Toluene (1.5 L) was added to the resultant sample, and the sample was transferred to a separating funnel and washed with water. The resultant was dried with MgSO₄ and then filtered and concentrated. The resultant sample was purified by silica gel column chromatography. The purified product was concentrated to dryness, and was then recrystallized, whereby a white powder (Compound No. 108) was obtained. The powder was purified by sublimation, whereby a white solid was obtained in an amount of 3.37 g in 40% yield.
FD-MS analysis C₄₂H₂₆O₂: theoretical value 562, observed value 562

### Synthesis Example 28 (Synthesis of Compound No. 281)

### (1) Synthesis of Compound 39

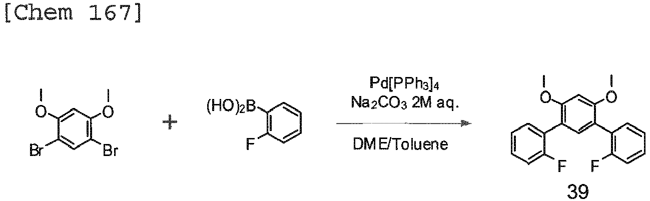

2,4-dibromo-1,5-dimethoxybenzene (88.8 g, 300 mmol), 2-fluorophenyl boric acid (100.7 g, 720 mmol), 2 M aqueous solution of Na₂CO₃ (600 mL), Pd[PPh₃]₄ (6.73 g, 6 mmol), DME (150 mL), and toluene (150 mL) were loaded into a three-necked flask, and the mixture was refluxed under an Ar atmosphere for 36 hours.

After the completion of the reaction, the obtained solution was cooled to room temperature. The resultant sample was transferred to a separating funnel, and water (500 mL) was charged into the funnel. Then, the mixture was extracted with toluene. The extract was dried with MgSO₄, and was then filtered and concentrated. The resultant sample was purified by silica gel column chromatography. The purified product was concentrated to dryness, and was then recrystallized, whereby a white solid was obtained in an amount of 86.5 g in 88% yield.
FD-MS analysis C₂₀H₁₆F₂O₂: theoretical value 326, observed value 326

### (2) Synthesis of Compound 40

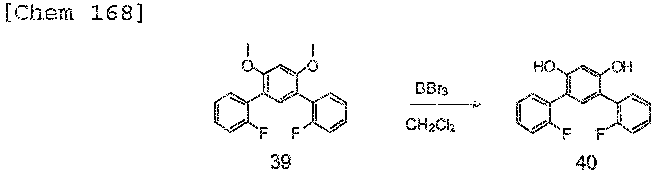

Compound 39 (48.3 g, 148 mmol) and CH₂Cl₂ (740 mL) were loaded into a three-necked flask, and the mixture was cooled to 0°C. Then, BBr₃ (89.0 g, 355 mmol) was added to the flask, and the resultant mixture was stirred at room temperature for 24 hours.

After the completion of the reaction, the obtained solution was cooled to -78°C, and inactivated using methanol (50 mL) and water (100 mL). The sample was transferred to a separating funnel. Then, the mixture was extracted with CH₂Cl₂. The extract was dried with MgSO₄, and was then filtered and concentrated. The obtained sample was purified by silica gel column chromatography. The purified product was concentrated to dryness, whereby a white solid was obtained in an amount of 44.14 g in 100% yield.
FD-MS analysis C₁₈H₁₂F₂O₂: theoretical value 298, observed value 298

### (3) Synthesis of Compound 41

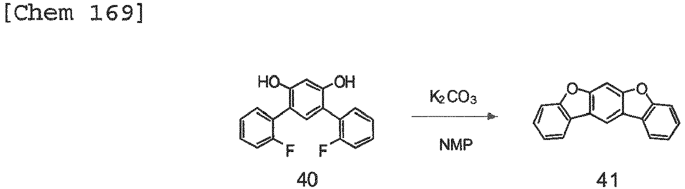

Compound 40 (44.14 g, 148 mmol) and NMP (888 mL) were loaded into a three-necked flask, and Compound 40 was dissolved completely. K₂CO₃ (81.8 g, 592 mmol) was added to the flask, followed by stirring at 200°C for 2 hours.

After the completion of the reaction, the obtained solution was cooled to room temperature. The sample was transferred to a separating funnel, toluene (2L) was added, and the resultant was washed with water. The resultant was dried with MgSO₄ and then filtered and concentrated. The resultant sample was purified by silica gel column chromatography. The purified product was concentrated to dryness, and was then recrystallized, whereby a white solid was obtained in an amount of 27.9 g in 73% yield.
FD-MS analysis C₁₈H₁₀O₂: theoretical value 258, observed value 258

### (4) Synthesis of Compound 42

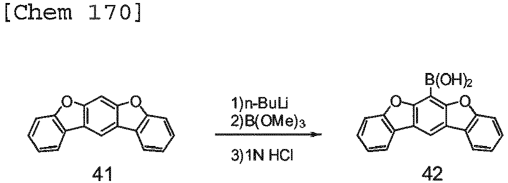

Compound 41 (12.9 g, 50 mmol) andTHF (300 mL) were loaded into a three-necked flask, and the mixture was cooled to -78°C. Then, n-BuLi (2.63 M solution in hexane, 20.0 ml, 52.5 mmol) was added to the flask, and the resultant mixture was stirred at room temperature under an Ar atmosphere for 1 hour. Next, the resultant was cooled to -78°C again, trimethyl borate (10.4 g, 100 mmol) was added to the resultant. After being stirred at -78°C for 10 minutes, the mixture was stirred at room temperature for 1 hour.

After the completion of the reaction, the resultant was concentrated to about the half volume. 1 N HCl (200 mL) was added to the concentrated resultant, followed by stirring at room temperature for 1 hour. The resultant sample was transferred to a separating funnel, and the mixture was extracted with ethyl acetate. The extract was dried with MgSO₄, and was then concentrated. The concentrated product was subjected to dispersion washing with a toluene/hexane mixed solvent, whereby a white solid was obtained in an amount of 13.7 g in 91% yield.

### (5) Synthesis of Compound No. 281

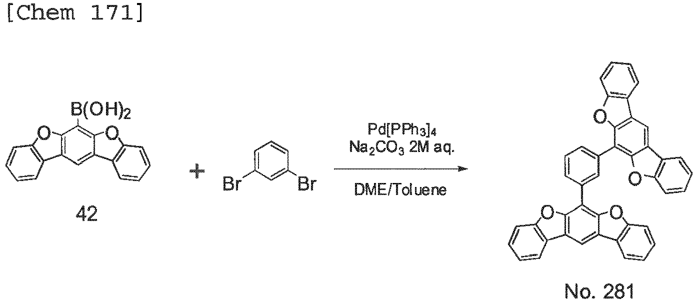

Compound 42 (3.8 g, 12.6 mmol), 1, 3-dibromobenzene (1.4 g, 6.0 mmol), a 2 M aqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 281) was obtained. The powder was purified by sublimation, whereby a white solid was obtained in an amount of 1.3 g in 37% yield.
FD-MS analysis C₄₂H₂₂O₄: theoretical value 590, observed value 590

### Synthesis Example 29 (Synthesis of Compound No. 284)

### (1) Synthesis of Compound 43

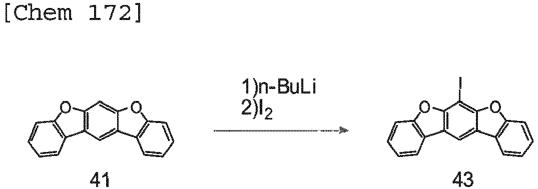

Compound 41 (2.69 g, 10.4 mmol) and THF (62 mL) were loaded into a three-necked flask, and the mixture was cooled to -78°C. Then, n-BuLi (1.66 M solution in hexane, 6.6 ml, 10.9 mmol) was added to the flask, and the resultant mixture was stirred at room temperature for 30 minutes. Next, the resultant was cooled to -78°C again, I₂ (2.69 g, 10.6 mmol) was added to the resultant. After being stirred at -78°C for 10 minutes, the mixture was stirred at room temperature for 1 hour.

After the completion of the reaction, water (30 mL) was added to inactivate the obtained solution, followed by concentration. After being subjected to dispersion washing with water, the sample was collected by filtration and then dissolved in toluene. After being dried with MgSO₄, the resultant was filtered and concentrated. The obtained sample was purified by silica gel column chromatography. The purified product was concentrated to dryness, and was then recrystallized, whereby a white solid was obtained in an amount of 3.77 g in 94% yield.
FD-MS analysis C₁₈H₉IO₂: theoretical value 384, observed value 384

### (2) Synthesis of Compound No. 284

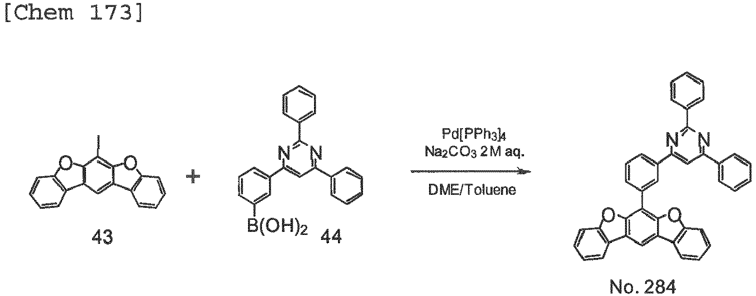

Compound 43 (2.3 g, 6.0 mmol), Compound 44 (2.2 g, 6.3 mmol), a 2 M aqueous solution of Na₂CO₃ (12 mL, 24 mmol), DME (12 mL), toluene (12 mL), and Pd[PPh₃]₄ (0.35 g, 0.30 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 284) was obtained. The powder was purified by sublimation, whereby a white solid was obtained in an amount of 1.0 g in 30% yield. FD-MS analysis C₄₀H₂₄N₂O₂: theoretical value 564, observed value 564

### Synthesis Example 30 (Synthesis of Compound No. 306 (not according to the invention))

### (1) Synthesis of Compound 46

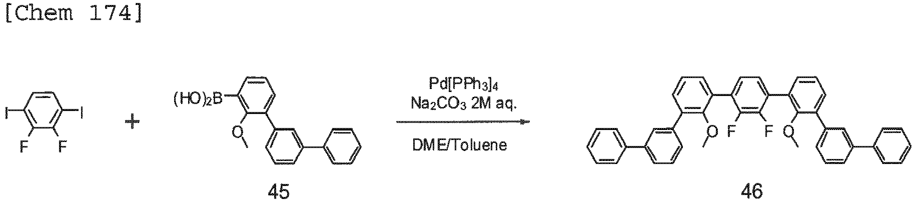

1,2-difluoro-3,6-diiodobenzene (7.3 g, 20.0 mmol), Compound 45 (12.8 g, 42.0 mmol), a 2 M aqueous solution of Na₂CO₃ (40 mL, 80.0 mmol), DME (40 mL), toluene (40 mL), and Pd[PPh₃]₄ (1.2 g, 1.0 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (100 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 8.2 g in 65% yield.
FD-MS analysis C₄₄H₃₂F₂O₂: theoretical value 630, observed value 630

### (2) Synthesis of Compound 47

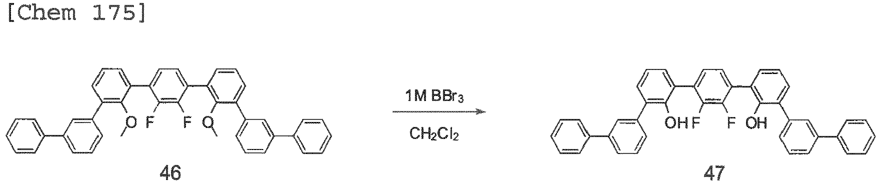

Compound 46 (8.2 g, 13.0 mmol), a 1 M solution of BBr₃ in CH₂Cl₂ (32 mL, 32.0 mmol), and CH₂Cl₂ (100 mL) were loaded into a three-necked flask, and the mixture was stirred under an Ar atmosphere at 0°C for 8 hours. After that, the mixture was left to stand at room temperature overnight.

After the completion of the reaction, the resultant was neutralized with a saturated aqueous solution of NaHCO₃. The resultant sample was transferred to a separating funnel, and was extracted with CH₂Cl₂. The resultant sample was purified by silica gel column chromatography, whereby a white solid was obtained in an amount of 7.6 g in 97% yield.
FD-MS analysis C₄₂H₂₈F₂O₂: theoretical value 602, observed value 602

### (3) Synthesis of Compound No. 306 (not according to the invention)

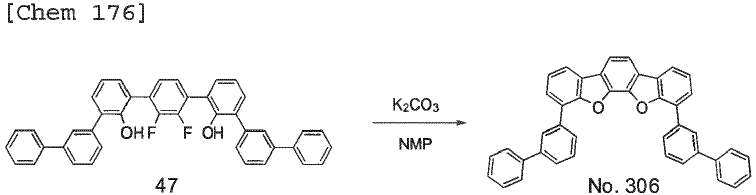

Compound 47 (7.6 g, 12.6 mmol), K₂CO₃ (7.0 g, 50.4 mmol), and NMP (50 mL) were loaded into a three-necked flask, and the mixture was stirred under an Ar atmosphere at 200°C for 3 hours.

After the completion of the reaction, the resultant was cooled to room temperature. Toluene (500 mL) was charged into the resultant sample. The mixture was transferred to a separating funnel, and was washed with water. The washed product was dried with MgSO₄, and was then filtered and concentrated. The resultant sample was purified by silica gel column chromatography. The purified product was concentrated to dryness, and was then recrystallized twice, whereby a white powder (Compound No. 306 (not according to the invention)) was obtained. The powder was purified by sublimation, whereby a white solid was obtained in an amount of 2.9 g in 41% yield.
FD-MS analysis C₄₂H₂₆O₂: theoretical value 562, observed value 562

### Synthesis Example 31 (Synthesis of Compound No. 329)

### (1) Synthesis of Compound 48

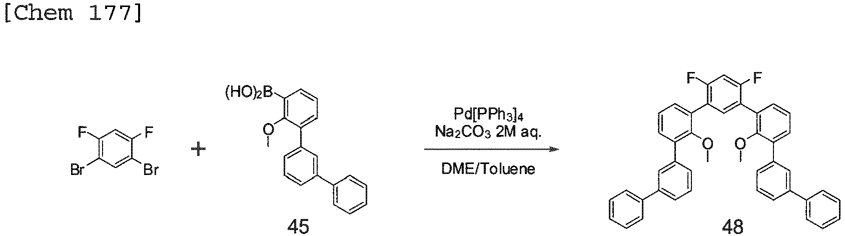

1,3-dibromo-4,6-difluorobenzene (5.4 g, 20.0 mmol), Compound 45 (12.8 g, 42.0 mmol), a 2 M aqueous solution of Na₂CO₃ (40 mL, 80.0 mmol), DME (40 mL), toluene (40 mL), and Pd[PPh₃]₄ (1.2 g, 1.0 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (100 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 7.2 g in 57% yield.
FD-MS analysis C₄₄H₃₂F₂O₂: theoretical value 630, observed value 630

### (2) Synthesis of Compound 49

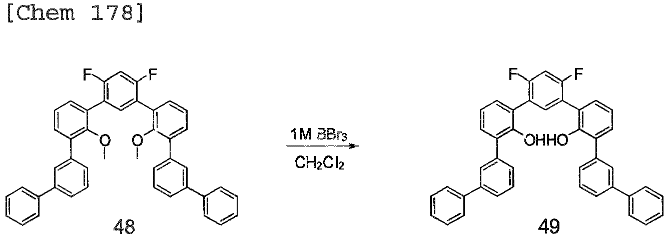

Compound 46 (7.2 g, 11.4 mmol), a 1 M solution of BBr₃ in CH₂Cl₂ (28 mL, 28.0 mmol), and CH₂Cl₂ (85 mL) were loaded into a three-necked flask, and the mixture was stirred under an Ar atmosphere at 0°C for 8 hours. After that, the mixture was left to stand at room temperature overnight.

After the completion of the reaction, the resultant was neutralized with a saturated aqueous solution of NaHCO₃. The resultant sample was transferred to a separating funnel, and was extracted with CH₂Cl₂. The resultant sample was purified by silica gel column chromatography, whereby a white solid was obtained in an amount of 6.9 g in 100% yield.
FD-MS analysis C₄₂H₂₈F₂O₂: theoretical value 602, observed value 602

### (3) Synthesis of Compound No. 329

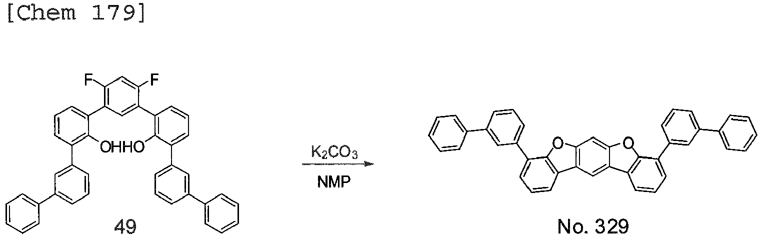

Compound 47 (6.9 g, 11.4 mmol), K₂CO₃ (6.3 g, 45.6 mmol), and NMP (50 mL) were loaded into a three-necked flask, and the mixture was stirred under an Ar atmosphere at 200°C for 3 hours.

After the completion of the reaction, the resultant was cooled to room temperature. Toluene (500 mL) was charged into the resultant sample. The mixture was transferred to a separating funnel, and was washed with water. The washed product was dried with MgSO₄, and was then filtered and concentrated. The resultant sample was purified by silica gel column chromatography. The purified product was concentrated to dryness, and was then recrystallized twice, whereby a white powder (Compound No. 329) was obtained. The powder was purified by sublimation, whereby a white solid was obtained in an amount of 2.3 g in 36% yield.
FD-MS analysis C₄₂H₂₆O₂: theoretical value 562, observed value 562

### Synthesis Example 32 (Synthesis of Compound No. 477)

### (1) Synthesis of Compound 50

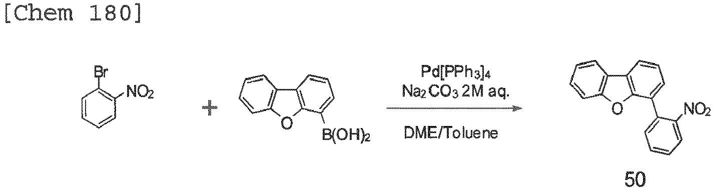

Compound 2-bromonitrobenzene (25.0 g, 123.8 mmol), 4-dibenzofuranboronic acid (31.5 g, 148.5 mmol), a 2 M aqueous solution of Na₂CO₃ (124 mL, 248 mmol), DME (250 mL), toluene (250 mL), and Pd [PPh₃]₄ (7.2 g, 6.2 mmol) were loaded into a three-necked flask, and the mixture was refluxed under an Ar atmosphere for 12 hours.

After the completion of the reaction, the resultant was cooled to room temperature. The resultant sample was transferred to a separating funnel, and water (500 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 24.0 g in 67% yield. FD-MS analysis C₁₈H₁₁NO₃: theoretical value 289, observed value 289

### (2) Synthesis of Compound 51

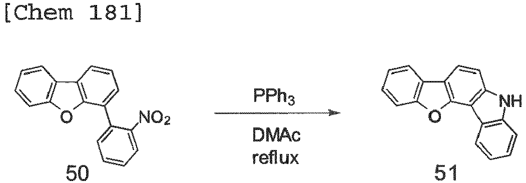

Compound 50 (24.0 g, 83.0 mmol), triphenylphosphine (54.4 g, 207.4 mmol), and dimethylacetamide (166 mL) were loaded into a three-necked flask, and the mixture was refluxed under an Ar atmosphere for 20 hours.

After the completion of the reaction, the resultant sample was cooled to room temperature. The resultant sample was transferred to a separating funnel, and water (400 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 14.5 g in 68% yield.
FD-MS analysis C₁₈H₁₁NO: theoretical value 257, observed value 257

### (3) Synthesis of Compound No. 477

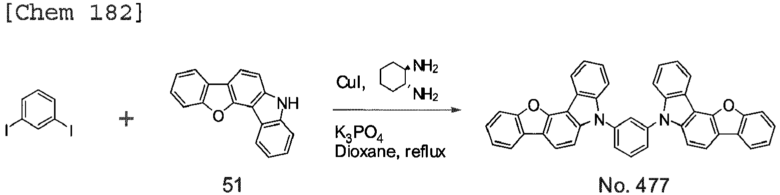

1, 3-diiodobenzene (3.3 g, 10. 0 mmol), Compound 51 (6.2 g, 24.0 mmol), CuI (1.9 g, 10 mmol), trans-cyclohexane-1,2-diamine (3.4 g, 30 mmol), K₃PO₄ (8.5 g, 40 mmol), and 1,4-dioxane (15 mL) were loaded into a three-necked flask, and the mixture was refluxed under an Ar atmosphere for 10 hours.

After the completion of the reaction, the resultant was cooled to room temperature. The resultant sample was transferred to a separating funnel, and water (100 mL) was charged into the funnel. Then, the mixture was extracted with CH₂Cl₂. The resultant sample was purified by column chromatography. The purified product was concentrated to dryness, and was then recrystallized twice, whereby a white powder (Compound No. 477) was obtained. The powder was purified by sublimation, whereby a white solid was obtained in an amount of 1.8 g in 30% yield.
FD-MS analysis C₄₂H₂₄N₂O₂: theoretical value 588, observed value 588

### Synthesis Example 33 (Synthesis of Compound No. 76 (not according to the invention))

### (1) Synthesis of Compound 52

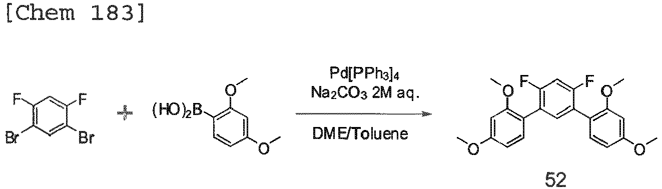

1,3-dibromo-4,6-difluorobenzene (6.8 g, 25.0 mmol), 2,4 -dimethoxyphenylboronic acid (10.0 g, 54. 9 mmol), and a 2 Maqueous solution of Na₂CO₃ (62 mL, 124 mmol), DME (60 mL), toluene (60 mL), and Pd [PPh₃]₄ (2.9 g, 2.5 mmol) were loaded into a three-necked flask, and the mixture was refluxed under an Ar atmosphere for 12 hours.

After the completion of the reaction, the resultant was cooled to room temperature. The resultant sample was transferred to a separating funnel, and water (500 mL) was charged into the funnel. Then, the mixture was extracted with toluene. The extract was dried with MgSO₄, and was then filtered and concentrated. The resultant sample was purified by silica gel column chromatography, whereby a white solid was obtained in an amount of 8.0 g in 83% yield.
FD-MS analysis C₂₂H₂₀F₂O₄: theoretical value 386, observed value 386

### (2) Synthesis of Compound 53

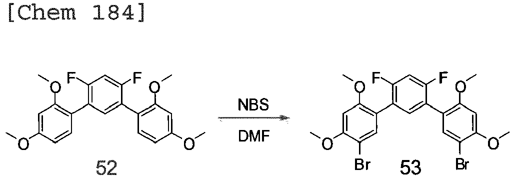

Compound 52 (8.0 g, 20.8 mmol), NBS (7.4 g, 41.6 mmol), and DMF (200 mL) were loaded into a three-necked flask, and the mixture was stirred under an Ar atmosphere at room temperature for 20 hours. After the completion of the reaction, the resultant sample was transferred to a separating funnel, and water (500mL) was charged into the funnel. Then, the mixture was extracted with AcOEt. The resultant sample was purified by column chromatography, whereby a white solid was obtained in an amount of 7.4 g in 65% yield.
FD-MS analysis C₂₂H₁₈Br₂F₂O₄: theoretical value 544, observed value 544

### (3) Synthesis of Compound 55

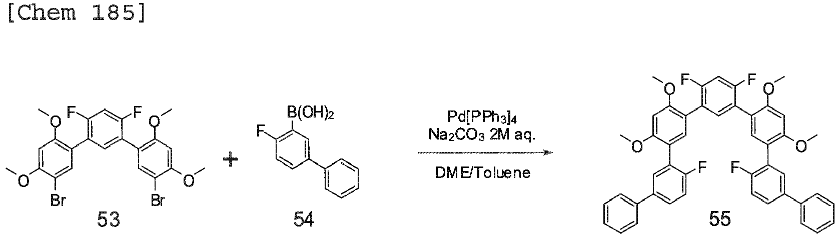

Compound 53 (7.4 g, 13.6 mmol), Compound 54 (12.8 g, 32.6 mmol), a 2 M aqueous solution of Na₂CO₃ (28 mL, 56.0 mmol), DME (28 mL), toluene (28 mL), and Pd[PPh₃]₄ (0.79 g, 0.68 mmol) were loaded into a three-necked flask, and the mixture was refluxed under an Ar atmosphere for 20 hours.

After the completion of the reaction, the resultant was cooled to room temperature. The resultant sample was transferred to a separating funnel, and water (300 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 6.2 g in 63% yield.
FD-MS analysis C₄₆H₃₄F₄O₄: theoretical value 726, observed value 726

### (4) Synthesis of Compound 56

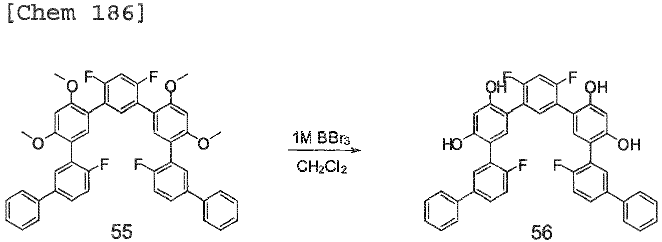

Compound 55 (6.2 g, 8.5 mmol), a 1 M solution of BBr₃ in CH₂Cl₂ (20.5 mL, 20.5 mmol), and CH₂Cl₂ (85 mL) were loaded into a three-necked flask, and the mixture was stirred under an Ar atmosphere at 0°C for 8 hours. After that, the mixture was left to stand at room temperature overnight.

After the completion of the reaction, the resultant was neutralized with a saturated aqueous solution of NaHCO₃. The resultant sample was transferred to a separating funnel, and was extracted with CH₂Cl₂. The resultant sample was purified by silica gel column chromatography, whereby a white solid was obtained in an amount of 5.1 g in 90% yield.
FD-MS analysis C₄₂H₂₆F₄O₄: theoretical value 670, observed value 670

### (5) Synthesis of Compound No. 76 (not according to the invention)

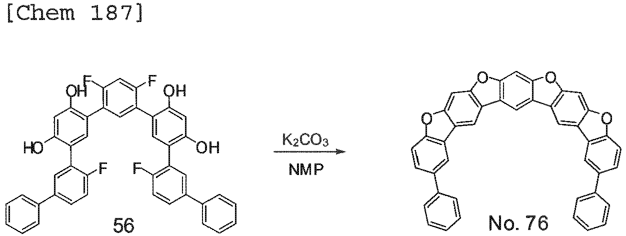

Compound 56 (5.1 g, 7.6 mmol), K₂CO₃ (4.2 g, 30.4 mmol), and NMP (80 mL) were loaded into a three-necked flask, and the mixture was refluxed under an Ar atmosphere at 200 °C for 4 hours. After the completion of the reaction, the resultant was cooled to room temperature. The resultant sample was transferred to a separating funnel, and water (500 mL) was charged into the funnel. Then, the mixture was extracted with toluene. The resultant sample was purified by column chromatography. The purified product was concentrated to dryness, and was then recrystallized twice, whereby a white powder (Compound No. 76 (not according to the invention)) was obtained. The powder was purified by sublimation, whereby a white solid was obtained in an amount of 1.4 g in 31% yield.
FD-MS analysis C₄₂H₂₂O₄: theoretical value 590, observed value 590

### Synthesis Example 34 (Synthesis of Compound No. 517)

### (1) Synthesis of Compound No. 57

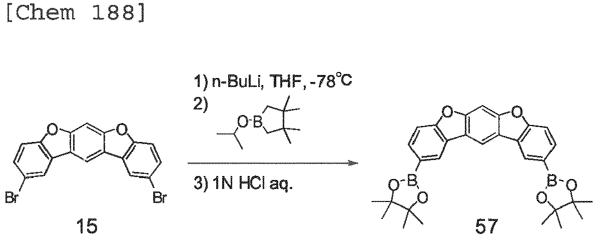

Under an Ar atmosphere, Compound 41 (5.0 g, 12.0 mmol) and THF (250 mL) were loaded into a three-necked flask, and then the mixture was cooled to -78°C. n-BuLi (2.64 M in hexane, 10.0 ml, 26.4 mmol) was added to the mixture, and then the whole was stirred at -78°C for 2 hours. Next, 2-isopropoxy-4,4,5,5-tetramethyl-1,3,2-dioxaborolane (5.59 g, 30.0 mmol) was added to the resultant, and then the mixture was stirred at -78°C for 2 hours. After that, the mixture was left to stand at room temperature overnight.

After the completion of the reaction, water (100 mL) was added to the mixture, and then the whole was stirred at room temperature for 1 hour. The reaction solution was concentrated so that THF might be removed by distillation. After that, the resultant sample was transferred to a separating funnel and extracted with CH₂Cl₂. The solution was dried with MgSO₄, and was then concentrated. The sample was recrystallized from toluene. Thus, a white solid was obtained in an amount of 2.77 g in 45% yield.

### (2) Synthesis of Compound No. 517

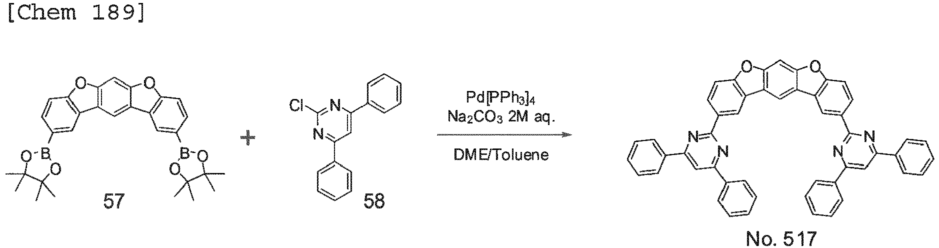

Compound 57 (2.77 g, 5.43 mmol), Compound 58 (3.48 g, 13.0 mmol), a 2 M aqueous solution of Na₂CO₃ (26 mL, 52 mmol), DME (25 mL), toluene (25 mL), and Pd[PPh₃]₄ (0.75 g, 0.65 mmol) were loaded into a three-necked flask, and the mixture was refluxed under an Ar atmosphere for 12 hours.

After the completion of the reaction, the resultant was cooled to room temperature. The resultant sample was transferred to a separating funnel, and water (500 mL) was charged into the funnel. Then, the mixture was extracted with toluene . The extract was dried with MgSO₄, and was then filtered and concentrated. The resultant sample was purified by silica gel column chromatography. The purified product was concentrated to dryness, and was then recrystallized twice, whereby a white powder (Compound No. 517) was obtained. The powder was purified by sublimation, whereby a white solid was obtained in an amount of 1.3 g in 33% yield.
FD-MS analysis C₅₀H₃₀N₄O₂: theoretical value 718, observed value 718

### Synthesis Example 35 (Synthesis of Compound No. 589)

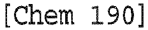

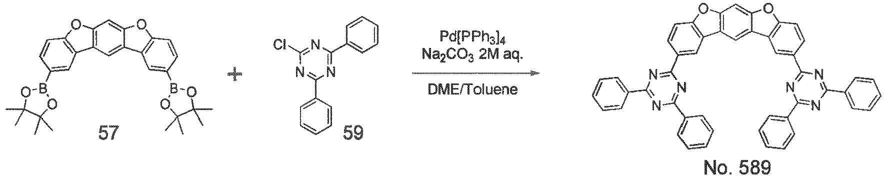

Compound 57 (2.77 g, 5.43 mmol), Compound 59 (3.48 g, 13.0 mmol), a 2 M aqueous solution of Na₂CO₃ (26 mL, 52 mmol), DME (25 mL), toluene (25 mL), and Pd[PPh₃]₄ (0.75 g, 0.65 mmol) were loaded into a three-necked flask, and the mixture was refluxed under an Ar atmosphere for 12 hours.

After the completion of the reaction, the resultant was cooled to room temperature. The resultant sample was transferred to a separating funnel, and water (500 mL) was charged into the funnel. Then, the mixture was extracted with toluene. The extract was dried with MgSO₄, and was then filtered and concentrated. The resultant sample was purified by silica gel column chromatography. The purified product was concentrated to dryness, and was then recrystallized twice, whereby a white powder (Compound No. 589) was obtained. The powder was purified by sublimation, whereby a white solid was obtained in an amount of 1.2 g in 30% yield.
FD-MS analysis C₄₈H₂₈N₆O₂: theoretical value 720, observed value 720

### Synthesis Example 36 (Synthesis of Compound No. 286) (not according to the invention)

### (1) Synthesis of Compound 60

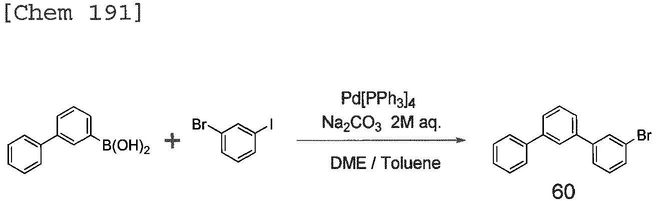

Compound 3-biphenylboronic acid (1.98 g, 10 mmol), 1-bromo-3-iodobenzene (2.83 g, 10 mmol), a 2 M aqueous solution of Na₂CO₃ (10 mL, 20 mmol), toluene (40 mL), and Pd[PPh₃]₄ (0.35 g, 0.30 mmol) were loaded into a three-necked flask, and the mixture was refluxed under an Ar atmosphere for 10 hours.

After the completion of the reaction, the resultant was cooled to room temperature. The resultant sample was transferred to a separating funnel, and water (500 mL) was charged into the funnel. Then, the mixture was extracted with toluene. The extract was dried with MgSO₄, and was then filtered and concentrated. The resultant sample was purified by silica gel column chromatography. The purified product was concentrated to dryness, whereby a colorless oily product was obtained in an amount of 2.9 g in 93% yield.
FD-MS analysis C₁₈H₁₃Br: theoretical value 309, observed value 309

### (2) Synthesis of Compound 61

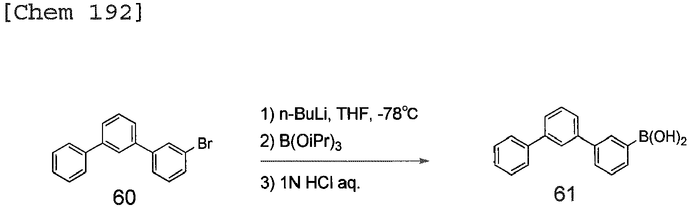

Compound 60 (2.9 g, 9.38 mmol) and THF (30 mL) were loaded into a three-necked flask under an Ar atmosphere, and the mixture was cooled to -78°C. Then, n-BuLi (1.59 M in n-hexane, 6.1 ml, 9.70 mmol) was added dropwise to the flask, and the resultant mixture was stirred at -78°C for 2 hours. Next, triisopropyl borate (5.45 g, 29.0 mmol) was added to the resultant, and the mixture was stirred at -78°C for 2 hours. After that, the resultant was left to stand at room temperature overnight. Then, 1 N HCl (20 mL) was charged into the resultant, and the mixture was stirred at room temperature for 1 hour. The resultant sample was concentrated, and was then transferred to a separating funnel. Water (100 mL) was charged into the funnel, and the mixture was extracted with CH₂Cl₂. The extract was dried with MgSO₄, and was then filtered and concentrated. The resultant sample was purified by recrystallization (toluene-hexane), whereby a white solid was obtained in an amount of 1.74 g in 68% yield.

### (3) Synthesis of Compound No. 286 (not according to the invention)

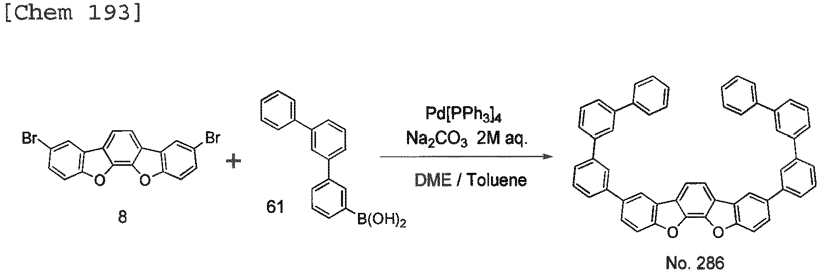

Compound 8 (1.20 g, 2.88 mmol), Compound 61 (1.74 g, 6.35 mmol), a 2 M aqueous solution of Na₂CO₃ (7.2 mL, 14.4 mmol), DME (10 mL), toluene (10 mL), and Pd[PPh₃]₄ (0.33 g, 0.29 mmol) were loaded into a three-necked flask, and the mixture was refluxed under an Ar atmosphere for 12 hours.

After the completion of the reaction, the resultant was cooled to room temperature. The resultant sample was transferred to a separating funnel, and water (500 mL) was charged into the funnel. Then, the mixture was extracted with toluene . The extract was dried with MgSO₄, and was then filtered and concentrated. The resultant sample was purified by silica gel column chromatography. The purified product was concentrated to dryness, and was then recrystallized twice, whereby a white powder (Compound No. 286 (not according to the invention)) was obtained. The powder was purified by sublimation, whereby a white solid was obtained in an amount of 0.9 g in 43% yield.
FD-MS analysis C₅₄H₃₄O₂: theoretical value 714, observed value 714

### Synthesis Example 37 (Synthesis of Compound No. 577) (1) Synthesis of Compound 62

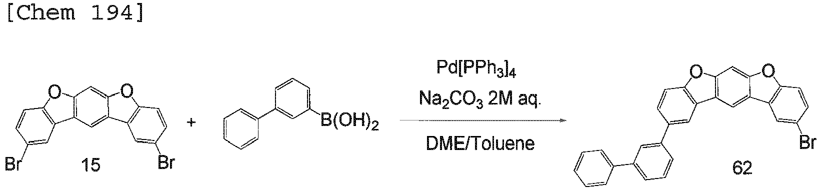

Compound 15 (16.6 g, 40 mmol), 3-biphenylboronic acid (7.9 g, 40 mmol), a 2 M aqueous solution of Na₂CO₃ (80 mL, 160 mmol), DME (120 mL), toluene (120 mL), and Pd[PPh₃]₄ (2.3 g, 2.0 mmol) were loaded into a three-necked flask, and the mixture was refluxed under an Ar atmosphere for 12 hours.

After the completion of the reaction, the resultant was cooled to room temperature. The resultant sample was transferred to a separating funnel, and water (200 mL) was charged into the funnel. Then, the mixture was extracted with toluene. The extract was dried with MgSO₄, and was then filtered and concentrated. The resultant sample was purified by silica gel column chromatography, whereby a white solid was obtained in an amount of 10.7 g in 54% yield. FD-MS analysis C₃₀H₁₇BrO₂: theoretical value 489, observed value 489

### (2) Synthesis of Compound No. 577

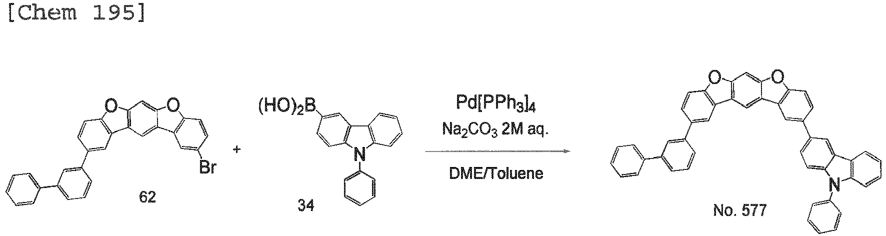

Compound 62 (4.89 g, 10 mmol), Compound 34 (3.16 g, 11 mmol), a 2 M aqueous solution of Na₂CO₃ (20 mL, 40 mmol), DME (100 mL), toluene (100 mL), and Pd[PPh₃]₄ (1.2 g, 1.0 mmol) were loaded into a three-necked flask, and the mixture was refluxed under an Ar atmosphere for 12 hours.

After the completion of the reaction, the resultant was cooled to room temperature. The resultant sample was transferred to a separating funnel, and water (200 mL) was charged into the funnel. Then, the mixture was extracted with toluene. The extract was dried with MgSO₄, and was then filtered and concentrated. The resultant sample was purified by silica gel column chromatography. The purified product was concentrated to dryness, and was then recrystallized twice, whereby a white powder (Compound No. 577) was obtained. The powder was purified by sublimation, whereby a white solid was obtained in an amount of 1.3 g in 20% yield. FD-MS analysis C₄₈H₂₉NO₂: theoretical value 651, observed value 651

### Synthesis Example 38 (Synthesis of Compound No. 294 (not according to the invention))

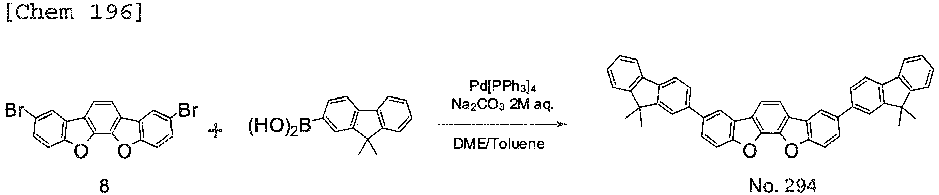

Compound 8 (2.5 g, 6.0 mmol), 9,9-dimethylfluorene-2-boronic acid (3.1 g, 13.2 mmol), a 2 Maqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 294 (not according to the invention) was obtained. The powder was purified by sublimation, whereby a white solid was obtained in an amount of 1.1 g in 30% yield.
FD-MS analysis C₄₈H₃₄O₂: theoretical value 642, observed value 642

### Synthesis Example 39 (Synthesis of Compound No. 304)

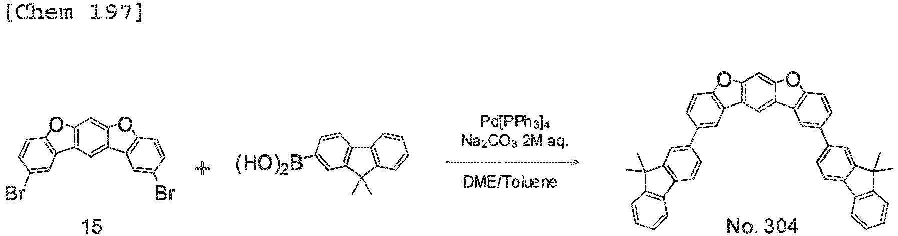

Compound 15 (2.5 g, 6.0 mmol), 9,9-dimethylfluorene-2-boronicacid (3.1g, 13.2 mmol), a 2 Maqueous solution of Na₂CO₃ (12 mL, 24 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.

After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 304) was obtained. The powder was purified by sublimation, whereby a white solid was obtained in an amount of 1.3 g in 35% yield.
FD-MS analysis C₄₈H₃₄O₂: theoretical value 642, observed value 642

An apparatus and measurement conditions adopted for field desorption mass spectrometry (FD-MS) in each of Synthesis Examples 1 to 39 are shown below.
Apparatus: HX110 (manufactured by JEOL Ltd.)
Conditions: accelerating voltage 8 kV
   scan range m/z=50 to 1500
   emitter kind: carbon
   emitter current: 0 mA→2 mA/min→40 mA (held for 10 minutes)

### Example 1 (not according to the invention)

### (Production of organic EL device)

A glass substrate provided with an ITO transparent electrode measuring 25 mm by 75 mm by 1.1 mm (manufactured by GEOMATEC Co. , Ltd.) was subjected to ultrasonic cleaning in isopropyl alcohol for 5 minutes. Further, the substrate was subjected to ultraviolet (UV)-ozone cleaning for 30 minutes.

The glass substrate provided with a transparent electrode thus cleaned was mounted on a substrate holder of a vacuum deposition apparatus. First, Compound A was deposited from the vapor onto the surface of the glass substrate on the side where a transparent electrode line was formed so as to cover the transparent electrode, whereby a hole transporting layer having a thickness of 30 nm was obtained.

Compound No. 11 (not according to the invention) as a host for phosphorescence and Ir (Ph-ppy)₃ as a dopant for phosphorescence were co-deposited from the vapor onto the hole transporting layer, whereby a phosphorescent layer having a thickness of 30 nm was obtained. The concentration of Ir(Ph-ppy)₃ was 5 mass%.

Subsequently, Compound B having a thickness of 10 nm, Compound C having a thickness of 20 nm, LiF having a thickness of 1 nm, and metal Al having a thickness of 80 nm were sequentially laminated on the phosphorescent layer, whereby a cathode was obtained. It should be noted that LiF as an electron injectable electrode was formed at a rate of 1 Å/min.

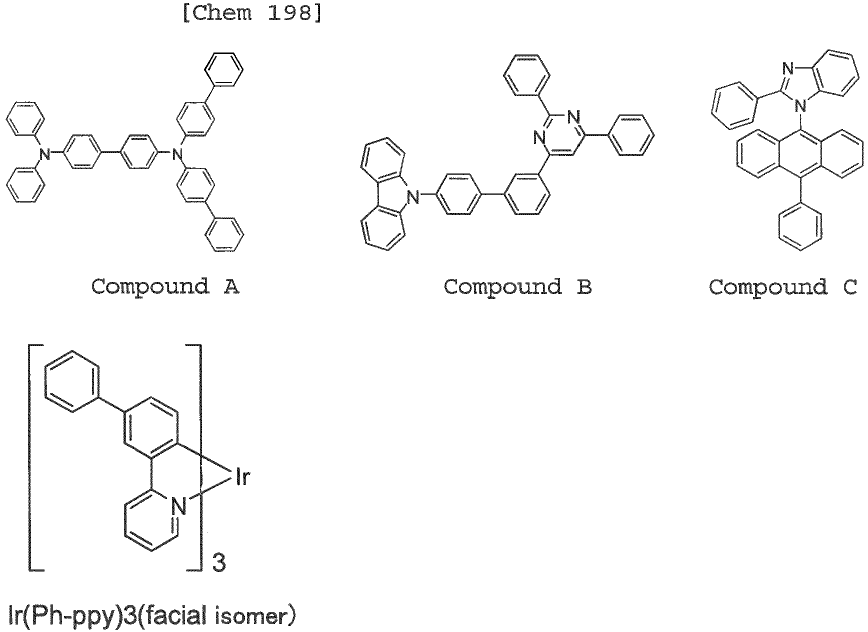

### (Evaluation of organic EL device for light emitting performance)

The organic EL device thus produced was caused to emit light by being driven with a direct current. The luminance (L) and the current density were measured. Then, the current efficiency (L/J) at a luminance of 1000 cd/m² was determined. Further, the lifetime of the device at a luminance of 20, 000 cd/m² was determined. Table 1 shows the results.

### Examples 2 to 34 (Examples 1 to 6, 11 to 14, 19 to 21, 24, 25, 30, 32 and 33 not according to the invention)

Organic EL devices were each produced in the same manner as in Example 1 except that a host material listed in Table 1 was used instead of Host Compound No. 11 (not according to the invention) in Example 1 (not according to the invention), and the devices were each evaluated in the same manner as in Example 1. Table 1 shows the results of the evaluation for light emitting performance.

### Comparative Examples 1 to 7

Organic EL devices were each produced in the same manner as in Example 1 except that the following compounds (a) to (g) described in EP 0908787 A were each used as a host material instead of Host Compound No. 11 (not according to the invention) in Example 1 (not according to the invention), and the devices were each evaluated in the same manner as in Example 1. Table 2 shows the results of the evaluation for light emitting performance.

### Comparative Examples 8 to 11

Organic EL devices were each produced in the same manner as in Example 1 except that the following compounds (h) to (k) described in WO 2006-122630 A1 were each used as a host material instead of Host Compound No. 11 (not according to the invention) in Example 1, and the devices were each evaluated in the same manner as in Example 1. Table 2 shows the results of the evaluation for light emitting performance.

### Comparative Examples 12 and 13

Organic EL devices were each produced in the same manner as in Example 1 except that the following compounds (1) and (m) described in WO 2007-063754 A1 were each used as a host material instead of Host Compound No. 11 (not according to the invention) in Example 1, and the devices were each evaluated in the same manner as in Example 1. Table 2 shows the results of the evaluation for light emitting performance.

### Comparative Example 14

An organic EL device was produced in the same manner as in Example 1 except that the following compound (n) described in US 2002-0132134 A and US 2003-0044646 A was used as a host material instead of Host Compound No. 11 (not according to the invention) in Example 1, and the device was evaluated in the same manner as in Example 1. Table 2 shows the results of the evaluation for light emitting performance.

### Comparative Example 15

An organic EL device was produced in the same manner as in Example 1 except that the following compound (o) described in JP 2008-81494 A was used as a host material instead of Host Compound No. 11 (not according to the invention) in Example 1, and the device was evaluated in the same manner as in Example 1. Table 2 shows the results of the evaluation for light emitting performance.

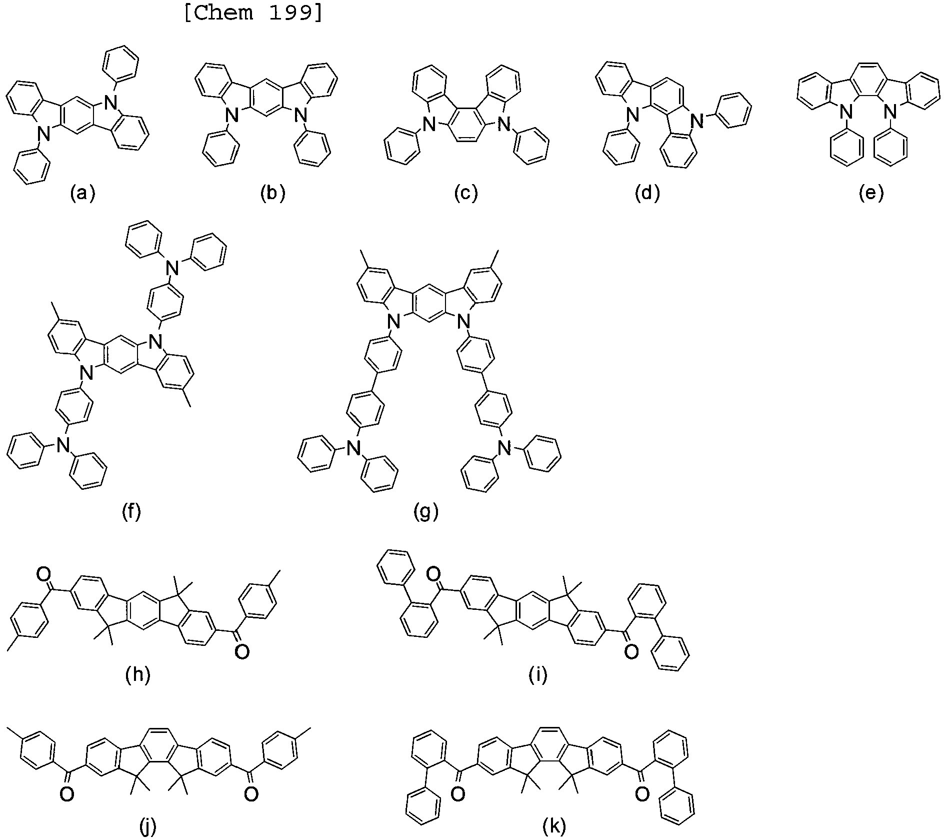

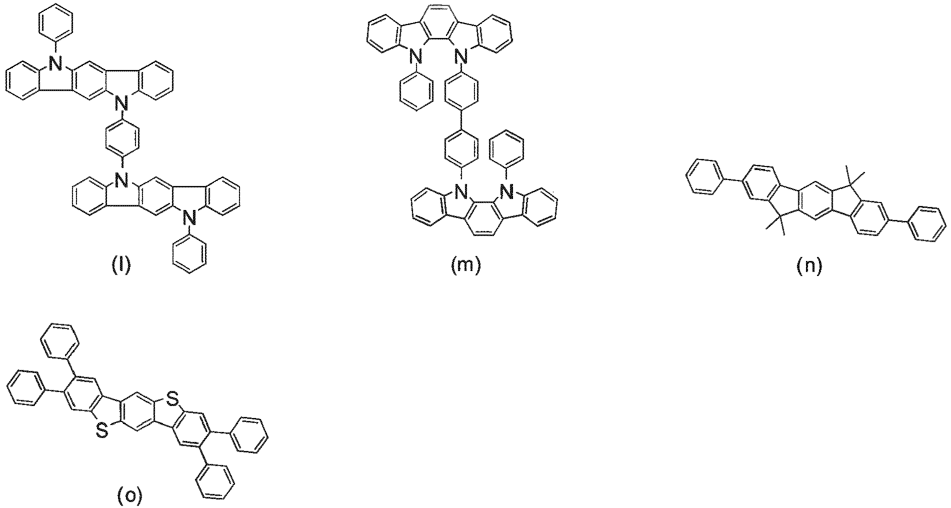

### [Table 1]

**Table 1**

| | Host Compound | Voltage (V) @20 mA/cm2 | Efficiency (cd/A) @1000 cd/m² | Lifetime (hr) @20,000 cd/m2 |
|---|---|---|---|---|
| Example 1* | (11) | 5.3 | 47.6 | 190 |
| Example 2* | (40) | 5.2 | 46.2 | 200 |
| Example 3* | (47) | 5.3 | 47.1 | 200 |
| Example 4* | (66) | 5.4 | 49.1 | 160 |
| Example 5* | (100) | 5.5 | 58.2 | 250 |
| Example 6* | (103) | 5.6 | 59.1 | 230 |
| Example 7 | (116) | 5.5 | 58.3 | 300 |
| Example 8 | (119) | 5.5 | 57.1 | 320 |
| Example 9 | (134) | 5.2 | 54.9 | 290 |
| Example 10 | (139) | 5.3 | 55.3 | 280 |
| Example 11* | (154) | 5.5 | 59.3 | 200 |
| Example 12* | (157) | 5.6 | 60.1 | 180 |
| Example 13* | (233) | 5.3 | 54.3 | 150 |
| Example 14* | (239) | 5.5 | 58.5 | 420 |
| Example 15 | (243) | 5.5 | 55.9 | 90 |
| Example 16 | (249) | 5.6 | 58.3 | 440 |
| Example 17 | (253) | 5.6 | 56.6 | 80 |
| Example 18 | (259) | 5.8 | 59.7 | 400 |
| Example 19* | (263) | 5.7 | 57.2 | 70 |
| Example 20* | (269) | 5.9 | 59.8 | 360 |
| Example 21* | (272) | 5.5 | 55.7 | 420 |
| Example 22 | (273) | 5.4 | 55.8 | 470 |
| Example 23 | (274) | 5.4 | 59.7 | 410 |
| Example 24* | (276) | 5.6 | 59.9 | 250 |
| Example 25* | (1) | 5.4 | 54.3 | 510 |
| Example 26 | (108) | 6.1 | 58.7 | 530 |
| Example 27 | (281) | 5.1 | 60.7 | 350 |
| Example 28 | (284) | 5.2 | 61.2 | 350 |
| Example 29 | (477) | 4.8 | 58.7 | 390 |
| Example 30* | (76) | 5.0 | 60.1 | 420 |
| Example 31 | (517) | 4.8 | 54.8 | 250 |
| Example 32* | (286) | 5.8 | 60.3 | 540 |
| Example 33* | (294) | 5.2 | 50.1 | 210 |
| Example 34 | (304) | 5.6 | 52.7 | 320 |

| | | | | |
|---|---|---|---|---|
| * not according to the invention | | | | |

### [Table 2]

**Table 2**

| | Host Compound | Voltage (V) @20 mA/cm2 | Efficiency (cd/A) @1000 cd/m² | Lifetime (hr) @20,000 cd/m² |
|---|---|---|---|---|
| Comparative Example 1 | (a) | 4.6 | 26.5 | 50 |
| Comparative Example 2 | (b) | 4.7 | 28.9 | 60 |
| Comparative Example 3 | (c) | 4.5 | 23.1 | 50 |
| Comparative Example 4 | (d) | 4.7 | 28.1 | 60 |
| Comparative Example 5 | (e) | 4.7 | 26.1 | 30 |
| Comparative Example 6 | (f) | 4.2 | 17.6 | 30 |
| Comparative Example 7 | (g) | 4.3 | 18.5 | 30 |
| Comparative Example 8 | (h) | 4.9 | 37.5 | 50 |
| Comparative Example 9 | (i) | 4.7 | 35.9 | 60 |
| Comparative Example 10 | (j) | 4.7 | 35.5 | 50 |
| Comparative Example 11 | (k) | 4.5 | 35.7 | 50 |
| Comparative Example 12 | (1) | 4.3 | 17.3 | 30 |
| Comparative Example 13 | (m) | 4.5 | 17.7 | 20 |
| Comparative Example 14 | (n) | 5.4 | 28.7 | 60 |
| Comparative Example 15 | (o) | 5.5 | 38.2 | 50 |

### Example 35

### (Production of organic EL device)

The glass substrate provided with a transparent electrode cleaned in the same manner as described above was mounted on a substrate holder of a vacuum deposition apparatus. First, Compound A was deposited from the vapor onto the surface of the glass substrate on the side where a transparent electrode line was formed so as to cover the transparent electrode, whereby a hole transporting layer having a thickness of 30 nm was obtained.

Compound No. 108 as a host for phosphorescence and Ir(Ph-ppy)₃ as a dopant for phosphorescence were co-deposited from the vapor onto the hole transporting layer, whereby a phosphorescent layer having a thickness of 30 nm was obtained. The concentration of Ir(Ph-ppy)₃ was 10 mass%.

Subsequently, Compound No. 92 (not according to the invention) having a thickness of 10 nm, Compound C having a thickness of 20 nm, LiF having a thickness of 1 nm, and metal A1 having a thickness of 80 nm were sequentially laminated on the phosphorescent layer, whereby a cathode was obtained. It should be noted that LiF as an electron injectable electrode was formed at a rate of 1 Å/min.

### (Evaluation of organic EL device for light emitting performance)

The organic EL device thus produced was caused to emit light by being driven with a direct current. The luminance (L) and the current density were measured. Then, the current efficiency (L/J) at a luminance of 1000 cd/m² was determined. Further, the lifetime of the device at a luminance of 20,000 cd/m² was determined. Table 3 shows the results.

### Examples 36 to 48 (Examples 36, 39, 40, 44 and 45 not according to the invention)

Organic EL devices were each produced in the same manner as in Example 35 except that a host compound and an electron transportable compound listed in Table 3 were used instead of Host Compound No. 108 and Electron Transportable Compound No. 92 (not according to the invention) in Example 35, and the devices were each evaluated in the same manner as in Example 35. Table 3 shows the results of the evaluation for light emitting performance.

### Comparative Example 16

An organic EL device was produced in the same manner as in Example 35 except that: CBP was used instead of Host Compound No. 108 in Example 35 ; andBAlq was used instead of Electron Transportable Compound No. 92 (not according to the invention) in Example 35. Then, the device was evaluated in the same manner as in Example 35. Table 3 shows the results of the evaluation for light emitting performance.

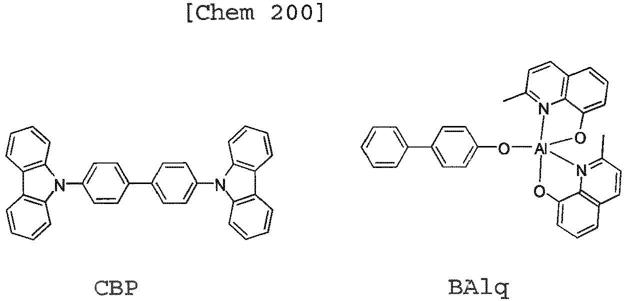

### [Table 3]

**Table 3**

| | Host Compound | Electron Transportable Compound | Voltage (V) @20 mA/cm2 | Efficiency (cd/A) @1000 cd/m2 | Lifetime (hr) @20, 000 cd/m2 |
|---|---|---|---|---|---|
| Example 35 | (108) | (92) | 5.8 | 60.2 | 580 |
| Example 36* | (108) | (306) | 5.7 | 62.1 | 560 |
| Example 37 | (108) | (108) | 6.0 | 63.7 | 600 |
| Example 38 | (108) | (329) | 5.9 | 62.3 | 550 |
| Example 39* | (1) | (92) | 4.8 | 55.6 | 550 |
| Example 40* | (103) | (92) | 5.5 | 66.1 | 590 |
| Example 41 | (119) | (92) | 5.7 | 68.9 | 570 |
| Example 42 | (108) | (517) | 4.8 | 62.1 | 560 |
| Example 43 | (1) | (517) | 4.7 | 59.7 | 600 |
| Example 44* | CBP | (92) | 5.7 | 48.5 | 100 |
| Example 45* | CBP | (306) | 5.6 | 49.6 | 120 |
| Example 46 | CBP | (108) | 5.9 | 48.2 | 100 |
| Example 47 | CBP | (329) | 5.8 | 49.3 | 120 |
| Example 48 | CBP | (517) | 5.2 | 44.2 | 150 |
| Comparative Example 16 | CBP | BAlq | 6.5 | 45.1 | 30 |

| | | | | | |
|---|---|---|---|---|---|
| * not according to the invention | | | | | |

Each of the organic EL devices of the comparative examples showed a lower current efficiency, was driven at a higher voltage, and had a shorter lifetime than those of each of the organic EL devices of the examples.

### Industrial Applicability

As described above in detail, the utilization of the polycyclic compound of the present invention as a material for an organic EL device can provide an organic EL device which shows high luminous efficiency, and has a long lifetime. Accordingly, the organic EL device of the present invention is extremely useful as, for example, a light source for various electronic instruments. In addition, the halogen compound of the present invention is suitable for an intermediate of the polycyclic compound. In addition, the polycyclic compound of the present invention can be effectively used also as a material for an organic electron device, and is extremely useful in an organic solar cell, organic semiconductor laser, a sensor using organic matter, or an organic TFT.

The following embodiments were originally disclosed, but are only according to the invention as far as specified in the claims:
[Item 1] A polycyclic compound represented by the following formula (1) or (2): 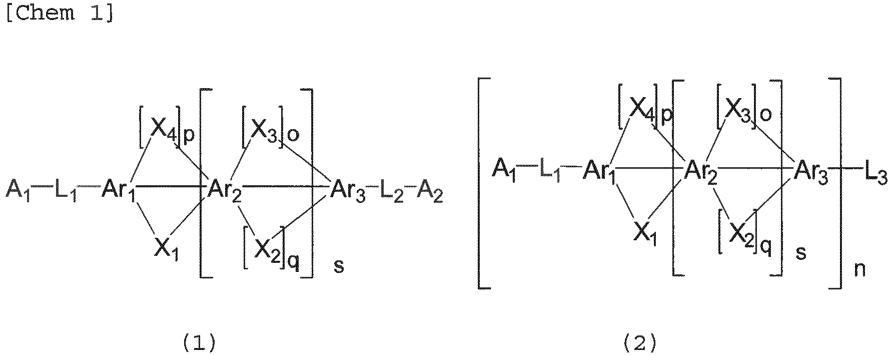 where:
   Ar₁, Ar₂, and Ar₃ each independently represent a substituted or unsubstituted aromatic hydrocarbon group having a ring formed of 6 carbon atoms or a substituted or unsubstituted aromatic heterocyclic group having a ring formed of 6 atoms, provided that, Ar₁, Ar₂, and Ar₃ each may have one substituent Y or a plurality of substituents Y's, in the case of the plurality of substituents Y's, the substituent Y's maybe different from eachother, Y represents an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with Ar₁, Ar₂, or Ar₃ through a carbon-carbon bond;
   X₁, X₂, X₃, and X₄ each independently represent oxygen (O), sulfur (S), N-R₁ or CR₂R₃;
   R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, when both X₁ and X₂ represent N-R₁, o and p each represent 0, and q represents 1, or when both X₁ and X₃ represent N-R₁, p and q each represent 0, and o represents 1, at least one R₁ represents a substituted or unsubstituted, monovalent fused aromatic heterocyclic group having a ring formed of 8 to 24 atoms;
   o, p, and q each represent 0 or 1, s represents 1, 2, or 3, and n represents 2, 3, or 4, and the compound represented by the formula (2) comprises a dimer using L₃ as a linking group for n=2, a trimer using L₃ as a linking group for n=3, or a tetramer using L₃ as a linking group for n=4;
   L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, monovalent or divalent aromatic heterocyclic group which has a ring formed of 3 to 24 atoms and which is linked with Ar₁ through a carbon-carbon bond;
   L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with Ar₃ through a carbon-carbon bond, provided that, when both X₁ and X₂ represent CR₂R₃, o and p each represent 0, q represents 1, and both L₁ and L₂ represent substituted or unsubstituted, divalent aromatic hydrocarbon groups each having a ring formed of 6 to 24 carbon atoms, or when both X₁ and X₃ represent CR₂R₃, p and q each represent 0, o represents 1, and both L₁ and L₂ represent substituted or unsubstituted, divalent aromatic hydrocarbon groups each having a rings formed of 6 to 24 carbon atoms, a case where L₁ and L₂ are simultaneously linked at para positions with respect to Ar₂ is excluded;
   when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with Ar₃ through a carbon-carbon bond, when n represents 3, L₃ represents a trivalent saturated hydrocarbon group having 1 to 20 carbonatoms, a substituted orunsubstituted, trivalent cyclic saturated hydrocarbon group having a ring formed of 3 to 20 carbon atoms, a trivalent silyl group, a silyl group, or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has a ring formed of 3 to 24 atoms and which is linked with Ar₃ through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with Ar₃ through a carbon-carbon bond, provided that, when both X₁ and X₂ represent CR₂R₃, o and p each represent 0, q represents 1, and both L₁ and L₃ represent substituted or unsubstituted, divalent, trivalent, or tetravalent aromatic hydrocarbon groups each having a ring formed of 6 to 24 carbon atoms, or when both X₁ and X₃ represent CR₂R₃, p and q each represent 0, o represents 1, and both L₁ and L₃ represent substituted or unsubstituted, divalent, trivalent, or tetravalent aromatic hydrocarbon groups each having a ring formed of 6 to 24 carbon atoms, a case where L₁ and L₃ are simultaneously linked at para positions with respect to Ar₂ is excluded;
   A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded;
   A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with L₂ through a carbon-carbon bond, provided that, when L₂ represents an alkyl or alkylene group having 1 to 20 carbon atoms, a case where A₂ represents a hydrogen atom is excluded, and, when X₁ and X₂ each represent oxygen (O), sulfur (S), or CR₂R₃, o and p each represent 0, q represents 1, both L₁ and L₂ represent single bonds, and both A₁ and A₂ represent hydrogen atom, or when X₁ and X₃ each represent oxygen (O), sulfur (S), or CR₂R₃, p and q each represent 0, o represents 1, both L₁ and L₂ represent single bonds, and both A₁ and A₂ represent hydrogen atom, Ar₂ has one substituent Y or a plurality of substituents Y's and a case where Y represents a methyl group or an unsubstituted phenyl group is excluded;
   A₁, A₂, L₁, L₂, and L₃ are each free of any carbonyl group;
   a case where the formula (1) has a structure represented by the following formula (3) is excluded; 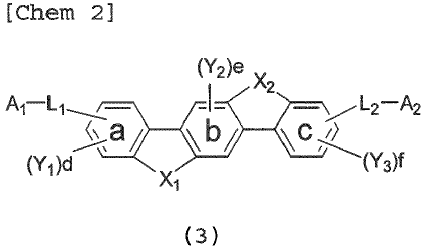
   provided that, X₁, X₂, A₁, A₂, L₁, and L₂ in the formula (3) has the same meaning of X₁, X₂, A₁, A₂, L₁, and L₂ in the formula (1), respectively;
   Y₁, Y₂, and Y₃ in the formula (3) each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with a benzene ring a, b, or c through a carbon-carbon bond, d and f each represent a number of 0, 1, 2, or 3, and e represents a number of 0, 1, or 2;
   a case where the formula (2) has a structure represented by the following formula (4) is excluded; 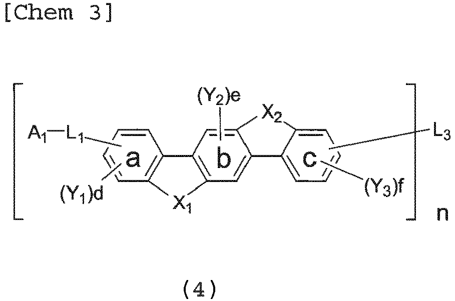
   provided that, X₁, X₂, A₁, L₁, L₃, and n in the formula (4) has the same meaning of X₁, X₂, A₁, L₁, L₃, and n in the formula (2), respectively; and
   Y₁, Y₂, and Y₃ in the formula (4) each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and whichis linked with the benzene ring a, b, or c through a carbon-carbon bond, d and f each represent a number of 0, 1, 2, or 3, and e represents a number of 0, 1, or 2.
[Item 2] The polycyclic compound according to item 1, wherein A₁ and/or A₂ each represent/represents a pyrimidyl group, triazyl group, or fluorenyl group having at least one substituent selected from a phenyl group, a biphenyl group, a terphenyl group, and a quaterphenyl group.
[Item 3] The polycyclic compound according to item 1, wherein A₁ and/or A₂ each represent/represents a carbazolyl group having at least one substituent selected from a phenyl group, a biphenyl group, a terphenyl group, and a quaterphenyl group.
[Item 4] The polycyclic compound according to item 1, wherein A₁ and/or A₂ each represent/represents a dibenzofuranyl group having at least one substituent selected from a phenyl group, a biphenyl group, a terphenyl group, and a quaterphenyl group.
[Item 5] The polycyclic compound according to item 1, wherein A₁ and/or A₂ each represent/represents an aromatic hydrocarbon group having a structure in which two or more benzene rings are bonded to each other at meta positions.
[Item 6] The polycyclic compound according to item 1, which is represented by one of the following formulae (5) and (6): 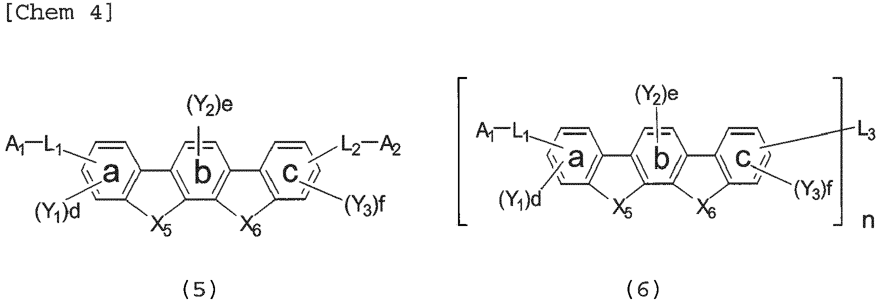 where:
   X₅ and X₆ each independently represent oxygen (O), sulfur (S), N-R₁, or CR₂R₃;
   R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, when both X₅ and X₆ represent N-R₁, at least one R₁ represents a substituted or unsubstituted, monovalent fused aromatic heterocyclic group having a ring formed of 8 to 24 atoms;
   n represents 2, 3, or 4, and the compound represented by the formula (6) comprises a dimer using L₃ as a linking group for n=2, a trimer using L₃ as a linking group for n=3, or a tetramer using L₃ as a linking group for n=4;
   L₁ represents a single bond, an alkylene group having 1 to 20 carbon atoms, a substituted or unsubstituted cycloalkylene group having a ring formed of 3 to 20 carbon atoms, a divalent silyl group or a divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
   L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond, provided that, when both X₅ and X₆ represent CR₂R₃ and both L₁ and L₂ represent substituted or unsubstituted, monovalent or divalent aromatic hydrocarbon groups each having a ring formed of 6 to 24 carbon atoms, a case where L₁ and L₂ are simultaneously linked at para positions with respect to a benzene ring b is excluded;
   when n represents 2, L₃ represents a single bond, an alkylene group having 1 to 20 carbon atoms, a substituted unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond, provided that, when both X₅ and X₆ represent CR₂R₃ and both L₁ and L₃ represent substituted or unsubstituted, divalent, trivalent, or tetravalent aromatic hydrocarbon groups each having a ring formed of 6 to 24 carbon atoms, a case where L₁ and L₃ are simultaneously linked at para positions with respect to the benzene ring b is excluded;
   A₁ represents a hydrogen atom, a substituted or unsubstituted cycloalkyl group having a ring formed of 3 to 20 carbon atoms, a silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkyl or alkylene group having 1 to 20 carbon atoms, a case where A₁ represents a hydrogen atom is excluded;
   A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
   Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of 0, 1, or 2, provided that, when X₅ and X₆ each represent oxygen (O), sulfur (S), or CR₂R₃, both L₁ and L₂ represent single bonds, and both A₁ and A₂ represent hydrogen atoms, the benzene ring b has one or two Y₂'s, and a case where Y₂ represents a methyl group or an unsubstituted phenyl group is excluded; and
   A₁, A₂, L₁, L₂, and L₃ are each free of any carbonyl group.
[Item 7] The polycyclic compound according to item 6, wherein the formula (5) is represented by one of the following formulae (5a) and (5b), and the formula (6) is represented by one of the following formulae (6a) and (6b): 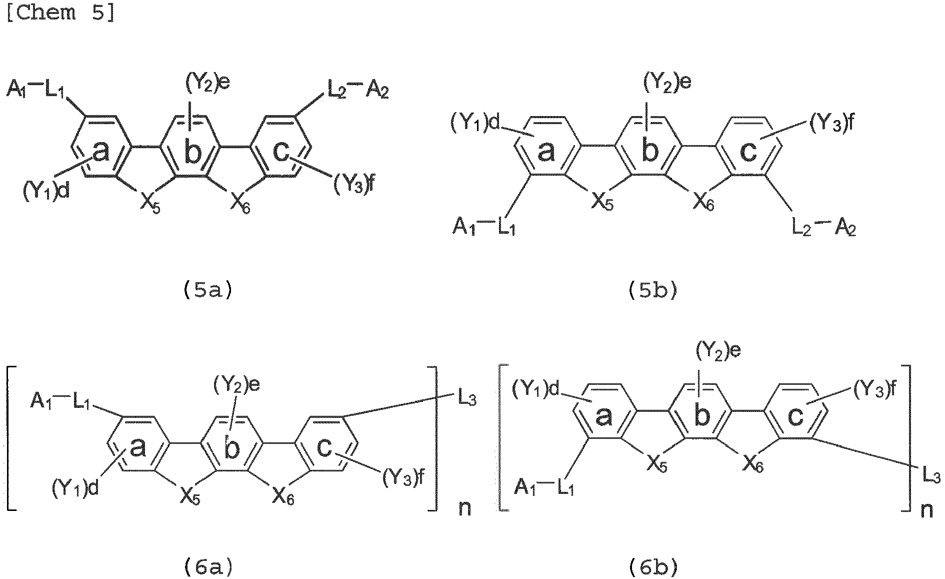 where X₅, X₆, L₁, L₂, L₃, A₁, A₂, Y₁, Y₂, Y₃, n, d, f, and e each have the same meaning as that described above.
[Item 8] The polycyclic compound according to item 1, wherein the polycyclic compound is represented by any one of the following formulae (7) to (12): 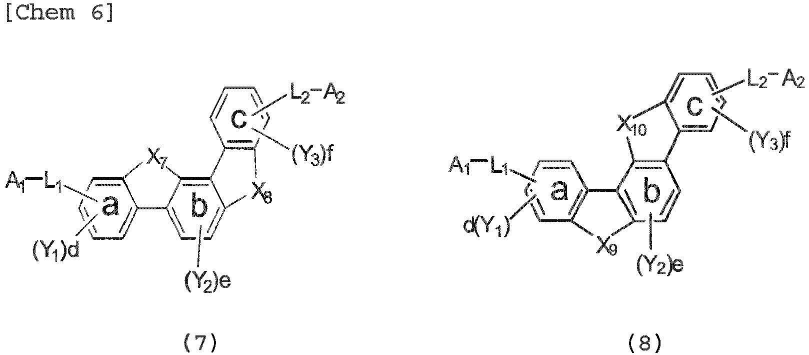 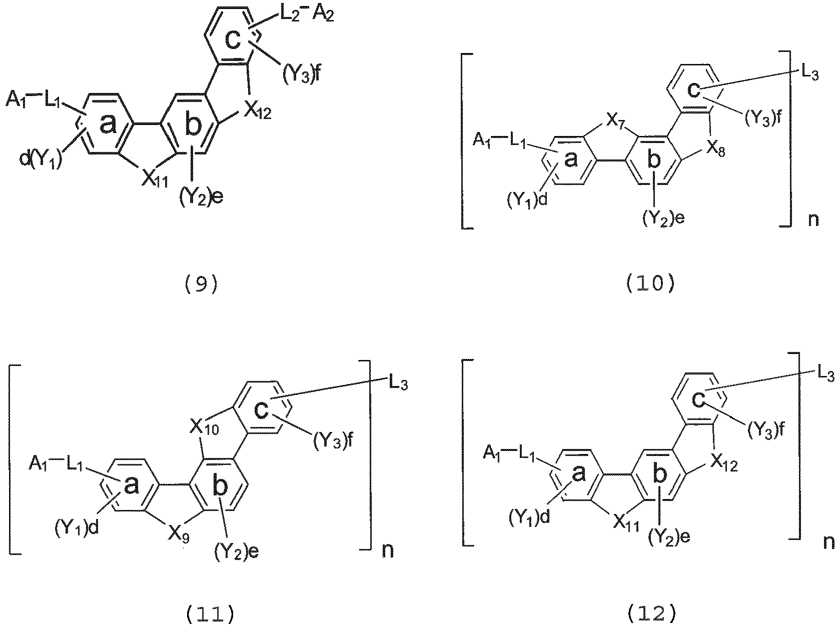 where:
   X₇, X₈, X₉, X₁₀, X₁₁, and X₁₂ each independently represent oxygen (O), sulfur (S), N-R₁ or CR₂R₃;
   R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or substituted or unsubstituted aromatic heterocyclic group having a ring formed of 3 to 24 atoms, provided that, when both X₇ and X₈, both X₉ and X₁₀, or both X₁₁ and X₁₂ represent N-R₁, at least one R₁ represents a substituted or unsubstituted, monovalent fused aromatic heterocyclic group having a ring formed of 8 to 24 atoms;
   n represents 2, 3, or 4, and the compound represented by any one of the formulae (10) to (12) comprises a dimer using L₃ as a linking group for n=2, a trimer using L₃ as a linking group for n=3, or a tetramer using L₃ as a linking group for n=4;
   L₁ represents a single bond, an alkylene group having 1 to 20 carbon atoms, a substituted or unsubstituted cycloalkylene group having a ring formed of 3 to 20 carbon atoms, a divalent silyl group or divalent silyl group having 2 to 20 carbon atoms, a substituted or unsubstituted, divalent aromatic hydrocarbon group having a ring formed of 6 to 24 carbon atoms, or a substituted or unsubstituted, monovalent or divalent aromatic heterocyclic group which has a ring formed of 3 to 24 atoms and which is linked with a benzene ring a through a carbon-carbon bond;
   L₂ represents a single bond, an alkylene group having 1 to 20 carbon atoms, a substituted or unsubstituted cycloalkylene group having a ring formed of 3 to 20 carbon atoms, a divalent silyl group or divalent silyl group having 2 to 20 carbon atoms, a substituted or unsubstituted, monovalent or divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond, provided that, when both X₇ and X₈, both X₉ and X₁₀, or both X₁₁ and X₁₂ represent CR₂R₃ and both L₁ and L₂ represent substituted or unsubstituted, monovalent or divalent aromatic hydrocarbon groups having a ring formed of 6 to 24 carbon atoms, a case where L₁ and L₂ are simultaneously linked at para positions with respect to a benzene ring b is excluded;
   when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond, provided that, when both X₇ and X₈, both X₉ and X₁₀, or both X₁₁ and X₁₂ represent CR₂R₃ and both L₁ and L₃ represent substituted or unsubstituted, divalent, trivalent, or tetravalent aromatic hydrocarbon groups having a ring formed of 6 to 24 carbon atoms, a case where L₁ and L₃ are simultaneously linked at para positions with respect to the benzene ring b is excluded;
   A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbonbond, provided that, when L₁ represents an alkylene group having 1 to 20 carbon atoms, a case where A₁ represents a hydrogen atom is excluded;
   A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
   Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of 0, 1, or 2, provided that, when both X₇ and X₈, both X₉ and X₁₀, or both X₁₁ and X₁₂ represent oxygen (O), sulfur (S), or CR₂R₃, both L₁ and L₂ represent single bonds, and both A₁ and A₂ represent hydrogen atoms, the benzene ring b has one or two Y₂'s, and a case where Y₂ represents a methyl group or an unsubstituted phenyl group is excluded; and
   A₁, A₂, L₁, L₂, and L₃ are each free of any carbonyl group.
[Item 9] The polycyclic compound according to item 8, wherein the formulae (7) to (12) are each represented by any one of the following formulae (7a) to (12a) and (7b) to (12b): 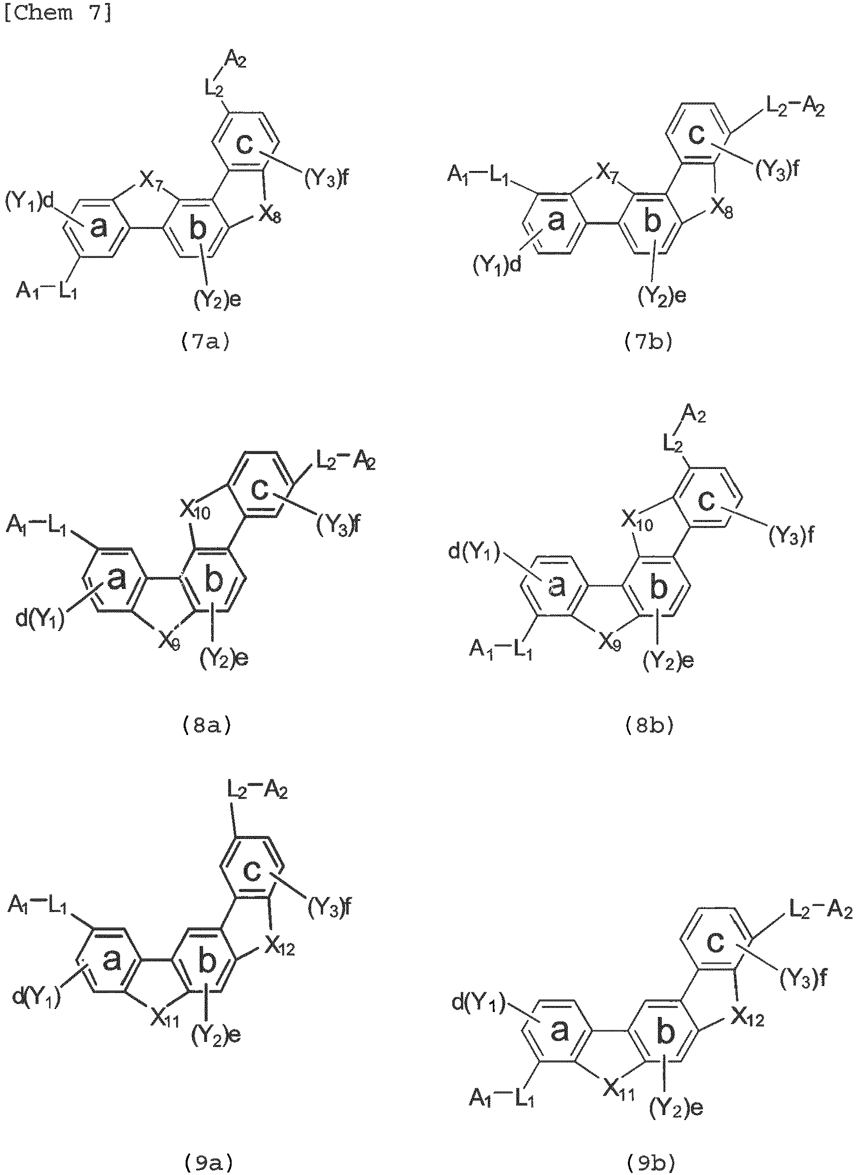 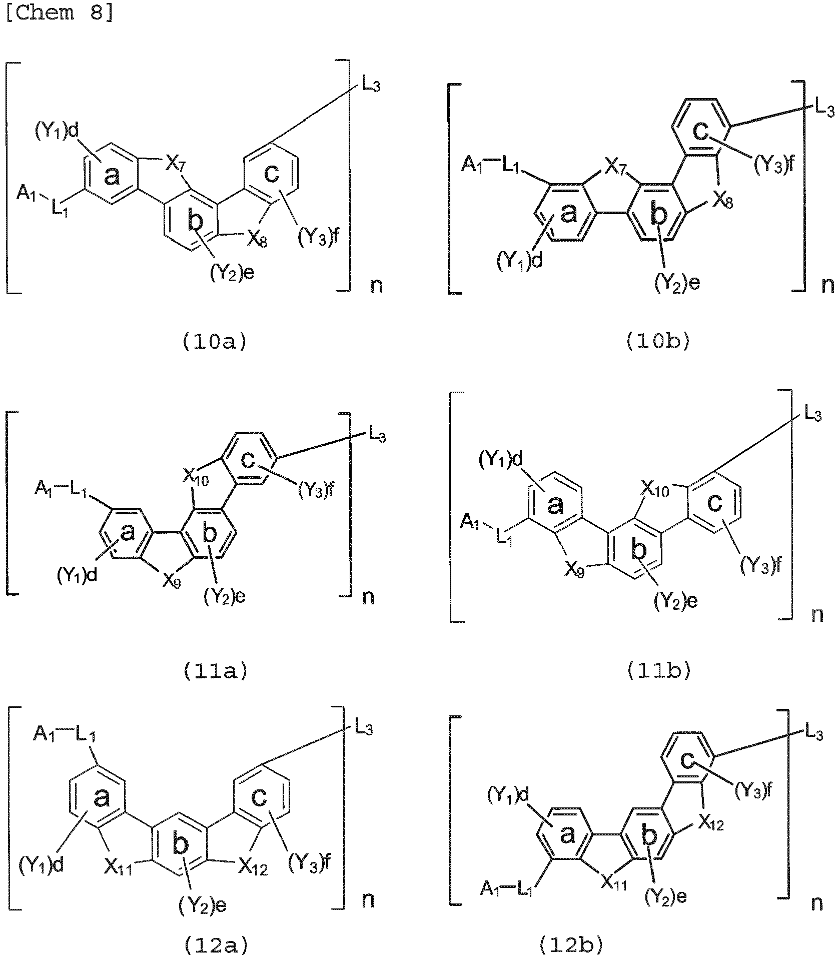 where X₇ to X₁₂, L₁, L₂, L₃, A₁, A₂, Y₁, Y₂, Y₃, n, d, f , and e each have the same meaning as that described above.
[Item 10] The polycyclic compound according to item 1, wherein the polycyclic compound is represented by one of the following formulae (13) and (14): 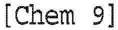 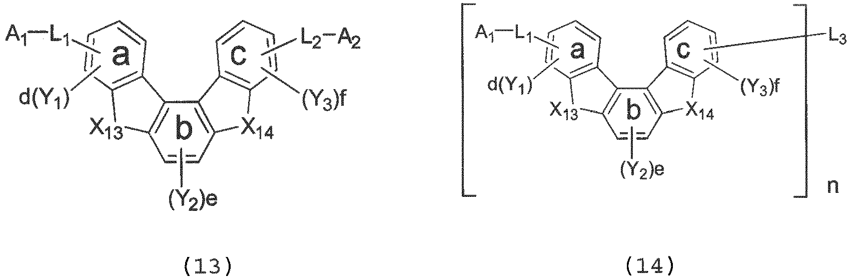 where:
   X₁₃, and X₁₄ each independently represent oxygen (O), sulfur (S), N-R₁, or CR₂R₃;
   R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or substituted or unsubstituted aromatic heterocyclic group having a ring formed of 3 to 24 atoms, provided that, when both X₁₃ and X₁₄ represent N-R₁, at least one R₁ represents a substituted or unsubstituted, monovalent fused aromatic heterocyclic group having a ring formed of 8 to 24 atoms;
   n represents 2, 3, or 4, and the compound represented by the formula (14) comprises a dimer using L₃ as a linking group for n=2, a trimer using L₃ as a linking group for n=3, or a tetramer using L₃ as a linking group for n=4;
   L₁ represents a single bond, an alkylene group having 1 to 20 carbon atoms, a substituted or unsubstituted cycloalkylene group having a ring formed of 3 to 20 carbon atoms, a divalent silyl group or divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
   L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond, provided that, when both X₁₃ and X₁₄ represent CR₂R₃ and both L₁ and L₂ represent substituted or unsubstituted, divalent aromatic hydrocarbon groups having a ring formed of 6 to 24 carbon atoms, a case where L₁ and L₂ are simultaneously linked at para positions with respect to a benzene ring b is excluded;
   when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond, provided that, when both X₁₃ and X₁₄ represent CR₂R₃ and both L₁ and L₃ represent substituted or unsubstituted, divalent, trivalent, or tetravalent aromatic hydrocarbon groups having a ring formed of 6 to 24 carbon atoms, a case where L₁ and L₃ are simultaneously linked at para positions with respect to the benzene ring b is excluded;
   A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded;
   A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
   Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of 0, 1, or 2, provided that, when both X₁₃ and X₁₄ represent oxygen (O), sulfur (S), or CR₂R₃, both L₁ and L₂ represent single bonds, and both A₁ and A₂ represent hydrogen atom, the benzene ring b has one or two Y₂'s, and a case where Y₂ represents a methyl group or an unsubstituted phenyl group is excluded; and
   A₁, A₂, L₁, L₂, and L₃ are each free of any carbonyl group.
[Item 11] The polycyclic compound according to item 10, wherein the formula (13) is represented by one of the following formulae (13a) and (13b), and the formula (14) is represented by one of the following formulae (14a) and (14b): 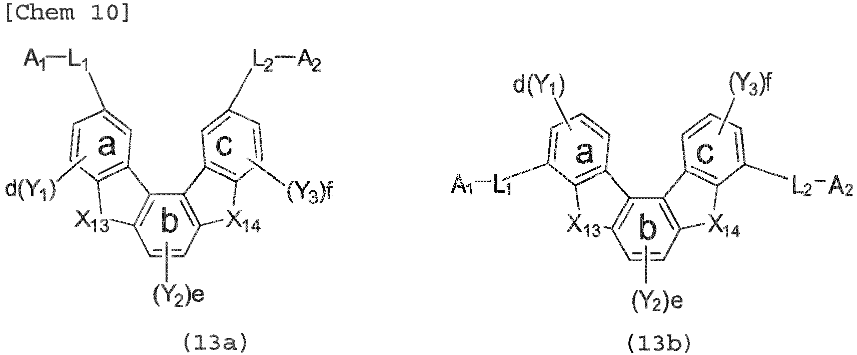 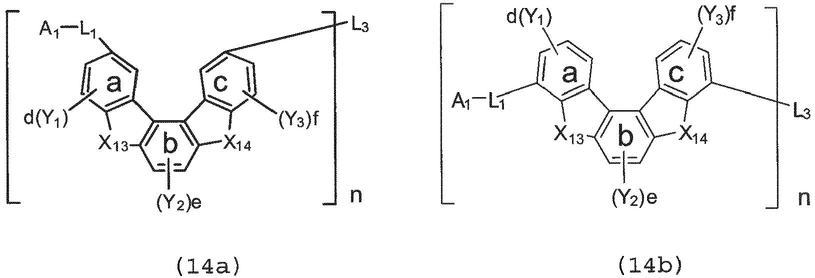 where X₁₃, X₁₄, L₁, L₂, L₃, A₁, A₂, Y₁, Y₂, Y₃, n, d, f , and e each have the same meaning as that described above.
[Item 12] The polycyclic compound according to item 1, wherein n represents 2 in the formula (2).
[Item 13] The polycyclic compound according to item 6, 8, or 10, wherein n represents 2 in any one of the formulae (6), (10) to (12), and (14) .
[Item 14] The polycyclic compound according to item 6, wherein a total number of substituents represented by Y₁, Y₂, and Y₃ in the formula (5) is 3 or less, and a total number of the substituents represented by Y₁, Y₂, and Y₃ in the structure of [ ]ₙ in the formula (6) is 3 or less.
[Item 15] The polycyclic compound according to item 8, wherein a total number of substituents represented by Y₁, Y₂, and Y₃ in the formulae (7) to (9) is 3 or less, and a total number of the substituents represented by Y₁, Y₂, and Y₃ in the structure of [ ]ₙ in the general formulae (10) to (12) is 3 or less.
[Item 16] The polycyclic compound according to item 10, wherein a total number of substituents represented by Y₁, Y₂, and Y₃ in the formula (13) is 3 or less, and a total number of the substituents represented by Y₁, Y₂, and Y₃ in the structure of [ ]ₙ in the formula (14) is 3 or less.
[Item 17] The polycyclic compound according to item 1, wherein both X₁ and X₂ or both X₃ and X₄ in the formula (1) or (2) represent N-R₁.
[Item 18] The polycyclic compound according to item 6, wherein both X₅ and X₆ in the formula (5) or (6) represent N-R₁.
[Item 19] The polycyclic compound according to item 8, wherein both X₇ and X₈, both X₉ and X₁₀, or both X₁₁ and X₁₂ in the formulae (7) to (12) represent N-R₁.
[Item 20] The polycyclic compound according to item 10, wherein both X₁₃ and X₁₄ in the formula (13) or (14) represent N-R₁.
[Item 21] The polycyclic compound according to item 1, wherein both X₁ and X₂ or both X₃ and X₄ in the formula (1) or (2) represent N-R₁, and at least one R₁ represents a dibenzofuran residue or a carbazole residue.
[Item 22] The polycyclic compound according to item 6, wherein both X₅ and X₆ in the formula (5) or (6) represent N-R₁, and at least one R₁ represents a dibenzofuran residue or a carbazole residue.
[Item 23] The polycyclic compound according to item 8, wherein both X₇ and X₈, both X₉ and X₁₀ or, both X₁₁ and X₁₂ in the formulae (7) to (12) represent N-R₁, and at least one R₁ represents a dibenzofuran residue or a carbazole residue.
[Item 24] The polycyclic compound according to item 10, wherein both X₁₃ and X₁₄ in the formula (13) or (14) represent N-R₁, and at least one R₁ represents a dibenzofuran residue or a carbazole residue.
[Item 25] The polycyclic compound according to item 1, wherein X₁ and X₂ or X₃ and X₄ in the formula (1) or (2) are each represented by N-R₁, and N-R₁ represented by X₁ and N-R₁ represented by X₂, or N-R₁ represented by X₃ and N-R₁ represented by X₄ are different from each other.
[Item 26] The polycyclic compound according to item 6, wherein X₅ and X₆ in the formula (5) or (6) are each represented by N-R₁, and N-R₁ represented by X₅ and N-R₁ represented by X₆ are different from each other.
[Item 27] The polycyclic compound according to item 8, wherein X₇ and X₈, X₉ and X₁₀, or X₁₁ and X₁₂ in the formulae (7) to (12) are each represented by N-R₁, and N-R₁ represented by X₇ and N-R₁ represented by X₈, or N-R₁ represented by X₉ and N-R₁ represented by X₁₀, or N-R₁ represented by X₁₁ and N-R₁ represented by X₁₂ are different from each other.
[Item 28] The polycyclic compound according to item 10, wherein X₁₃ and X₁₄ in the formula (13) or (14) are each represented by N-R₁, and N-R₁ represented by X₁₃ and N-R₁ represented by X₁₄ are different from each other.
[Item 29] The polycyclic compound according to item 6, wherein both X₅ and X₆ in the formula (5) or (6) represent CR₂R₃.
[Item 30] The polycyclic compound according to item 8, wherein both X₁₁ and X₁₂ in the formula (9) or (12) represent CR₂R₃.
[Item 31] The polycyclic compound according to item 10, wherein both X₁₃ and X₁₄ in the formula (13) or (14) represent CR₂R₃ .
[Item 32] The polycyclic compound according to item 1, wherein at least one of X₁ and X₂ or X₃ and X₄ in the formula (1) or (2) represents an oxygen atom.
[Item 33] The polycyclic compound according to item 6, wherein at least one of X₅ and X₆ in the formula (5) or (6) represents an oxygen atom.
[Item 34] The polycyclic compound according to item 8, wherein at least one of X₇ and X₂, X₉ and X₁₀, or X₁₁ and X₁₂ in the formula (7) to (12) represents an oxygen atom.
[Item 35] The polycyclic compound according to item 10, wherein at least one of X₁₃ and X₁₄ in the formula (13) or (14) represents an oxygen atom.
[Item 36] The polycyclic compound according to item 1, which is a benzofurano dibenzofuran derivative in which both X₁ and X₂ or both X₃ and X₄ in the formula (1) or (2) represent oxygen atoms.
[Item 37] The polycyclic compound according to item 6, which is a benzofurano dibenzofuran derivative in which both X₅ and X₆ in the formula (5) or (6) represent oxygen atoms.
[Item 38] The polycyclic compound according to item 8, which is a benzofurano dibenzofuran derivative in which both X₇ and X₈, both X₉ and X₁₀, or both X₁₁ and X₁₂ in the formulae (7) to (12) represent oxygen atoms.
[Item 39] The polycyclic compound according to item 10, which is a benzofurano dibenzofuran derivative in which both X₁₃ and X₁₄ in the formula (13) or (14) represent oxygen atoms.
[Item 40] The polycyclic compound according to item 6, which is a benzofurano dibenzofuran derivative represented by one of the following formulae (15) and (16): 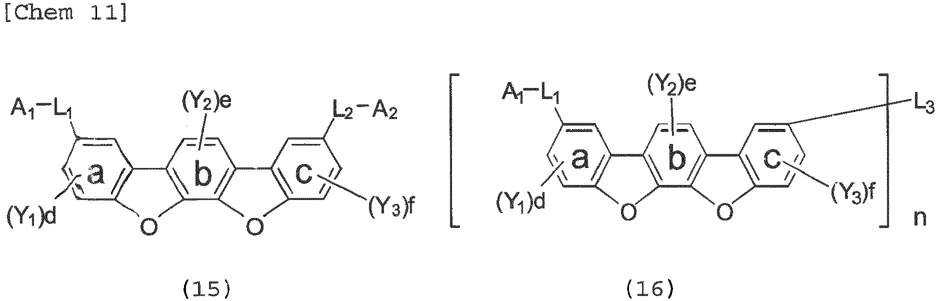 where:
   n represents 2, 3, or 4, and the compound represented by the formula (16) comprises a dimer using L₃ as a linking group for n=2, a trimer using L₃ as a linking group for n=3, or a tetramer using L₃ as a linking group for n=4;
   L₁ represents a single bond, an alkylene group having 1 to 20 carbon atoms, a substituted or unsubstituted cycloalkylene group having a ring formed of 3 to 20 carbon atoms, a divalent silyl group or a divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
   L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond;
   when n represents 2, L₃ represents a single bond, an alkylene group having 1 to 20 carbon atoms, a substituted or unsubstituted cycloalkylene group having a ring formed of 3 to 20 carbon atoms, a divalent silyl group or a silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond;
   A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded;
   A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
   Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of 0, 1, or 2, provided that, when both L₁ and L₂ represent single bonds, and both A₁ and A₂ represent hydrogen atom, the benzene ring b has one or two Y₂'s, and a case where Y₂ represents a methyl group or an unsubstituted phenyl group is excluded; and
   A₁, A₂, L₁, L₂, and L₃ are each free of any carbonyl group.
[Item 41] The polycyclic compound according to item 8, which is a benzofurano dibenzofuran derivative represented by one of the following formulae (17) and (18): 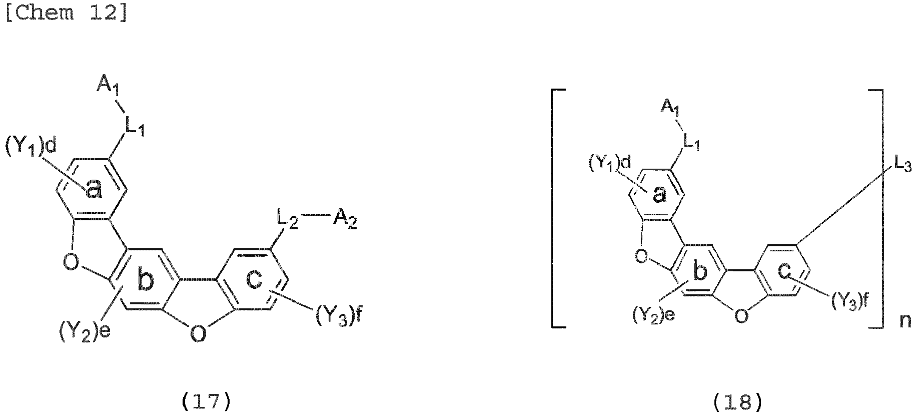 where:
   n represents 2, 3, or 4, and the compound represented by the formula (18) comprises a dimer using L₃ as a linking group for n=2, a trimer using L₃ as a linking group for n=3, or a tetramer using L₃ as a linking group for n=4;
   L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
   L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond;
   when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond;
   A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbonbond, provided that, when L₁ represents analkylene group having 1 to 20 carbon atoms, a case where A₁ represents a hydrogen atom is excluded;
   A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
   Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of 0, 1, or 2, provided that, when both L₁ and L₂ represent single bonds, and both A₁ and A₂ represent hydrogen atom, the benzene ring b has one or two Y₂'s, and a case where Y₂ represents a methyl group or an unsubstituted phenyl group is excluded; and
   A₁, A₂, L₁, L₂, and L₃ are each free of any carbonyl group.
[Item 42] A polycyclic, which is represented by one of the following formulae (19) to (23): 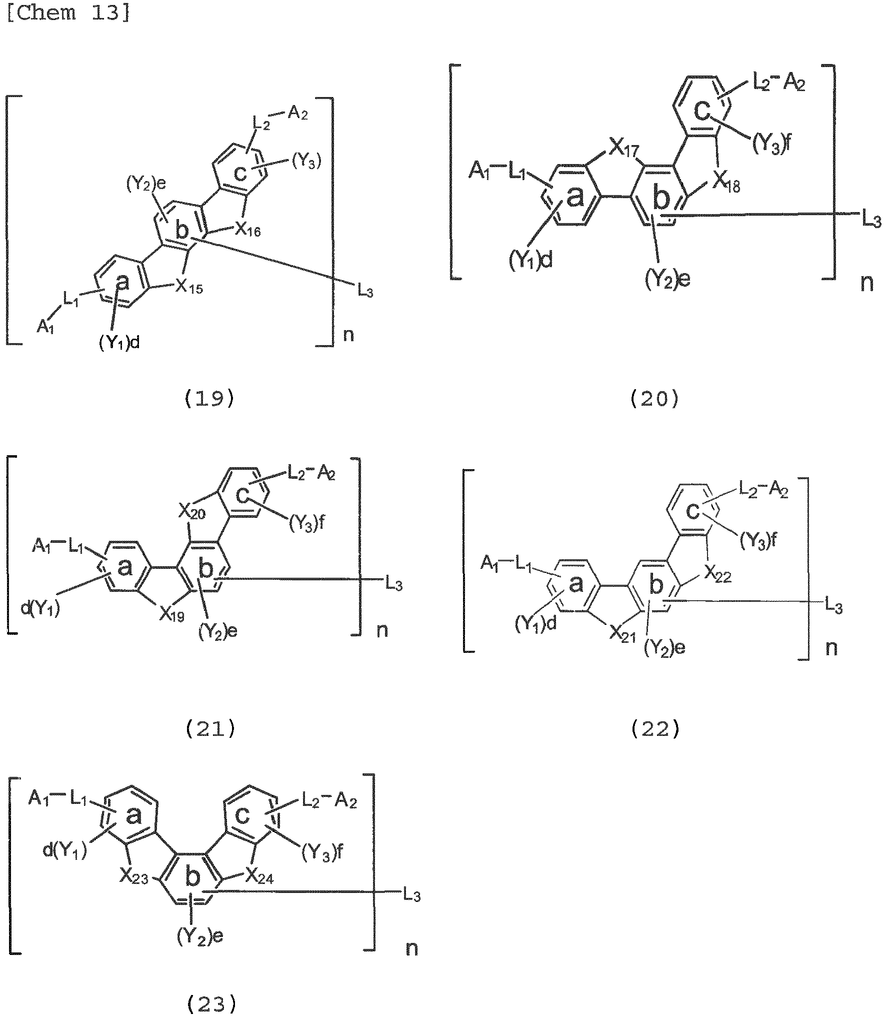 where:
   X₁₅, X₁₆, X₁₇, X₁₈, X₁₉, X₂₀, X₂₁, X₂₂, X₂₃, and X₂₄ each independently represent oxygen (O), sulfur (S), N-R₁, or CR₂R₃;
   R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, when both X₁₅ and X₁₆, both X₁₇ and X₁₈, both X₁₉ and X₂₀, both X₂₁ and X₂₂, or both X₂₃ and X₂₄ represent N-R₁, at least one R₁ represents a substituted or unsubstituted, monovalent fused aromatic heterocyclic group having a ring formed of 8 to 24 atoms;
   n represents 2, 3, or 4, and the compound represented by any one of the formulae (19) to (23) comprise a dimer using L₃ as a linking group for n=2, a trimer using L₃ as a linking group for n=3, or a tetramer using L₃ as a linking group for n=4;
   L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
   L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond, provided that, when both X₁₅ and X₁₆, both X₁₇ and X₁₈, both X₁₉ and X₂₀, both X₂₁ and X₂₂, or both X₂₃ and X₂₄ represent CR₂R₃ and both L₁ and L₂ represent substituted or unsubstituted, monovalent or divalent aromatic hydrocarbon groups each having a rings formed of 6 to 24 carbon atoms, a case where L₁ and L₂ are simultaneously linked at para positions with respect to a benzene ring b is excluded;
   when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring b through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring b through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring b through a carbon-carbon bond;
   A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded;
   A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
   Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, a number of each of d and f is 0, 1, 2, or 3, and a number of e is 0 or 1; and
   A₁, A₂, L₁, L₂, and L₃ are each free of a carbonyl group.
[Item 43] The polycyclic compound according to item 42, which is a benzofurano dibenzofuran derivative represented by the following formula (24): 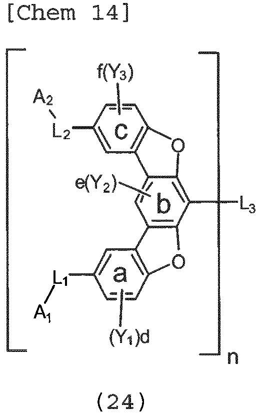 where:
   n represents 2, 3, or 4, and the compound represented by the formula (24) comprises a dimer using L₃ as a linking group for n=2, a trimer using L₃ as a linking group for n=3, or a tetramer using L₃ as a linking group for n=4;
   L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
   L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond;
   when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring b through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring b through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring b through a carbon-carbon bond;
   A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded;
   A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbonbond, provided that, when L₂ represents an alkylene group having 1 to 20 carbon atoms, a case where A₂ represents a hydrogen atom is excluded;
   Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, a number of each of d and f is 0, 1, 2, or 3, and a number of e is 0 or 1; and
   A₁, A₂, L₁, L₂, and L₃ are each free of any carbonyl group.
[Item 44] A polycyclic compound, which is represented by any one of the following formulae (25) to (29): 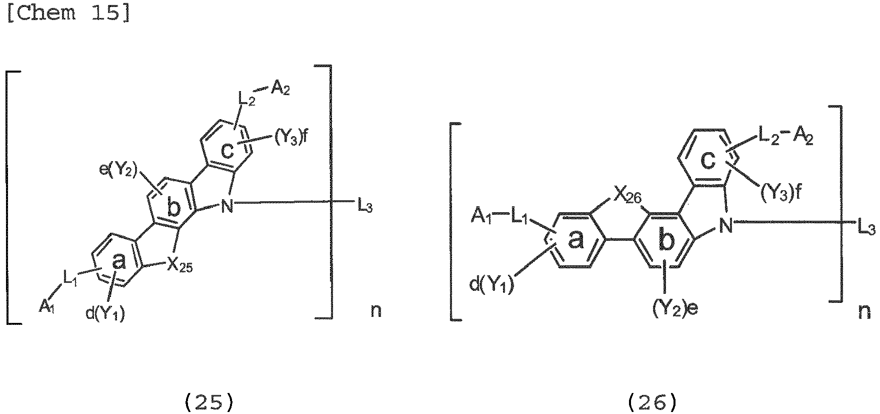 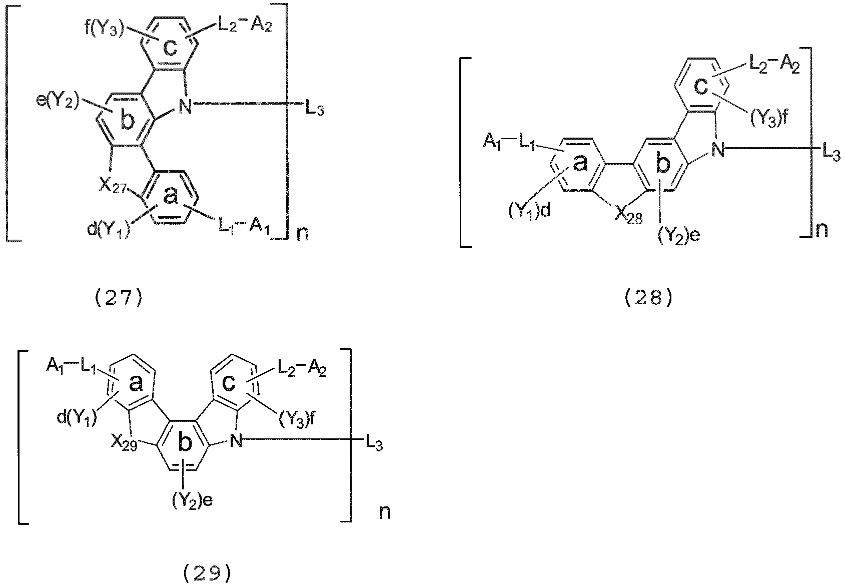 where:
   X₂₅, X₂₆, X₂₇, X₂₈, and X₂₉ each independently represent oxygen (O), sulfur (S), N-R₁, or CR₂R₃;
   R₂ and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms;
   n represents 2, 3, or 4, and the compound represented by any one of the formulae (25) to (29) comprises a dimer using L₃ as a linking group for n=2, a trimer using L₃ as a linking group for n=3, or a tetramer using L₃ as a linking group for n=4;
   L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, monovalent or divalent aromatic heterocyclic group which has a ring formed of 3 to 24 atoms and which is linked with a benzene ring a through a carbon-carbon bond;
   L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond;
   when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has a ring formed of 3 to 24 atoms, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms;
   A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded;
   A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
   Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of 0, 1, or 2; and A₁, A₂, L₁, L₂, and L₃ are each free of any carbonyl group.
[Item 45] A material for an organic electron device, wherein the material for an organic electron device is a benzofurano dibenzofuran derivative.
[Item 46] A material for an organic electron device, wherein the material for an organic electron device is a benzofurano dibenzofuran derivative in which both X₁ and X₂ or both X₃ and X₄ in the formula (1) or (2) according to item 1 represent oxygen atoms.
[Item 47] A halogen compound represented by the following formula (30) : 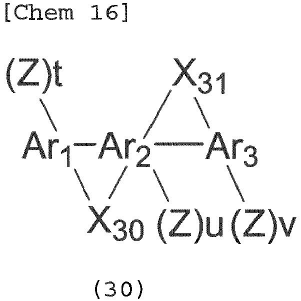 where:
   Ar₁, Ar₂, and Ar₃ each independently represent a substituted or unsubstituted aromatic hydrocarbon group having a ring formed of 6 carbon atoms or a substituted or unsubstituted aromatic heterocyclic group having a ring formed of 6 atoms, provided that, Ar₁, Ar₂, and Ar₃ each may have one substituent Y or a plurality of substituents Y's, in the case of the plurality of substituents Y's, the substituent Y's may be different from each other, Y represents an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with Ar₁, Ar₂, or Ar₃ through a carbon-carbon bond;
   X₃₀ and X₃₁ each independently represent oxygen (O), sulfur (S), or N-R₁;
   R₁ represents an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, when both X₃₀ and X₃₁ represent N-R₁, at least one R₁ represents a substituted or unsubstituted, monovalent fused aromatic heterocyclic group having a ring formed of 8 to 24 atoms;
   Z represents a halogen atom that comprises a fluorine atom, a chlorine atom, a bromine atom, and an iodine atom;
   t, u, and v each represent 0 or 1; provided that, t+u+v≥1;
   a case where the formula (30) has a structure represented by the following formula (31) is excluded; 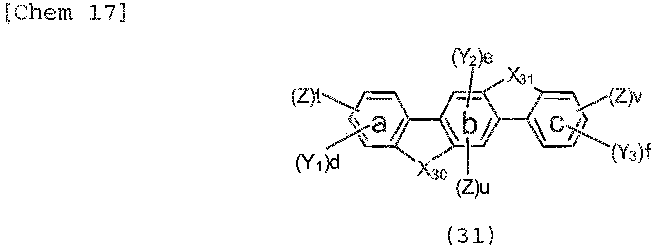
   provided that, X₃₀, X₃₁, Z, t, and u in the formula (31) has the same meaning of X₃₀, X₃₁, Z, t, and u in the formula (30), respectively; and
   Y₁, Y₂, and Y₃ in the formula (31) each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted a silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of 0, 1, or 2.
[Item 48] The halogen compound according to item 47, wherein the halogen compound is represented by the following formula (32): 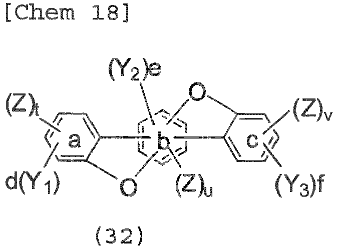 where:
   Y₁, Y₂, and Y₃ each independently represent a hydrogen atom, an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with a benzene ring a, b, or c through a carbon-carbon bond;
   d and f each represent a number of 0, 1, 2, or 3, and e represents a number of 0, 1, or 2;
   Z represents a halogen atom that comprises a fluorine atom, a chlorine atom, a bromine atom, or an iodine atom; and
   t, u, and v each represent 0 or 1, provided that, t+u+v≥1.
[Item 49] The halogen compound according to item 47, wherein the halogen compound is represented by one of the following formulae (33) to (35): 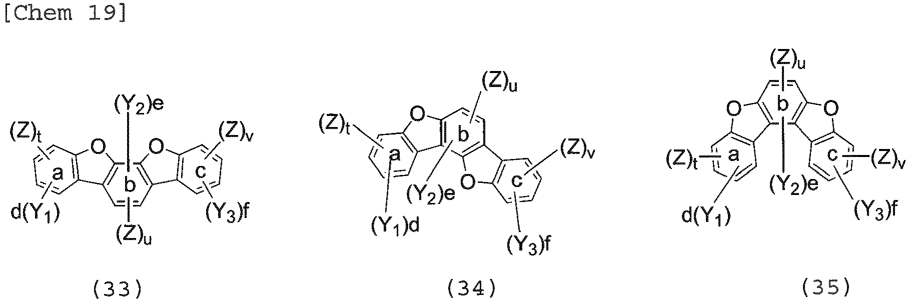 where:
   Y₁, Y₂, and Y₃ each independently represent a hydrogen atom, an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a unsubstituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with a benzene ring a, b, or c through a carbon-carbon bond, d and f each represent a number of 0, 1, 2, or 3, and e represents a number of 0, 1, or 2;
   Z represents a halogen atom that comprises a fluorine atom, a chlorine atom, a bromine atom, or an iodine atom; and
   t, u, and v each represent 0 or 1, provided that t+u+v≥1,
[Item 50] The halogen compound according to item 47, wherein the halogen compound is represented by the following formula (36) or (37) : 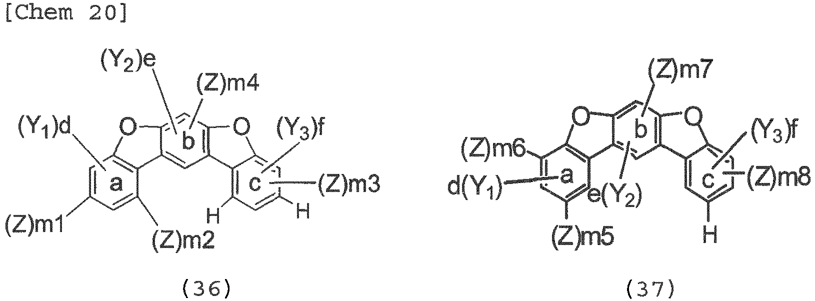 where:
   Y₁, Y₂, and Y₃ each independently represent a hydrogen atom, an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with a benzene ring a, b, or c through a carbon-carbon bond, d and f each represent a number of 0, 1, 2, or 3, and e represents a number of 0, 1, or 2;
   Z represents a halogen atom that comprises a fluorine atom, a chlorine atom, a bromine atom, or an iodine atom; and
   ml to m8 each represent 0 or 1, provided that, m1+m2=1, m5+m6≤1, m6+m7+m8≥1.
[Item 51] An organic electroluminescence device comprising one or more organic thin film layers including a light emitting layer between a cathode and an anode, wherein at least one layer of the organic thin film layers contains a polycyclic compound having a π-conjugated heteroacene skeleton crosslinked with a carbon atom, nitrogen atom, oxygen atom, or sulfur atom.
[Item 52] The organic electroluminescence device according to Item 51, wherein the polycyclic compound comprises the polycyclic compound according to any one of items 1, 42, and 44.
[Item 53] The organic electroluminescence device according to item 51, wherein the light emitting layer contains the polycyclic compound as a host material.
[Item 54] The organic electroluminescence device according to item 51, wherein the light emitting layer further contains a phosphorescent material.
[Item 55] The organic electroluminescence device according to item 51, wherein the light emitting layer contains a host material and a phosphorescent material, and the phosphorescent material comprises an orthometalated complex of an iridium (Ir), osmium (Os), or platinum (Pt) metal.
[Item 56] The organic electroluminescence device according to Item 51, further comprising an electron injecting layer between the light emitting layer and the cathode, wherein the electron injecting layer contains a nitrogen-containing ring derivative.
[Item 57] The organic electroluminescence device according to Item 51, further comprising an electron transporting layer between the light emitting layer and the cathode, wherein the electron transporting layer contains the polycyclic compound.
[Item 58] The organic electroluminescence device according to Item 57, wherein the light emitting layer contains the polycyclic compound as a host material.
[Item 59] The organic electroluminescence device according to Item 57, wherein the light emitting layer contains, as a host material, the polycyclic compound represented by the formula (5) or (6) according to item 6, or the formula (9) or (12) according to item 8.
[Item 60] The organic electroluminescence device according to item 57, wherein the light emitting layer contains, as a host material, the polycyclic compound represented by the formula (15) or (16) according to item 40, or the formula (17) or (18) according to item 41.
[Item 61] The organic electroluminescence device according to item 51, further comprising a hole transporting layer between the light emitting layer and the anode, wherein the hole transporting layer contains the polycyclic compound.
[Item 62] The organic electroluminescence device according to item 51, further comprising a reducing dopant at an interfacial region between the cathode and the organic thin film layers.

## Claims

1. A polycyclic compound represented by any one of the following formulae (7) to (12): 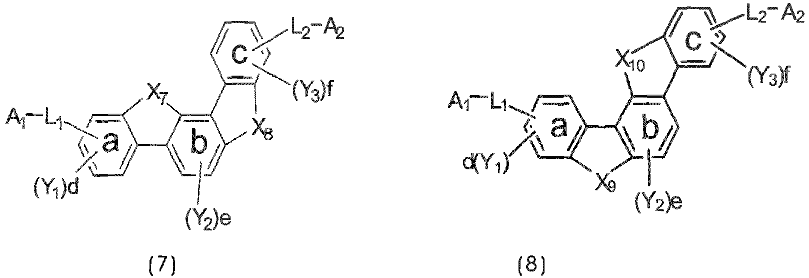 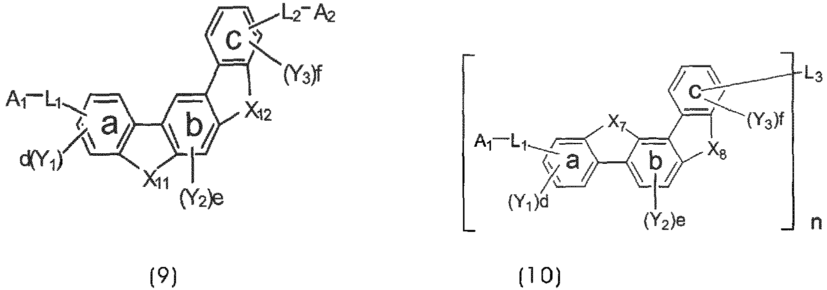 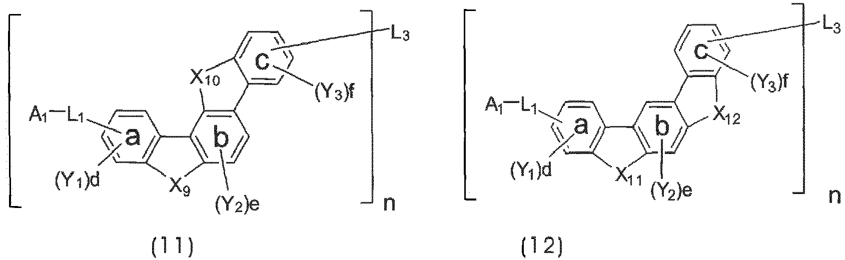 where:
X₇, X₈, X₉, X₁₀, X₁₁, and X₁₂ each independently represent oxygen (O), sulfur (S), N-R₁ or CR₂R₃; provided that X₇, X₉ and X₁₁ in the general formulae (7) to (12) each independently represent oxygen (O), sulfur (S) or CR₂R₃, or X₈, X₁₀ and X₁₂ in the general formulae (7) to (12) each independently represent oxygen (O), sulfur (S) or CR₂R₃;
R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group, wherein the aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted aromatic heterocyclic group having a ring formed of 3 to 24 atoms;
n represents 2, 3, or 4, and the compound represented by any one of the formulae (10) to (12) comprises a dimer using L₃ as a linking group for n = 2, a trimer using L₃ as a linking group for n = 3, or a tetramer using L₃ as a linking group for n=4;
L₁ represents a single bond, an alkylene group having 1 to 20 carbon atoms, a substituted or unsubstituted cycloalkylene group having a ring formed of 3 to 20 carbon atoms, a divalent silyl group or divalent silyl group having 2 to 20 carbon atoms, a substituted or unsubstituted, divalent aromatic hydrocarbon group, wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with a benzene ring a through a carbon-carbon bond;
L₂ represents a single bond, an alkylene group having 1 to 20 carbon atoms, a substituted or unsubstituted cycloalkylene group having a ring formed of 3 to 20 carbon atoms, a divalent silyl group or divalent silyl group having 2 to 20 carbon atoms, a substituted or unsubstituted, divalent aromatic hydrocarbon group, wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with a benzene ring c through a carbon-carbon bond,
provided that, when both X₇ and X₈, both X₉ and X₁₀, or both X₁₁ and X₁₂ represent CR₂R₃ and both L₁ and L₂ represent the substituted or unsubstituted, divalent aromatic hydrocarbon groups, a case where L₁ is linked to the benzene ring a at the para position with respect to the benzene ring b and L₂ is linked to the benzene ring c at the para position with respect to the benzene ring b is excluded;
when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group, wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with the benzene ring c through a carbon-carbon bond,
when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group, wherein the trivalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, trivalent aromatic heterocyclic group which has 3 to 24 atoms and which is linked with the benzene ring c through a carbon-carbon bond, or
when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group, wherein the tetravalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, tetravalent aromatic heterocyclic group which has a ring formed of 3 to 24 atoms and which is linked with the benzene ring c through a carbon-carbon bond,
provided that, when both X₇ and X₈, both X₉ and X₁₀, or both X₁₁ and X₁₂ represent CR₂R₃ and both L₁ and L₃ represent the substituted or unsubstituted, divalent, trivalent, or tetravalent aromatic hydrocarbon groups, a case where L₁ is linked to the benzene ring a at the para position with respect to the benzene ring b and L₃ is linked to the benzene ring c at the para position with respect to the benzene ring b is excluded;
A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group, wherein the aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or an aromatic heterocyclic group which has a ring formed of 3 to 24 atoms and which is linked with L₁ through a carbon-carbon bond,
provided that, when L₁ represents an alkylene group having 1 to 20 carbon atoms, a case where A₁ represents a hydrogen atom is excluded;
A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring, wherein the aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or an aromatic heterocyclic group which has a ring formed of 3 to 24 atoms and which is linked with L₂ through a carbon-carbon bond,
provided that, when L₂ represents an alkylene group having 1 to 20 carbon atoms, a case where A₂ represents a hydrogen atom is excluded;
Y,, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group, wherein the aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted aromatic heterocyclic group which has a ring formed of 3 to 24 atoms and which is linked with the benzene ring a, b, or c through a carbon-carbon bond,
d and f each represent a number of 0, 1, 2, or 3, and e represents a number of 0, 1, or 2,
provided that, when both X₇ and X₈, both X₉ and X₁₀, or both X₁₁ and X₁₂ represent oxygen (O), sulfur (S), or CR₂R₃, both L₁ and L₂ represent single bonds, and both A₁ and A₂ represent hydrogen atoms, the benzene ring b has one or two Y₂'s, and a case where Y₂ represents a methyl group or an unsubstituted phenyl group is excluded;
A₁, A₂, L₁, L₂, and L₃ are each free of any carbonyl group, and
the substituent that may substitute each of the groups in formulae (7) to (12) includes alkyl groups each having 1 to 6 carbon atoms, a phenyl group, a pyridyl group, a carbazolyl group, and a dibenzofuranyl group,
provided that the following compound is excluded:

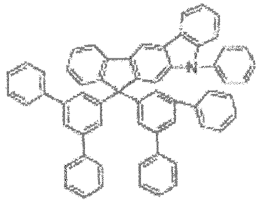

2. The polycyclic compound according to claim 1, wherein the formulae (7) to (12) are each represented by any one of the following formulae (7a) to (12a) and (7b) to (12b): 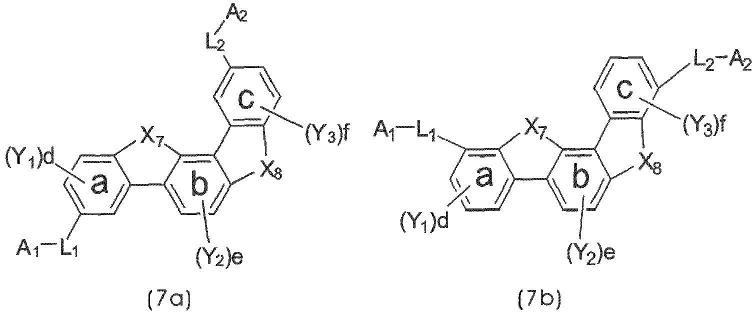 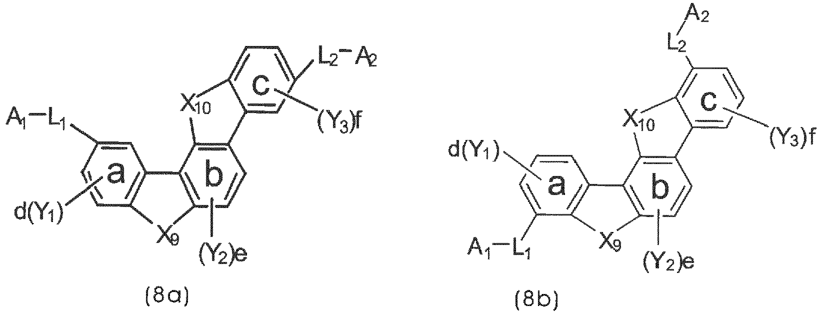 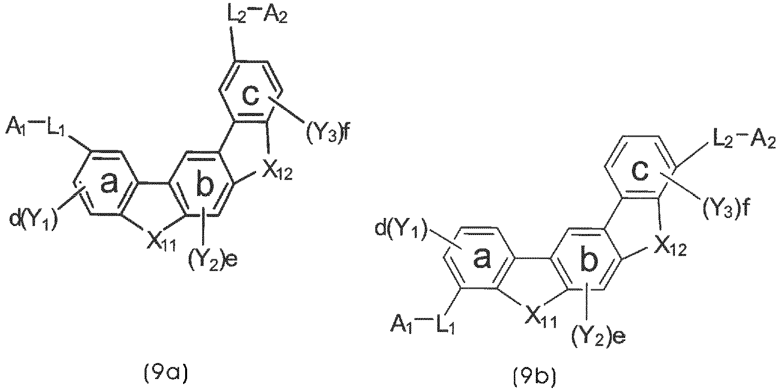 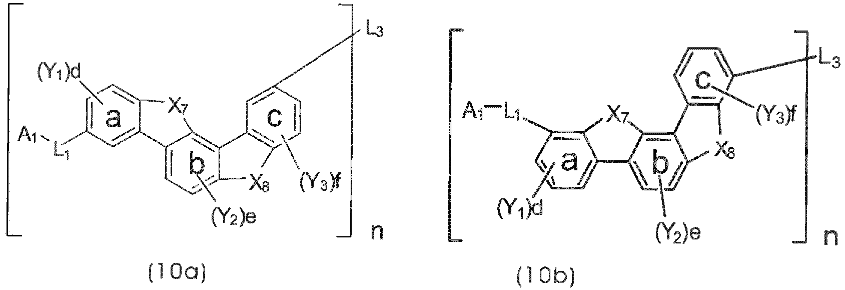 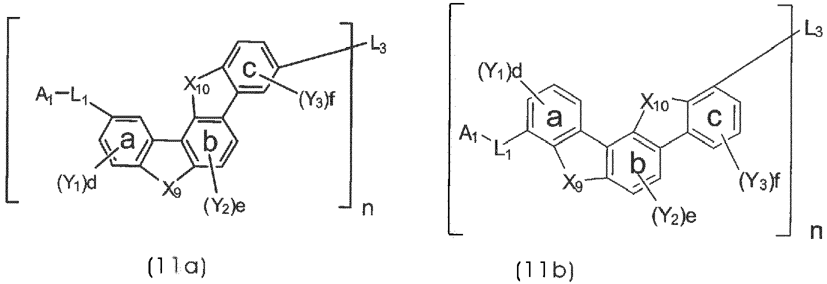 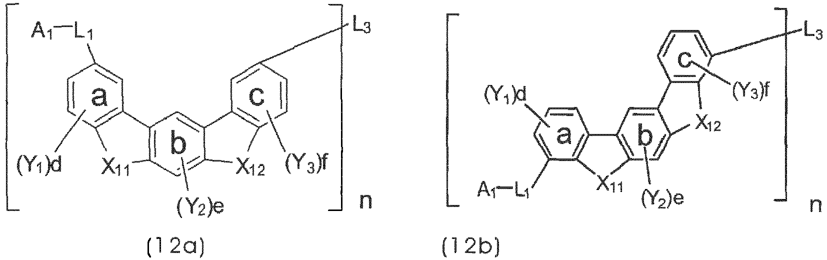 where X₇ to X₁₂, L₁, L₂, L₃, A₁, A₂, Y₁, Y₂, Y₃, n, d, f, and e each have the same meaning as that described above.

3. The polycyclic compound according to claim 1, wherein n represents 2 in the formulae (10) to (12).

4. The polycyclic compound according to claim 1, wherein a total number of substituents represented by Y₁, Y₂, and Y₃ in the formulae (7) to (9) is 3 or less, and a total number of the substituents represented by Y₁, Y₂, and Y₃ in the structure of []ₙ in the general formulae (10) to (12) is 3 or less.

5. The polycyclic compound according to claim 1, wherein both X₁₁ and X₁₂ in the formula (9) or (12) represent CR₂R₃.

6. The polycyclic compound according to claim 1, wherein at least one of X₇ and X₈, X₉ and X₁₀, or X₁₁ and X₁₂ in the formulae (7) to (12) represents an oxygen atom.

7. The polycyclic compound according to claim 1, which is a benzofurano dibenzofuran derivative in which both X₇ and X₈, both X₉ and X₁₀, or both X₁₁ and X₁₂ in the formulae (7) to (12) represent oxygen atoms.

8. The polycyclic compound according to claim 1, which is a benzofurano dibenzofuran derivative represented by one of the following formulae (17) and (18): 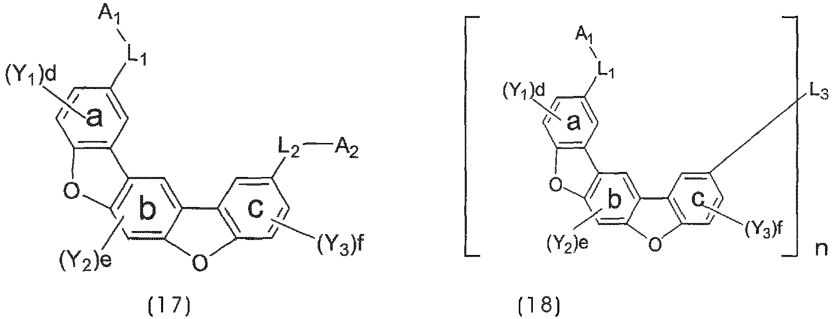 where:
n represents 2, 3, or 4, and the compound represented by the formula (18) comprises a dimer using L₃ as a linking group for n=2, a trimer using L₃ as a linking group for n=3, or a tetramer using L₃ as a linking group for n=4;
L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group, wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with a benzene ring a through a carbon-carbon bond;
L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group, wherein the aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with a benzene ring c through a carbon-carbon bond;
when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group, wherein the divalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, divalent aromatic heterocyclic group which has a ring formed of 3 to 24 atoms and which is linked with the benzene ring c through a carbon-carbon bond,
when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group, wherein the trivalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, trivalent aromatic heterocyclic group which has 3 to 24 atoms and which is linked with the benzene ring c through a carbon-carbon bond, or
when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group, wherein the tetravalent aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted, tetravalent aromatic heterocyclic group which has a ring formed of 3 to 24 atoms and which is linked with the benzene ring c through a carbon-carbon bond;
A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group, wherein the aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or an aromatic heterocyclic group which has a ring formed of 3 to 24 atoms and which is linked with L₁ through a carbon-carbon bond,
provided that, when L₁ represents an alkylene group having 1 to 20 carbon atoms, a case where A₁ represents a hydrogen atom is excluded;
A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group, wherein the aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or an aromatic heterocyclic group which has a ring formed of 3 to 24 atoms and which is linked with L₂ through a carbon-carbon bond,
provided that, when L₂ represents an alkylene group having 1 to 20 carbon atoms, a case where A₂ represents a hydrogen atom is excluded;
Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group, wherein the aromatic hydrocarbon group is a residue of benzene, naphthalene, biphenyl, terphenyl, fluorene, phenanthrene, triphenylene, perylene, chrysene, fluoranthene, benzofluorene, benzotriphenylene, benzochrysene, or anthracene, or a substituted or unsubstituted aromatic heterocyclic group which has a ring formed of 3 to 24 atoms and which is linked with the benzene ring a, b, or c through a carbon-carbon bond,
d and f each represent a number of 0, 1, 2, or 3, and e represents a number of 0, 1, or 2,
provided that, when both L₁ and L₂ represent single bonds, and both A₁ and A₂ represent hydrogen atom, the benzene ring b has one or two Y₂'s, and a case where Y₂ represents a methyl group or an unsubstituted phenyl group is excluded; and
A₁, A₂, L₁, L₂, and L₃ are each free of any carbonyl group.

## Patentansprüche

1. Polycyclische Verbindung, die durch eine der folgenden Formeln (7) bis (12) dargestellt wird: 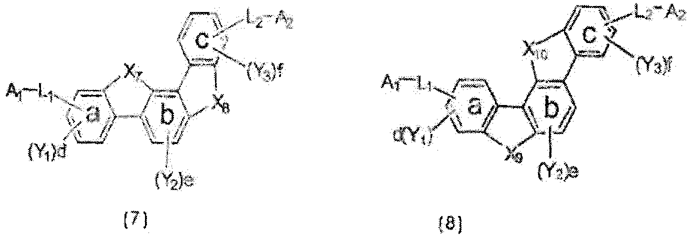 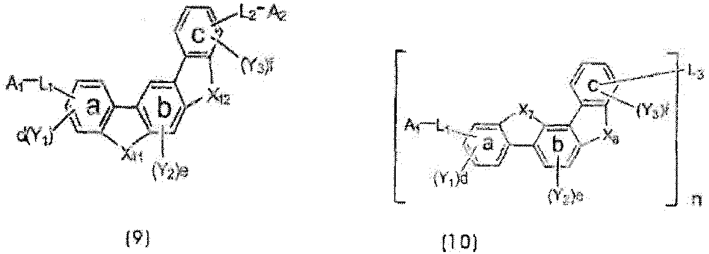 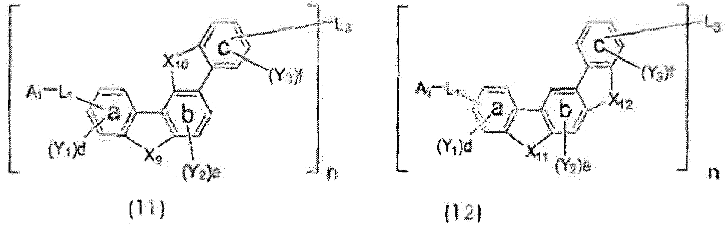 worin
X₇, X₈, X₂, X₁₀, X₁₁ und X₁₂ jeweils unabhängig voneinander Sauerstoff (O), Schwefel (S), N-R₁ oder CR₂R₂ darstellen; mit der Maßgabe, dass X₇, X₉ und X₁₁ in den allgemeinen Formeln (7) bis (12) jeweils unabhängig voneinander für Sauerstoff (O), Schwefel (S) oder CR₂R₃ oder X₈, X₁₀ und X₁₂ in den allgemeinen Formeln (7) bis (12) jeweils unabhängig voneinander für Sauerstoff (O), Schwefel (S) oder CR₂R₃ stehen;
R₁, R₂ und R₃ jeweils unabhängig voneinander eine Alkylgruppe mit 1 bis 20 Kohlenstoffatomen, eine substituierte oder unsubstituierte Cycloalkylgruppe mit einem aus 3 bis 20 Kohlenstoffatomen gebildeten Ring, eine Aralkylgruppe mit 7 bis 24 Kohlenstoffatomen, eine Silylgruppe oder eine substituierte Silylgruppe mit 3 bis 20 Kohlenstoffatomen, eine substituierte oder unsubstituierte aromatische Kohlenwasserstoffgruppe, worin die aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine substituierte oder unsubstituierte aromatische heterocyclische Gruppe mit einem aus 3 bis 24 Atomen gebildeten Ring darstellen;
n 2, 3 oder 4 darstellt und die durch eine der Formeln (10) bis (12) dargestellte Verbindung ein Dimer unter Verwendung von L₃ als Verbindungsgruppe für n = 2, ein Trimer unter Verwendung von L₃ als Verbindungsgruppe für n = 3, oder ein Tetramer unter Verwendung von L₃ als Verbindungsgruppe für n = 4 umfasst;
L₁ eine Einfachbindung, eine Alkylengruppe mit 1 bis 20 Kohlenstoffatomen, eine substituierte oder unsubstituierte Cycloalkylengruppe mit einem aus 3 bis 20 Kohlenstoffatomen gebildeten Ring, eine zweiwertige Silylgruppe oder eine zweiwertige Silylgruppe mit 2 bis 20 Kohlenstoffatomen, eine substituierte oder unsubstituierte, zweiwertige aromatische Kohlenwasserstoffgruppe, worin die zweiwertige aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine substituierte oder unsubstituierte, zweiwertige aromatische heterocyclische Gruppe mit einem aus 3 bis 24 Atomen gebildeten Ring der über eine Kohlenstoff-Kohlenstoff-Bindung mit einem Benzolring a verbunden ist, darstellt;
L₂ eine Einfachbindung, eine Alkylengruppe mit 1 bis 20 Kohlenstoffatomen, eine substituierte oder unsubstituierte Cycloalkylengruppe mit einem aus 3 bis 20 Kohlenstoffatomen gebildeten Ring, eine zweiwertige Silylgruppe oder eine zweiwertige Silylgruppe mit 2 bis 20 Kohlenstoffatomen, eine substituierte oder unsubstituierte, zweiwertige aromatische Kohlenwasserstoffgruppe, worin die zweiwertige aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine substituierte oder unsubstituierte, zweiwertige aromatische heterocyclische Gruppe mit einem aus 3 bis 24 Atomen gebildeten Ring der über eine Kohlenstoff-Kohlenstoff-Bindung mit einem Benzolring c verbunden ist, darstellt,
mit der Maßgabe, dass, wenn sowohl X₇ als auch X₈, sowohl X₉ als auch X₁₀ oder sowohl X₁₁ als auch X₁₂ beide CR₂R₃ darstellen und sowohl L₁ als auch L₂ die substituierten oder unsubstituierten, zweiwertigen aromatischen Kohlenwasserstoffgruppen darstellen, ein Fall ausgenommen ist, worin L₁ an den Benzolring a in para-Position in Bezug auf den Benzolring b gebunden ist und L₂ an den Benzolring c in der para-Position in Bezug auf den Benzolring b gebunden ist;
wenn n 2 darstellt, L₃ eine Einfachbindung, eine Alkylengruppe mit 1 bis 20 Kohlenstoffatomen, eine substituierte oder unsubstituierte Cycloalkylengruppe mit einem aus 3 bis 20 Kohlenstoffatomen gebildeten Ring, eine zweiwertige Silylgruppe oder eine substituierte zweiwertige Silylgruppe mit 2 bis 20 Kohlenstoffatome, eine substituierte oder unsubstituierte, zweiwertige aromatische Kohlenwasserstoffgruppe, worin die zweiwertige aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine substituierte oder unsubstituierte, zweiwertige aromatische heterocyclische Gruppe mit einem aus 3 bis 24 Atomen gebildeten Ring der über eine Kohlenstoff-Kohlenstoff-Bindung mit dem Benzolring c verbunden ist, darstellt,
wenn n 3 darstellt, L₃ eine dreiwertige gesättigte Kohlenwasserstoffgruppe mit 1 bis 20 Kohlenstoffatomen, eine substituierte oder unsubstituierte dreiwertige cyclische gesättigte Kohlenwasserstoffgruppe mit einem aus 3 bis 20 Kohlenstoffatomen gebildeten Ring, eine dreiwertige Silylgruppe oder eine substituierte dreiwertige Silylgruppe mit 1 bis 20 Kohlenstoffatomen, eine substituierte oder unsubstituierte dreiwertige aromatische Kohlenwasserstoffgruppe, worin die dreiwertige aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine substituierte oder unsubstituierte, dreiwertige aromatische heterocyclische Gruppe mit 3 bis 24 Kohlenstoffatomen die über eine Kohlenstoff-Kohlenstoff-Bindung mit dem Benzolring c verbunden ist, darstellt, oder
wenn n 4 darstellt, L₃ eine vierwertige gesättigte Kohlenwasserstoffgruppe mit 1 bis 20 Kohlenstoffatomen, eine substituierte oder unsubstituierte, vierwertige cyclische gesättigte Kohlenwasserstoffgruppe mit einem aus 3 bis 20 Kohlenstoffatomen gebildeten Ring, ein Siliciumatom, eine substituierte oder unsubstituierte, vierwertige aromatische Kohlenwasserstoffgruppe, worin die vierwertige aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine substituierte oder unsubstituierte, vierwertige aromatische heterocyclische Gruppe mit einem aus 3 bis 24 Atomen gebildeten Ring der über eine Kohlenstoff-Kohlenstoff-Bindung mit dem Benzolring c verbunden ist, darstellt,
mit der Maßgabe, dass, wenn sowohl X₇ als auch X₈, sowohl X₉ als auch X₁₀ oder sowohl X₁₁ als auch X₁₂ beide CR₂R₃ darstellen und sowohl L₁ als auch L₃ die substituierten oder unsubstituierten, zweiwertigen, dreiwertigen oder vierwertigen aromatischen Kohlenwasserstoffgruppen darstellen, ein Fall ausgenommen ist, worin L₁ an den Benzolring a in para-Position in Bezug auf den Benzolring b gebunden ist und L₃ an den Benzolring c in der para-Position in Bezug auf den Benzolring b gebunden ist;
A₁ ein Wasserstoffatom, eine substituierte oder unsubstituierte Cycloalkylgruppe mit einem aus 3 bis 20 Kohlenstoffatomen gebildeten Ring, eine Silylgruppe oder eine substituierte Silylgruppe mit 3 bis 20 Kohlenstoffatomen, eine substituierte oder unsubstituierte aromatische Kohlenwasserstoffgruppe, worin die aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine aromatische heterocyclische Gruppe mit einem aus 3 bis 24 Atomen gebildeten Ring der über eine Kohlenstoff-Kohlenstoff-Bindung an L₁ gebunden ist, darstellt,
mit der Maßgabe, dass, wenn L₁ eine Alkylengruppe mit 1 bis 20 Kohlenstoffatomen darstellt, ein Fall, in dem A₁ ein Wasserstoffatom darstellt, ausgenommen ist;
A₂ ein Wasserstoffatom, eine substituierte oder unsubstituierte Cycloalkylgruppe mit einem aus 3 bis 20 Kohlenstoffatomen gebildeten Ring, eine Silylgruppe oder eine substituierte Silylgruppe mit 3 bis 20 Kohlenstoffatomen, eine substituierte oder unsubstituierte aromatische Kohlenwasserstoffgruppe mit einem Ring, worin die aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine aromatische heterocyclische Gruppe mit einem aus 3 bis 24 Atomen gebildeten Ring der über eine Kohlenstoff-Kohlenstoff-Bindung an L₂ gebunden ist, darstellt;
mit der Maßgabe, dass, wenn L₂ eine Alkylengruppe mit 1 bis 20 Kohlenstoffatomen darstellt, ein Fall, in dem A₂ ein Wasserstoffatom darstellt, ausgenommen ist;
Y₁, Y₂ und Y₃ jeweils eine Alkylgruppe mit 1 bis 20 Kohlenstoffatomen, eine substituierte oder unsubstituierte Cycloalkylgruppe mit einem aus 3 bis 20 Kohlenstoffatomen gebildeten Ring, eine Alkoxygruppe mit 1 bis 20 Kohlenstoffatomen, eine Aralkylgruppe mit 7 bis 24 Kohlenstoffatomen, eine Silylgruppe oder eine substituierte Silylgruppe mit 3 bis 20 Kohlenstoffatomen, eine substituierte oder unsubstituierte aromatische Kohlenwasserstoffgruppe, worin die aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine substituierte oder unsubstituierte aromatische heterocyclische Gruppe mit einem aus 3 bis 24 Atomen gebildeten Ring der über eine Kohlenstoff-Kohlenstoff-Bindung mit dem Benzolring a, b oder c verbunden ist, darstellt,
d und f jeweils eine Zahl von 0, 1, 2 oder 3 darstellen und e eine Zahl von 0, 1 oder 2 darstellt,
mit der Maßgabe, dass, wenn sowohl X₇ als auch X₈, sowohl X₉ als auch X₁₀ oder sowohl X₁₁ als auch X₁₂ beide Sauerstoff (O), Schwefel (S) oder CR₂R₃, sowohl L₁ als auch L₂ Einfachbindungen und sowohl A₁ als auch A₂ Wasserstoffatome darstellen, der Benzolring b ein oder zwei Y₂ aufweist, und ein Fall ausgenommen ist, in dem Y₂ eine Methylgruppe oder eine unsubstituierte Phenylgruppe darstellt;
A₁, A₂, L₁, L₂ und L₃ jeweils frei von einer Carbonylgruppe sind, und
der Substituent, der jede der Gruppen in den Formeln (7) bis (12) ersetzen kann, Alkylgruppen mit jeweils 1 bis 6 Kohlenstoffatomen, eine Phenylgruppe, eine Pyridylgruppe, eine Carbazolylgruppe und eine Dibenzofuranylgruppe einschließt,
mit der Maßgabe, dass die folgende Verbindung ausgenommen ist:

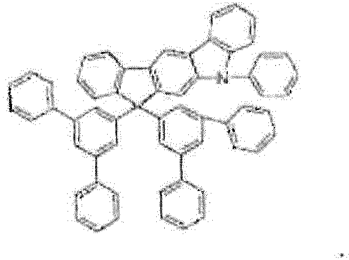

2. Die polycyclische Verbindung nach Anspruch 1, worin die Formeln (7) bis (12) jeweils durch eine der folgenden Formeln (7a) bis (12a) und (7b) bis (12b) dargestellt sind: 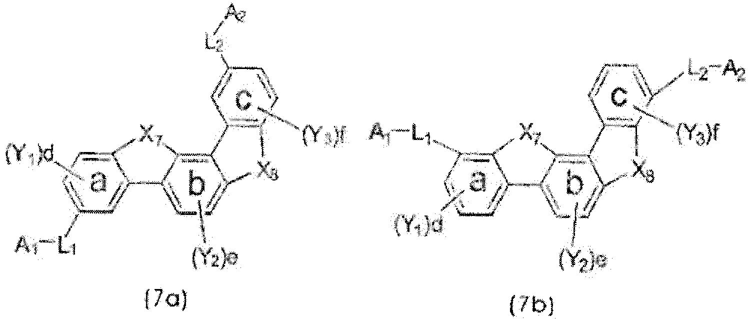 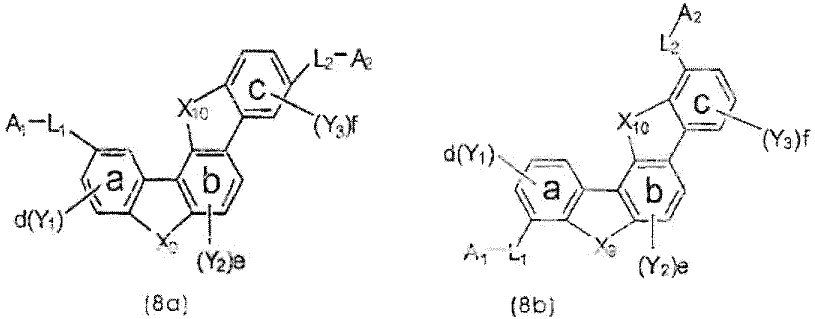 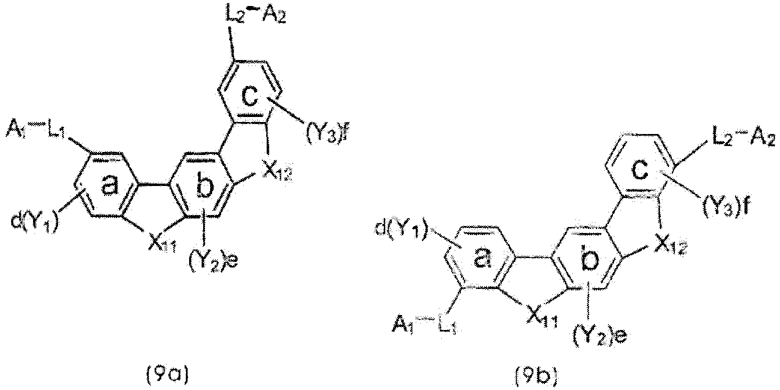 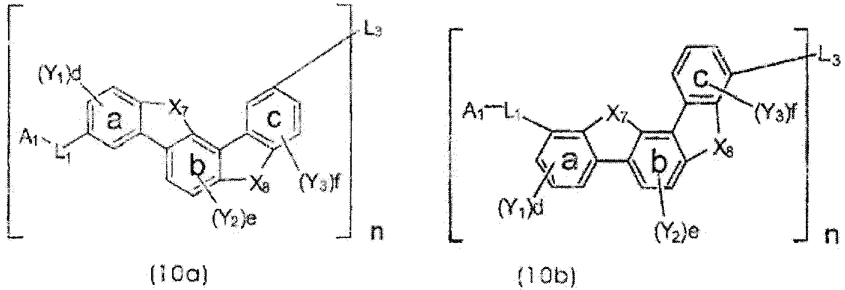 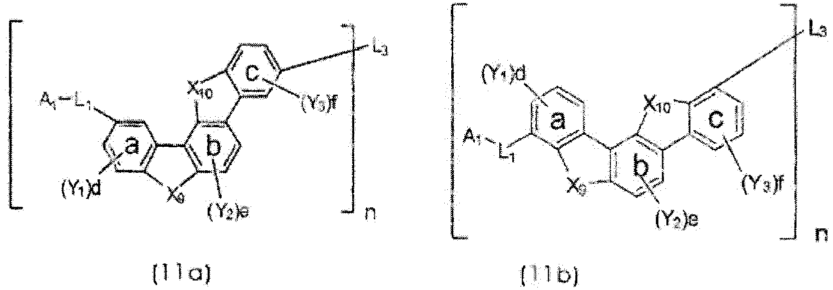 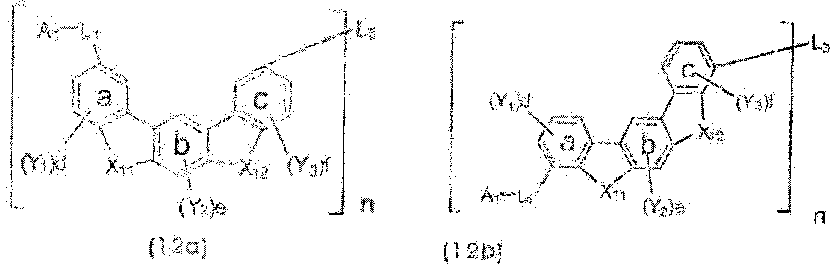 worin X₇ bis X₁₂, L₁, L₂, L₃, A₁, A₂, Y₁, Y₂, Y₃, n, d, f, und e jeweils die gleiche Bedeutung aufweisen wie oben beschrieben.

3. Die polycyclische Verbindung nach Anspruch 1, worin n in den Formeln (7) bis (12) 2 darstellt.

4. Die polycyclische Verbindung nach Anspruch 1, worin eine Gesamtzahl von Substituenten, dargestellt durch Y₁, Y₂ und Y₃ in den Formeln (7) bis (9), 3 oder weniger ist und eine Gesamtzahl der Substituenten, dargestellt durch Y₁, Y₂ und Y₃ in der Struktur []ₙ in den allgemeinen Formeln (10) bis (12), 3 oder weniger ist.

5. Die polycyclische Verbindung nach Anspruch 1, worin sowohl X₁₁ als auch X₁₂ in der Formel (9) oder (12) CR₂R₃ darstellen.

6. Die polycyclische Verbindung nach Anspruch 1, worin mindestens eines von X₇ und X₈, X₉ und X₁₀ oder X₁₁ und X₁₂ in den Formeln (7) bis (12) ein Sauerstoffatom darstellt.

7. Die polycyclische Verbindung nach Anspruch 1, die ein Benzofurandibenzofuranderivat ist, worin sowohl X₇ als auch X₈, sowohl X₉ als auch X₁₀, oder sowohl X₁₁ als auch X₁₂ in den Formeln (7) bis (12) Sauerstoffatome darstellen.

8. Die polycyclische Verbindung nach Anspruch 1, die ein Benzofurandibenzofuranderivat ist, das durch eine der folgenden Formeln (17) und (18) dargestellt wird: 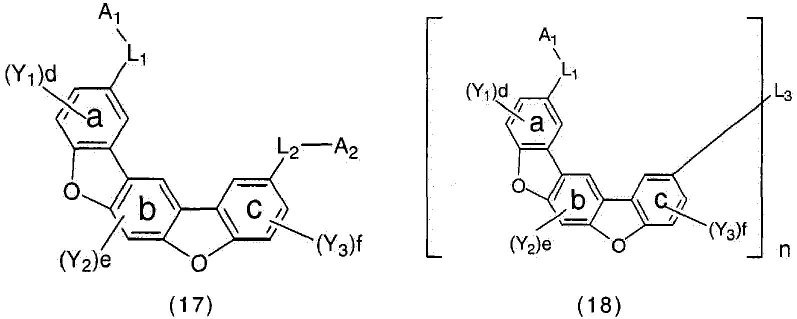 worin
n 2, 3 oder 4 darstellt und die durch die Formel (18) dargestellte Verbindung ein Dimer unter Verwendung von L₃ als Verbindungsgruppe für n = 2, ein Trimer unter Verwendung von L₃ als Verbindungsgruppe für n = 3, oder ein Tetramer unter Verwendung von L₃ als Verbindungsgruppe für n = 4 umfasst;
L₁ eine Einfachbindung, eine Alkylengruppe mit 1 bis 20 Kohlenstoffatomen, eine substituierte oder unsubstituierte Cycloalkylengruppe mit einem aus 3 bis 20 Kohlenstoffatomen gebildeten Ring, eine zweiwertige Silylgruppe oder eine zweiwertige Silylgruppe mit 2 bis 20 Kohlenstoffatomen, eine substituierte oder unsubstituierte, zweiwertige aromatische Kohlenwasserstoffgruppe, worin die zweiwertige aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine substituierte oder unsubstituierte, zweiwertige aromatische heterocyclische Gruppe mit einem aus 3 bis 24 Atomen gebildeten Ring der über eine Kohlenstoff-Kohlenstoff-Bindung mit einem Benzolring a verbunden ist, darstellt;
L₂ eine Einfachbindung, eine Alkylengruppe mit 1 bis 20 Kohlenstoffatomen, eine substituierte oder unsubstituierte Cycloalkylengruppe mit einem aus 3 bis 20 Kohlenstoffatomen gebildeten Ring, eine zweiwertige Silylgruppe oder eine zweiwertige Silylgruppe mit 2 bis 20 Kohlenstoffatomen, eine substituierte oder unsubstituierte, zweiwertige aromatische Kohlenwasserstoffgruppe, worin die zweiwertige aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine substituierte oder unsubstituierte, zweiwertige aromatische heterocyclische Gruppe mit einem aus 3 bis 24 Atomen gebildeten Ring der über eine Kohlenstoff-Kohlenstoff-Bindung mit einem Benzolring c verbunden ist, darstellt,
wenn n 2 darstellt, L₃ eine Einfachbindung, eine Alkylengruppe mit 1 bis 20 Kohlenstoffatomen, eine substituierte oder unsubstituierte Cycloalkylengruppe mit einem aus 3 bis 20 Kohlenstoffatomen gebildeten Ring, eine zweiwertige Silylgruppe oder eine substituierte zweiwertige Silylgruppe mit 2 bis 20 Kohlenstoffatome, eine substituierte oder unsubstituierte, zweiwertige aromatische Kohlenwasserstoffgruppe, worin die zweiwertige aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine substituierte oder unsubstituierte, zweiwertige aromatische heterocyclische Gruppe mit einem aus 3 bis 24 Atomen gebildeten Ring der über eine Kohlenstoff-Kohlenstoff-Bindung mit dem Benzolring c verbunden ist, darstellt,
wenn n 3 darstellt, L₃ eine dreiwertige gesättigte Kohlenwasserstoffgruppe mit 1 bis 20 Kohlenstoffatomen, eine substituierte oder unsubstituierte dreiwertige cyclische gesättigte Kohlenwasserstoffgruppe mit einem aus 3 bis 20 Kohlenstoffatomen gebildeten Ring, eine dreiwertige Silylgruppe oder eine substituierte dreiwertige Silylgruppe mit 1 bis 20 Kohlenstoffatomen, eine substituierte oder unsubstituierte dreiwertige aromatische Kohlenwasserstoffgruppe, worin die dreiwertige aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine substituierte oder unsubstituierte, dreiwertige aromatische heterocyclische Gruppe mit 3 bis 24 Kohlenstoffatomen die über eine Kohlenstoff-Kohlenstoff-Bindung mit dem Benzolring c verbunden ist, darstellt, oder
wenn n 4 darstellt, L₃ eine vierwertige gesättigte Kohlenwasserstoffgruppe mit 1 bis 20 Kohlenstoffatomen, eine substituierte oder unsubstituierte, vierwertige cyclische gesättigte Kohlenwasserstoffgruppe mit einem aus 3 bis 20 Kohlenstoffatomen gebildeten Ring, ein Siliciumatom, eine substituierte oder unsubstituierte, vierwertige aromatische Kohlenwasserstoffgruppe, worin die vierwertige aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine substituierte oder unsubstituierte, vierwertige aromatische heterocyclische Gruppe mit einem aus 3 bis 24 Atomen gebildeten Ring der über eine Kohlenstoff-Kohlenstoff-Bindung mit dem Benzolring c verbunden ist, darstellt,
A₁ ein Wasserstoffatom, eine substituierte oder unsubstituierte Cycloalkylgruppe mit einem aus 3 bis 20 Kohlenstoffatomen gebildeten Ring, eine Silylgruppe oder eine substituierte Silylgruppe mit 3 bis 20 Kohlenstoffatomen, eine substituierte oder unsubstituierte aromatische Kohlenwasserstoffgruppe, worin die aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine aromatische heterocyclische Gruppe mit einem aus 3 bis 24 Atomen gebildeten Ring der über eine Kohlenstoff-Kohlenstoff-Bindung an L₁ gebunden ist, darstellt,
mit der Maßgabe, dass, wenn L₁ eine Alkylengruppe mit 1 bis 20 Kohlenstoffatomen darstellt, ein Fall, in dem A₁ ein Wasserstoffatom darstellt, ausgenommen ist;
A2 ein Wasserstoffatom, eine substituierte oder unsubstituierte Cycloalkylgruppe mit einem aus 3 bis 20 Kohlenstoffatomen gebildeten Ring, eine Silylgruppe oder eine substituierte Silylgruppe mit 3 bis 20 Kohlenstoffatomen, eine substituierte oder unsubstituierte aromatische Kohlenwasserstoffgruppe mit einem Ring, worin die aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine aromatische heterocyclische Gruppe mit einem aus 3 bis 24 Atomen gebildeten Ring der über eine Kohlenstoff-Kohlenstoff-Bindung an L₂ gebunden ist, darstellt;
mit der Maßgabe, dass, wenn L₂ eine Alkylengruppe mit 1 bis 20 Kohlenstoffatomen darstellt, ein Fall, in dem A₂ ein Wasserstoffatom darstellt, ausgenommen ist;
Y₁, Y₂ und Y₃ jeweils eine Alkylgruppe mit 1 bis 20 Kohlenstoffatomen, eine substituierte oder unsubstituierte Cycloalkylgruppe mit einem aus 3 bis 20 Kohlenstoffatomen gebildeten Ring, eine Alkoxygruppe mit 1 bis 20 Kohlenstoffatomen, eine Aralkylgruppe mit 7 bis 24 Kohlenstoffatomen, eine Silylgruppe oder eine substituierte Silylgruppe mit 3 bis 20 Kohlenstoffatomen, eine substituierte oder unsubstituierte aromatische Kohlenwasserstoffgruppe, worin die aromatische Kohlenwasserstoffgruppe ein Rest von Benzol, Naphthalin, Biphenyl, Terphenyl, Fluoren, Phenanthren, Triphenylen, Perylen, Chrysen, Fluoranthen, Benzofluoren, Benzotriphenylen, Benzochrysen oder Anthracen ist, oder eine substituierte oder unsubstituierte aromatische heterocyclische Gruppe mit einem aus 3 bis 24 Atomen gebildeten Ring der über eine Kohlenstoff-Kohlenstoff-Bindung mit dem Benzolring a, b oder c verbunden ist, darstellt,
d und f jeweils eine Zahl von 0, 1, 2 oder 3 darstellen und e eine Zahl von 0, 1 oder 2 darstellt,
mit der Maßgabe, dass, wenn sowohl L₁ als auch L₂ Einfachbindungen und sowohl A₁ als auch A₂ Wasserstoffatome darstellen, der Benzolring b ein oder zwei Y₂ aufweist, und ein Fall ausgenommen ist, in dem Y₂ eine Methylgruppe oder eine unsubstituierte Phenylgruppe darstellt; und
A₁, A₂, L₁, L₂ und L₃ jeweils frei von einer Carbonylgruppe sind.

## Revendications

1. Composé polycyclique représenté par l'une quelconque des formules (7) à (12) suivantes : 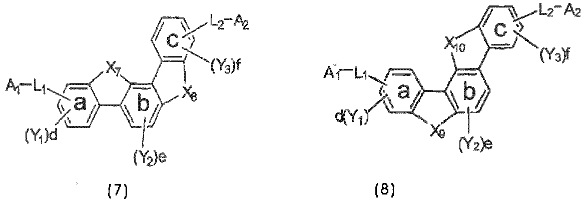 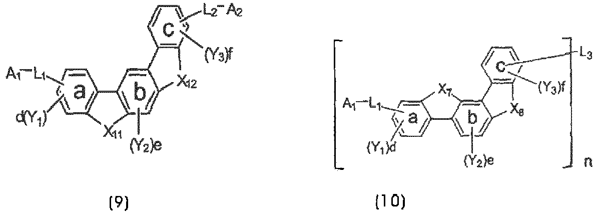 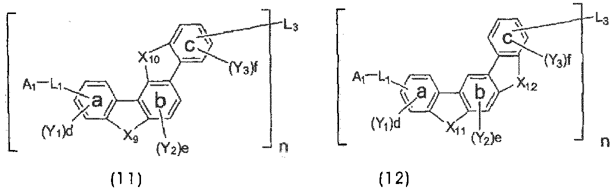 où :
X₇, X₈, X₉, X₁₀, X₁₁ et X₁₂ représentent chacun indépendamment un atome d'oxygène (O), un atome de soufre (S), N-R₁ ou CR₂R₃ ; à condition que X₇, X₉ et X₁₁ dans les formules générales (7) à (12) représentent chacun indépendamment un atome d'oxygène (O), un atome de soufre (S) ou CR₂R₃, ou X₈, X₁₀ et X₁₂ dans les formules générales (7) à (12) représentent chacun indépendamment un atome d'oxygène (O), un atome de soufre (S) ou CR2R3 ;
R₁, R₂ et R₃ représentent chacun indépendamment un groupe alkyle ayant 1 à 20 atome(s) de carbone, un groupe cycloalkyle substitué ou non substitué ayant un noyau formé de 3 à 20 atomes de carbone, un groupe aralkyle ayant 7 à 24 atomes de carbone, un groupe silyle ou un groupe silyle substitué ayant de 3 à 20 atomes de carbone, un groupe hydrocarboné aromatique substitué ou non substitué, où le groupe hydrocarboné aromatique est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène ou d'anthracène, ou un groupe hétérocyclique aromatique substitué ou non substitué ayant un noyau formé de 3 à 24 atomes ;
n représente 2, 3 ou 4, et le composé représenté par l'une quelconque des formules (10) à (12) comprend un dimère utilisant L₃ comme groupe de liaison pour n = 2, un trimère utilisant L₃ comme groupe de liaison pour n = 3, ou un tétramère utilisant L₃ comme groupe de liaison pour n = 4 ;
L₁ représente une liaison simple, un groupe alkylène ayant 1 à 20 atome(s) de carbone, un groupe cycloalkylène substitué ou non substitué ayant un noyau formé de 3 à 20 atomes de carbone, un groupe silyle divalent ou un groupe silyle divalent ayant 2 à 20 atomes de carbone, un groupe hydrocarboné aromatique divalent substitué ou non substitué, où le groupe hydrocarboné aromatique divalent est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène ou d'anthracène ou un groupe hétérocyclique aromatique divalent substitué ou non substitué qui a un noyau formé de 3 à 24 atomes et qui est lié à un noyau benzénique a par une liaison carbone-carbone ;
L₂ représente une liaison simple, un groupe alkylène ayant 1 à 20 atome(s) de carbone, un groupe cycloalkylène substitué ou non substitué ayant un noyau formé de 3 à 20 atomes de carbone, un groupe silyle divalent ou un groupe silyle divalent ayant 2 à 20 atomes de carbone, un groupe hydrocarboné aromatique divalent substitué ou non substitué, où le groupe hydrocarboné aromatique divalent est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène ou d'anthracène ou un groupe hétérocyclique aromatique divalent substitué ou non substitué qui a un noyau formé de 3 à 24 atomes et qui est lié à un noyau benzénique c par une liaison carbone-carbone,
à condition que, lorsque, X₇ et X₈, X₉ et X₁₀, ou X₁₁ et X₁₂ représentent tous deux CR₂R₃ et L₁ et L₂ représentent tous deux des groupes hydrocarbonés aromatiques divalents substitués ou non substitués, le cas où L₁ est lié au noyau benzénique a à la position para par rapport au noyau benzénique b et L₂ est lié au noyau benzénique c à la position para par rapport au noyau benzénique b soit exclu ;
lorsque n représente 2, L₃ représente une liaison simple, un groupe alkylène ayant 1 à 20 atome(s) de carbone, un groupe cycloalkylène substitué ou non substitué ayant un noyau formé de 3 à 20 atomes de carbone, un groupe silyle divalent ou un groupe silyle divalent substitué ayant 2 à 20 atomes de carbone, un groupe hydrocarboné aromatique divalent substitué ou non substitué, où le groupe hydrocarboné aromatique divalent est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène ou d'anthracène ou un groupe hétérocyclique aromatique divalent substitué ou non substitué qui a un noyau formé de 3 à 24 atomes et qui est lié au noyau benzénique c par une liaison carbone-carbone,
lorsque n représente 3, L₃ représente un groupe hydrocarboné saturé trivalent ayant 1 à 20 atome(s) de carbone, un groupe hydrocarboné saturé cyclique trivalent substitué ou non substitué ayant un noyau formé de 3 à 20 atomes de carbone, un groupe silyle trivalent ou un groupe silyle trivalent substitué ayant 1 à 20 atome(s) de carbone, un groupe hydrocarboné aromatique trivalent substitué ou non substitué, où le groupe hydrocarboné aromatique trivalent est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène ou d'anthracène ou un groupe hétérocyclique aromatique trivalent substitué ou non substitué qui a 3 à 24 atomes et qui est lié au noyau benzénique c par une liaison carbone-carbone, ou
lorsque n représente 4, L₃ représente un groupe hydrocarboné saturé tétravalent ayant 1 à 20 atome(s) de carbone, un groupe hydrocarboné saturé cyclique tétravalent substitué ou non substitué ayant un noyau formé de 3 à 20 atomes de carbone, un atome de silicium, un groupe hydrocarboné aromatique tétravalent substitué ou non substitué, où le groupe hydrocarboné aromatique tétravalent est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène ou d'anthracène ou un groupe hétérocyclique aromatique tétravalent substitué ou non substitué qui a un noyau formé de 3 à 24 atomes et qui est lié au noyau benzénique c par une liaison carbone-carbone,
à condition que, lorsque, X₇ et X₈, X₉ et X₁₀, ou X₁₁ et X₁₂ représentent tous deux CR₂R₃ et L₁ et L₃ représentent tous deux les groupes hydrocarbonés aromatiques divalents, trivalents ou tétravalents, substitués ou non substitués, le cas où L₁ est lié au noyau benzénique a à la position para par rapport au noyau benzénique b et L₃ est lié au noyau benzénique c à la position para par rapport au noyau benzénique b soit exclu ;
A₁ représente un atome d'hydrogène, un groupe cycloalkyle substitué ou non substitué ayant un noyau formé de 3 à 20 atomes de carbone, un groupe silyle ou un groupe silyle substitué ayant 3 à 20 atomes de carbone, un groupe hydrocarboné aromatique substitué ou non substitué, où le groupe hydrocarboné aromatique est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène ou d'anthracène ou un groupe hétérocyclique aromatique qui a un noyau formé de 3 à 24 atomes et qui est lié à L₁ par une liaison carbone-carbone,
à condition que, lorsque L₁ représente un groupe alkylène ayant 1 à 20 atome(s) de carbone, le cas où A₁ représente un atome d'hydrogène soit exclu ;
A₂ représente un atome d'hydrogène, un groupe cycloalkyle substitué ou non substitué ayant un noyau formé de 3 à 20 atomes de carbone, un groupe silyle ou un groupe silyle substitué ayant 3 à 20 atomes de carbone, un groupe hydrocarboné aromatique substitué ou non substitué ayant un noyau, où le groupe hydrocarboné aromatique est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène ou d'anthracène ou un groupe hétérocyclique aromatique qui a un noyau formé de 3 à 24 atomes et qui est lié à L₂ par une liaison carbone-carbone,
à condition que, lorsque L₂ représente un groupe alkylène ayant 1 à 20 atome(s) de carbone, le cas où A₂ représente un atome d'hydrogène soit exclu ;
Y₁, Y₂ et Y₃ représentent chacun un groupe alkyle ayant 1 à 20 atome(s) de carbone, un groupe cycloalkyle substitué ou non substitué ayant un noyau formé de 3 à 20 atomes de carbone, un groupe alcoxy ayant 1 à 20 atome(s) de carbone, un groupe aralkyle ayant 7 à 24 atomes de carbone, un groupe silyle ou un groupe silyle substitué ayant 3 à 20 atomes de carbone, un groupe hydrocarboné aromatique substitué ou non substitué, où le groupe hydrocarboné aromatique est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène ou d'anthracène ou un groupe hétérocyclique aromatique substitué ou non substitué qui a un noyau formé de 3 à 24 atomes et qui est lié au noyau benzénique a, b ou c par une liaison carbone-carbone,
d et f représentent chacun un nombre égal à 0, 1, 2 ou 3, et e représente un nombre égal à 0, 1 ou 2,
à condition que, lorsque X₇ et X₈, X₉ et X₁₀, ou X₁₁ et X₁₂ représentent tous deux un atome d'oxygène (O), un atome de soufre (S), ou CR₂R₃, L₁ et L₂ représentent tous deux des liaisons simples, et A₁ et A₂ représentent tous deux des atomes d'hydrogène, le noyau benzénique b ait un ou deux Y₂'s, et le cas où Y₂ représente un groupe méthyle ou un groupe phényle non substitué soit exclu ;
A₁, A₂, L₁, L₂, et L₃ sont chacun exempts de tout groupe carbonyle, et
le substituant qui peut substituer chacun des groupes des formules (7) à (12) comporte des groupes alkyle ayant chacun 1 à 6 atome(s) de carbone, un groupe phényle, un groupe pyridyle, un groupe carbazolyle, et un groupe dibenzofuranyle,
à condition que le composé suivant soit exclu :

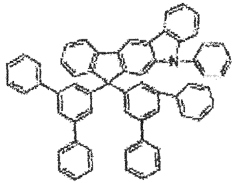

2. Composé polycyclique selon la revendication 1, dans lequel les formules (7) à (12) sont chacune représentées par l'une quelconque des formules (7a) à (12a) et (7b) à (12b) suivantes : 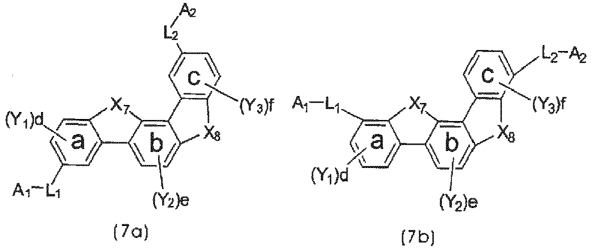 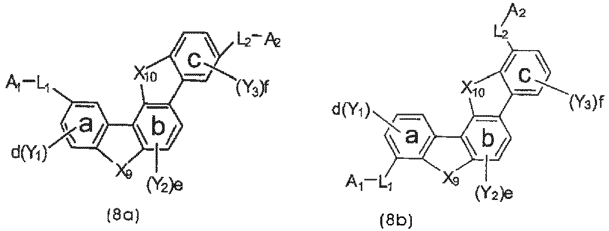 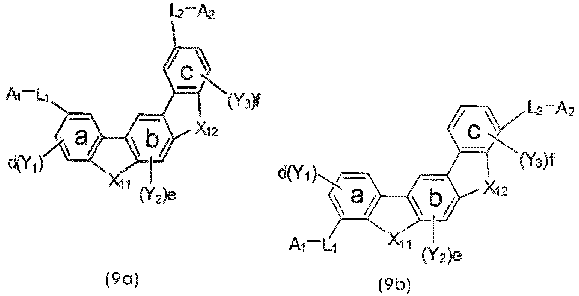 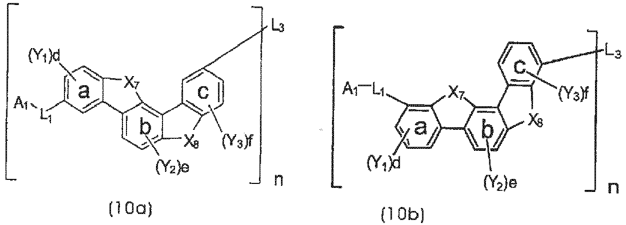 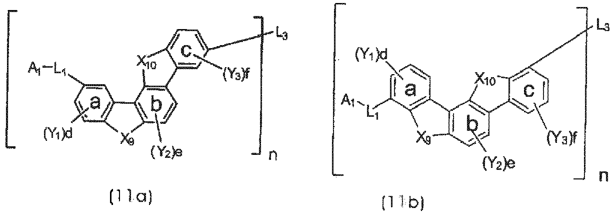 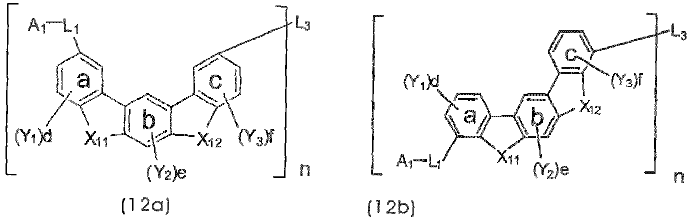 où X₇ à X₁₂, L₁, L₂, L₃, A₁, A₂, Y₁, Y₂, Y₃, n, d, f, et e ont chacun la même signification que celle décrite ci-dessus.

3. Composé polycyclique selon la revendication 1, dans lequel n représente 2 dans les formules (10) à (12).

4. Composé polycyclique selon la revendication 1, dans lequel un nombre total de substituants représentés par Y₁, Y₂, et Y₃ dans les formules (7) à (9) est inférieur ou égal à 3, et un nombre total des substituants représentés par Y₁, Y₂, et Y₃ dans la structure de []ₙ dans les formules générales (10) à (12) est inférieur ou égal à 3.

5. Composé polycyclique selon la revendication 1, dans lequel X₁₁ et X₁₂ dans la formule (9) ou (12) représentent tous deux CR₂R₃.

6. Composé polycyclique selon la revendication 1, dans lequel au moins l'un de X₇ et X₈, X₉ et X₁₀, ou X₁₁ et X₁₂ dans les formules (7) à (12) représente un atome d'oxygène.

7. Composé polycyclique selon la revendication 1, qui est un dérivé de benzofurano dibenzofurane dans lequel X₇ et X₈, X₉ et X₁₀, ou X₁₁ et X₁₂ dans les formules (7) à (12) représentent tous deux des atomes d'oxygène.

8. Composé polycyclique selon la revendication 1, qui est un dérivé de benzofurano dibenzofurane représenté par l'une des formules suivantes (17) et (18) suivantes : 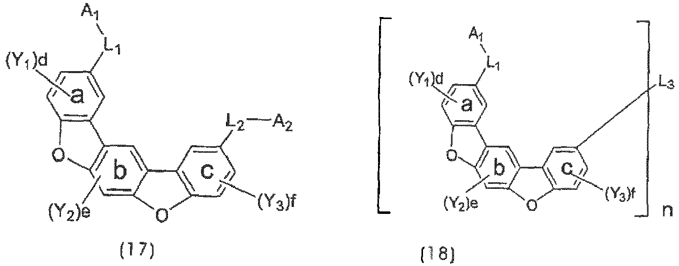 où :
n représente 2, 3, ou 4, et le composé représenté par la formule (18) comprend un dimère utilisant L₃ comme groupe de liaison pour n = 2, un trimère utilisant L₃ comme groupe de liaison pour n = 3, ou un tétramère utilisant L₃ comme groupe de liaison pour n = 4 ;
L₁ représente une liaison simple, un groupe alkylène ayant 1 à 20 atome(s) de carbone, un groupe cycloalkylène substitué ou non substitué ayant un noyau formé de 3 à 20 atomes de carbone, un groupe silyle divalent ou un groupe silyle divalent substitué ayant 2 à 20 atomes de carbone, un groupe hydrocarboné aromatique divalent substitué ou non substitué, où le groupe hydrocarboné aromatique divalent est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène, ou d'anthracène, ou un groupe hétérocyclique aromatique divalent substitué ou non substitué qui a un noyau formé de 3 à 24 atomes et qui est lié à un noyau benzénique a par une liaison carbone-carbone ;
L₂ représente une liaison simple, un groupe alkylène ayant 1 à 20 atome(s) de carbone, un groupe cycloalkylène substitué ou non substitué ayant un noyau formé de 3 à 20 atomes de carbone, un groupe silyle divalent ou un groupe silyle divalent substitué ayant 2 à 20 atomes de carbone, un groupe hydrocarboné aromatique divalent substitué ou non substitué, où le groupe hydrocarboné aromatique est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène, ou d'anthracène, ou un groupe hétérocyclique aromatique divalent substitué ou non substitué qui a un noyau formé de 3 à 24 atomes et qui est lié à un noyau benzénique c par une liaison carbone-carbone ;
lorsque n représente 2, L₃ représente une liaison simple, un groupe alkylène ayant 1 à 20 atome(s) de carbone, un groupe cycloalkylène substitué ou non substitué ayant un noyau formé de 3 à 20 atomes de carbone, un groupe silyle divalent ou un groupe silyle divalent substitué ayant 2 à 20 atomes de carbone, un groupe hydrocarboné aromatique divalent substitué ou non substitué, où le groupe hydrocarboné aromatique divalent est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène, ou d'anthracène, ou un groupe hétérocyclique aromatique divalent substitué ou non substitué qui a un noyau formé de 3 à 24 atomes et qui est lié au noyau benzénique c par une liaison carbone-carbone,
lorsque n représente 3, L₃ représente un groupe hydrocarboné saturé trivalent ayant 1 à 20 atome(s) de carbone, un groupe hydrocarboné saturé cyclique trivalent substitué ou non substitué ayant un noyau formé de 3 à 20 atomes de carbone, un groupe silyle trivalent ou un groupe silyle trivalent substitué ayant 1 à 20 atome(s) de carbone, un groupe hydrocarboné aromatique trivalent substitué ou non substitué, où le groupe hydrocarboné aromatique trivalent est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène, ou d'anthracène, ou un groupe hétérocyclique aromatique trivalent substitué ou non substitué qui a 3 à 24 atomes et qui est lié au noyau benzénique c par une liaison carbone-carbone, ou
lorsque n représente 4, L₃ représente un groupe hydrocarboné saturé tétravalent ayant 1 à 20 atome(s) de carbone, un groupe hydrocarboné saturé cyclique tétravalent substitué ou non substitué ayant un noyau formé de 3 à 20 atomes de carbone, un atome de silicium, un groupe hydrocarboné aromatique tétravalent substitué ou non substitué, où le groupe hydrocarboné aromatique tétravalent est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène, ou d'anthracène, ou un groupe hétérocyclique aromatique tétravalent substitué ou non substitué qui a un noyau formé de 3 à 24 atomes et qui est lié au noyau benzénique c par une liaison carbone-carbone ;
A₁ représente un atome d'hydrogène, un groupe cycloalkyle substitué ou non substitué ayant un noyau formé de 3 à 20 atomes de carbone, un groupe silyle ou un groupe silyle substitué ayant 3 à 20 atomes de carbone, un groupe hydrocarboné aromatique substitué ou non substitué, où le groupe hydrocarboné aromatique est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène, ou d'anthracène, ou un groupe hétérocyclique aromatique qui a un noyau formé de 3 à 24 atomes et qui est lié à L₁ par une liaison carbone-carbone,
à condition que, lorsque L₁ représente un groupe alkylène ayant 1 à 20 atome(s) de carbone, le cas où A₁ représente un atome d'hydrogène soit exclu ;
A₂ représente un atome d'hydrogène, un groupe cycloalkyle substitué ou non substitué ayant un noyau formé de 3 à 20 atomes de carbone, un groupe silyle ou un groupe silyle substitué ayant 3 à 20 atomes de carbone, un groupe hydrocarboné aromatique substitué ou non substitué, où le groupe hydrocarboné aromatique est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène, ou d'anthracène, ou un groupe hétérocyclique aromatique qui a un noyau formé de 3 à 24 atomes et qui est lié à L₂ par une liaison carbone-carbone,
à condition que, lorsque L₂ représente un groupe alkylène ayant 1 à 20 atome(s) de carbone, le cas où A₂ représente un atome d'hydrogène soit exclu ;
Y₁, Y₂, et Y₃ représentent chacun un groupe alkyle ayant 1 à 20 atome(s) de carbone, un groupe cycloalkyle substitué ou non substitué ayant un noyau formé de 3 à 20 atomes de carbone, un groupe alcoxy ayant 1 à 20 atome(s) de carbone, un groupe aralkyle ayant 7 à 24 atomes de carbone, un groupe silyle ou un groupe silyle substitué ayant 3 à 20 atomes de carbone, un groupe hydrocarboné aromatique substitué ou non substitué, où le groupe hydrocarboné aromatique est un résidu de benzène, de naphtalène, de biphényle, de terphényle, de fluorène, de phénanthrène, de triphénylène, de pérylène, de chrysène, de fluoranthène, de benzofluorène, de benzotriphénylène, de benzochrysène, ou d'anthracène, ou un groupe hétérocyclique aromatique substitué ou non substitué qui a un noyau formé de 3 à 24 atomes et qui est lié au noyau benzénique a, b ou c par une liaison carbone-carbone,
d et f représentent chacun un nombre égal à 0, 1, 2 ou 3, et e représente un nombre égal à 0, 1 ou 2,
à condition que, lorsque L₁ et L₂ représentent tous deux des liaisons simples, et A₁ et A₂ représentent tous deux un atome d'hydrogène, le noyau benzénique b ait un ou deux Y₂'s, et le cas où Y₂ représente un groupe méthyle ou un groupe phényle non substitué soit exclu ; et
A₁, A₂, L₁, L₂, et L₃ sont chacun exempts de tout groupe carbonyle.
